(19) Europäisches Patentamt
European Patent Office
Office européen des brevets

(11) **EP 4 714 948 A1**

(12) **EUROPEAN PATENT APPLICATION**
published in accordance with Art. 153(4) EPC

(43) Date of publication:
**25.03.2026 Bulletin 2026/13**

(21) Application number: **24806639.1**

(22) Date of filing: **16.05.2024**

(51) International Patent Classification (IPC):
***C07D 401/14*** *(2006.01)* ***C07D 487/10*** *(2006.01)*
***C07D 405/14*** *(2006.01)* ***C07D 403/14*** *(2006.01)*
***A61K 31/506*** *(2006.01)* ***A61K 31/496*** *(2006.01)*
***A61P 35/00*** *(2006.01)* ***A61P 29/00*** *(2006.01)*
***A61P 9/00*** *(2006.01)* ***A61P 37/06*** *(2006.01)*
***A61P 3/00*** *(2006.01)* ***A61P 35/02*** *(2006.01)*

(52) Cooperative Patent Classification (CPC):
**A61K 31/496; A61K 31/506; A61K 31/513; A61K 31/517; A61K 31/55; A61P 1/00; A61P 3/00; A61P 9/00; A61P 29/00; A61P 31/00; A61P 35/00; A61P 35/02; A61P 37/06; C07D 401/14; C07D 403/14;** (Cont.)

(86) International application number:
**PCT/CN2024/093652**

(87) International publication number:
**WO 2024/235289 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 CN 202310557533**

(71) Applicant: **TYK Medicines, Inc.**
**Huzhou, Zhejiang 313100 (CN)**

(72) Inventors:
- **LIANG, Apeng**
  **Huzhou, Zhejiang 313100 (CN)**
- **HAN, Shunxun**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **DONG, Shengli**
  **Huzhou, Zhejiang 313100 (CN)**
- **NIU, Chengshan**
  **Huzhou, Zhejiang 313100 (CN)**
- **WU, Yusheng**
  **Huzhou, Zhejiang 313100 (CN)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

# (54) COMPOUND USED FOR EGFR PROTEIN DEGRADATION, AND USE THEREOF

(57) The present invention relates to a compound used for EGFR protein degradation, and a use thereof. Specifically, the compound of the present invention has a structure as shown in formula 1, wherein the definition of each group and substituent is as described in the description. Further disclosed are a method for preparing the compound, and a use of the compound in the prevention and/or treatment of EGFR-related diseases.

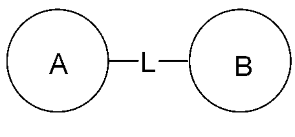

Formula I

EP 4 714 948 A1

(52) Cooperative Patent Classification (CPC): (Cont.)
**C07D 405/14; C07D 471/04; C07D 487/08; C07D 487/10**

## Description

### Technical field

The present invention relates to the field of medical technology, specifically to a compound used for EGFR protein degradation, and use thereof.

### Background

The HER family of receptor tyrosine kinases are mediators of cell growth, differentiation and survival. This receptor family includes four distinct members, namely the epidermal growth factor receptor (EGFR, ErbB1 or HER1), HER2 (ErbB2), HER3 (ErbB3) and HER4 (ErbB4). Upon ligand binding, the receptor forms homodimers or heterodimers, and the subsequent activation of endogenous tyrosine kinase activity leads to receptor autophosphorylation and the activation of downstream signaling molecules. The aberrant activation of EGFR, resulting from overexpression or mutation, has been shown to be implicated in multiple types of human cancers, including colorectal cancer, pancreatic cancer, glioma, head and neck cancer, and lung cancer, particularly non-small cell lung cancer (NSCLC). Various EGFR-targeted agents have been developed over the years, with three generations of these drugs already in clinical use.

In the actual clinical practice, patients treated with the first generation or second generation EGFR inhibitors, usually develop the drug-resistant mutation of EGFRT790M within 8- 12 months, which leads to the loss of therapeutic efficacy of the drug. Although, the later marketed third-generation EGFR inhibitors, such as osimertinib and ametinib, can effectively overcome the EGFRT790M mutation resistance. However, after a period of use, drug-resistant mutations such as EGFRC797S will still emerge, leading to disease progression.

The frequent emergence of drug resistance mutations during treatment with EGFR small-molecule tyrosine kinase inhibitors has become an urgent clinical challenge. Although some EGFR variant inhibitor compounds, such as EAI045, have been reported to overcome C797S resistance, the clinical efficacy is limited. In recent years, a series of PROTAC-type compounds capable of overcoming C797S resistance by degrading EGFR proteins have been reported in some patents (WO2019149922, WO2021127561), which has become a new research direction. Although some progress has been made with EGFR allosteric inhibitors and EGFR degraders, there is still a need to find more clinically valuable EGFR protein modulating drugs for the treatment of diseases caused by current EGFR dysregulation, especially in the field of EGFR-positive non-small cell lung cancer.

### Summary

The purpose of the present invention is to provide a compound of Formula I and its use for preventing and/or treating of EGFR-related diseases.

In the first aspect of the present invention, provided is a compound, the compound is a compound of Formula I, or a pharmaceutically acceptable salt thereof, a stereoisomer, a tautomer, a hydrate, a solvate, an isotopic compound, or a prodrug thereof,

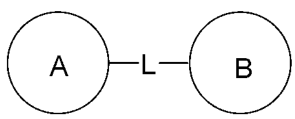

Formula I

wherein,

L is selected from the group consisting of:

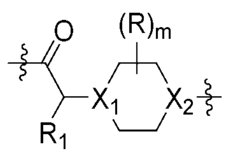
,
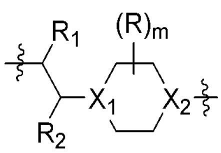
,

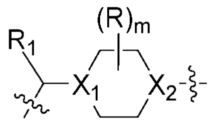
,
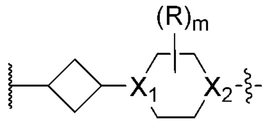
and
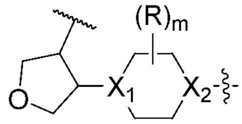
;

ring A is selected from the group consisting of:

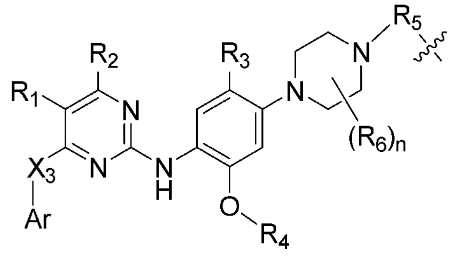

and

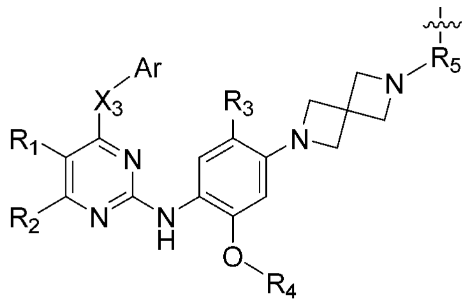
;

ring B is selected from the group consisting of:

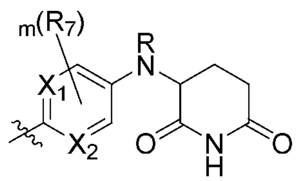
,

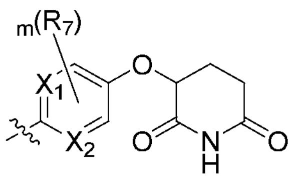
,
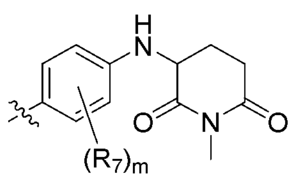
and
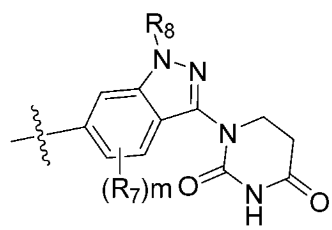
;

in each formula:

each of $X_1$ and $X_2$ is independently selected from the group consisting of: CR and N;
each $X_3$ is independently selected from the group consisting of: NH, O and none;
each Ar is independently selected from the group consisting of: substituted or unsubstituted phenyl, substituted or unsubstituted 5-10 membered heteroaryl containing 1, 2, or 3 heteroatoms selected from N, O and S,

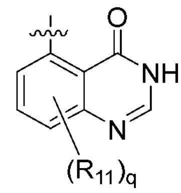 and 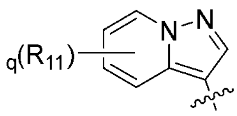;

the "substituted" refers to being substituted by one or more (such as 2, 3, or 4) substituents selected from the group consisting of: halogen, $C_{1-6}$ alkyl, $C_{1-6}$ haloalkyl, hydroxyl substituted $C_{1-6}$ alkyl, $C_{3-6}$ cycloalkyl, $C_{1-6}$ alkyl-NH -, $C_{1-6}$ alkoxy, $C_{1-6}$ haloalkoxy, $C_{3-6}$ halocycloalkyl, cyano, oxo, - C(O)$NR_9R_{10}$, -CO-$C_{1-6}$ alkyl, - CO-$C_{3-6}$ cycloalkyl, -CO-$C_{1-6}$ haloalkyl, -CO-$C_{3-6}$ halocycloalkyl, and 5-6 membered heteroaryl containing 1, 2, or 3 heteroatoms selected from N, O and S;

each $R_1$ is independently selected from the group consisting of: hydrogen, deuterium, halogen, $C_{1-6}$ alkyl, $C_{1-6}$ haloalkyl, $C_{3-6}$ cycloalkyl, $C_{3-6}$ halocycloalkyl and cyano;

each of $R_2$ and $R_3$ is independently selected from the group consisting of: hydrogen, deuterium, halogen, $C_{1-6}$ alkyl, $C_{2-6}$ alkenyl, $C_{2-6}$ alkynyl, $C_{3-6}$ cycloalkyl, $C_{1-6}$ alkyl-NH-, $C_{1-6}$ alkoxy, $C_{1-6}$ haloalkoxy, $C_{1-6}$ haloalkyl, cyano, $C_{3-6}$ halocycloalkyl, - $NR_9C(O)R_{10}$, - C(O)$NR_9R_{10}$, methylsulfonyl,

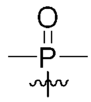,

unsubstituted or $C_{1-6}$ alkyl substituted 5-10 membered heteroaryl containing 1, 2, or 3 heteroatoms selected from N, O and S;

each $R_4$ is independently selected from the group consisting of: $C_{1-6}$ alkyl, $C_{1-6}$ haloalkyl, $C_{3-6}$ cycloalkyl and $C_{3-6}$ halocycloalkyl;

each $R_5$ is independently selected from the group consisting of: absent, $C_{1-6}$ alkyl, NR,

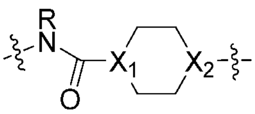, 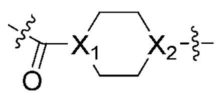, and 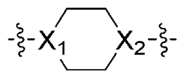;

each of $R_6$, $R_7$, and $R_8$ is independently selected from the group consisting of: H, halogen, $C_{1-6}$ alkyl, $C_{3-6}$ cycloalkyl, $C_{1-6}$ alkyl-NH-, $C_{1-6}$ alkoxy, $C_{1-6}$ haloalkoxy, $C_{1-6}$ haloalkyl, cyano, $C_{3-6}$ halocycloalkyl-$NR_9C(O)R_{10}$, -C(O)$NR_9R_{10}$, methylsulfonyl,

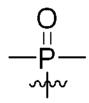,

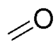,

and hydroxyl, or $R_6$ together with the attached ring form a 3-6 membered ring;

each $R_9$ is independently selected from the group consisting of: H, $C_{1-6}$ alkyl, $C_{1-6}$ haloalkyl, $C_{3-6}$ cycloalkyl and $C_{3-6}$ halocycloalkyl;

each $R_{10}$ is independently selected from the group consisting of: H, $C_{1-6}$ alkyl, $C_{2-6}$ alkenyl, $C_{2-6}$ alkynyl, $C_{3-6}$ cycloalkyl, $C_{1-6}$ alkoxy, $C_{1-6}$ haloalkoxy, $C_{1-6}$ haloalkyl, $C_{3-6}$ halocycloalkyl, methylsulfonyl, and

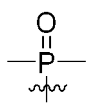;

each of m, n, and q is independently selected from the group consisting of: 0, 1, 2, 3, 4 and 5;

each $R_{11}$ is independently selected from the group consisting of: H, halogen, $C_{1-6}$ alkyl, $C_{1-6}$ haloalkyl, $C_{3-6}$ cycloalkyl and $C_{3-6}$ halocycloalkyl; and
each R is independently selected from the group consisting of: H, $C_{1-6}$ alkyl, hydroxyl, halogen and $C_{1-6}$ haloalkyl.

In another preferred embodiment, at least one of $X_1$ and $X_2$ is N.

In another preferred embodiment, the 5-10 membered heteroaryl containing 1, 2, or 3 heteroatoms selected from N, O and S is selected from the group consisting of: pyridyl

( 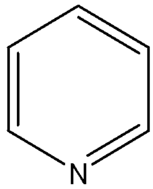 ),

thiazolyl

( 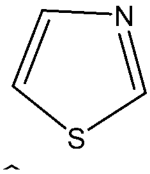 ),

indolyl

( 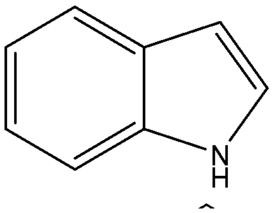 ),

quinolinyl

( 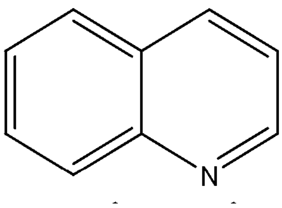 ), 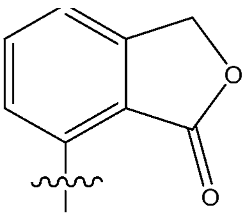 , 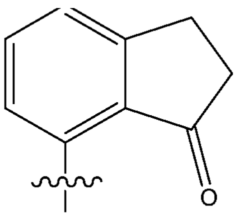 , 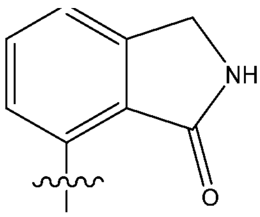

and

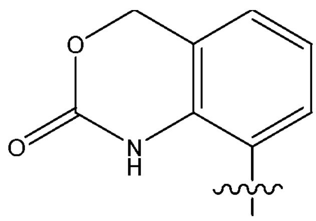 .

In another preferred embodiment, the 5-6 membered heteroaryl containing 1, 2, or 3 heteroatoms selected from N, O and S is selected from the group consisting of:

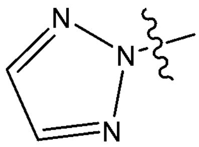, 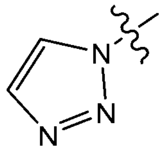, 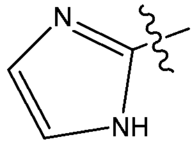, 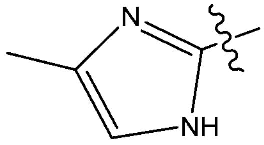 and 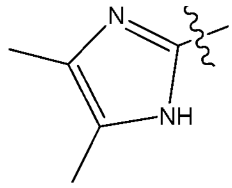.

In another preferred embodiment, each Ar is independently selected from the group consisting of: substituted phenyl,

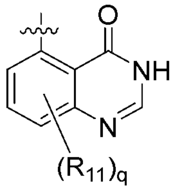 and 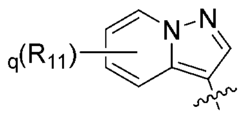;

the "substituted" refers to being substituted by one substituent selected from the group consisting of: - $C(O)NR_9R_{10}$, and 5-6 membered heteroaryl containing 1, 2, or 3 N atoms;

$R_{11}$, and q are as defined in claim 1;
$R_9$ is selected from the group consisting of: H and $C_{1-6}$ alkyl;
$R_{10}$ is selected from the group consisting of: H and $C_{1-6}$ alkyl;
and, $R_9$ and $R_{10}$ are not simultaneously H.

In another preferred embodiment, each Ar is independently selected from the group consisting of: substituted phenyl,

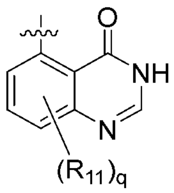, and 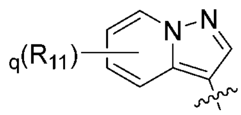;

the "substituted" refers to being substituted by one substituent selected from the group consisting of: - $C(O)NR_9R_{10}$, and 5-6 membered heteroaryl containing 1, 2, or 3 N atoms;

$R_{11}$, and q are as defined in claim 1;
$R_9$ is H; and
$R_{10}$ is $C_{1-6}$ alkyl.

In another preferred embodiment, one of $R_9$ and $R_{10}$ is H, and the other is $C_{1-6}$ alkyl (preferably, $C_{1-3}$ alkyl, more preferably, $C_1$ alkyl).
In another preferred embodiment, one of $R_9$ and $R_{10}$ is H, and the other is a $C_{1-6}$ alkyl selected from the group consisting of: methyl, ethyl, propyl, isopropyl, butyl, isobutyl, tert butyl, neopentyl, tert-pentyl, and hexyl.
In another preferred embodiment, Ar is a substituted phenyl, and the "substituted" refers to being substituted by $-C(O)NR_9R_{10}$.
In another preferred embodiment, both $X_1$ and $X_2$ are N; or one of $X_1$ and $X_2$ is N, and the other is CR.
In another preferred embodiment, in L, $X_1$ is N, and $X_2$ is CR.
In another preferred embodiment, in L, $X_1$ is CR, and $X_2$ is N.
In another preferred embodiment, in L, $X_1$ is N, and $X_2$ is N.
In another preferred embodiment, in ring B, $X_1$ is CR, and $X_2$ is CR.
In another preferred embodiment, in ring B, $X_1$ is CR, and $X_2$ is N. In another preferred embodiment, $R_1$ is selected from the group consisting of: hydrogen, halogen, trifluoromethyl, and cyano.
In another preferred embodiment, $R_1$ is a halogen selected from the group consisting of: F, Cl, Br, and I.
In another preferred embodiment, $R_2$ is H.

In another preferred embodiment, $R_3$ is selected from the group consisting of: H, $C_{1-6}$ alkyl, $C_{2-6}$ alkenyl, $C_{2-6}$ alkynyl, and $C_{3-6}$ cycloalkyl.

In another preferred embodiment, $R_3$ is selected from the group consisting of: $C_{1-6}$ alkyl, $C_{2-6}$ alkenyl, $C_{2-6}$ alkynyl, and $C_{3-6}$ cycloalkyl.

In another preferred embodiment, $R_3$ is a $C_{1-6}$ alkyl selected from the group consisting of: methyl, ethyl, propyl, isopropyl, butyl, isobutyl, tert butyl, neopentyl, tert-pentyl, and hexyl.

In another preferred embodiment, the compound is represented by Formula II, Formula III, Formula IV, Formula V, Formula VI, and Formula VII,

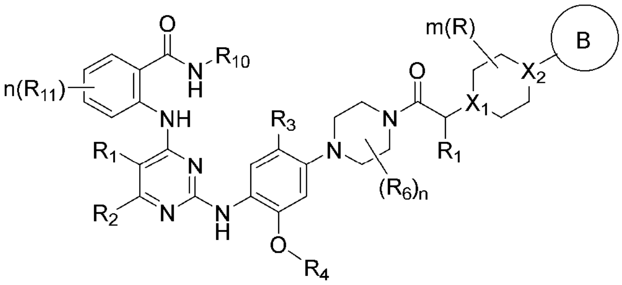

Formula II ,

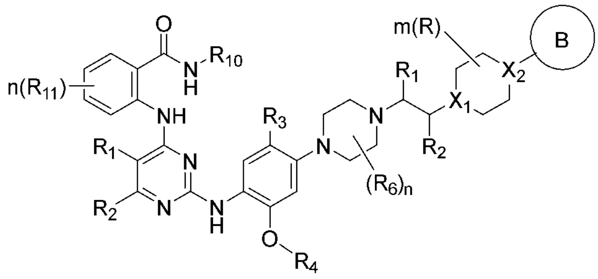

Formula III

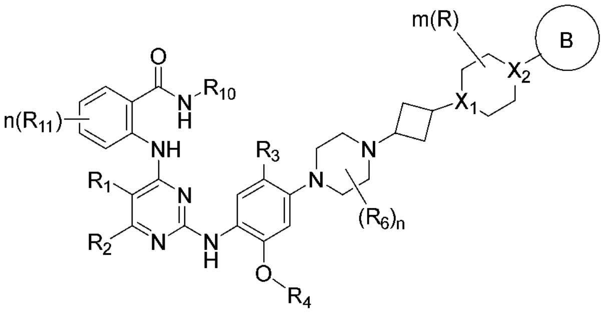

Formula IV ,

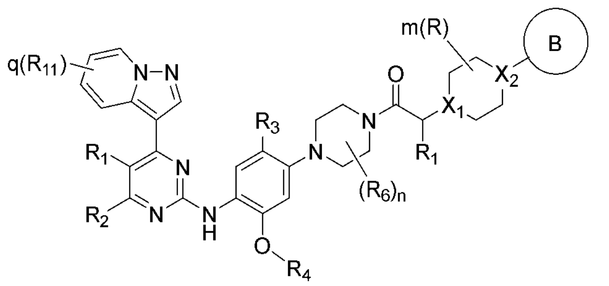

Formula V ,

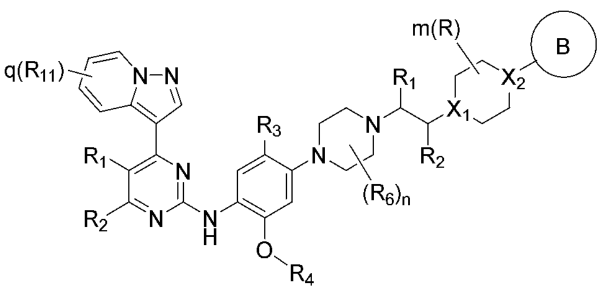

Formula VI ,

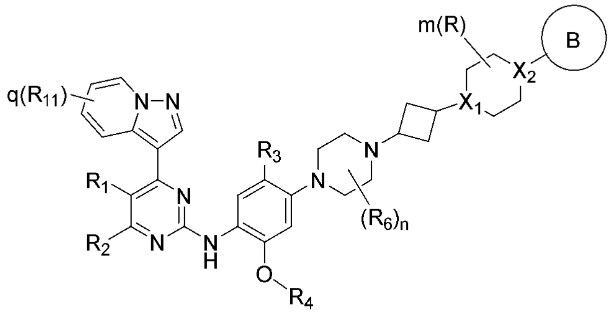

Formula VII ,

wherein, $R_4$ is a $C_{1-6}$ alkyl selected from the group consisting of: methyl, ethyl, propyl, isopropyl, butyl, isobutyl, tert butyl, neopentyl, tert-pentyl, and hexyl.

In another preferred embodiment, $R_6$ together with its attached ring form a 3- to 6-membered ring, thereby forming a spiro, fused or bridged ring, preferably, $R_6$ is $C_{1-4}$ alkylene and together with its attached ring form

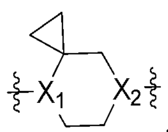 , 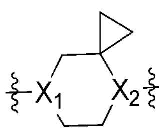 , 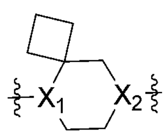 ,

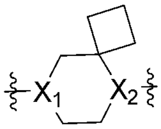, 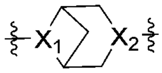, 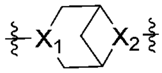, 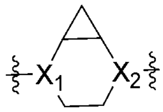, and 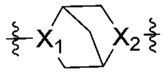,

preferably, $R_6$ is methylene;
wherein, $X_1$ and $X_2$ are defined as above.

In another preferred embodiment, the compound is selected from the group consisting of:

| | |
|---|---|
| 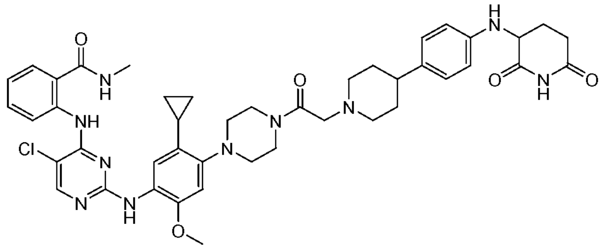<br>T-001 | 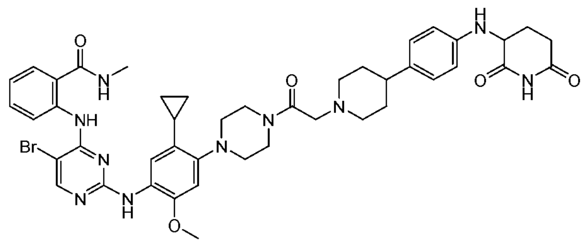<br>T-002 |
| 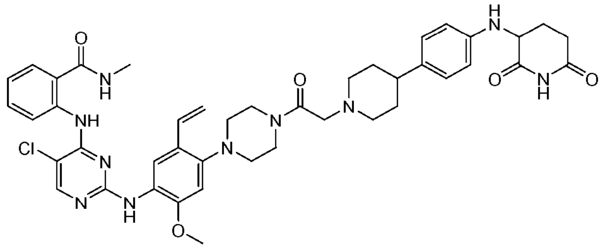<br>T-003 | 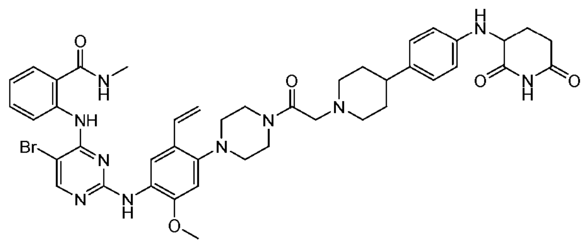<br>T-004 |
| 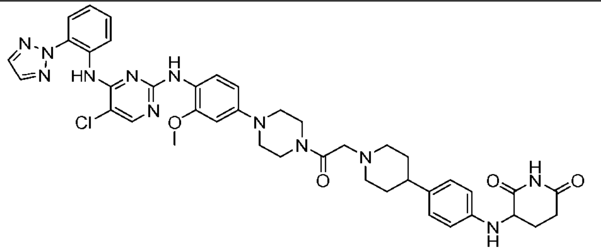<br>T-005 | 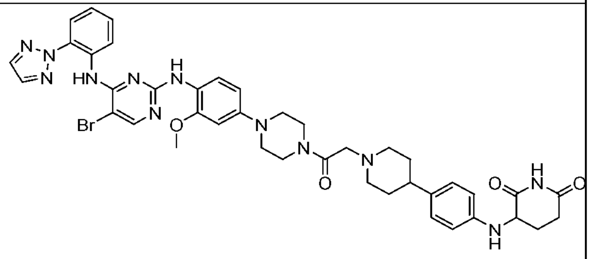<br>T-006 |
| 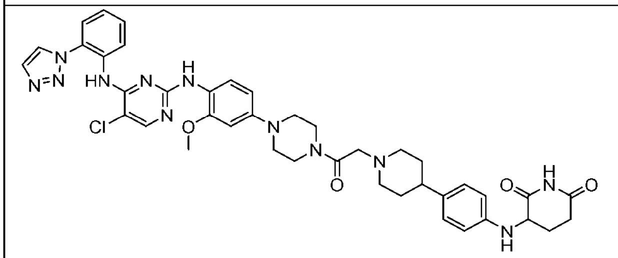<br>T-007 | 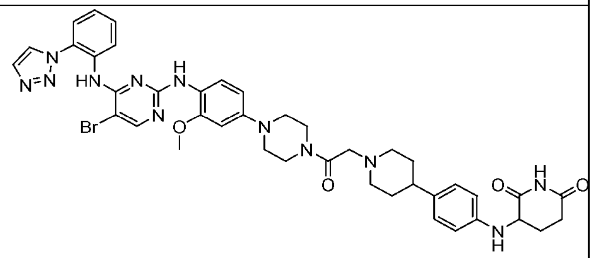<br>T-008 |
| 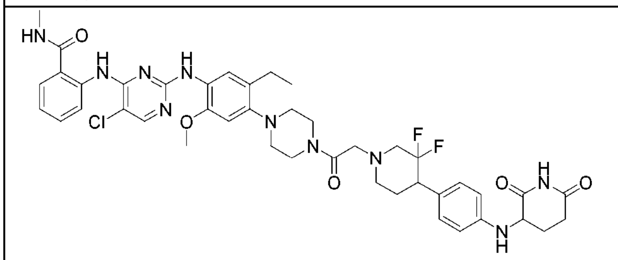<br>T-009 | 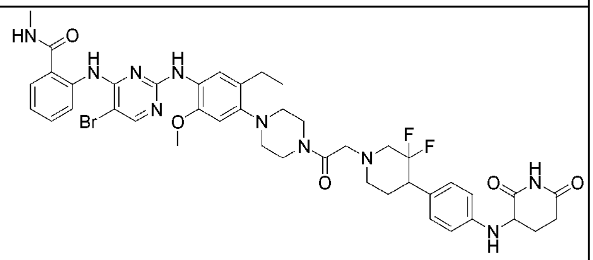<br>T-010 |
| 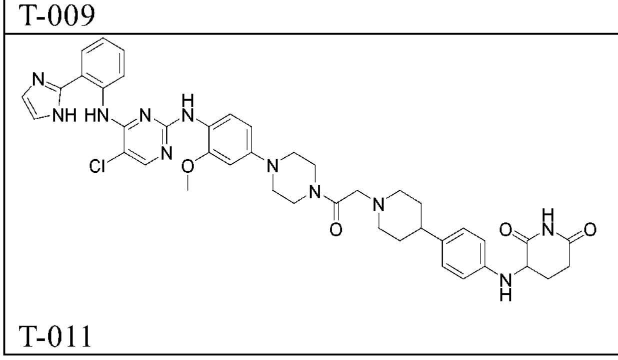<br>T-011 | 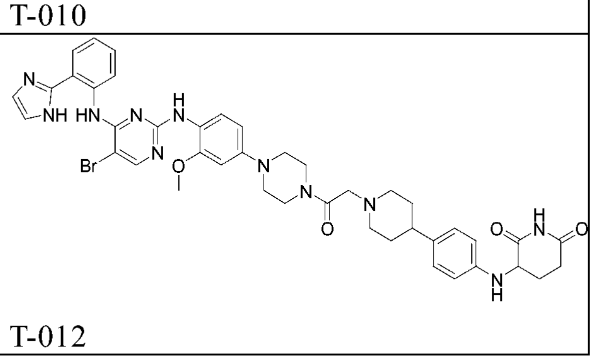<br>T-012 |

| | |
|---|---|
| 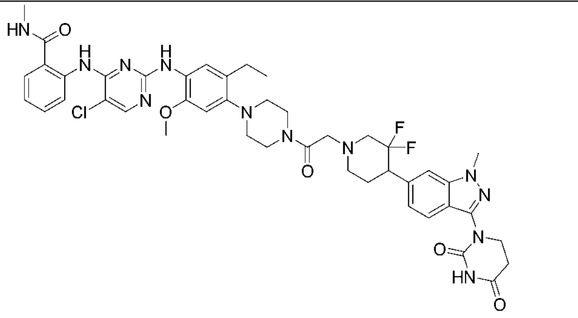<br>T-013 | 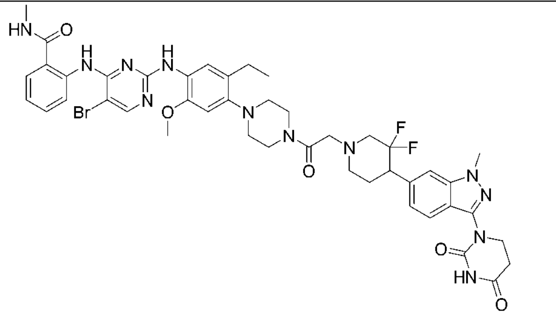<br>T-014 |
| 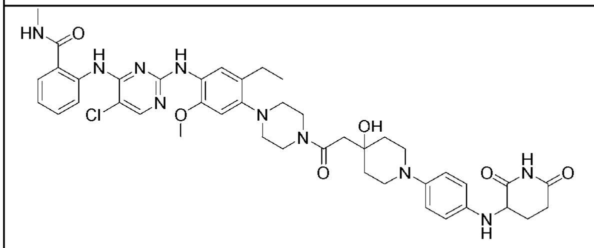<br>T-015 | 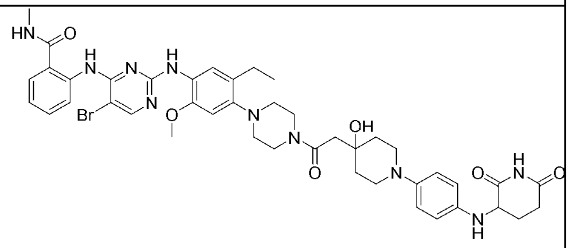<br>T-016 |
| 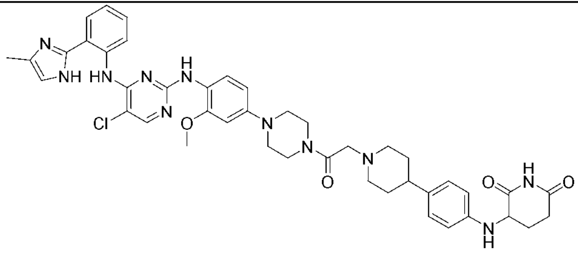<br>T-017 | 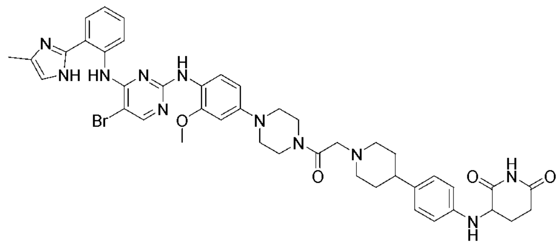<br>T-018 |
| 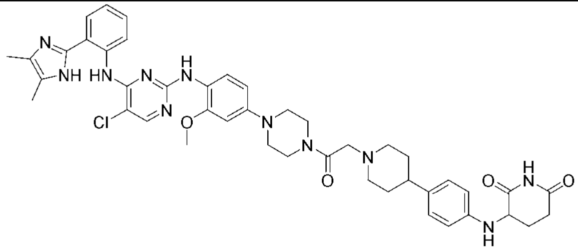<br>T-019 | 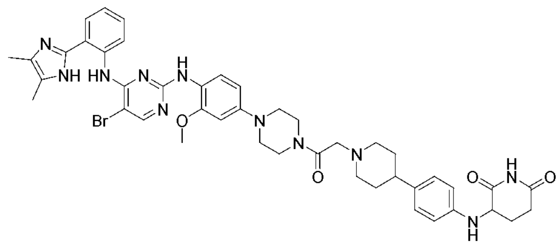<br>T-020 |
| 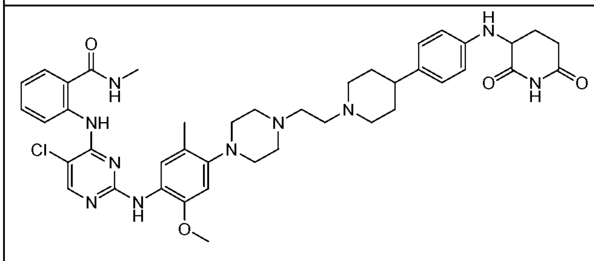<br>T-021 | 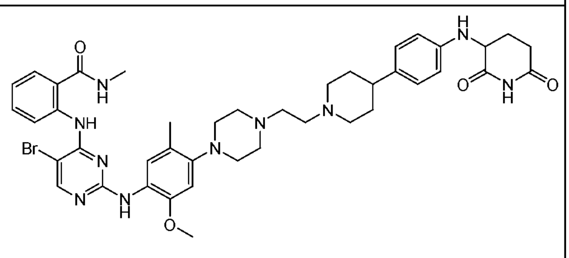<br>T-022 |
| 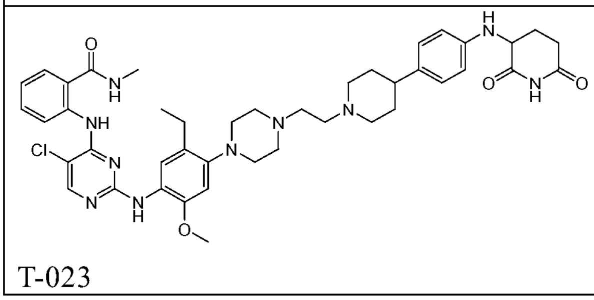<br>T-023 | 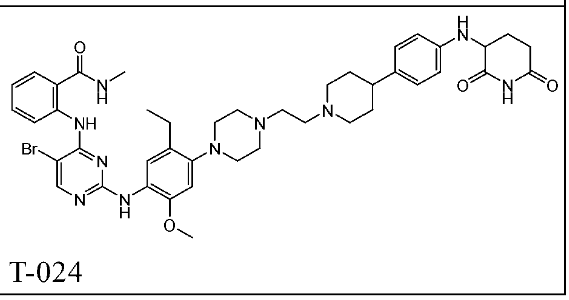<br>T-024 |

| | |
|---|---|
| 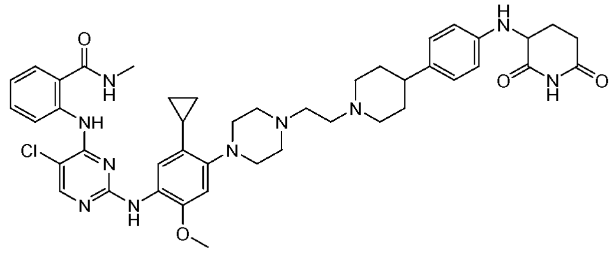 T-025 | 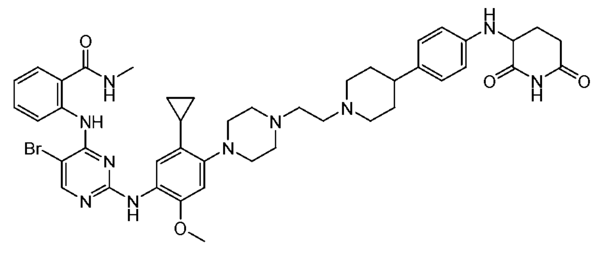 T-026 |
| 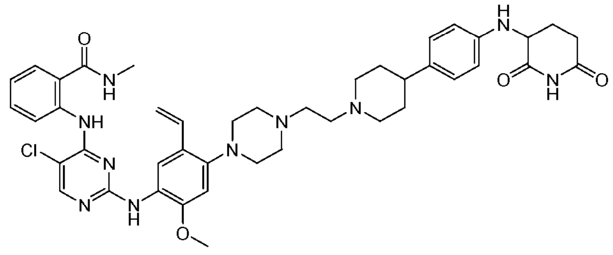 T-027 | 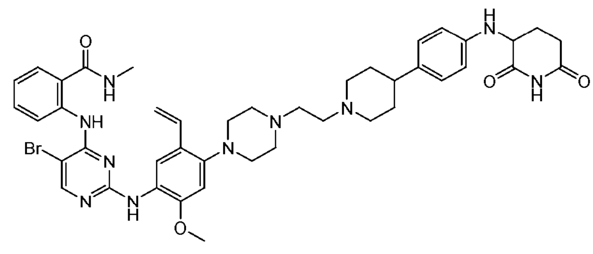 T-028 |
| 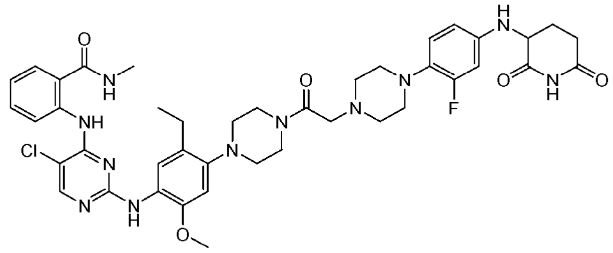 T-029 | 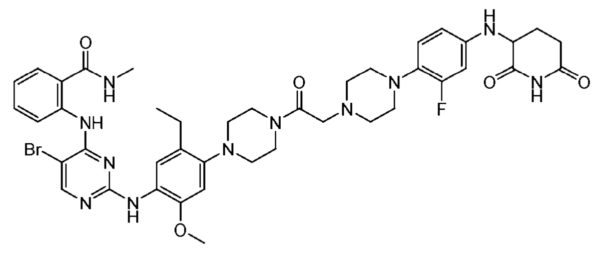 T-030 |
| 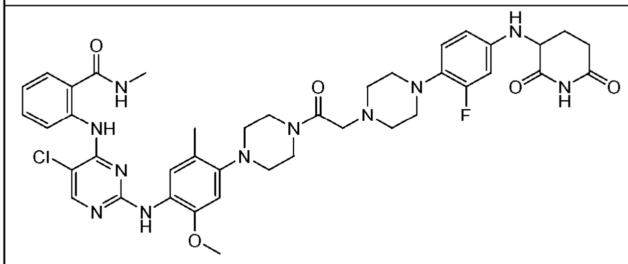 T-031 | 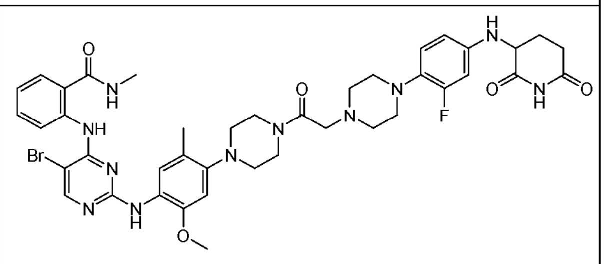 T-032 |
| 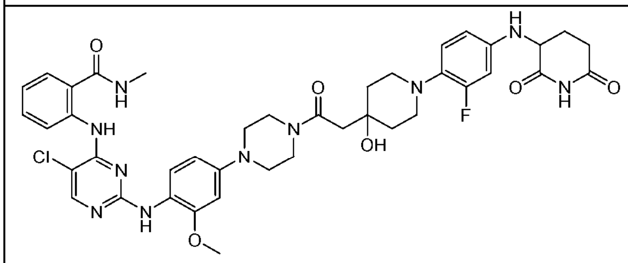 T-033 | 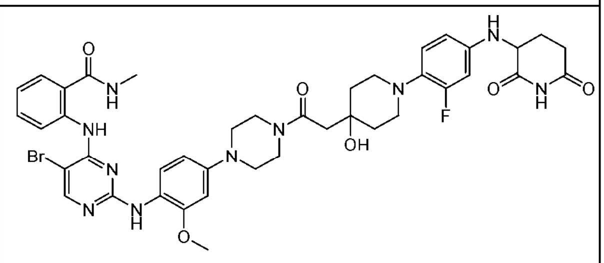 T-034 |
| 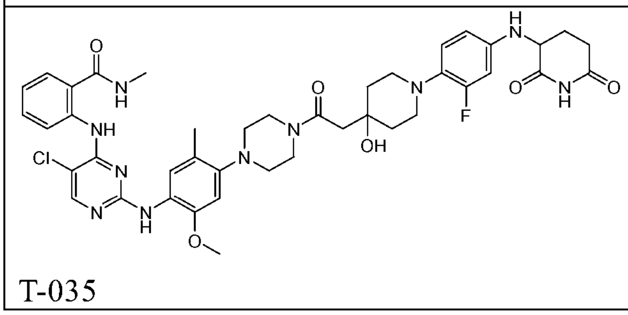 T-035 | 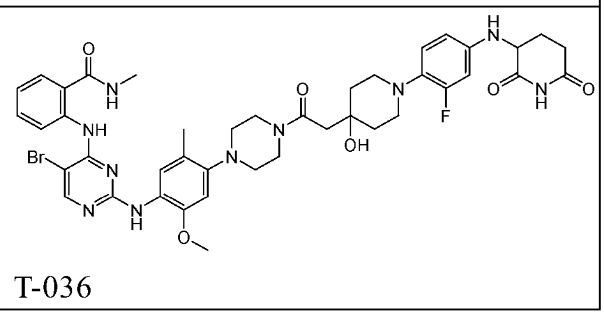 T-036 |

| | |
|---|---|
| 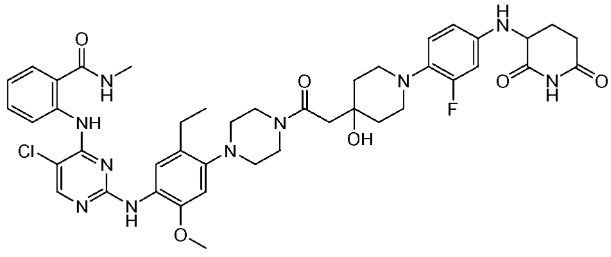<br>T-037 | 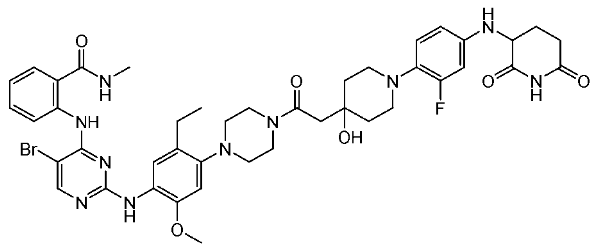<br>T-038 |
| 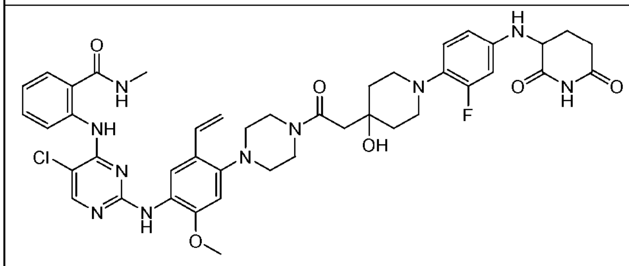<br>T-039 | 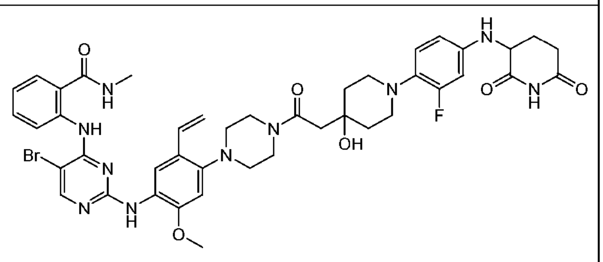<br>T-040 |
| 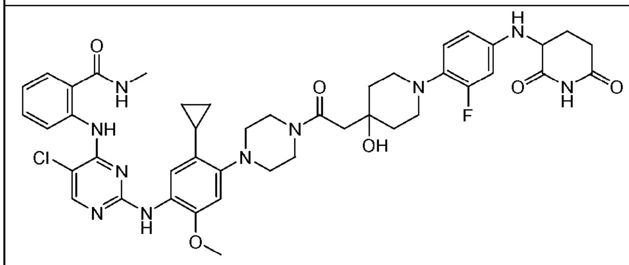<br>T-041 | 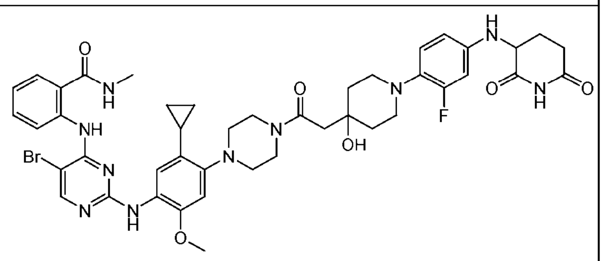<br>T-042 |
| 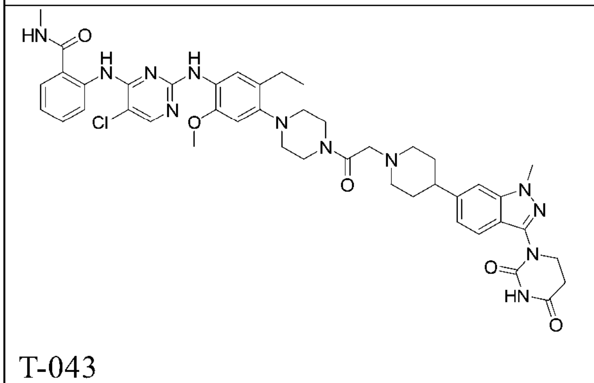<br>T-043 | 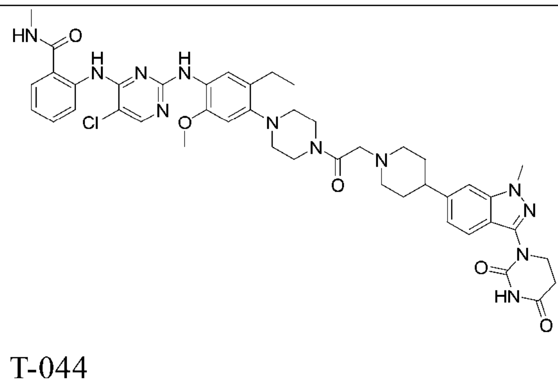<br>T-044 |
| 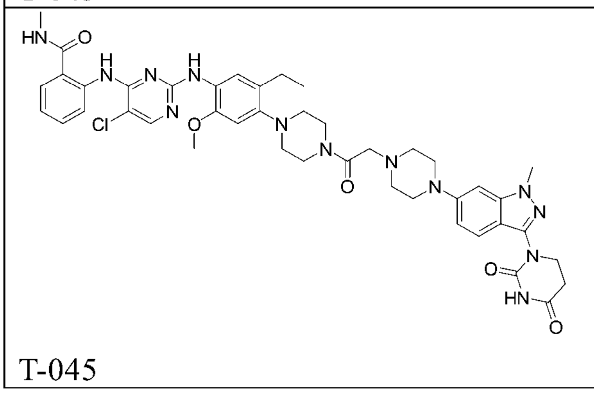<br>T-045 | 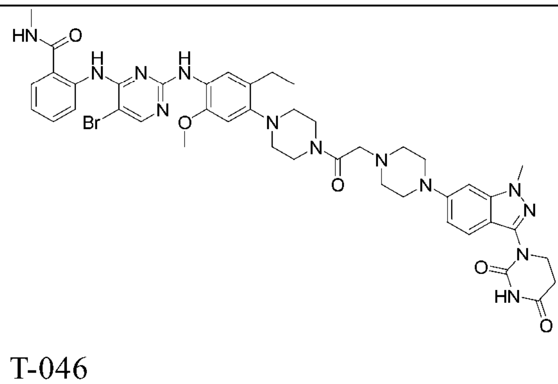<br>T-046 |

| | |
|---|---|
| 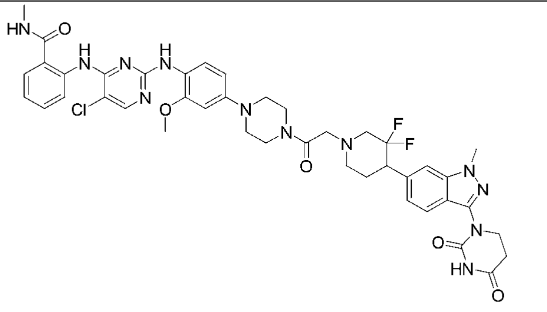 T-047 | 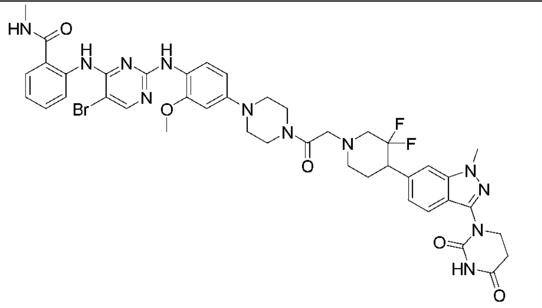 T-048 |
| 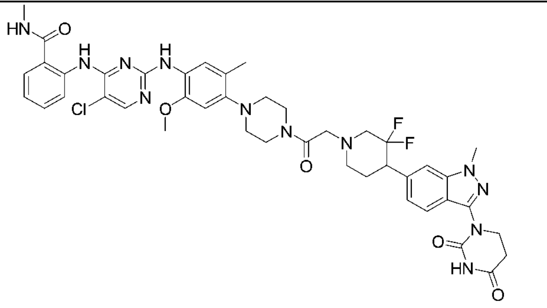 T-049 | 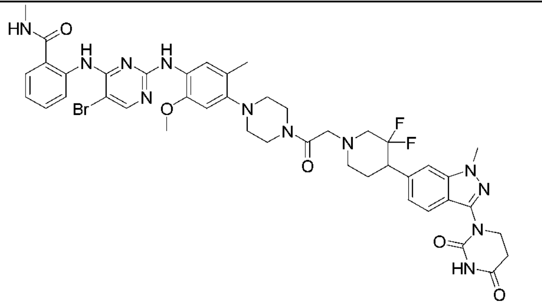 T-050 |
| 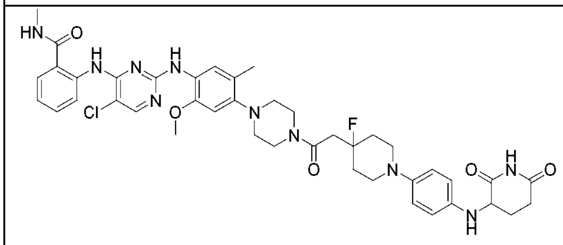 T-051 | 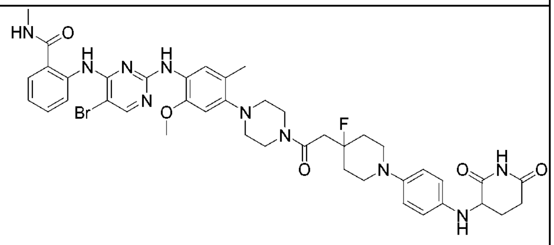 T-052 |
| 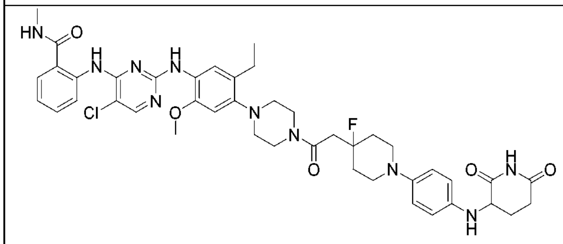 T-053 | 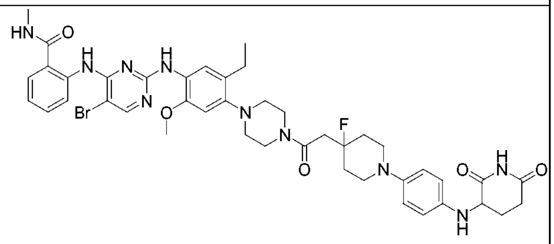 T-054 |
| 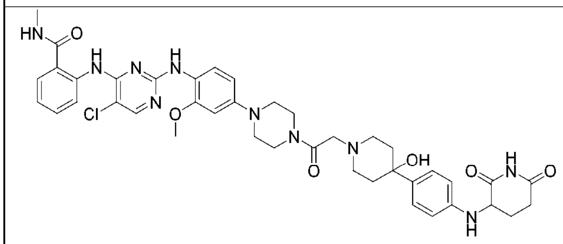 T-055 | 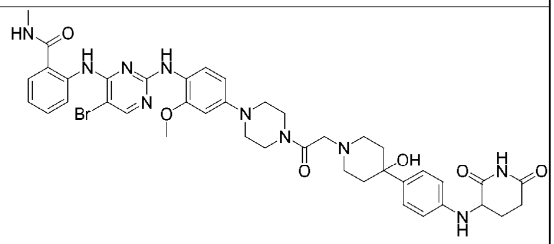 T-056 |
| 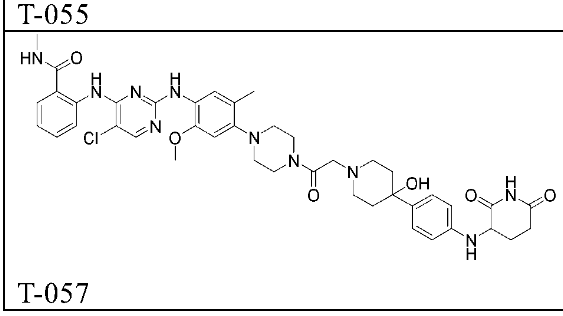 T-057 | 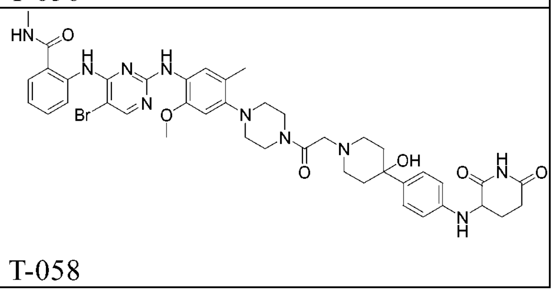 T-058 |

| | |
|---|---|
| 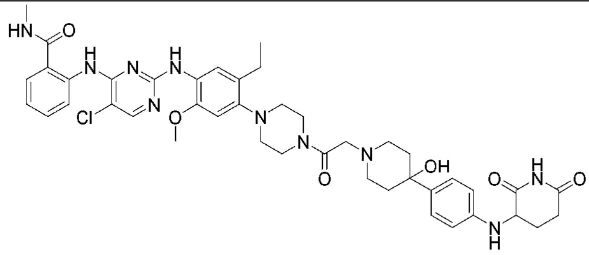<br>T-059 | 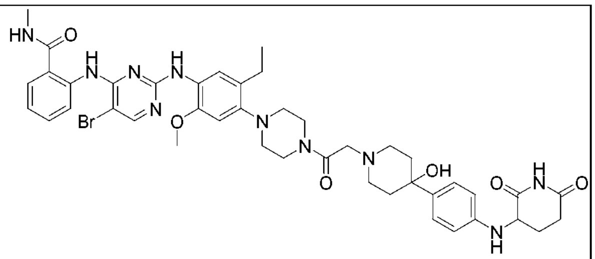<br>T-060 |
| 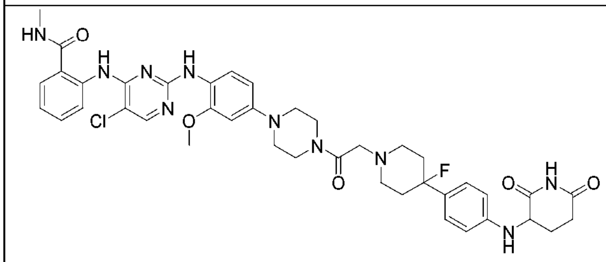<br>T-061 | 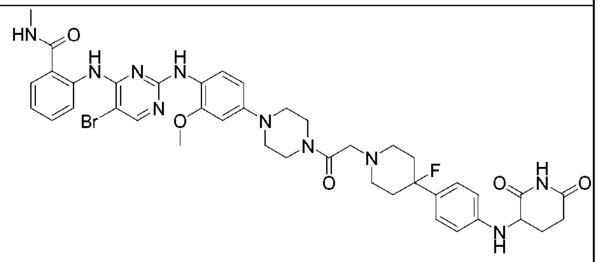<br>T-062 |
| 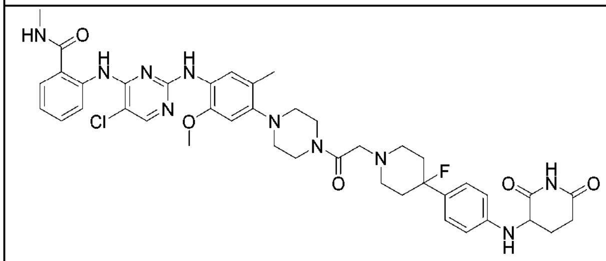<br>T-063 | 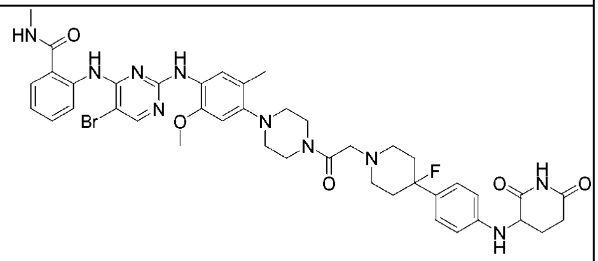<br>T-064 |
| 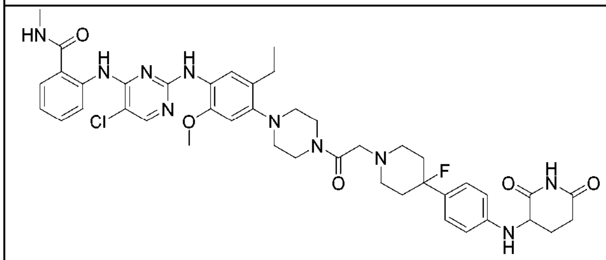<br>T-065 | 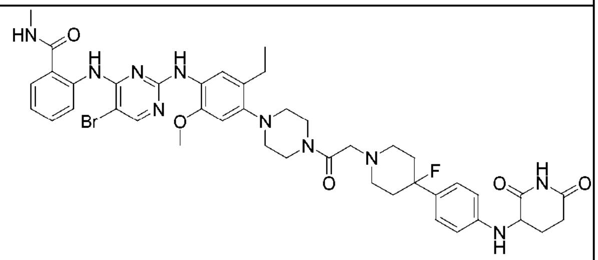<br>T-066 |
| 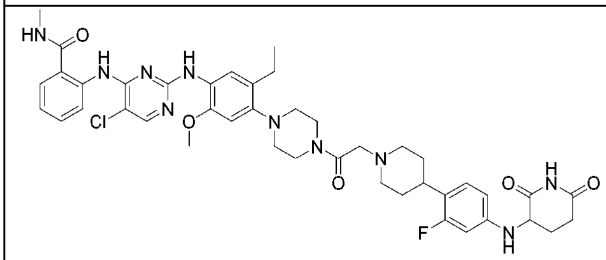<br>T-067 | 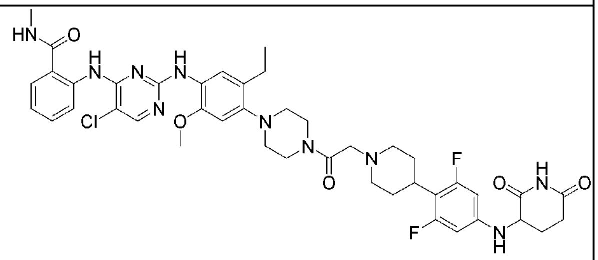<br>T-068 |
| 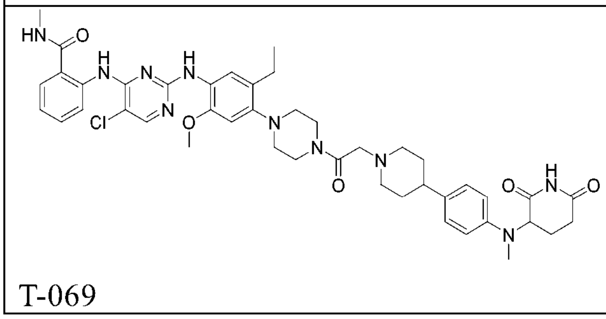<br>T-069 | 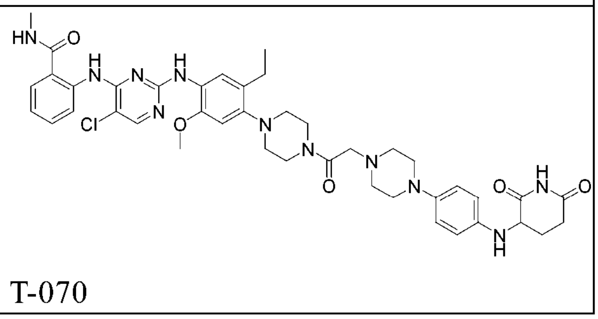<br>T-070 |

| | |
|---|---|
| 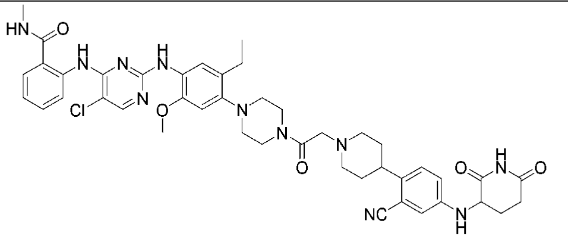 T-071 | 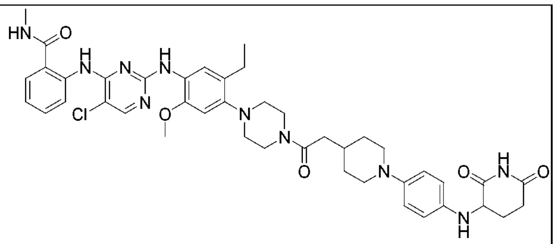 T072 |
| 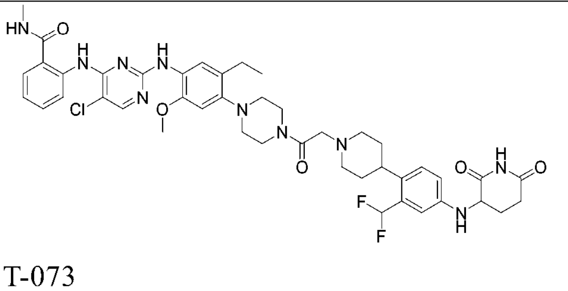 T-073 | 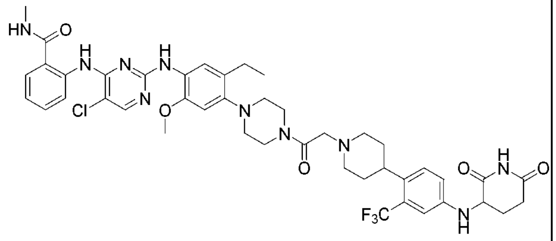 T-074 |
| 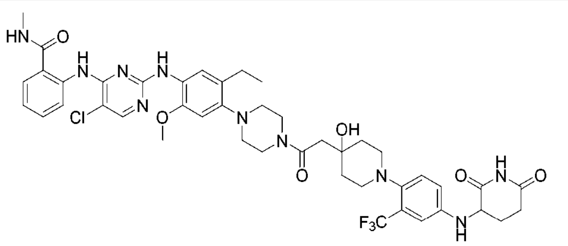 T-075 | 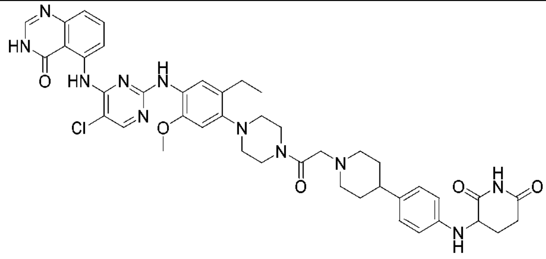 T-076 |
| 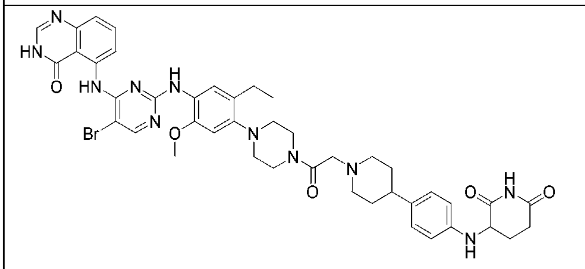 T-077 | 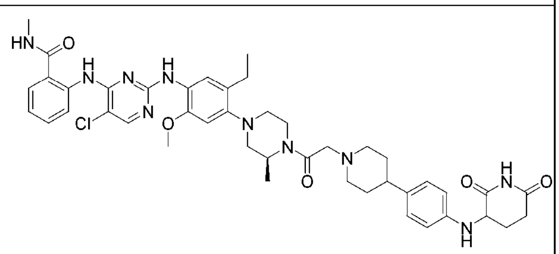 T-078 |
| 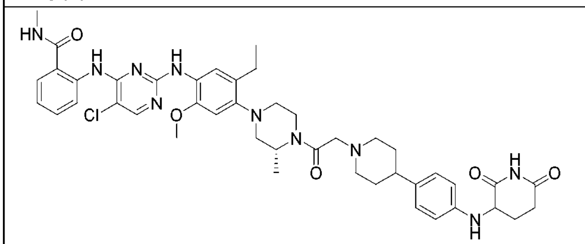 T-079 | 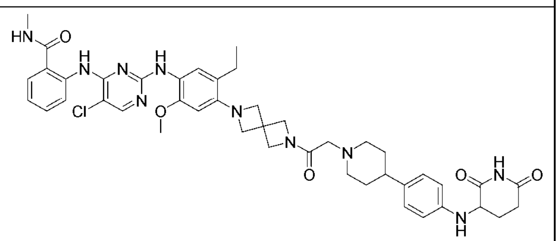 T-080 |
| 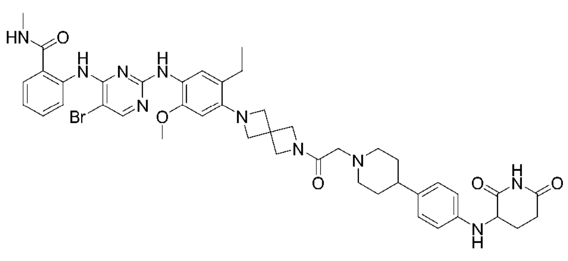 T-081 | 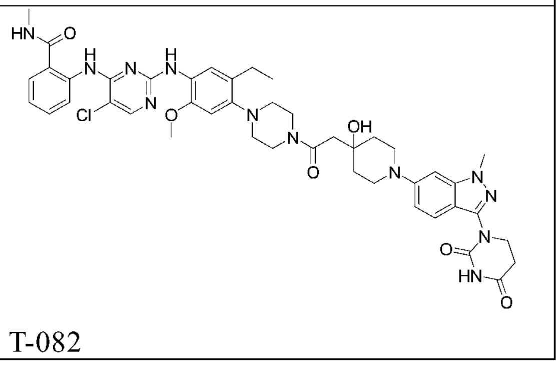 T-082 |

| | |
|---|---|
| 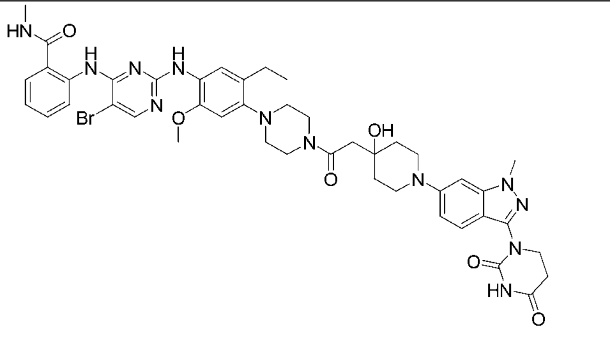 T-083 | 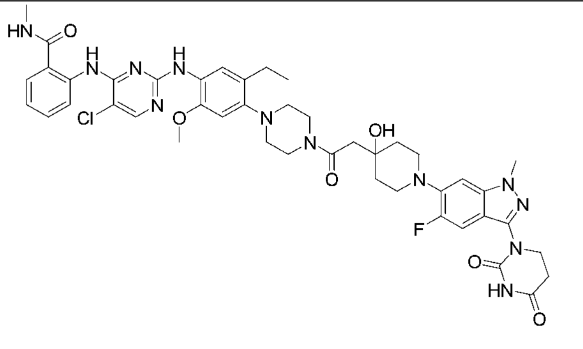 T-084 |
| 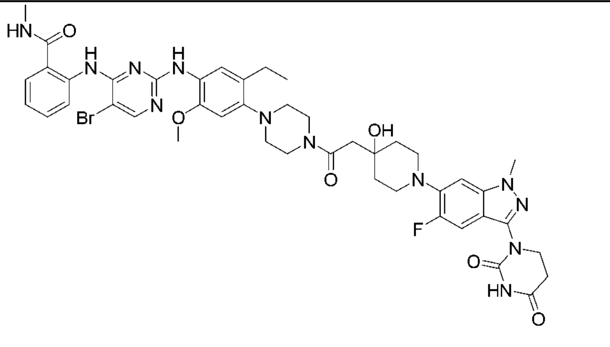 T-085 | 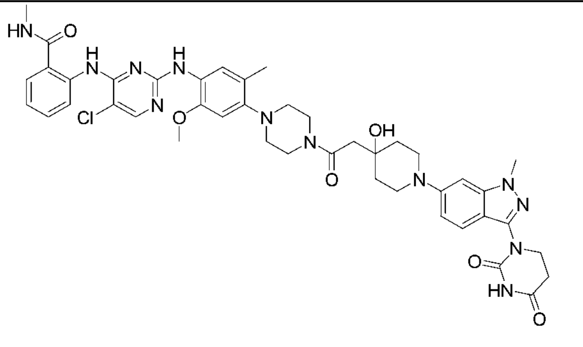 T-086 |
| 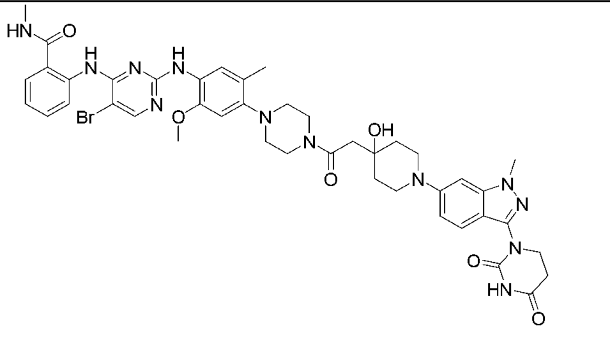 T-087 | 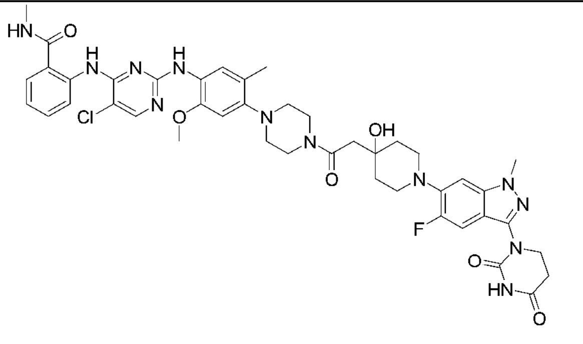 T-088 |
| 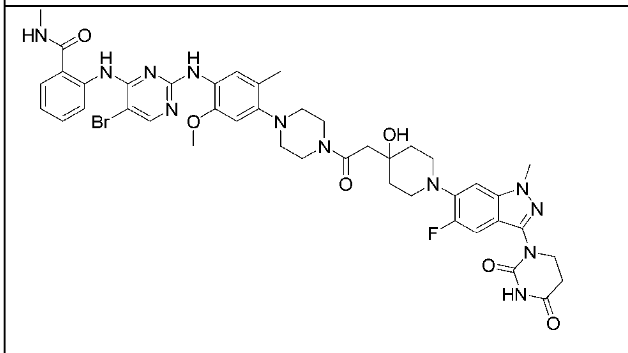 T-089 | 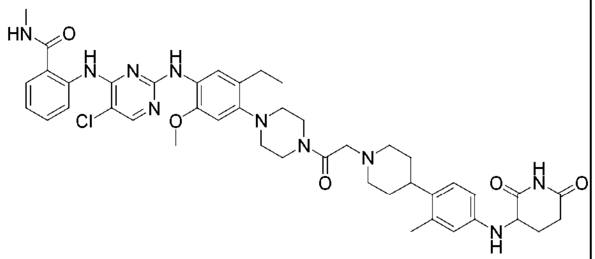 T-090 |
| 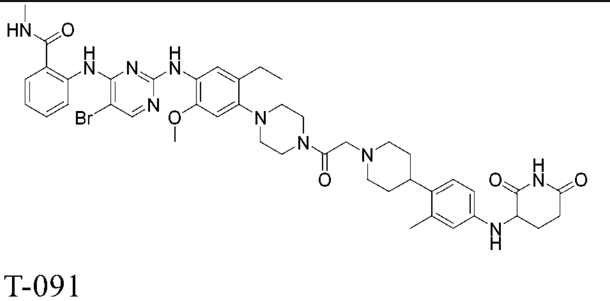 T-091 | 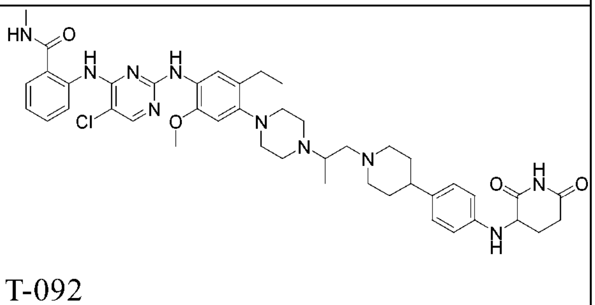 T-092 |

| | |
|---|---|
| 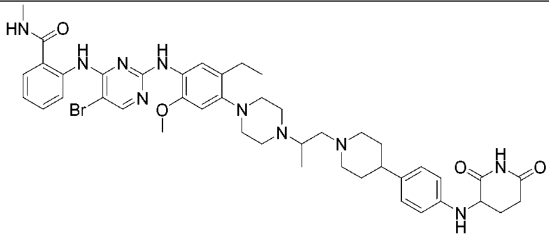 T-093 | 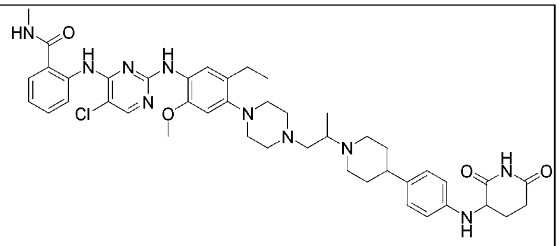 T-094 |
| 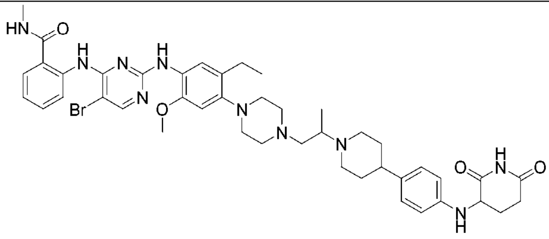 T-095 | 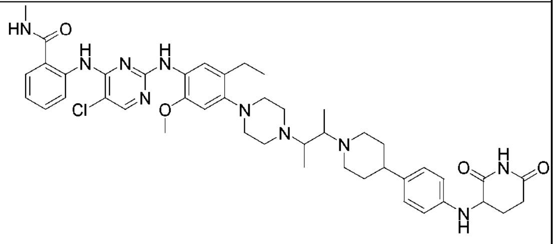 T-096 |
| 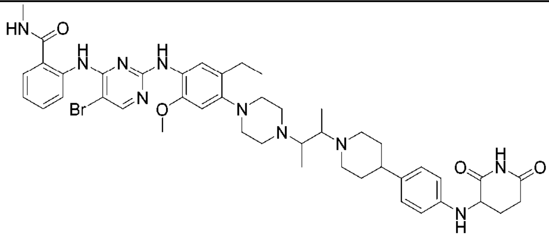 T-097 | 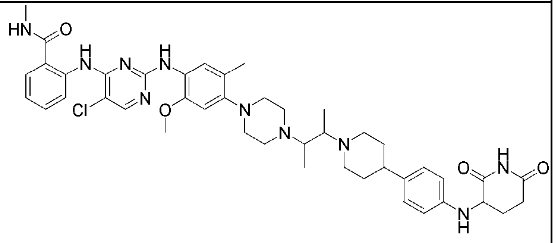 T-098 |
| 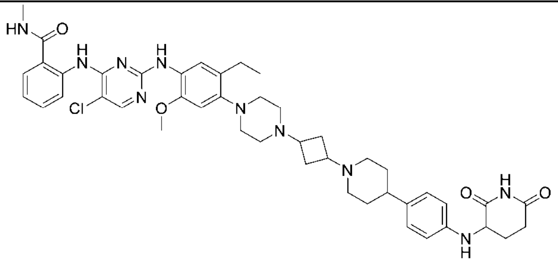 T-099 | 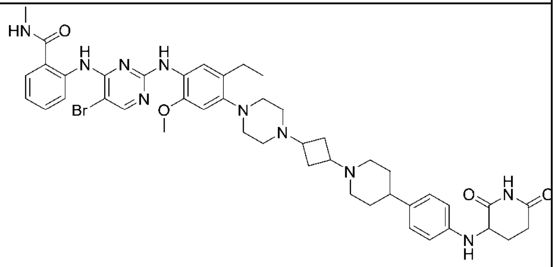 T-100 |
| 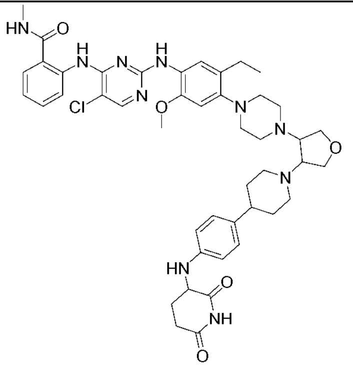 T-101 | 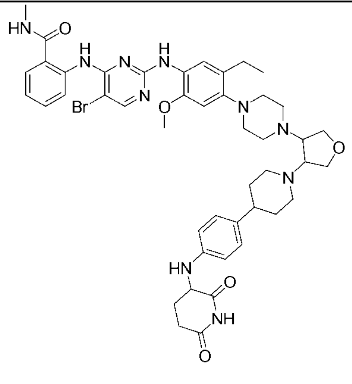 T-102 |
| 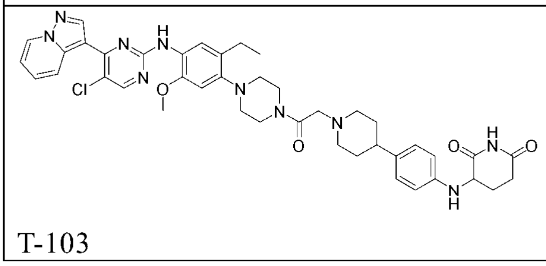 T-103 | 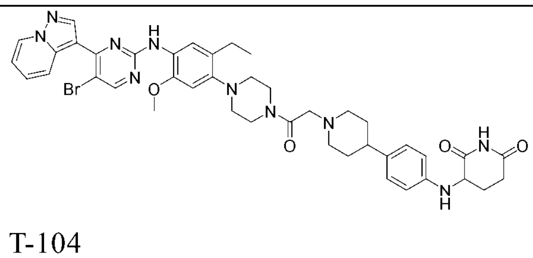 T-104 |

| | |
|---|---|
| 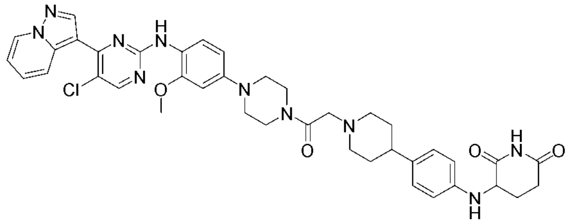 T-105 | 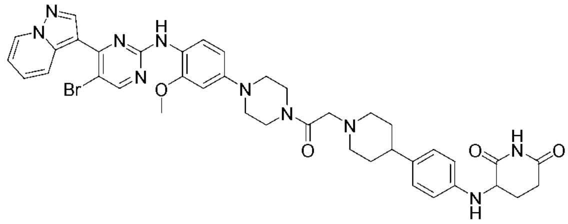 T-106 |
| 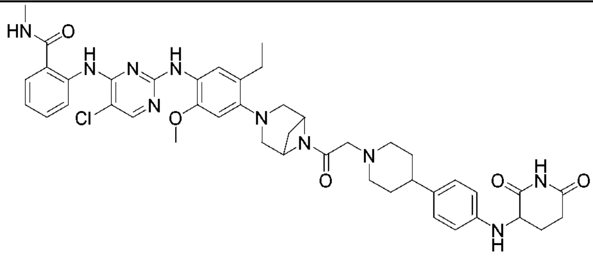 T-107 | 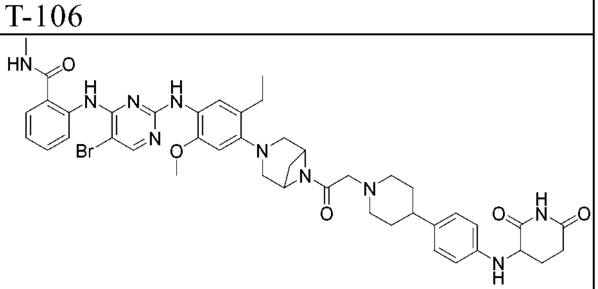 T-108 |
| 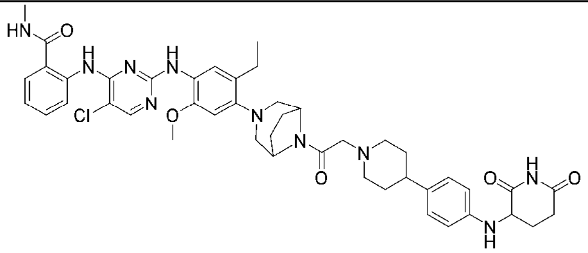 T-109 | 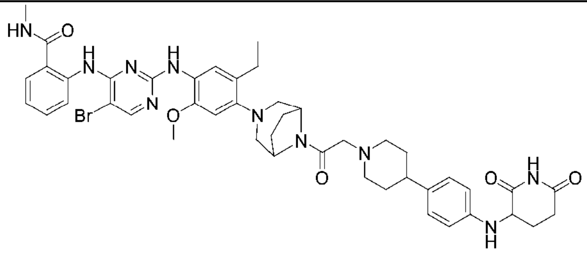 T-110 |
| 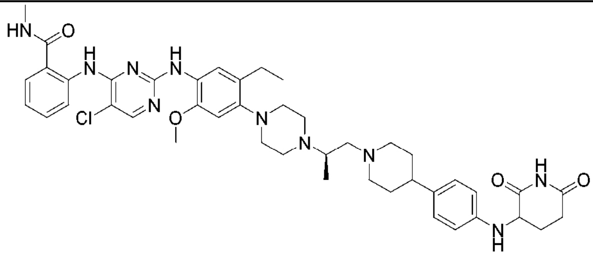 T-111 | 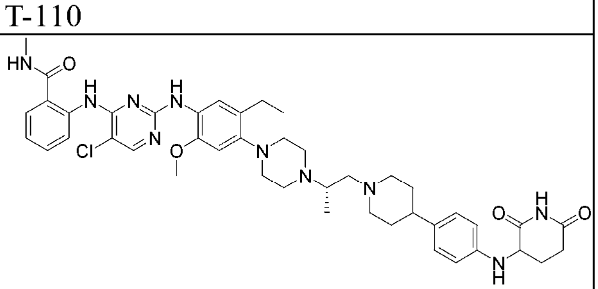 T-112 |
| 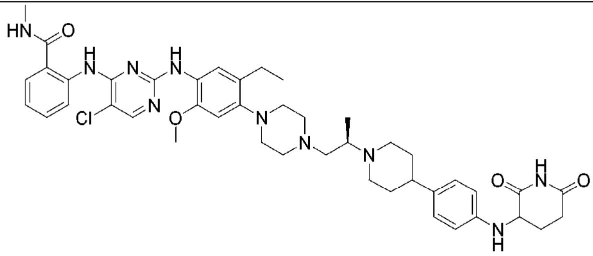 T-113 | 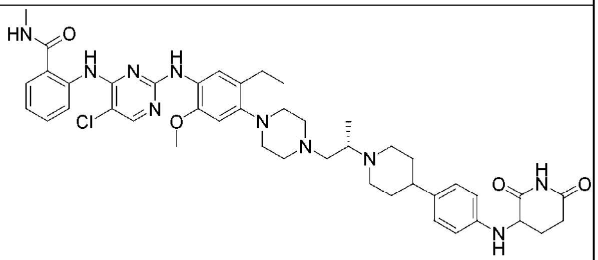 T-114 |
| 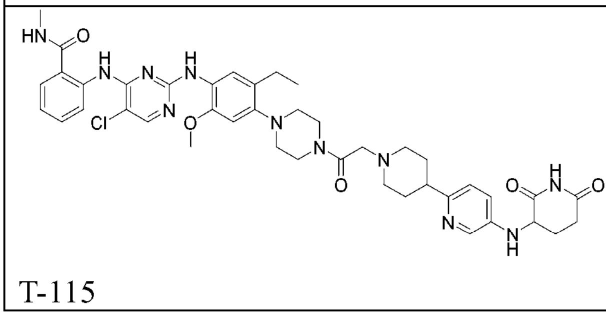 T-115 | 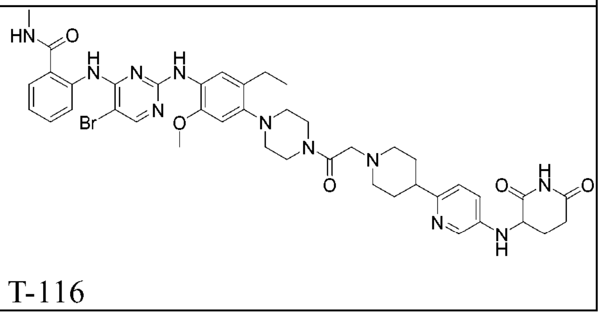 T-116 |

| | |
|---|---|
| 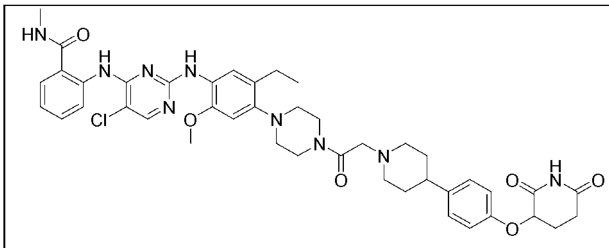 T-117 | 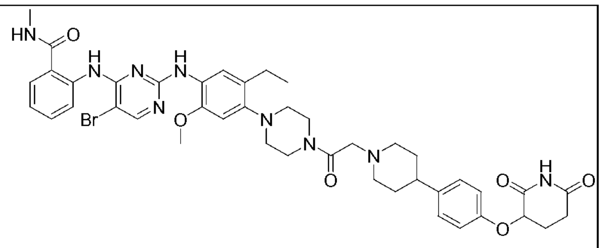 T-118 |
| 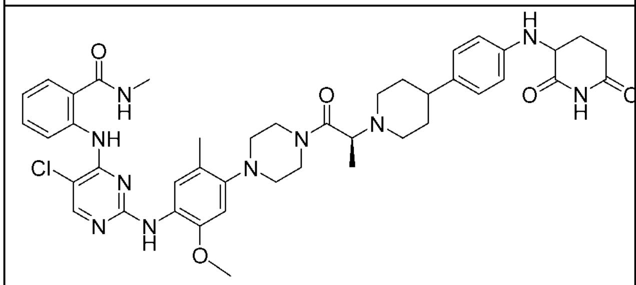 T-119 | 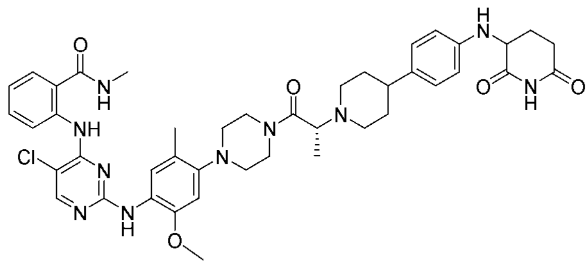 T-120 |
| 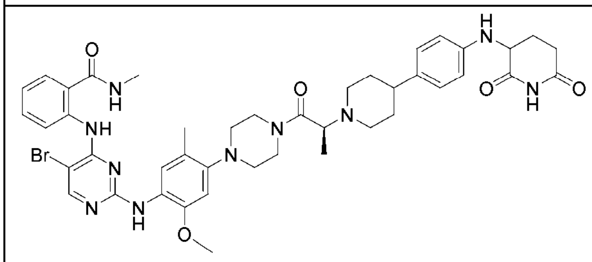 T-121 | 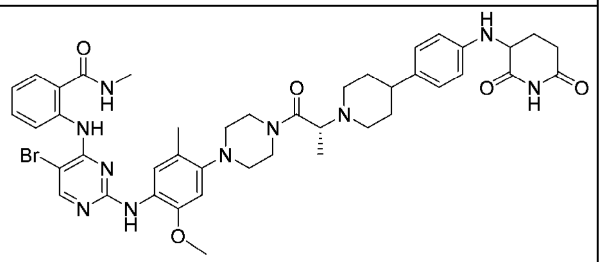 T-122 |
| 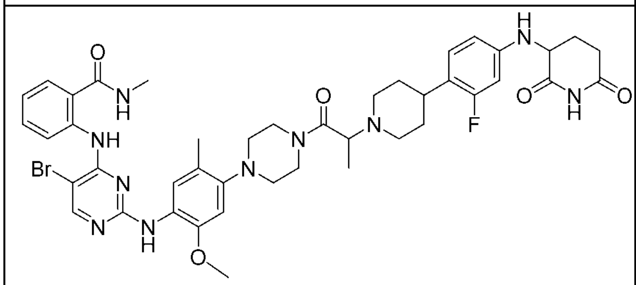 T-123 | 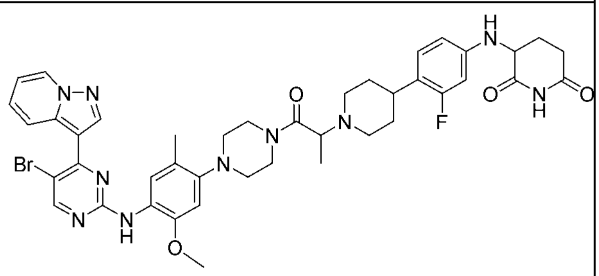 T-124 |
| 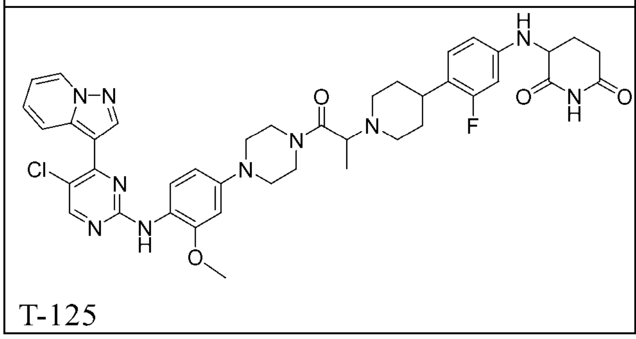 T-125 | 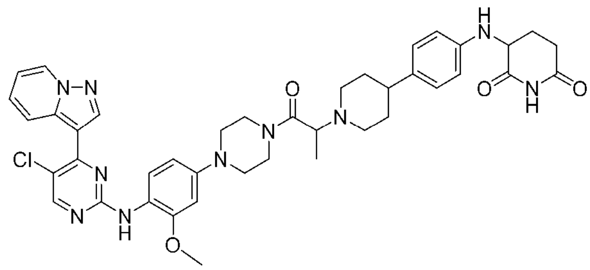 T-126 |

.

In another preferred embodiment, the pharmaceutically acceptable salt is an inorganic acid salt or an organic acid salt;

the inorganic acid salt is selected from the group consisting of: hydrochloride, hydrobromide, hydriodate, sulfate, bisulfate, nitrate, phosphate, and acid phosphate;

the organic acid salt is selected from the group consisting of: formate, acetate, trifluoroacetate, propionate, pyruvate, hydroxyacetate, oxalate, malonate, fumarate, maleate, lactate, malate, citrate, tartrate, methanesulfonate, ethanesulfonate, benzenesulfonate, p-toluenesulfonate, salicylate, picrate, glutamate, ascorbate, camphorate, and camphorsulfonate.

In another preferred embodiment, the compound is not selected from the group consisting of:

| | |
|---|---|
| 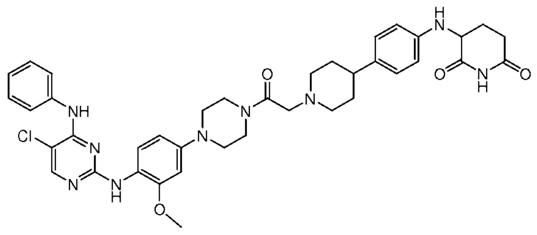 T-01 | 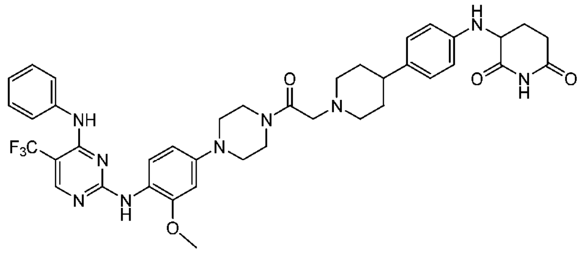 T-02 |
| 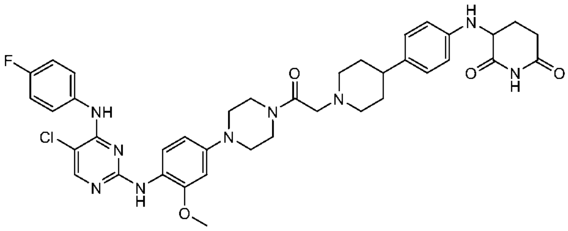 T-03 | 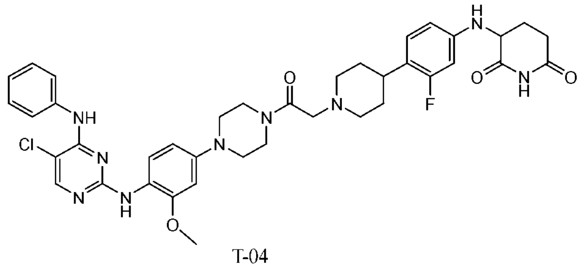 T-04 |
| 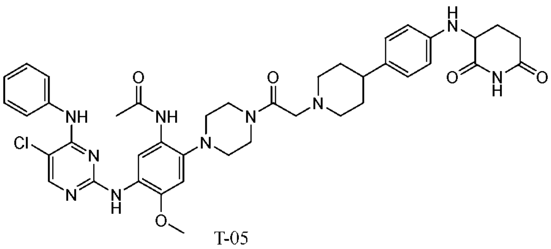 T-05 | 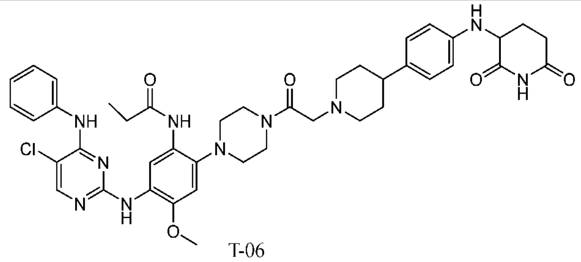 T-06 |
| 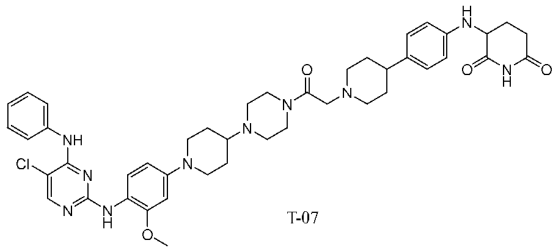 T-07 | 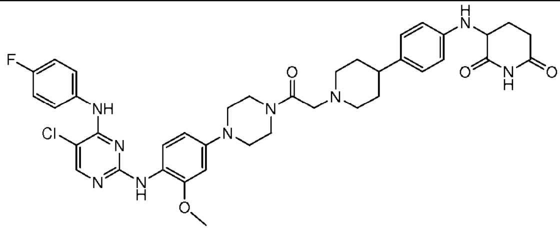 T-08 |
| 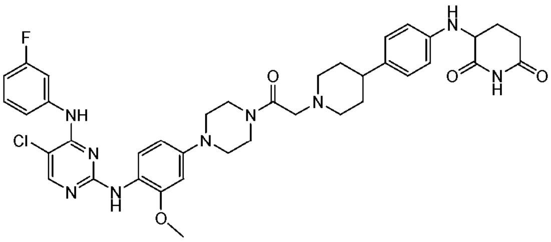 T-09 | 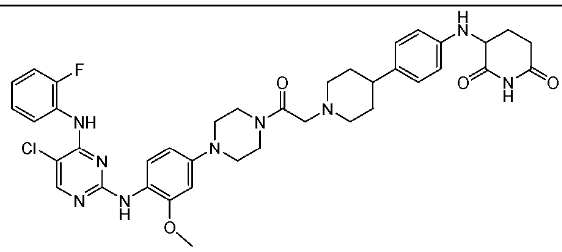 T-10 |

| | |
|---|---|
| 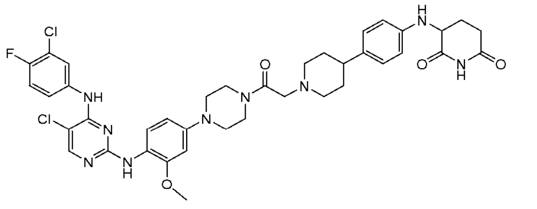 T-11 | 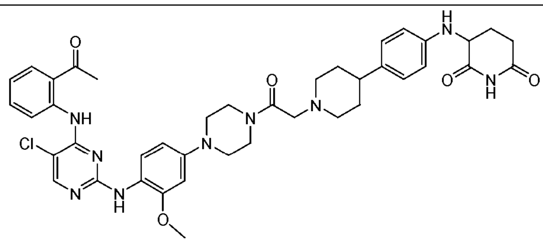 T-12 |
| 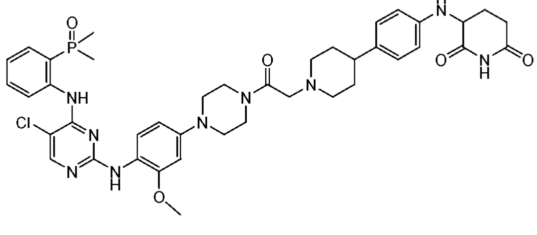 T-13 | 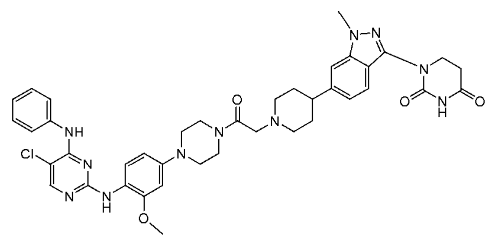 T-14 |
| 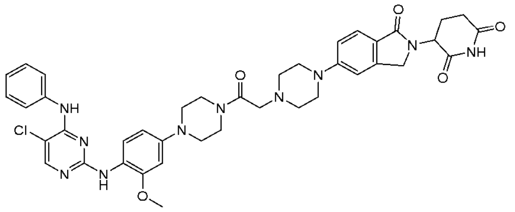 T-15 | 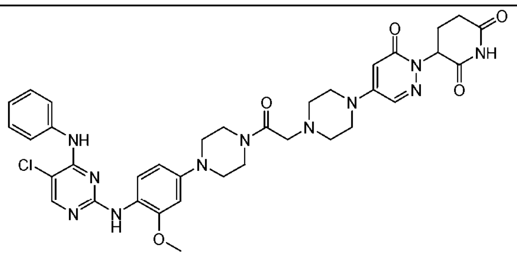 T-16 |
| 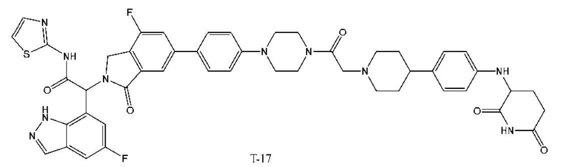 T-17 | 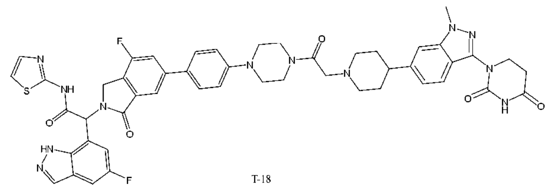 T-18 |
| 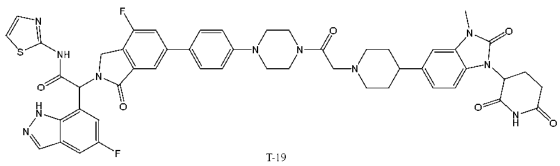 T-19 | 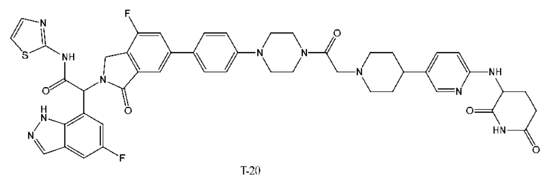 T-20 |
| 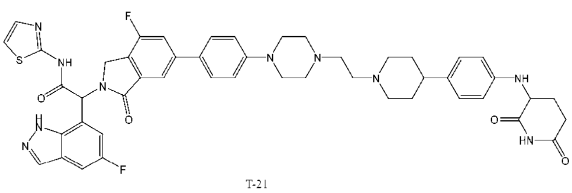 T-21 | 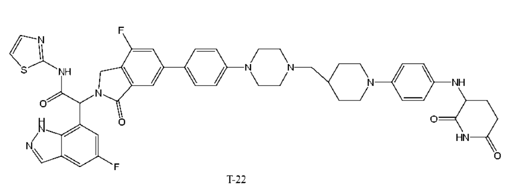 T-22 |
| 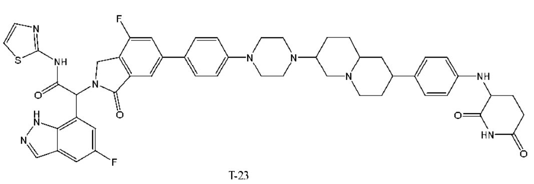 T-23 | 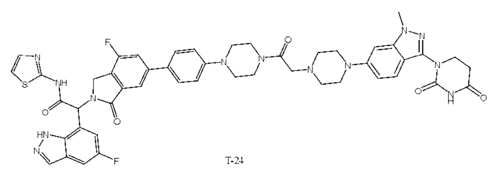 T-24 |

| | |
|---|---|
| 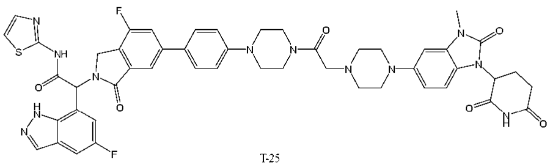<br>T-25 | 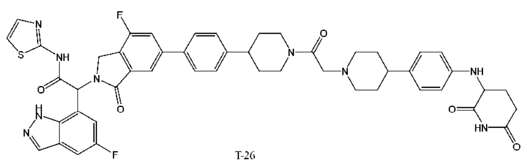<br>T-26 |
| 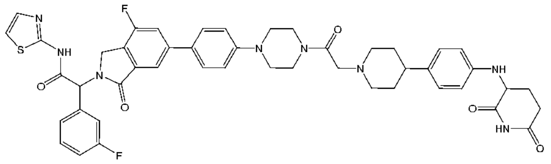<br>T-27 | 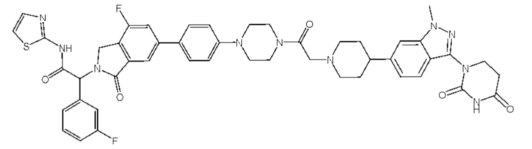<br>T-28 |
| 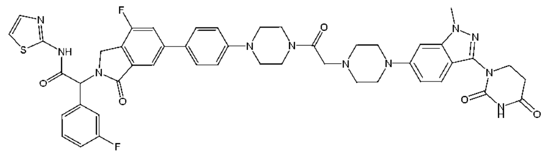<br>T-29 | 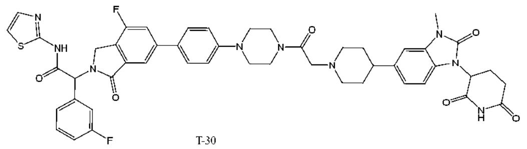<br>T-30 |
| 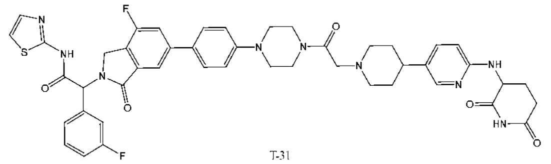<br>T-31 | 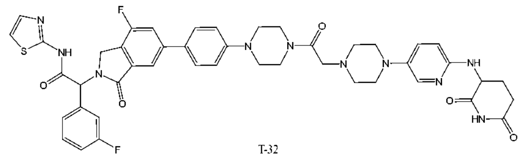<br>T-32 |
| 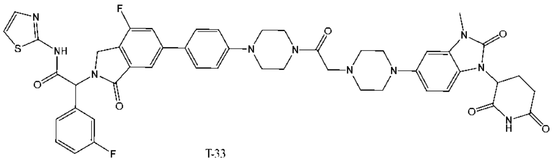<br>T-33 | 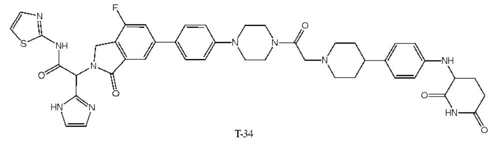<br>T-34 |
| 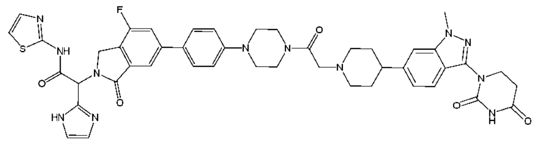<br>T-35 | 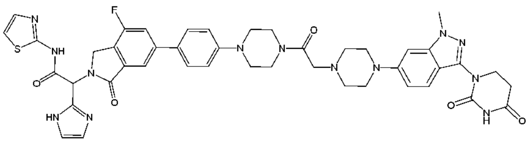<br>T-36 |
| 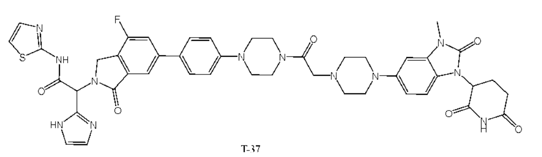<br>T-37 | 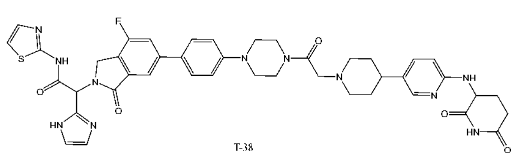<br>T-38 |

| | |
|---|---|
| 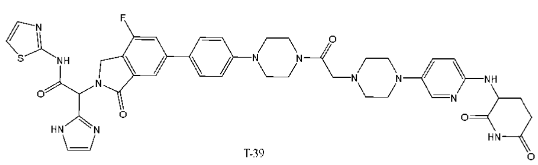<br>T-39 | 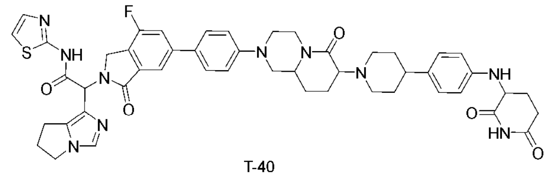<br>T-40 |
| 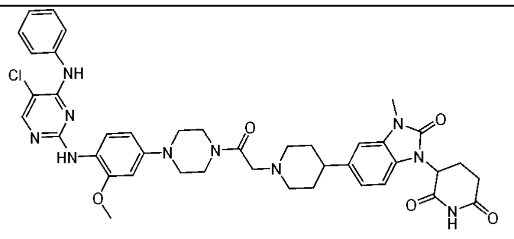<br>T-41 | 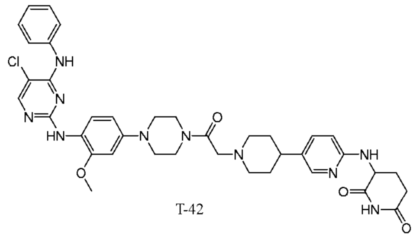<br>T-42 |
| 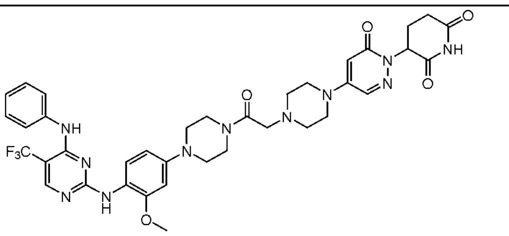<br>T-43 | 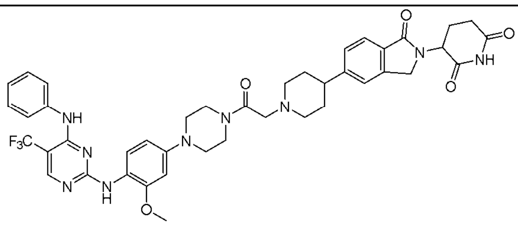<br>T-44 |
| 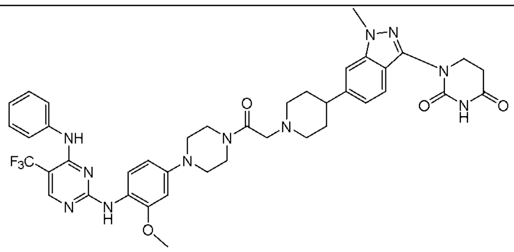<br>T-45 | 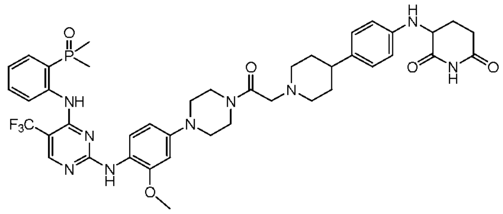<br>T-46 |
| 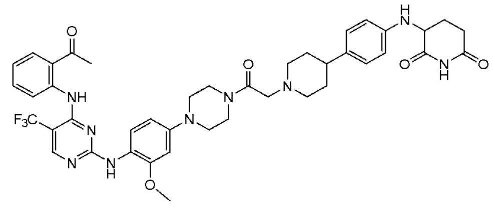<br>T-47 | 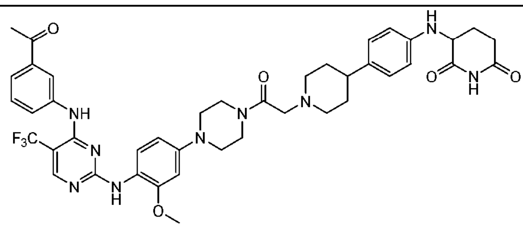<br>T-48 |
| 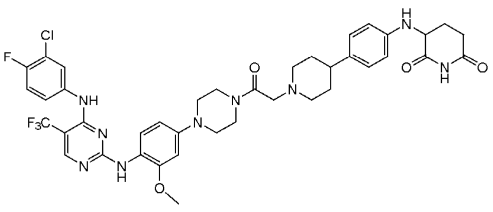<br>T-49 | 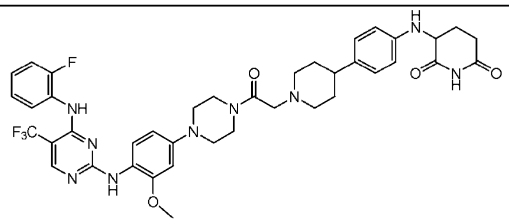<br>T-50 |
| 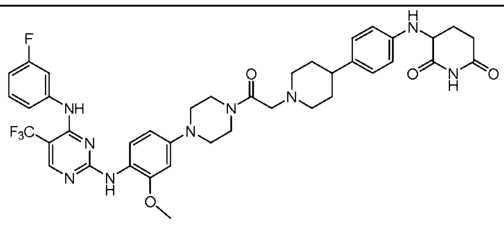<br>T-51 | 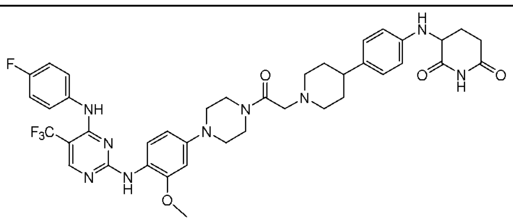<br>T-52 |

| | |
|---|---|
| 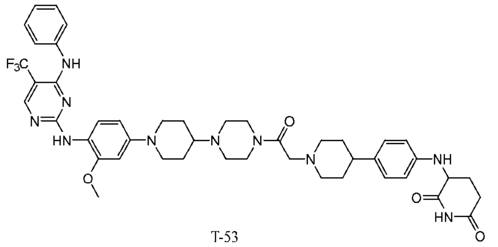 T-53 | 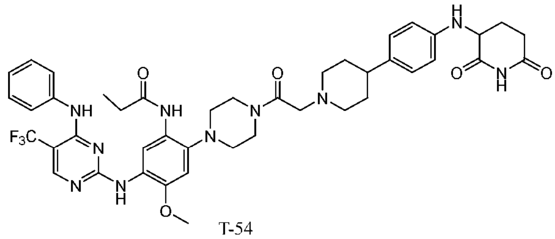 T-54 |
| 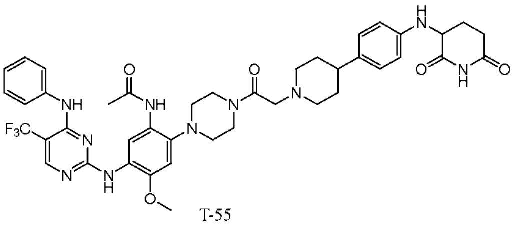 T-55 | 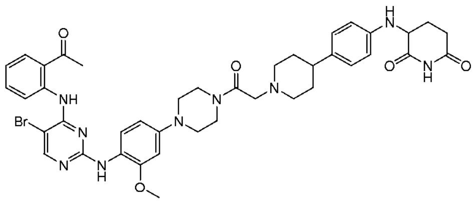 T-56 |
| 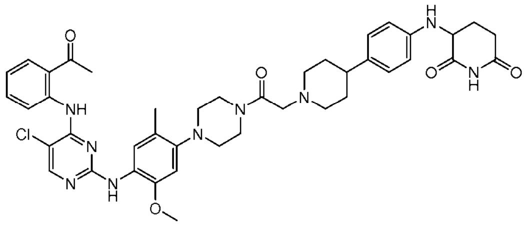 T-57 | 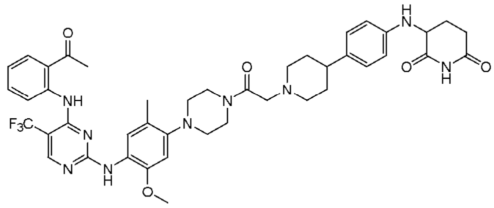 T-58 |
| 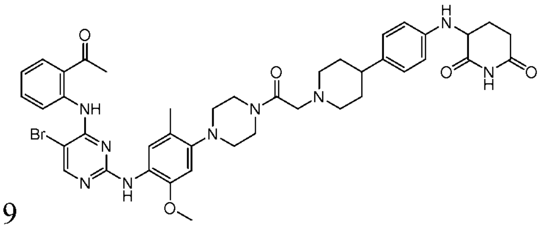 T-59 | 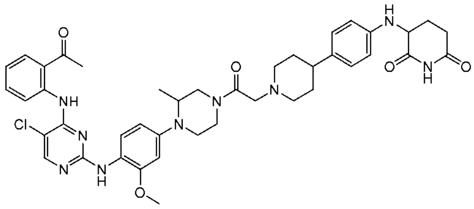 T-60 |
| 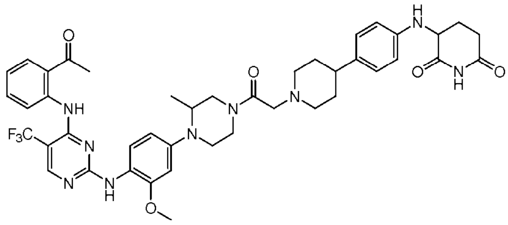 T-61 | 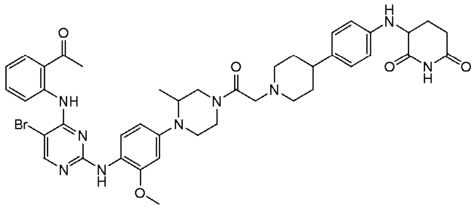 T-62 |
| 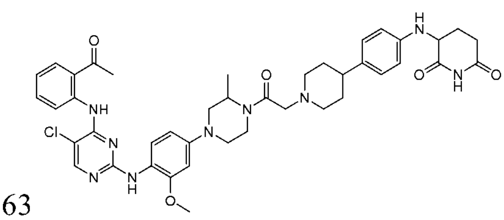 T-63 | 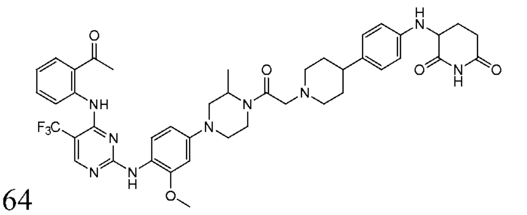 T-64 |
| 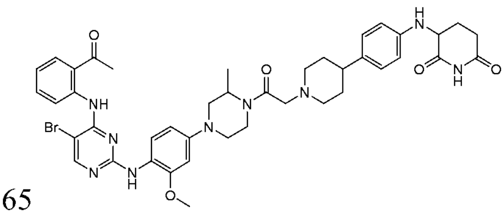 T-65 | 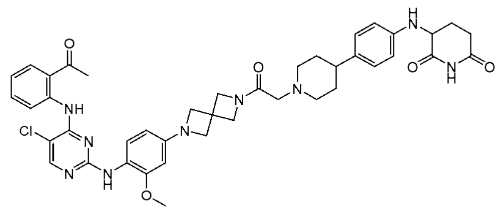 T-66 |

| | |
|---|---|
| 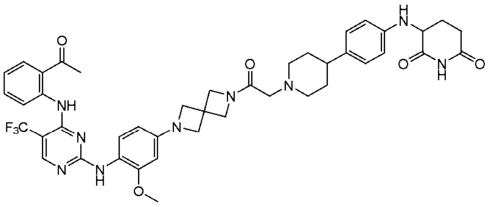 T-67 | 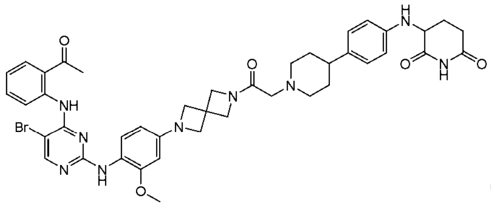 T-68 |
| 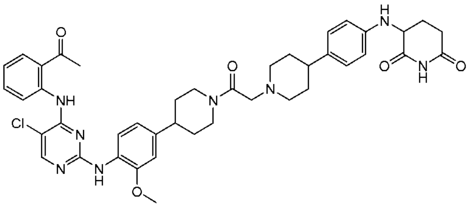 T-69 | 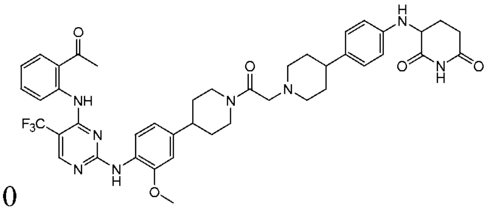 T-70 |
| 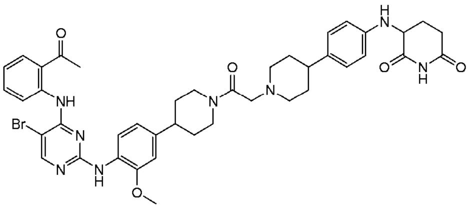 T-71 | 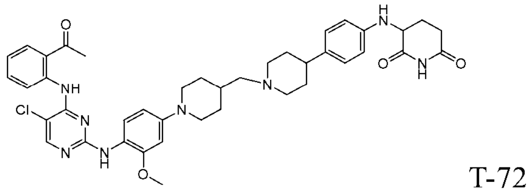 T-72 |
| 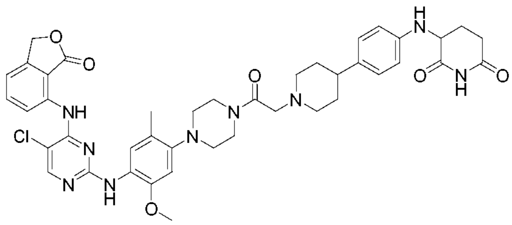 T-73 | 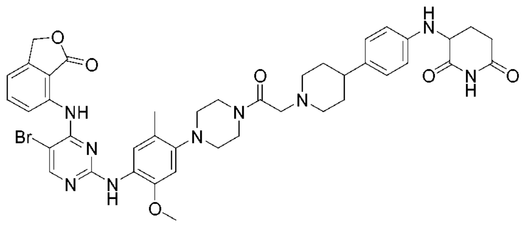 T-74 |
| 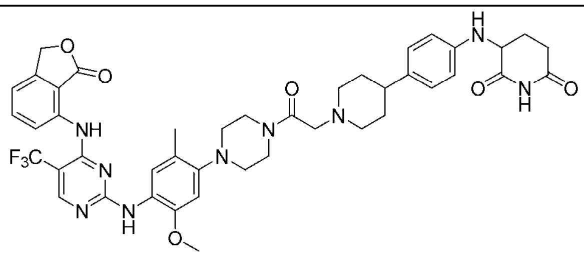 T-75 | 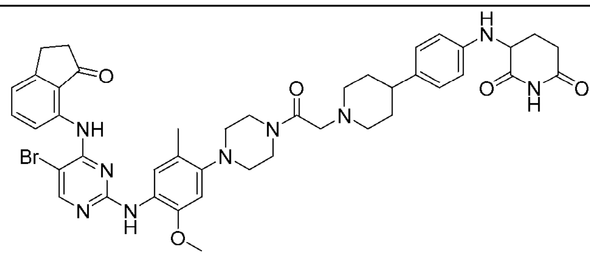 T-76 |
| 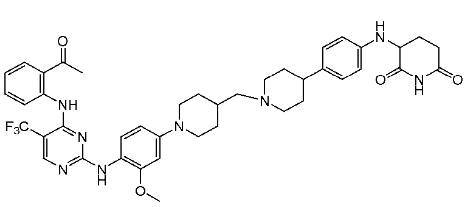 T-77 | 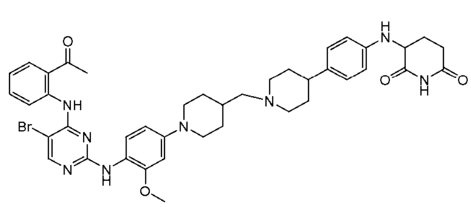 T-78 |
| 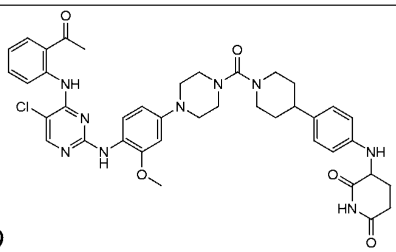 T-79 | 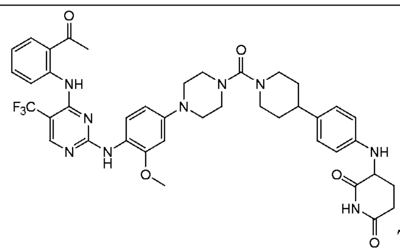 T-80 |

| | |
|---|---|
| 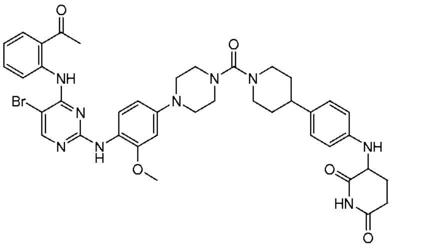 T-81 | 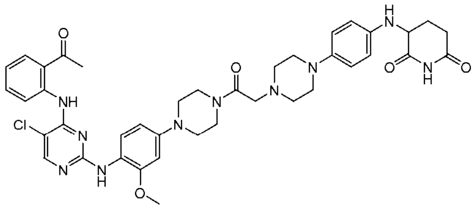 T-82 |
| 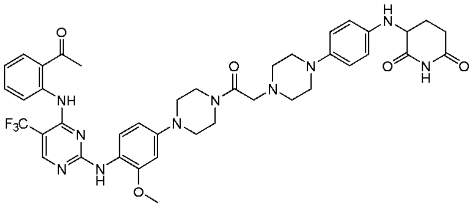 T-83 | 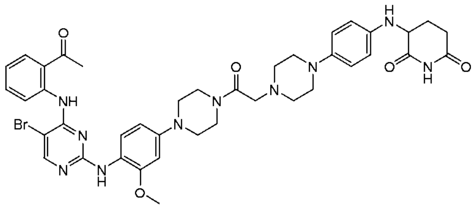 T-84 |
| 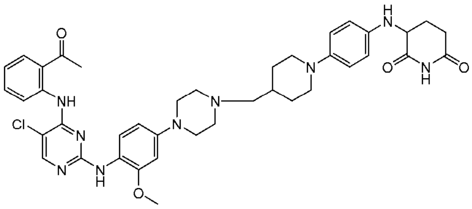 T-85 | 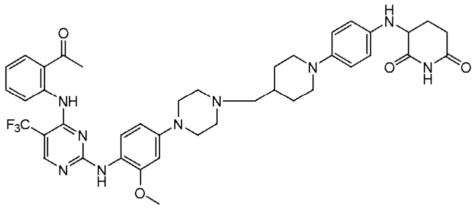 T-86 |
| 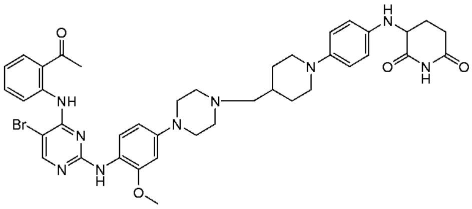 T-87 | 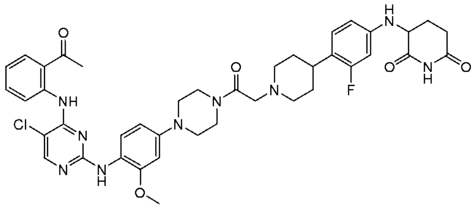 T-88 |
| 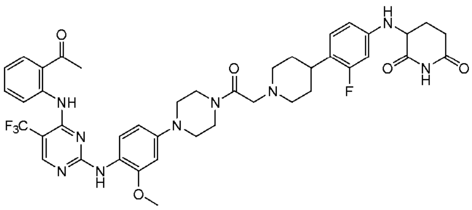 T-89 | 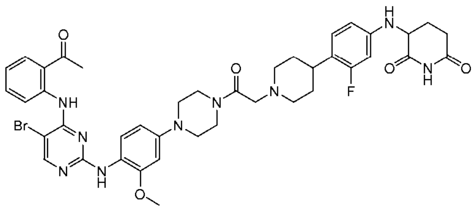 T-90 |
| 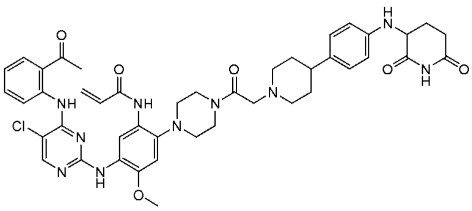 T-91 | 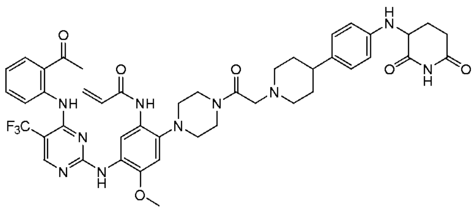 T-92 |
| 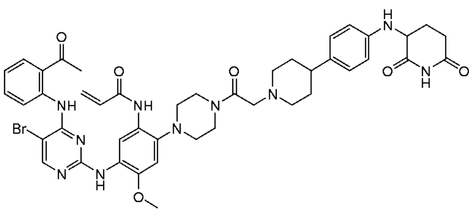 T-93 | 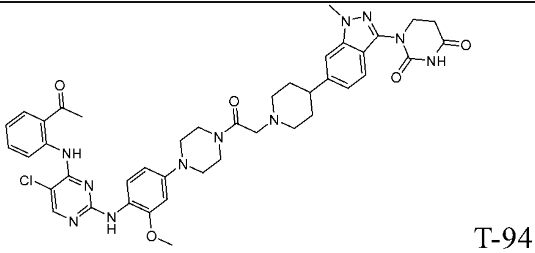 T-94 |

| | |
|---|---|
| 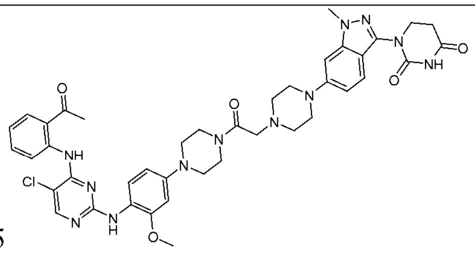 T-95 | 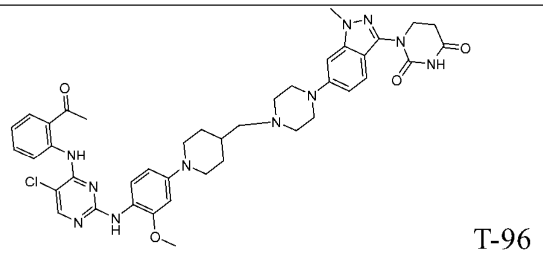 T-96 |
| 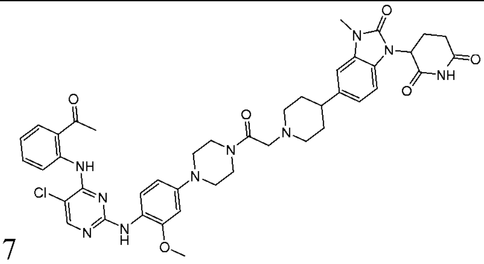 T-97 | 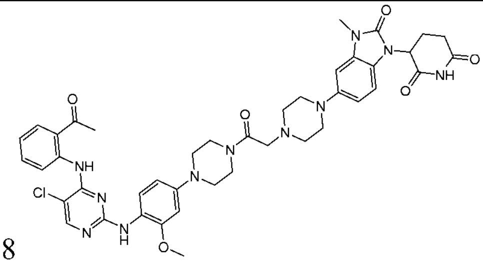 T-98 |
| 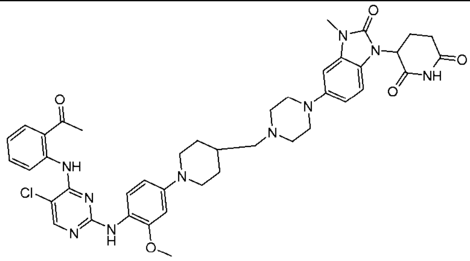 T-99 | 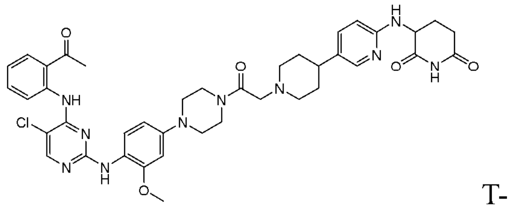 T-100 |
| 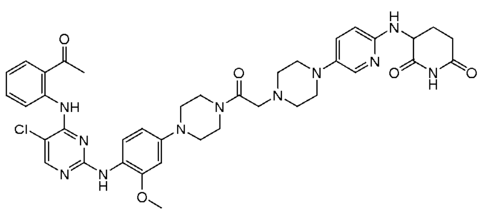 T-101 | 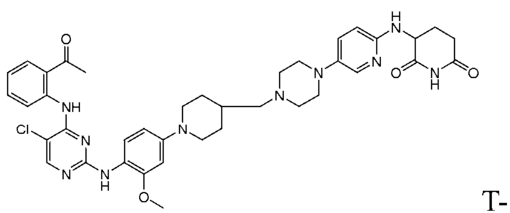 T-102 |
| 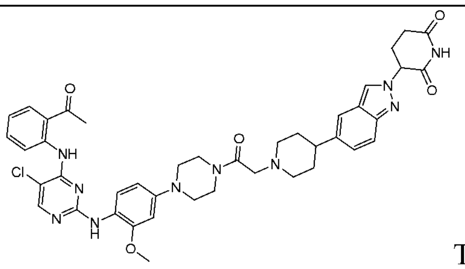 T-103 | 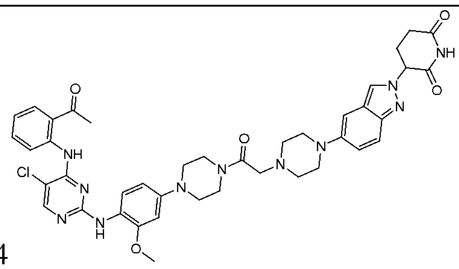 T-104 |
| 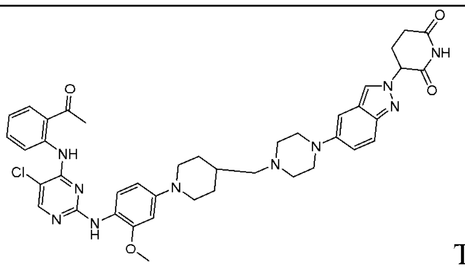 T-105 | 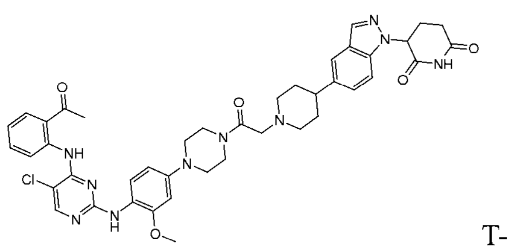 T-106 |
| 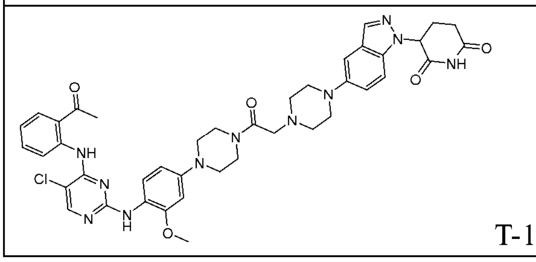 T-107 | 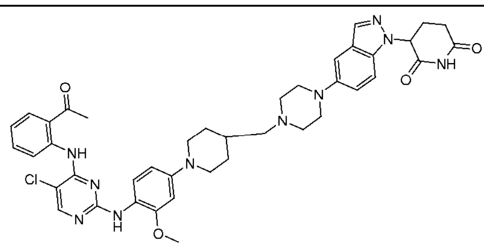 T-108 |

| | |
|---|---|
| 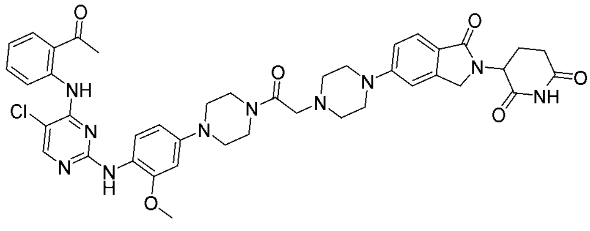 T-109 | 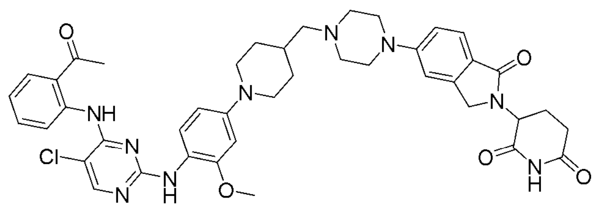 T-110 |
| 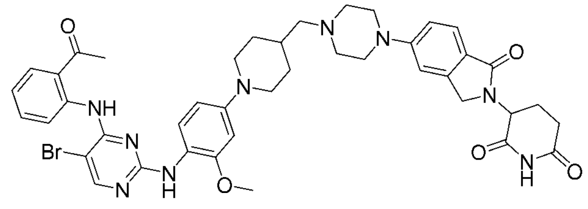 T-111 | 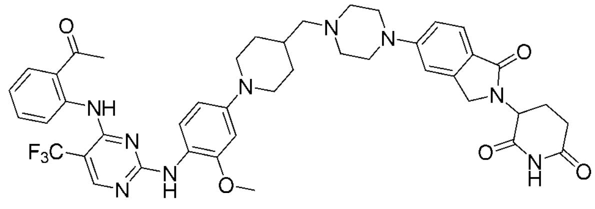 T-112 |
| 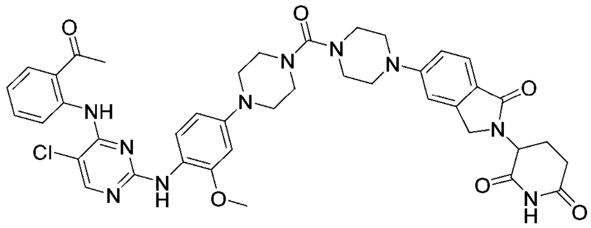 T-113 | 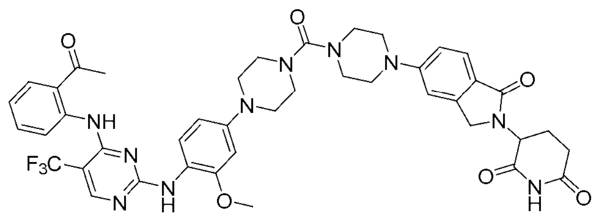 T-114 |
| 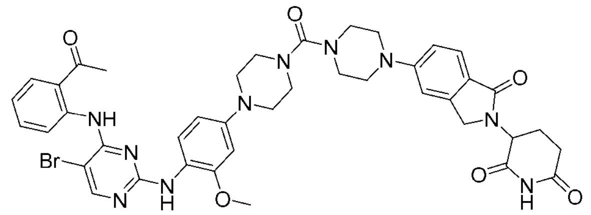 T-115 | 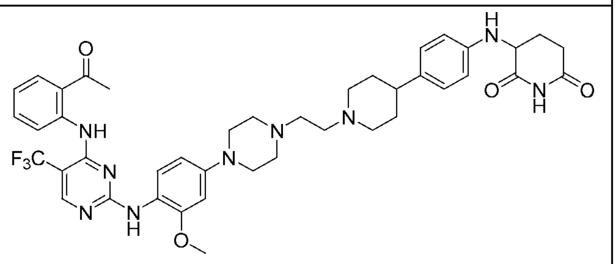 T-116 |
| 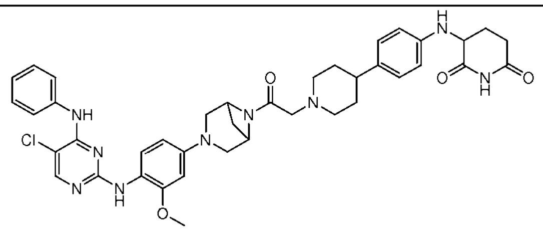 T-117 | 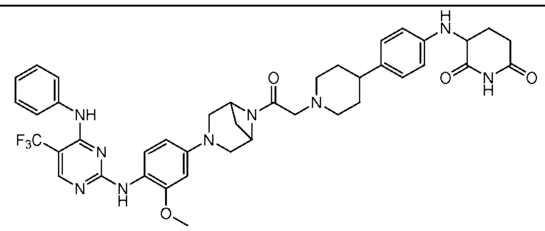 T-118 |
| 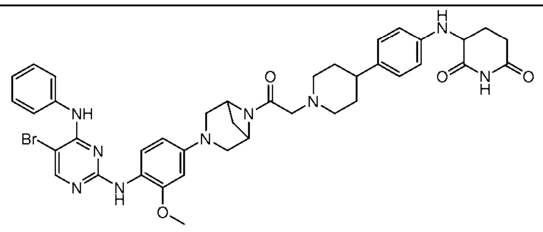 T-119 | 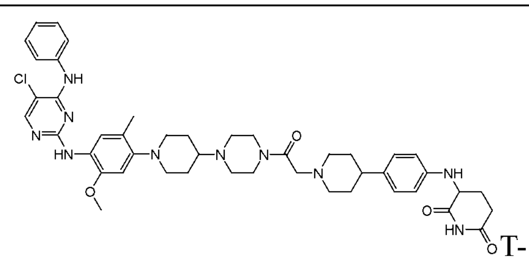 T-120 |

| | |
|---|---|
| 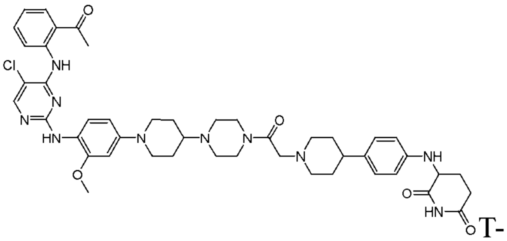 T-121 | 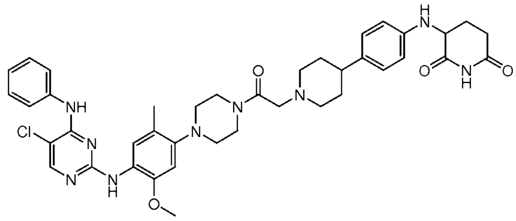 T-<br>122 |
| 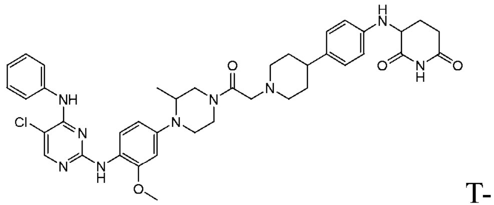 T-123 | 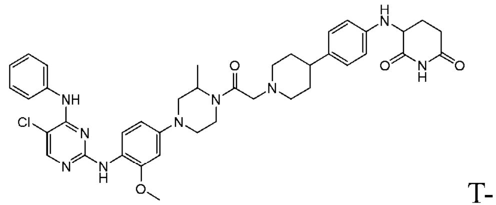 T-124 |
| 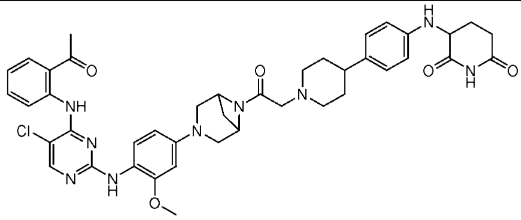 T-<br>125 | 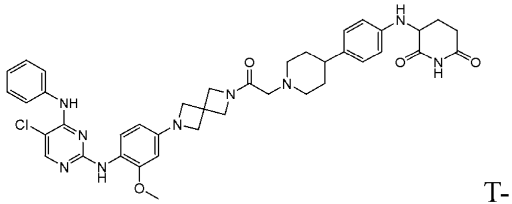 T-126 |
| 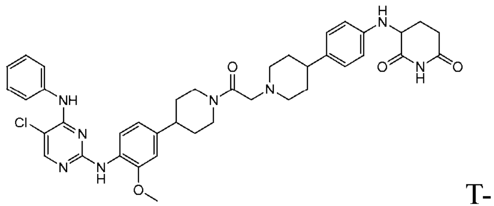 T-127 | 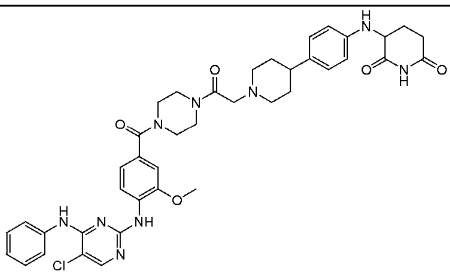 T-128 |
| 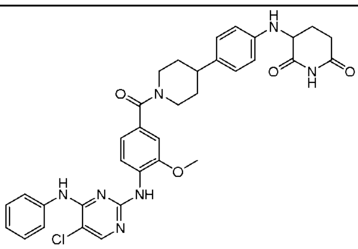 T-129 | 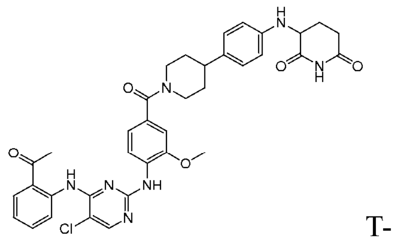 T-130 |
| 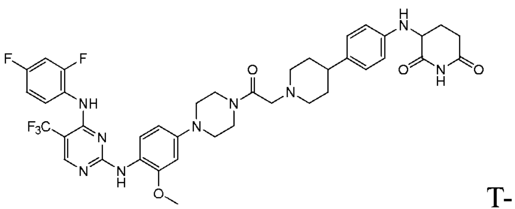 T-131 | 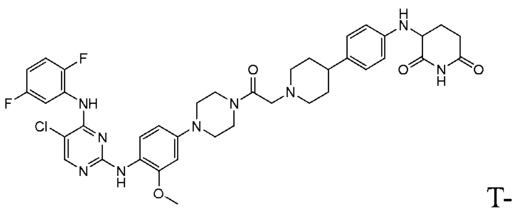 T-132 |
| 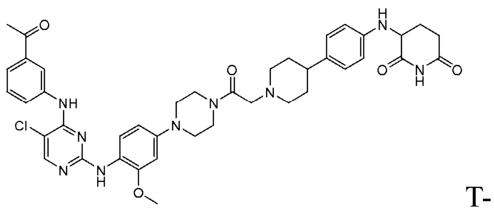 T-133 | 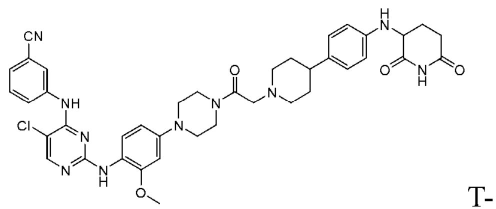 T-134 |

| | |
|---|---|
| 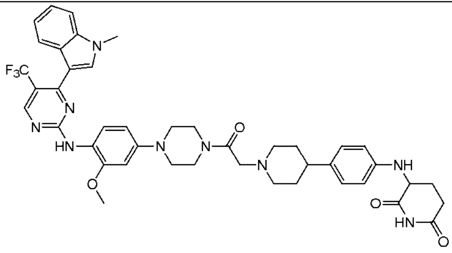 T-135 | 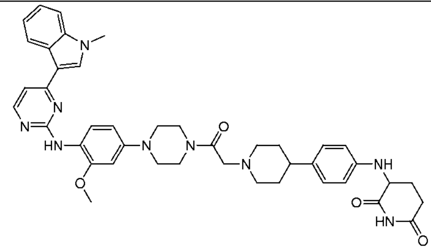 T-136 |
| 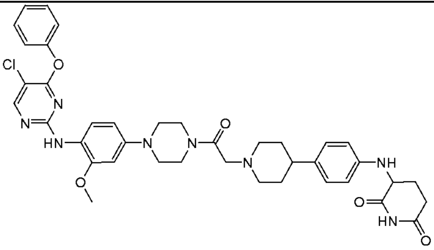 T-137 | 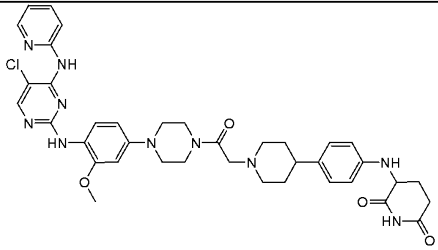 T-138 |
| 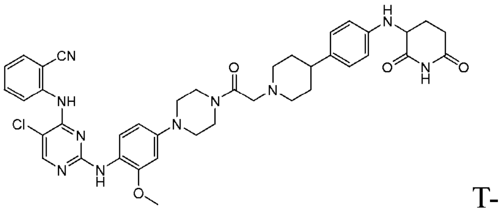 T-139 | 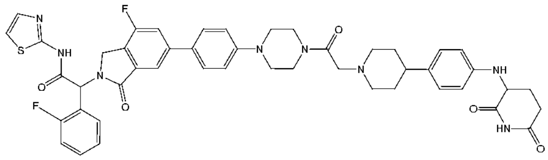 T-140 |
| 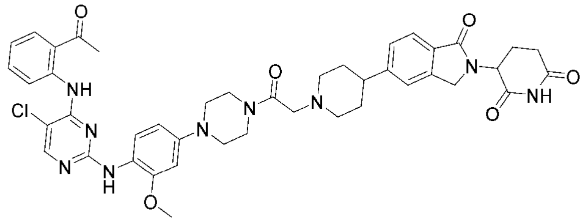 T-141 | 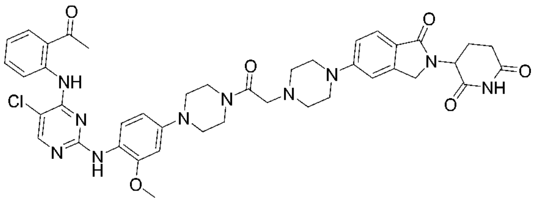 T-142 |
| 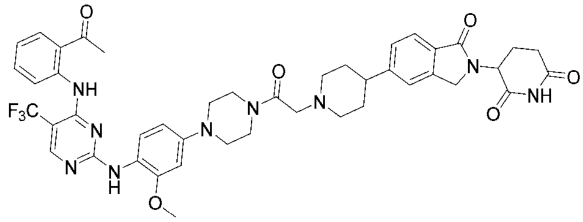 T-143 | 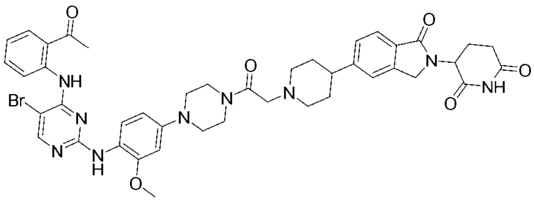 T-144 |
| 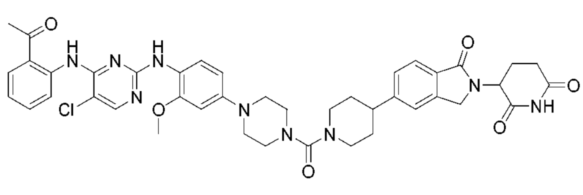 T-145 | 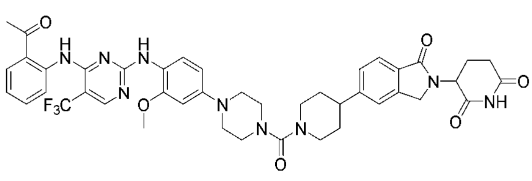 T-146 |
| 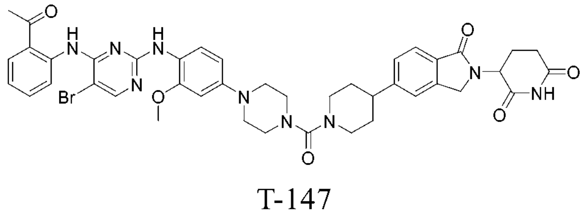 T-147 | 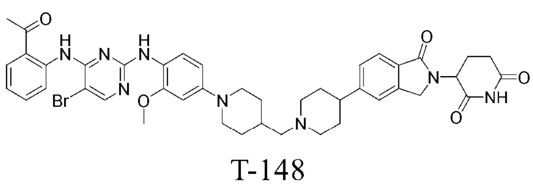 T-148 |

| | |
|---|---|
| 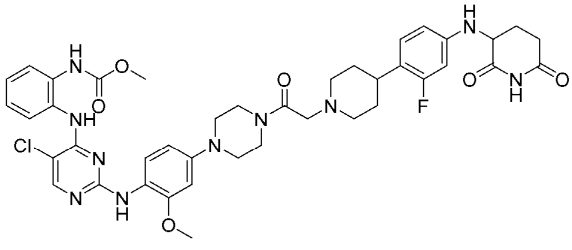 T-149 | 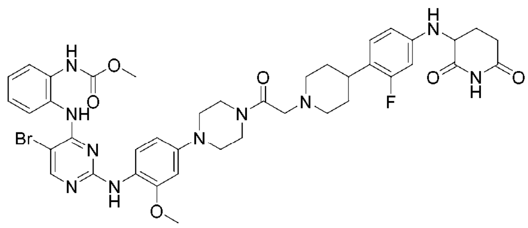 T-150 |
| 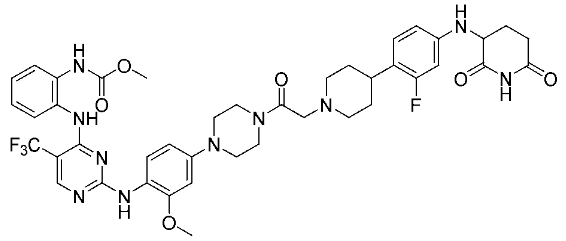 T-151 | 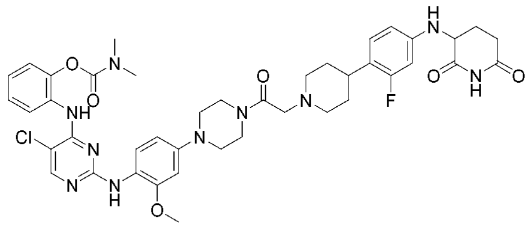 T-152 |
| 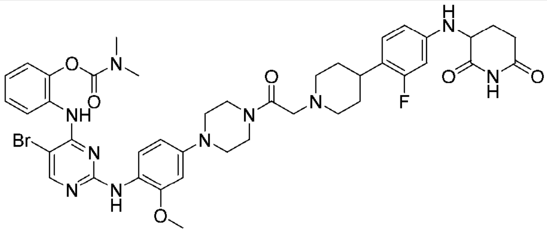 T- 153 | 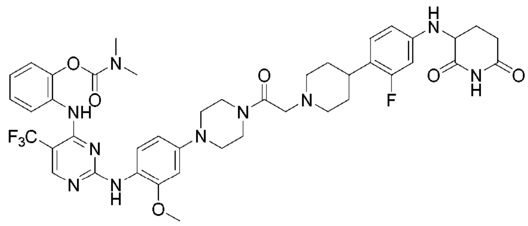 T-154 |
| 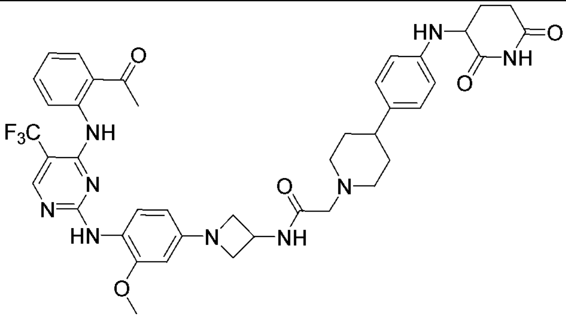 T-155 | 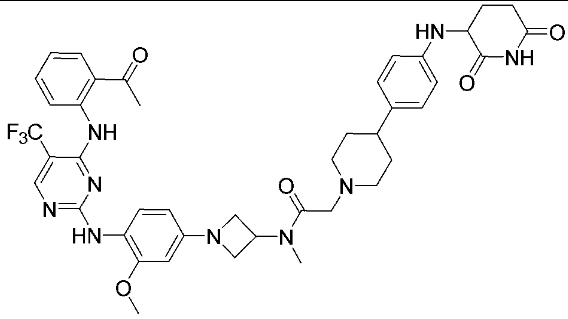 T-156 |
| 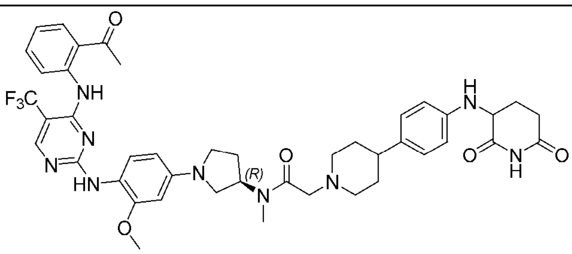 T-157 | 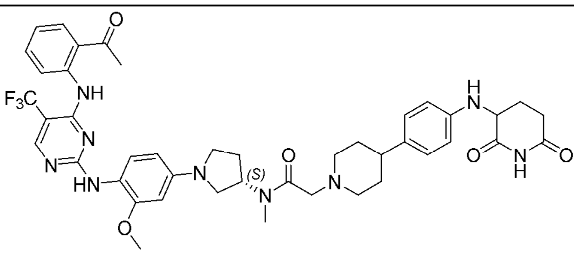 T-158 |
| 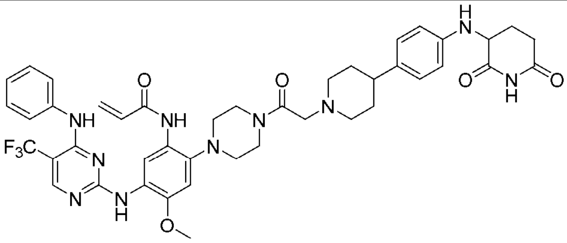 T-159 | 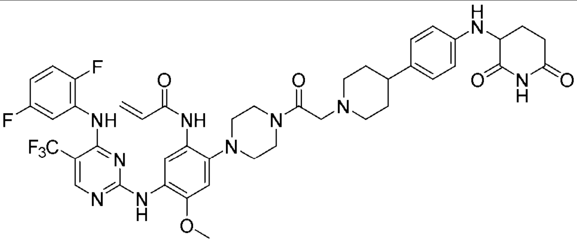 T-160 |

| | |
|---|---|
| 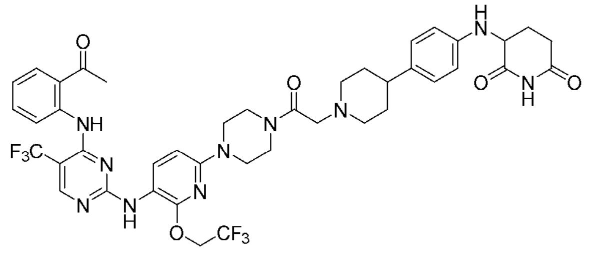 T-161 | 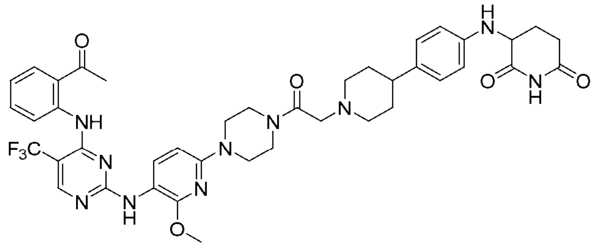 T-162 |
| 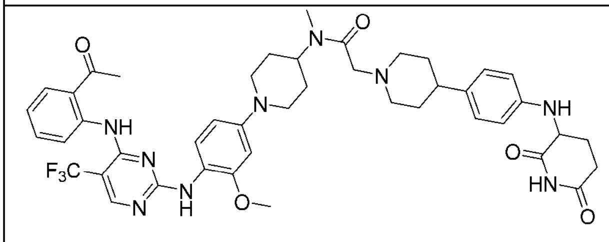 T-163 | 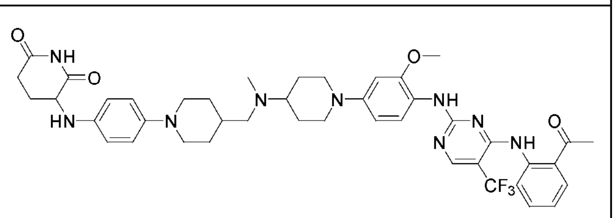 T-164 |
| 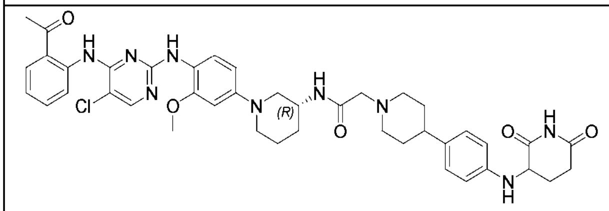 T-165 | 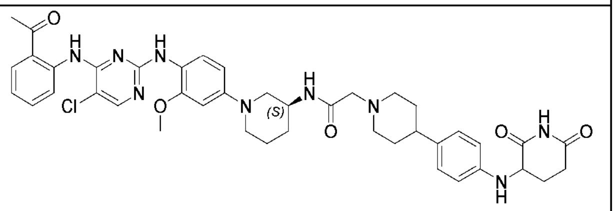 T-166 |
| 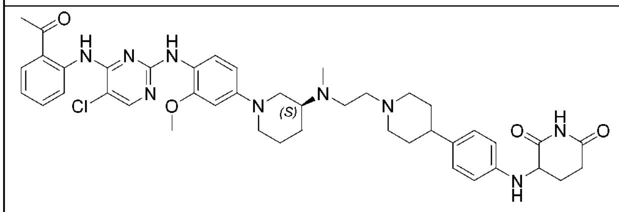 T-167 | 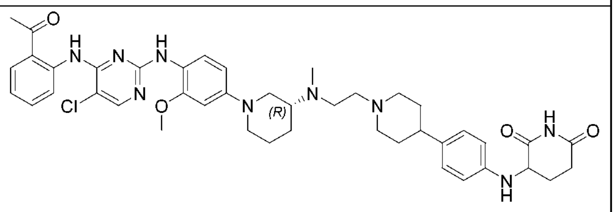 T-168 |
| 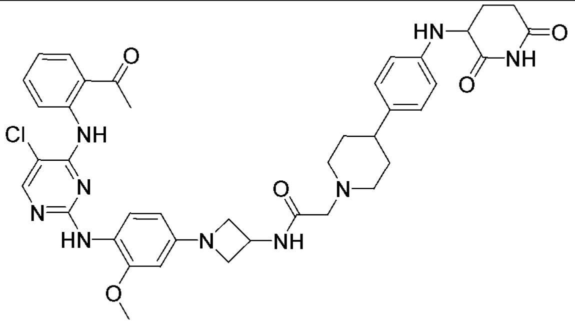 T-169 | 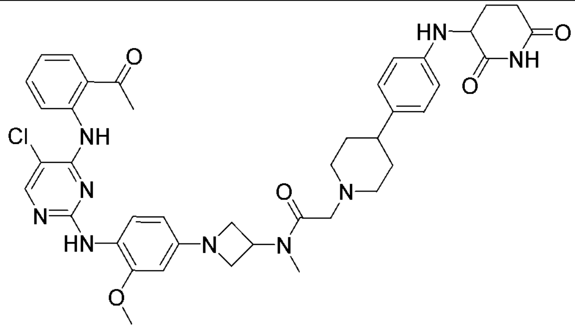 T-170 |
| 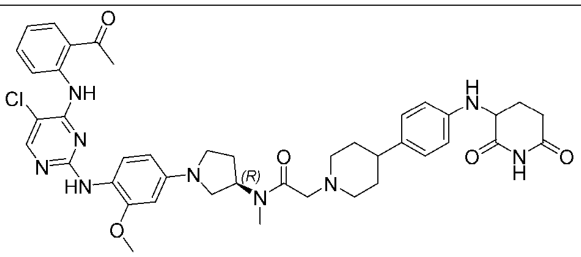 T-171 | 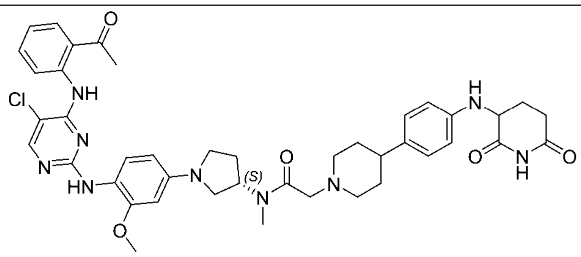 T-172 |

| | |
|---|---|
| 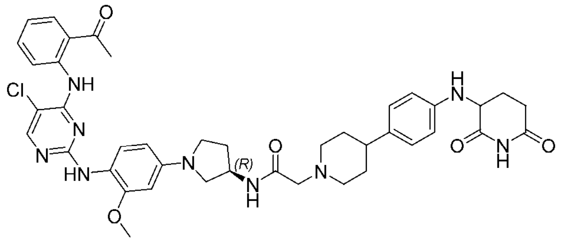<br>T-173 | 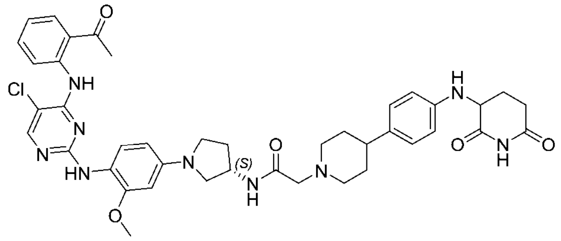<br>T-174 |
| 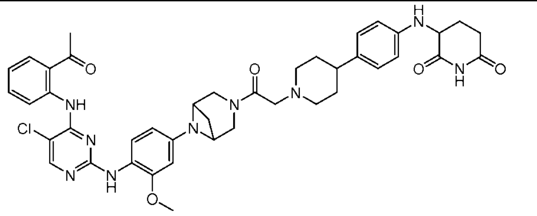<br>T-175 | 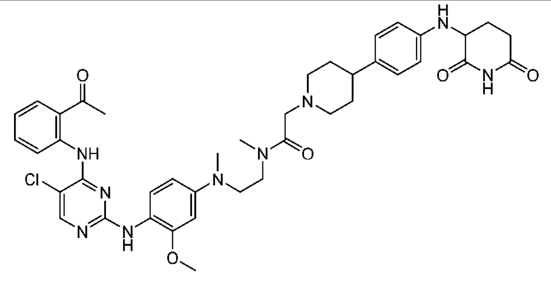<br>T-176 |
| 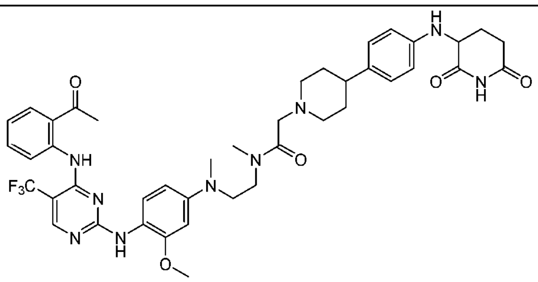<br>T-177 | 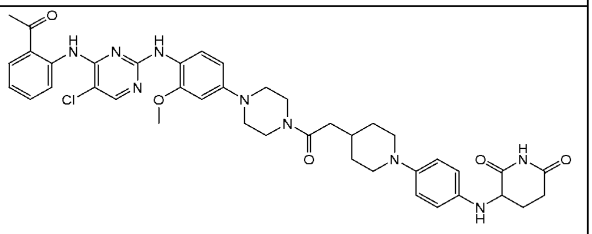<br>T-178 |
| 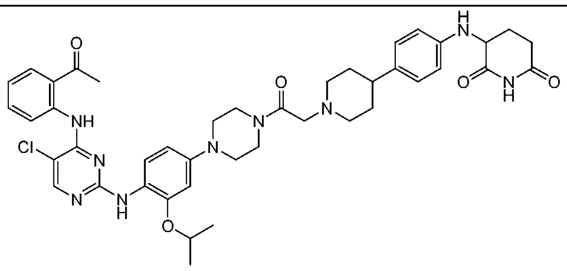<br>T-179 | 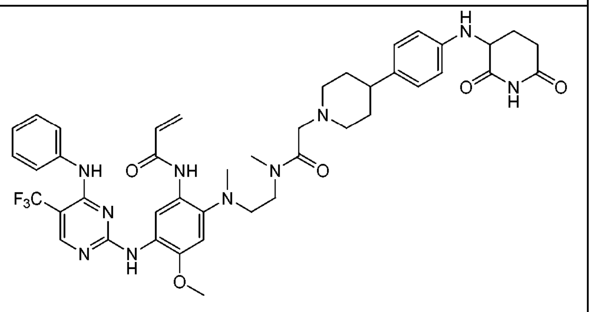<br>T-180 |
| 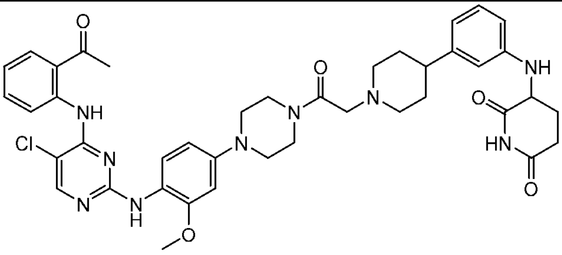<br>T-181 | 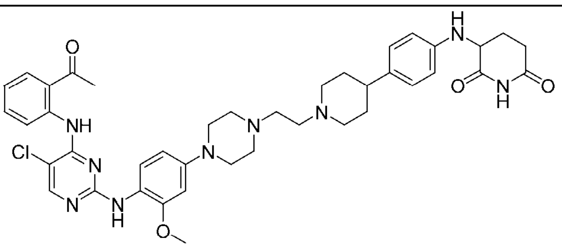<br>T-182 |
| 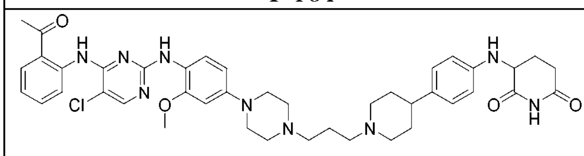<br>T-183 | 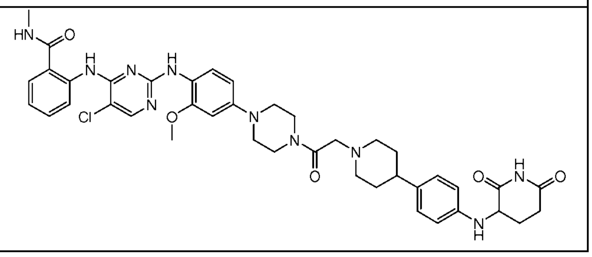 |

| | |
|---|---|
| | T-184 |
| 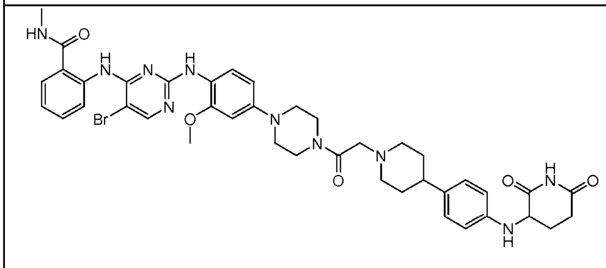 T-185 | 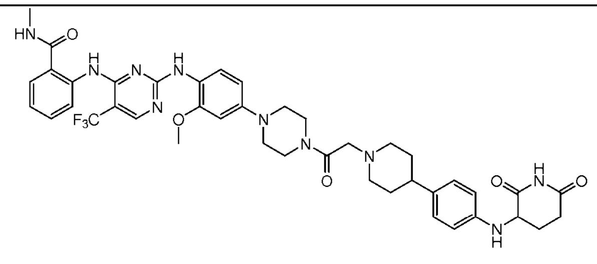 T-186 |
| 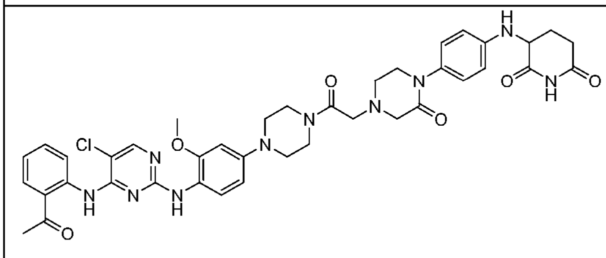 T-187 | 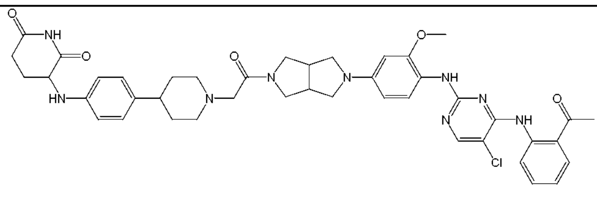 T-188 |
| 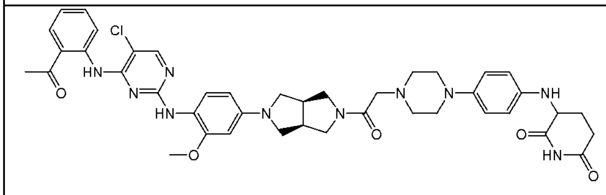 T-189 | 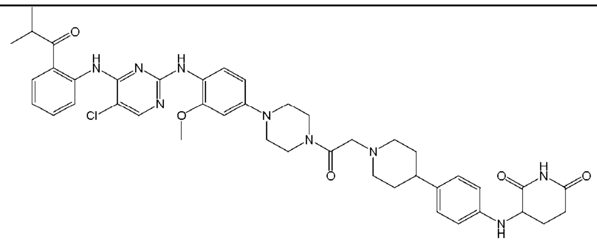 T-190 |
| 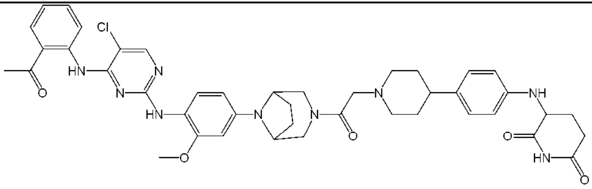 T-191 | 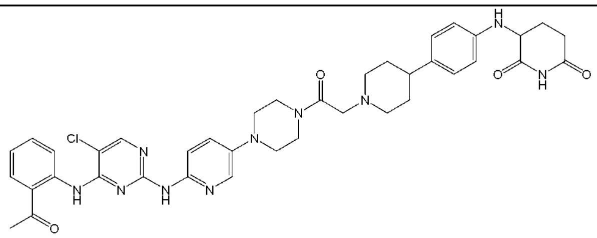 T-192 |
| 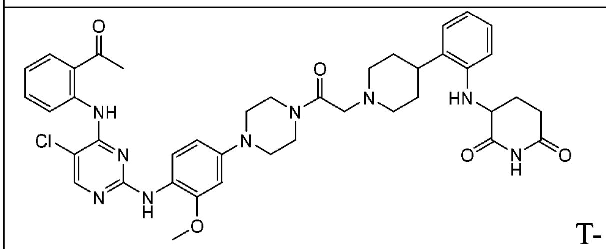 T- 193 | 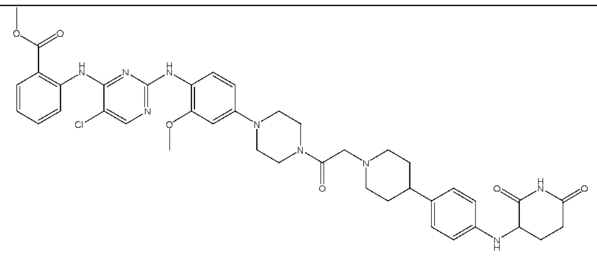 T-194 |

| | |
|---|---|
| 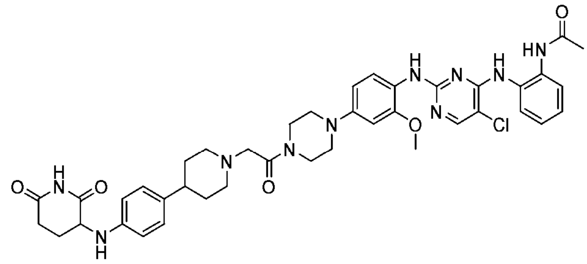<br>T-195 | 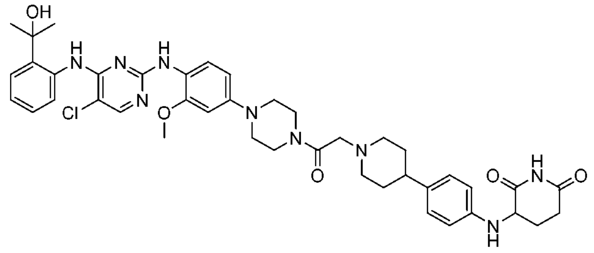<br>T-196 |
| 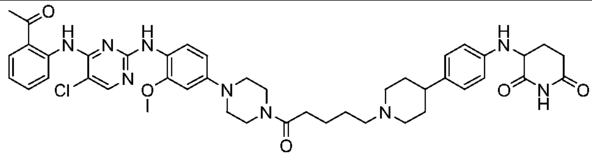<br>T-197 | 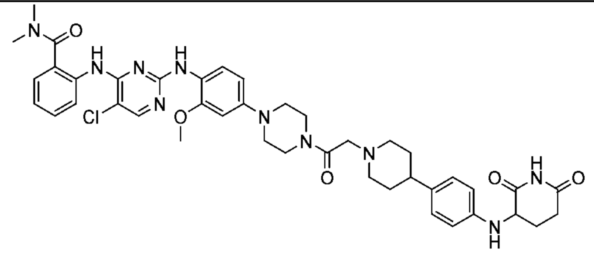<br>T-198 |
| 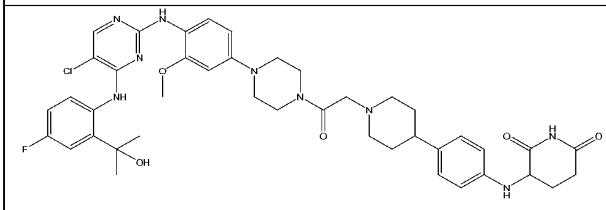<br>T-199 | 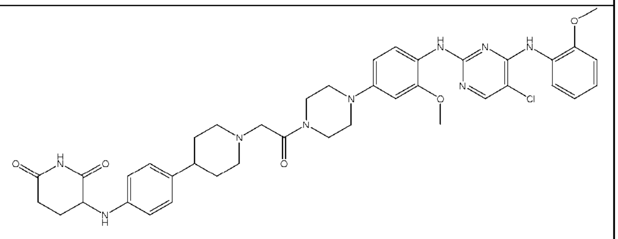<br>T-200 |
| 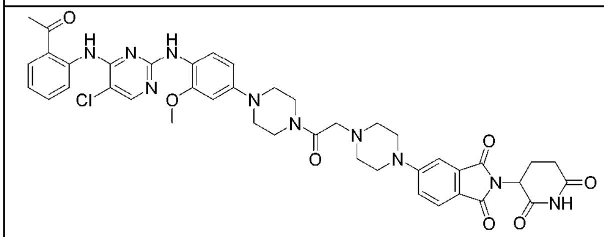<br>T-201 | 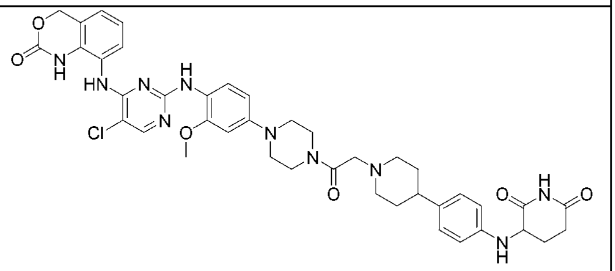<br>T-202 |
| 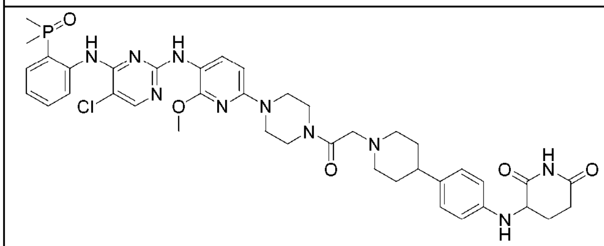<br>T-203 | 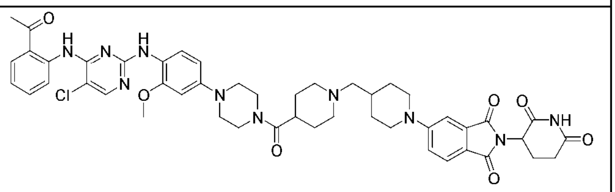<br>T-204 |
| 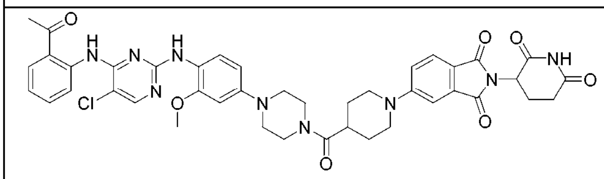<br>T-205 | 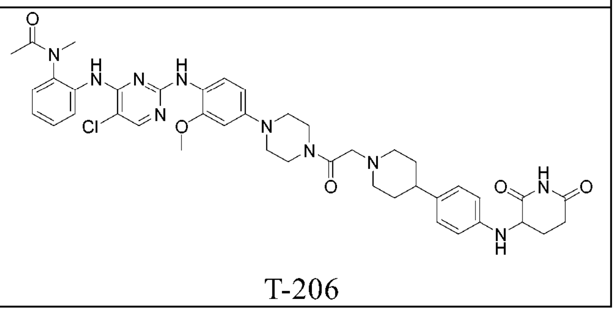<br>T-206 |

| | |
|---|---|
| 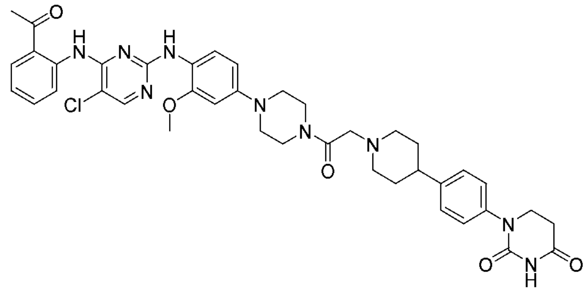<br>T-207 | 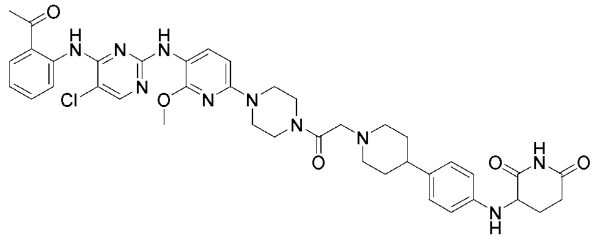<br>T-208 |
| 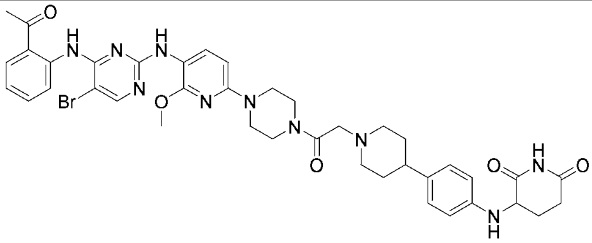<br>T-209 | 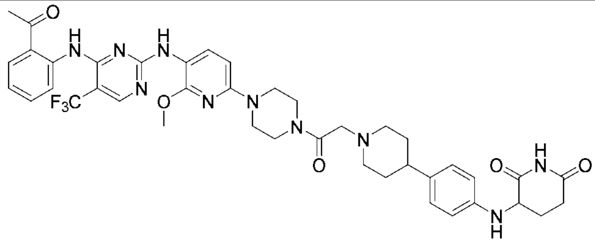<br>T-210 |
| 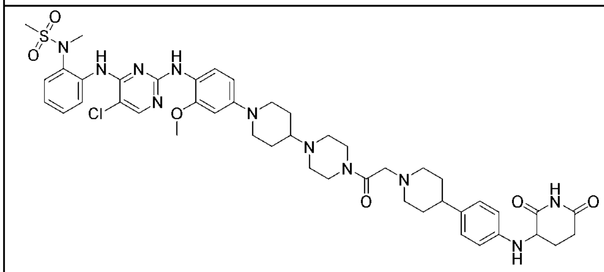<br>T-211 | 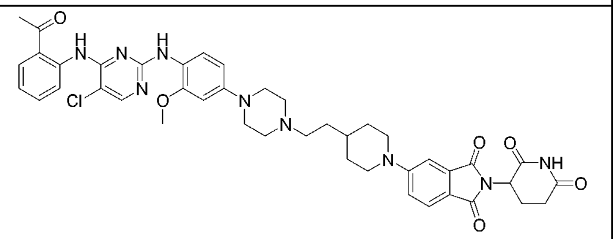<br>T-212 |
| 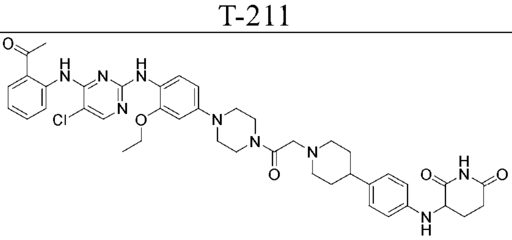<br>T-213 | 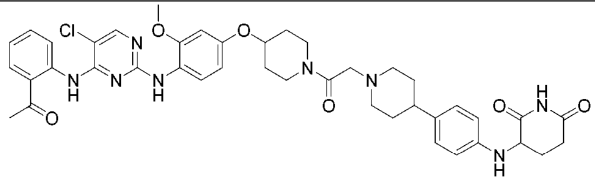<br>T-214 |
| 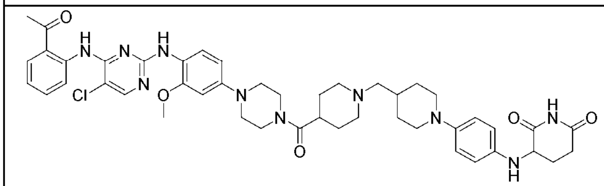<br>T-215 | 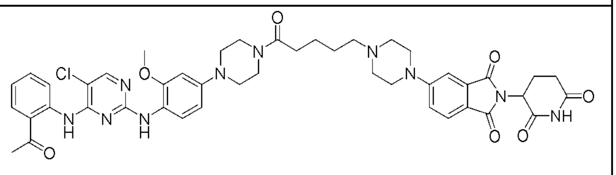<br>T-216 |
| 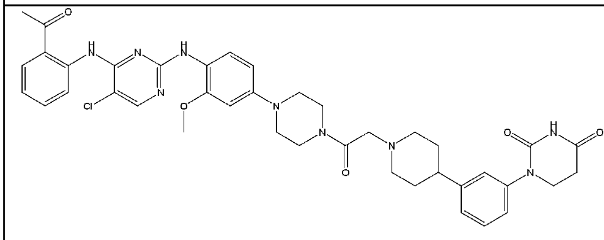<br>T-217 | 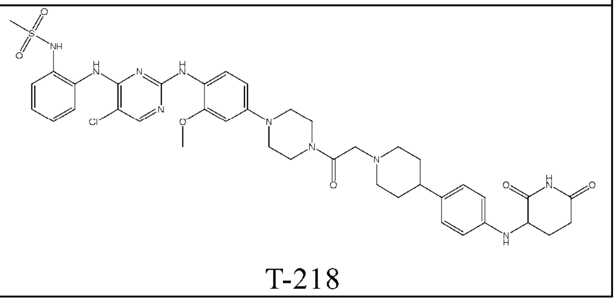<br>T-218 |

| | |
|---|---|
| 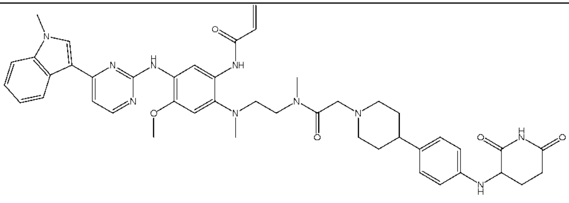 T-219 | 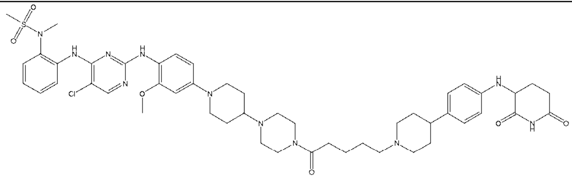 T-220 |
| 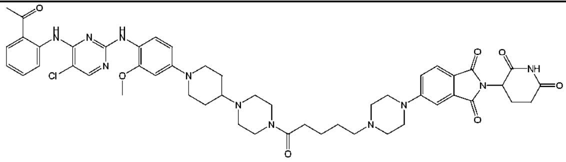 T-221 | 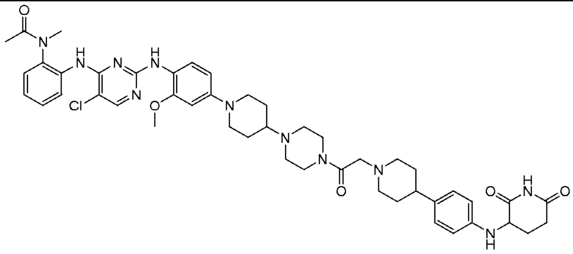 T-222 |
| 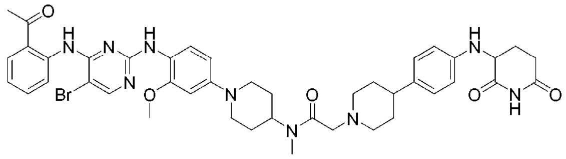 T-223 | 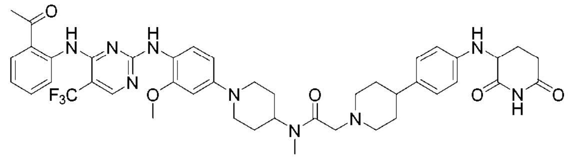 T-224 |
| 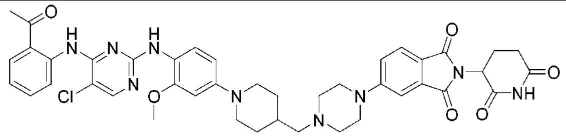 T-225 | 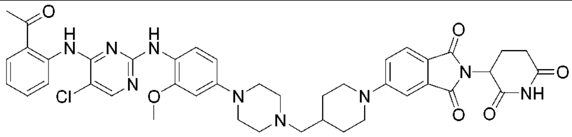 T-226 |
| 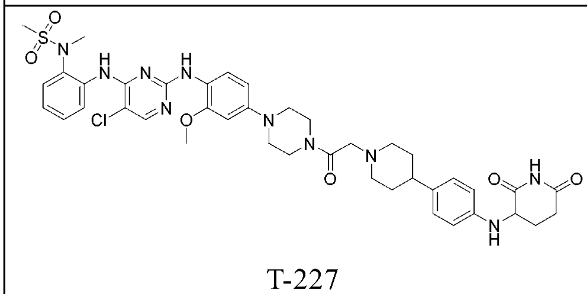 T-227 | 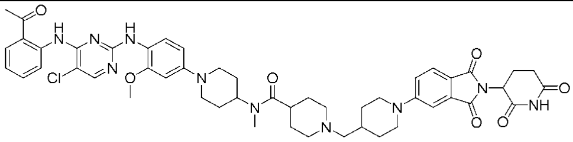 T-228 |
| 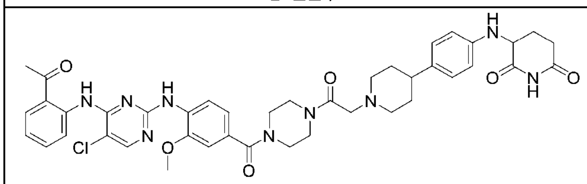 T-229 | 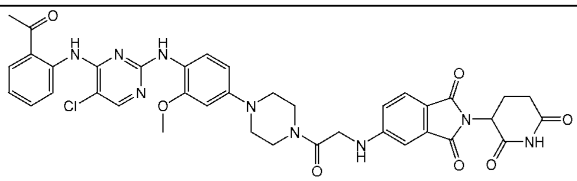 T-230 |
| 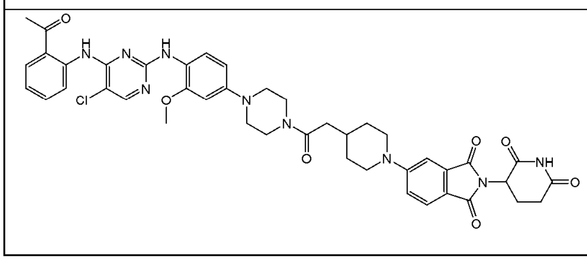 | 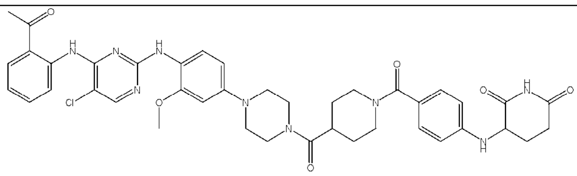 T-232 |

| | |
|---|---|
| T-231 | |
| 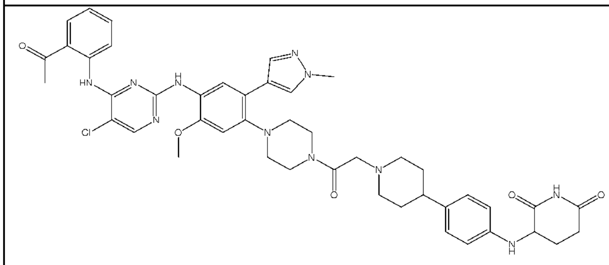 T-233 | 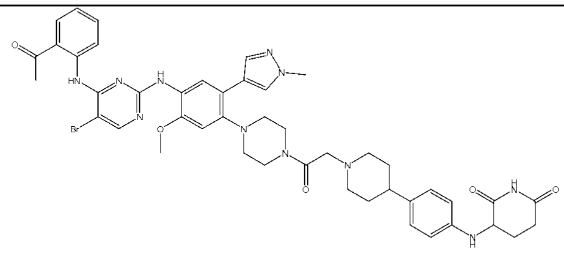 T-234 |
| 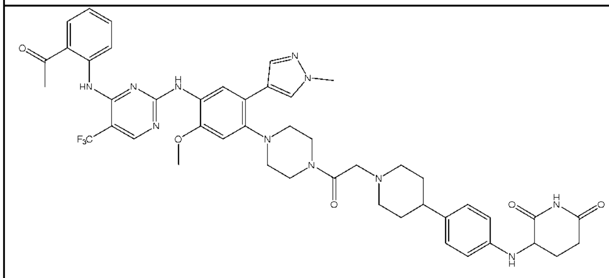 T-235 | 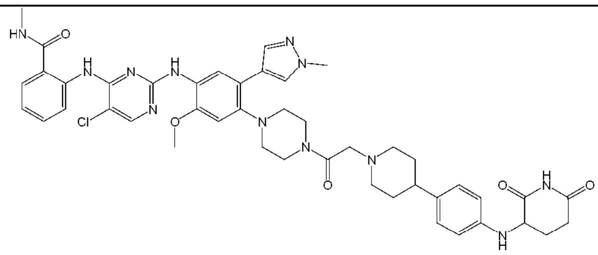 T-236 |
| 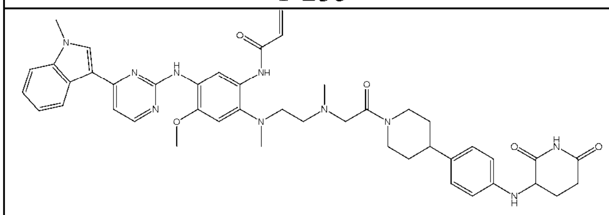 T-237 | 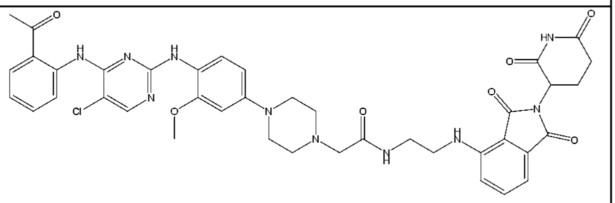 T-238 |
| 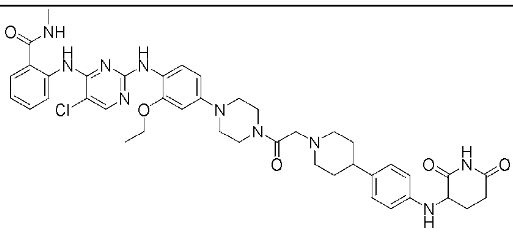 T-239 | 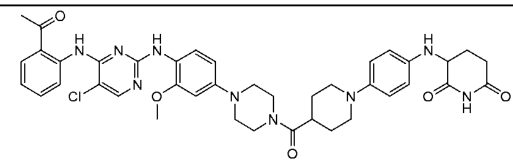 T-240 |
| 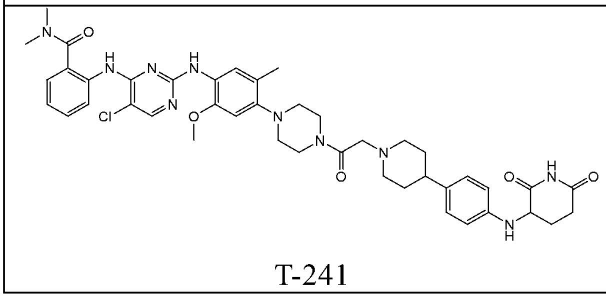 T-241 | 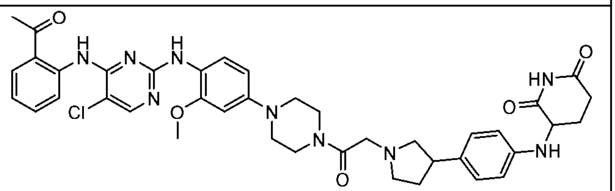 T-242 |

| | |
|---|---|
| 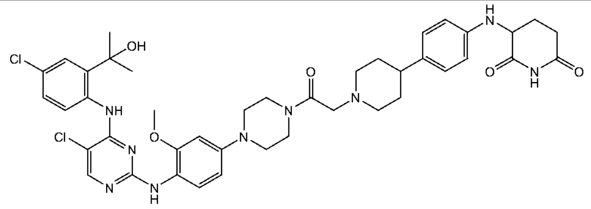 T-243 | 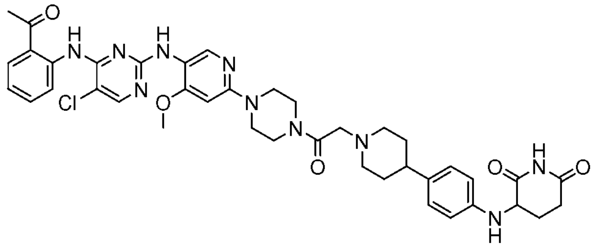 T-244 |
| 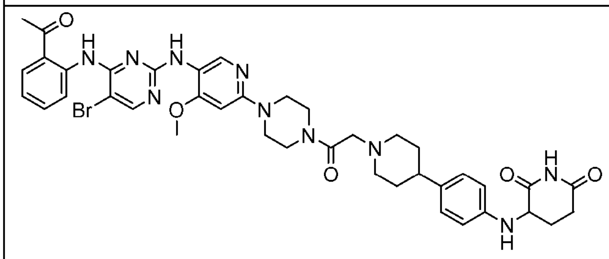 T-245 | 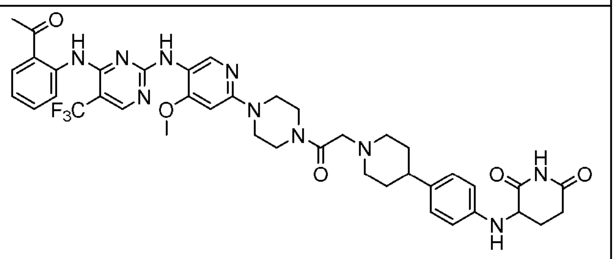 T-246 |
| 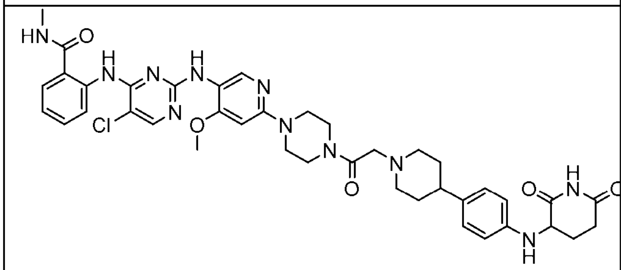 T-247 | 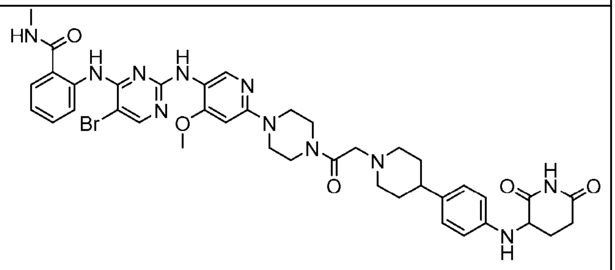 T-248 |
| 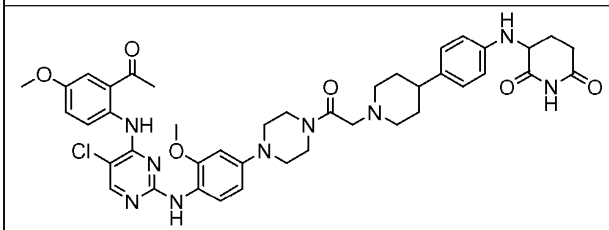 T-249 | 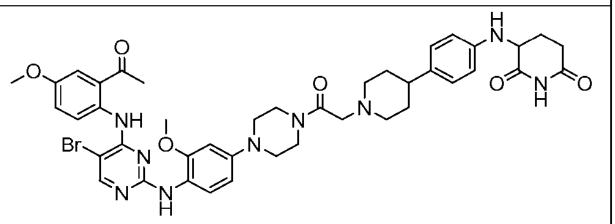 T-250 |
| 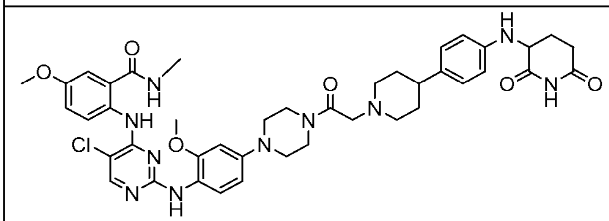 T-251 | 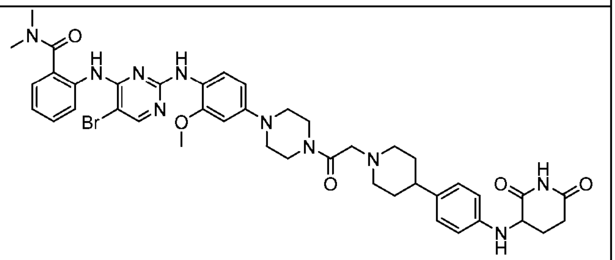 T-252 |
| 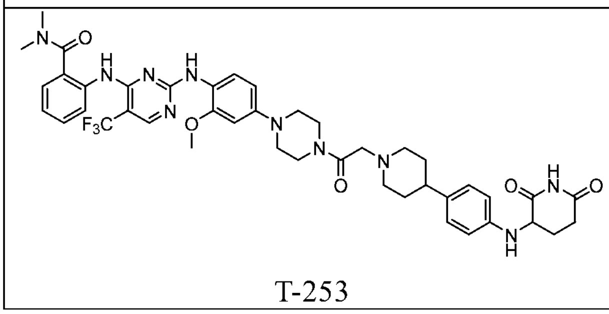 T-253 | 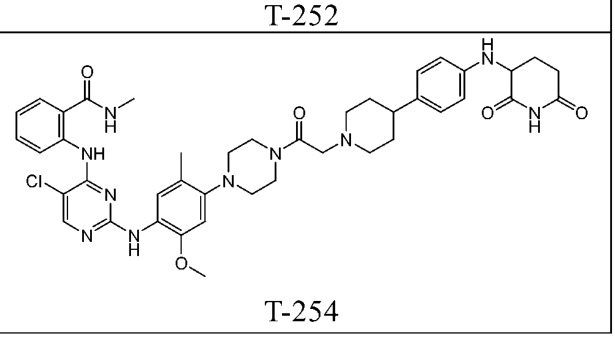 T-254 |

| | |
|---|---|
| 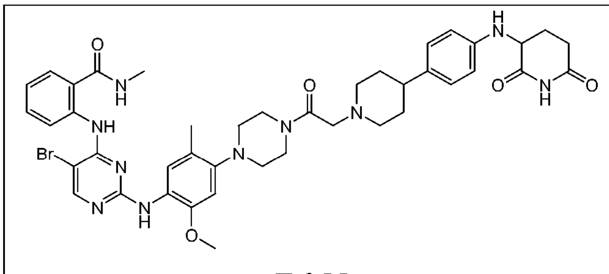<br>T-255 | 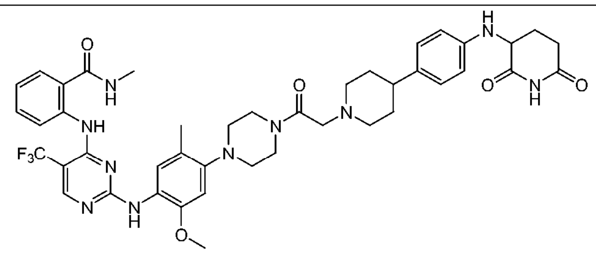<br>T-256 |
| 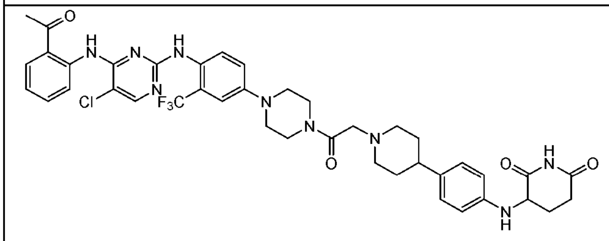<br>T-257 | 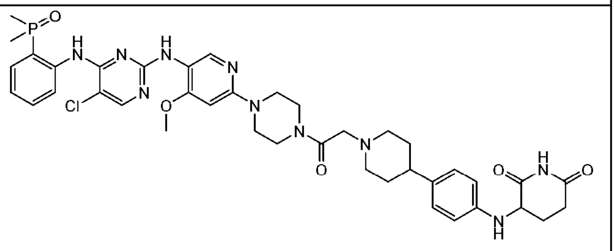<br>T-258 |
| 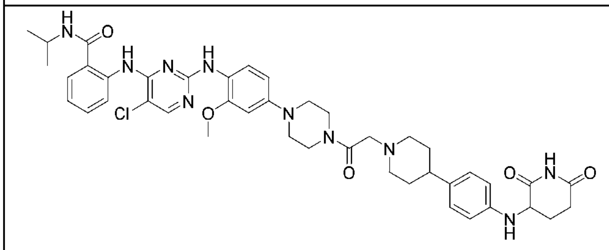<br>T-259 | 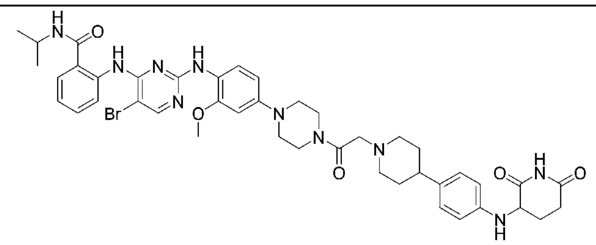<br>T-260 |
| 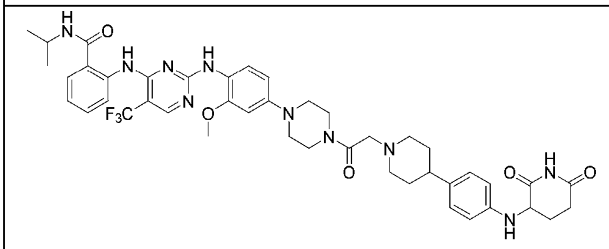<br>T-261 | 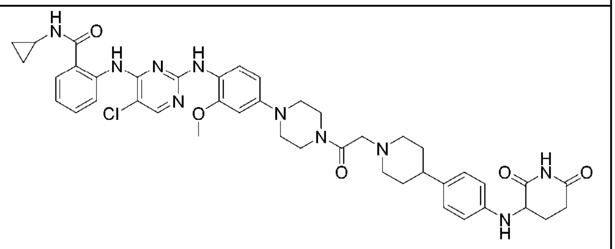<br>T-262 |
| 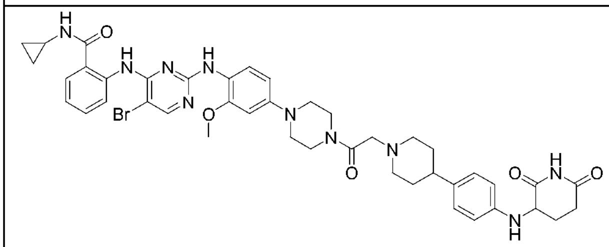<br>T-263 | 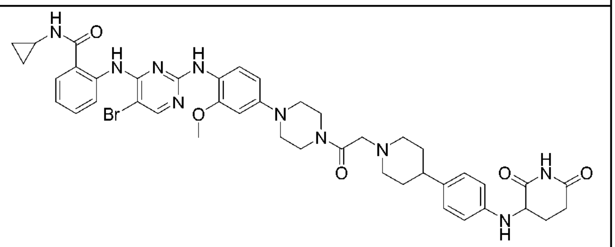<br>T-264 |
| 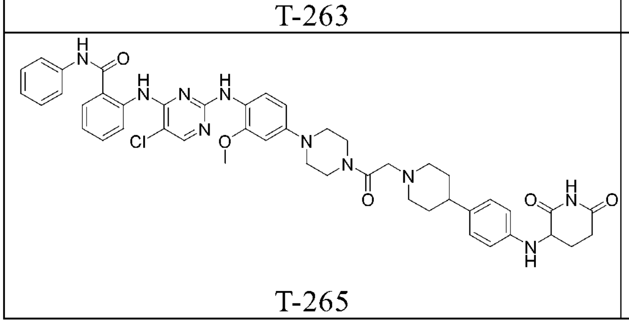<br>T-265 | 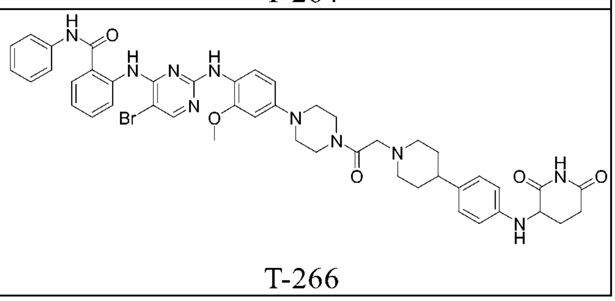<br>T-266 |

| | |
|---|---|
| 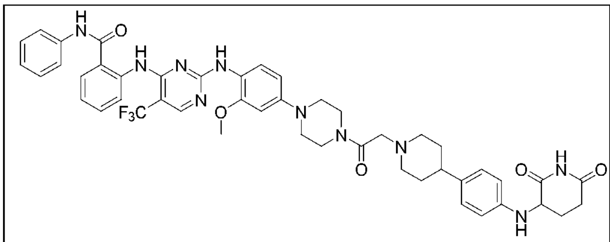<br>T-267 | 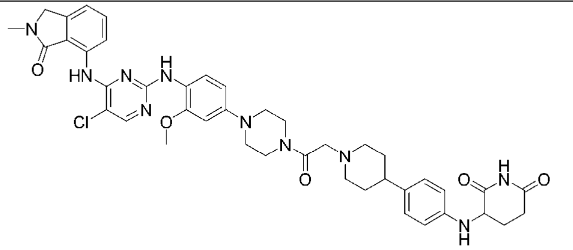<br>T-268 |
| 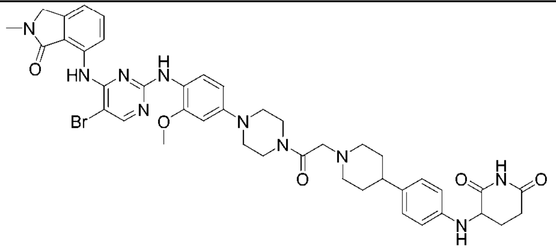<br>T-269 | 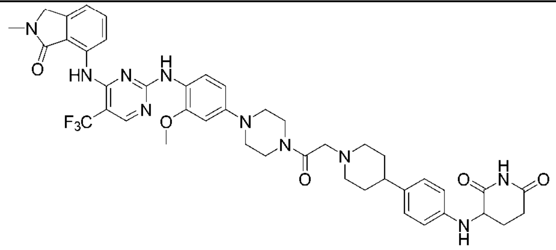<br>T-270 |
| 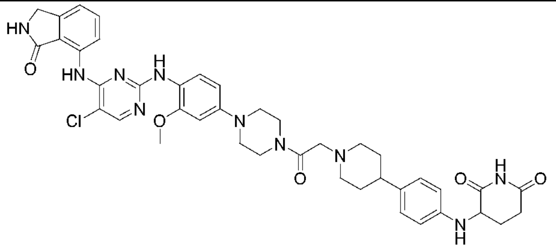<br>T-271 | 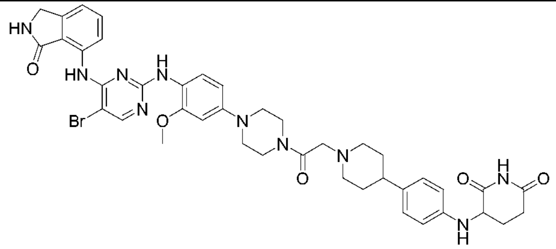<br>T-272 |
| 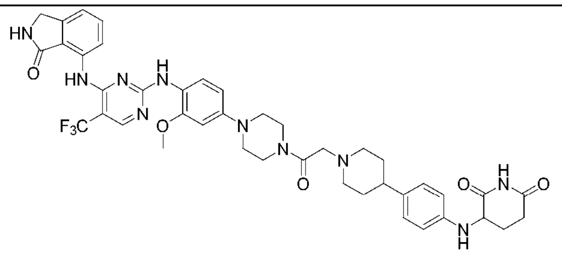<br>T-273 | 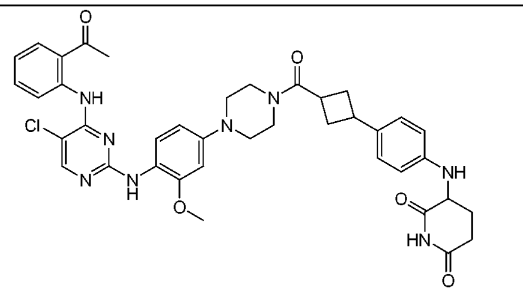<br>T-274 |
| 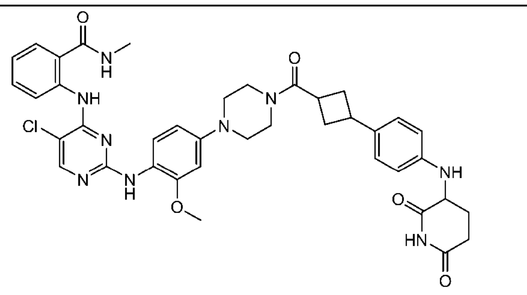<br>T-275 | 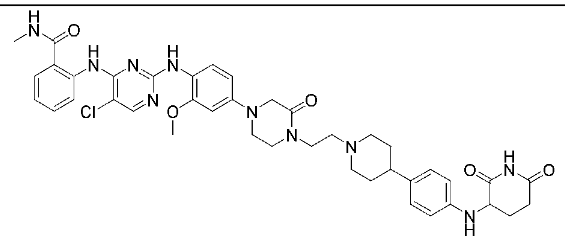<br>T-276 |
| 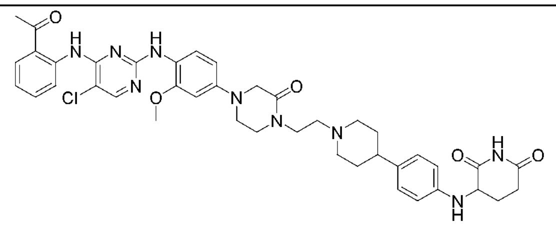<br>T-277 | 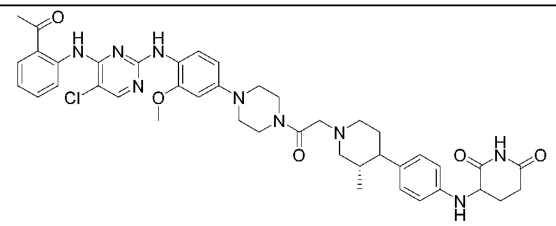<br>T-278 |

| | |
|---|---|
| 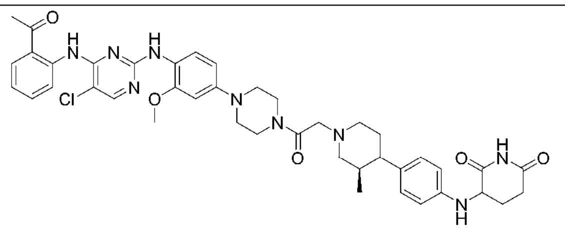 T-279 | 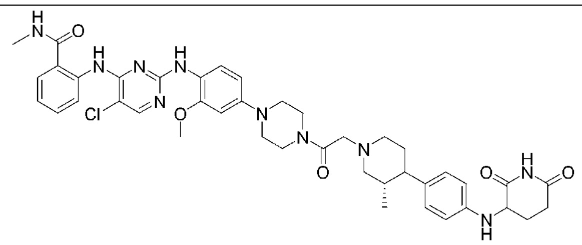 T-280 |
| 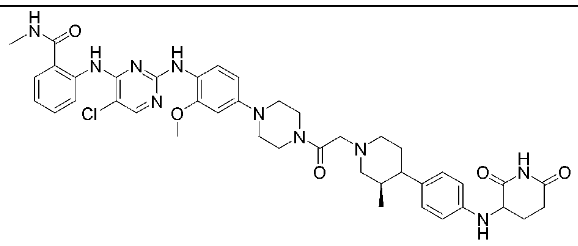 T-281 | 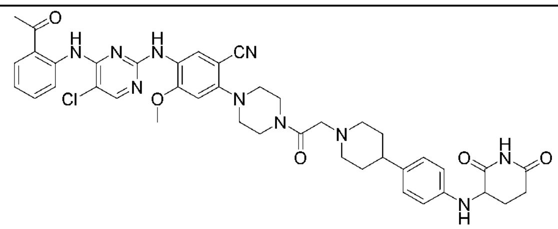 T-282 |
| 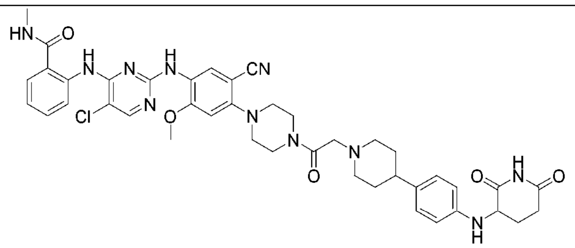 T-283 | 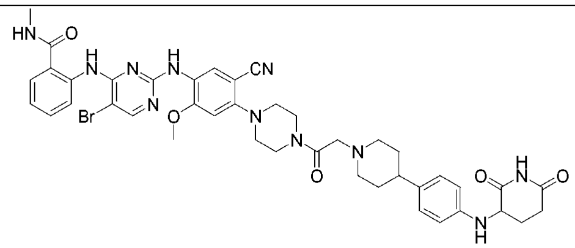 T-284 |
| 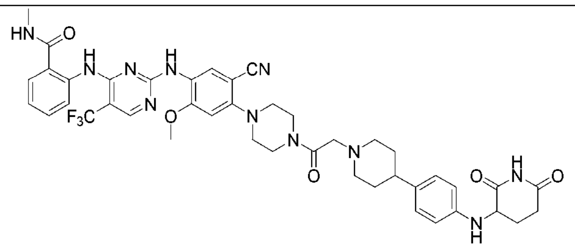 T-285 | 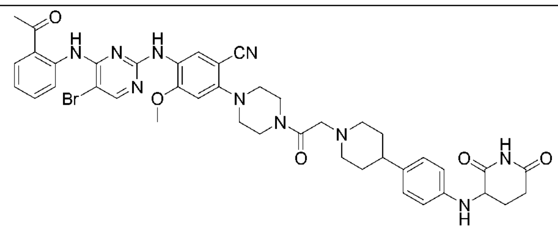 T-286 |
| 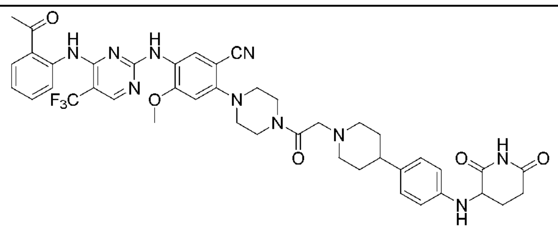 T-287 | 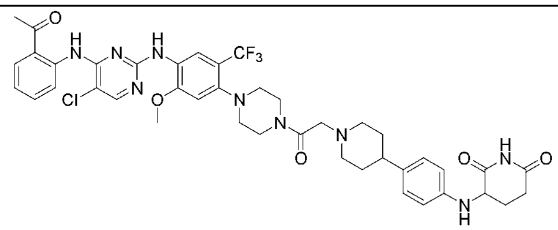 T-288 |
| 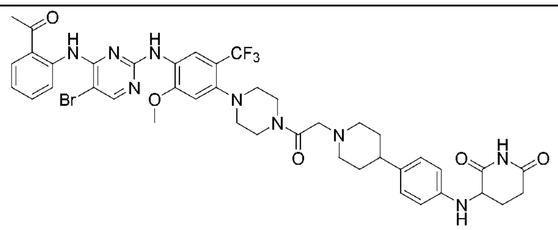 T-289 | 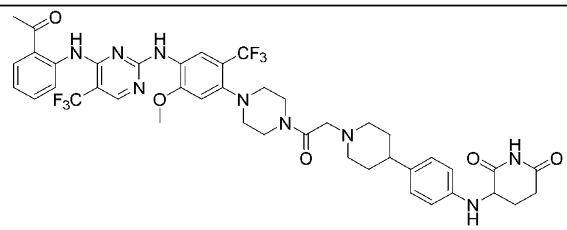 T-290 |

| | |
|---|---|
| 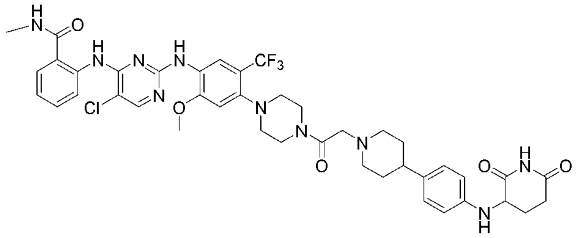<br>T-291 | 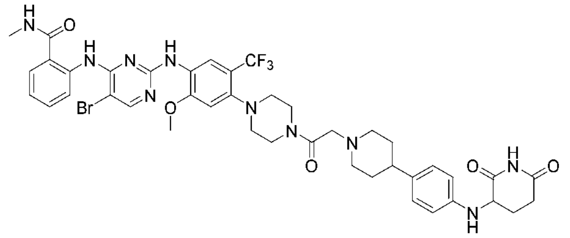<br>T-292 |
| 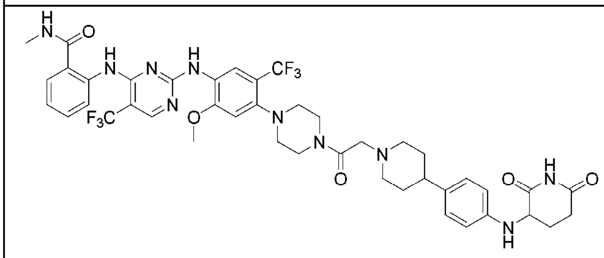<br>T-293 | 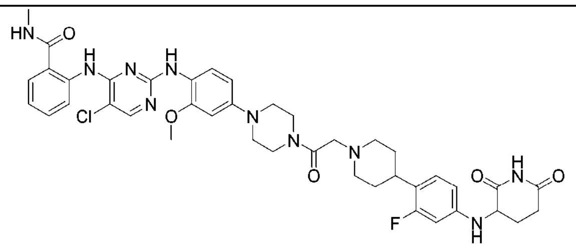<br>T-294 |
| 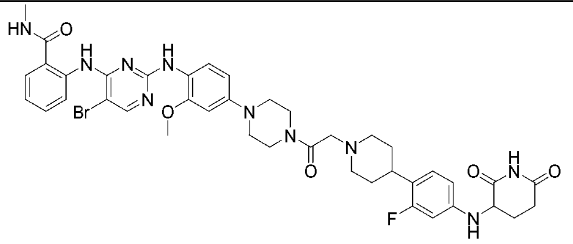<br>T-295 | 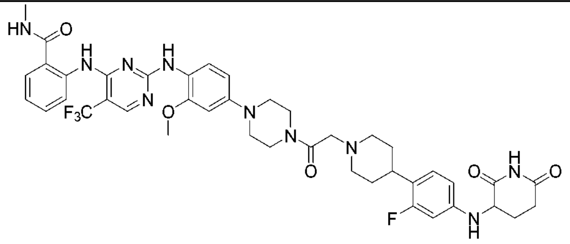<br>T-296 |
| 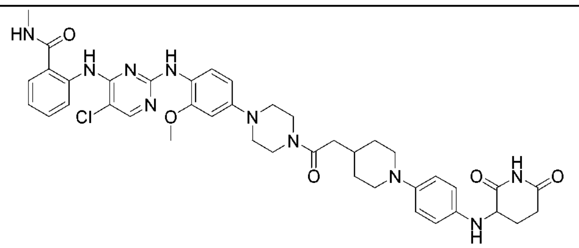<br>T-297 | 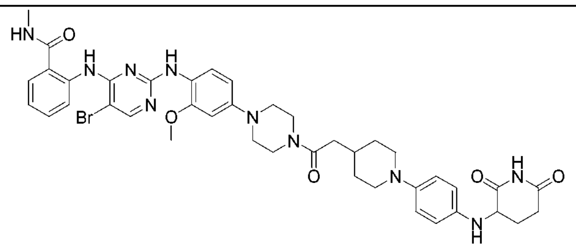<br>T-298 |
| 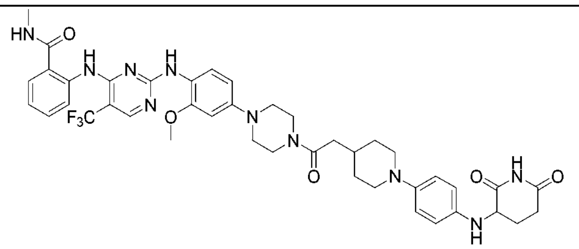<br>T-299 | 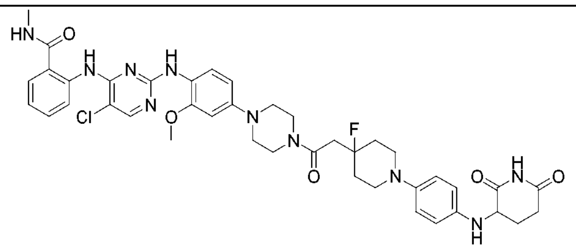<br>T-300 |
| 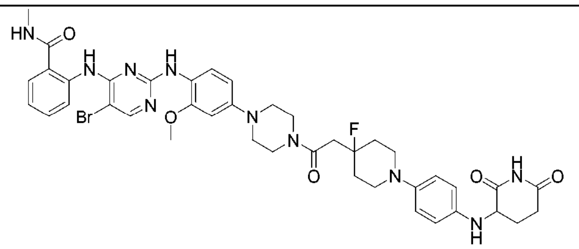<br>T-301 | 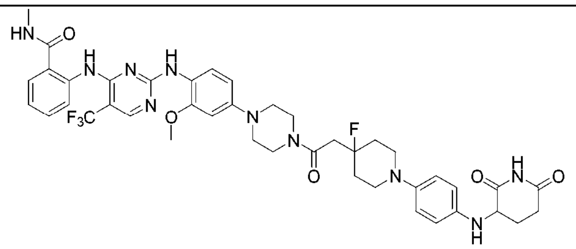<br>T-302 |

| | |
|---|---|
| 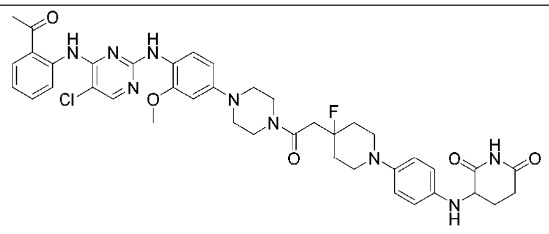 T-303 | 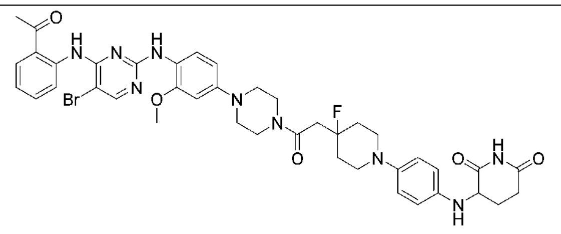 T-304 |
| 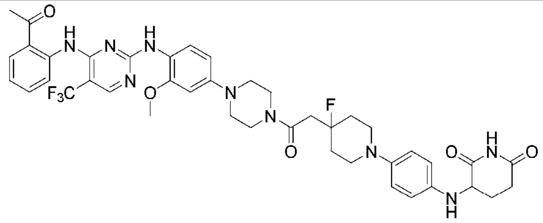 T-305 | 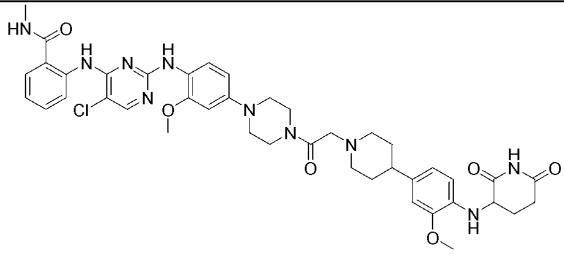 T-306 |
| 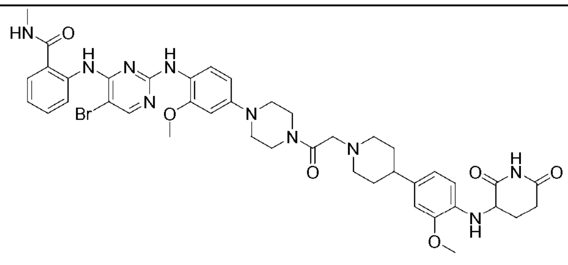 T-307 | 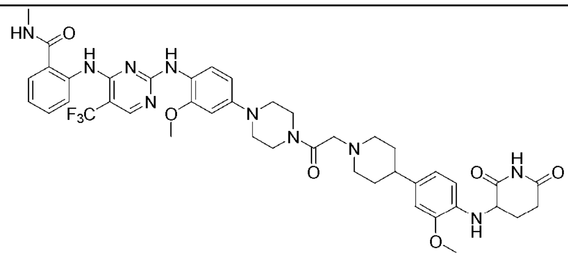 T-308 |
| 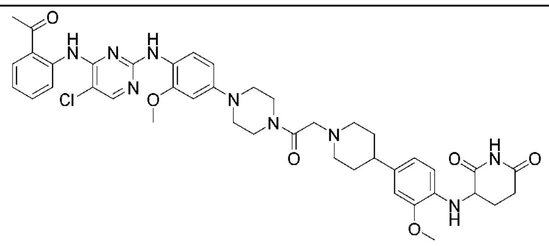 T-309 | 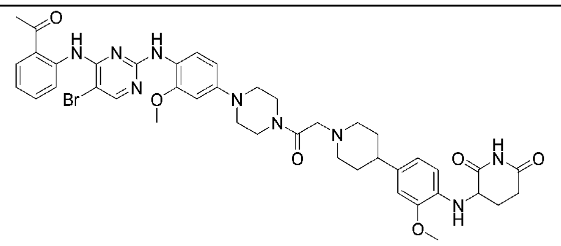 T-310 |
| 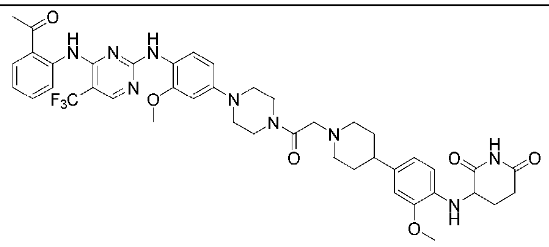 T-311 | 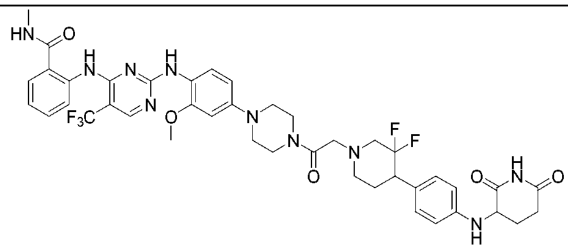 T-312 |
| 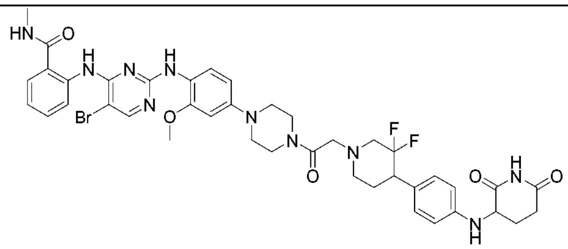 T-313 | 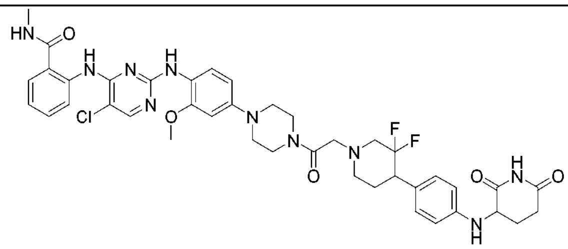 T-314 |

| | |
|---|---|
| 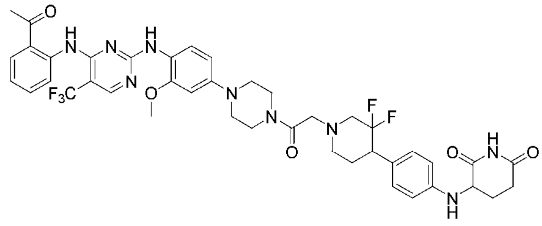<br>T-315 | 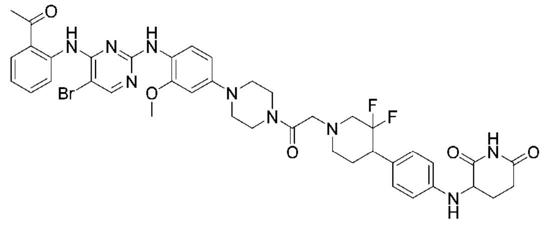<br>T-316 |
| 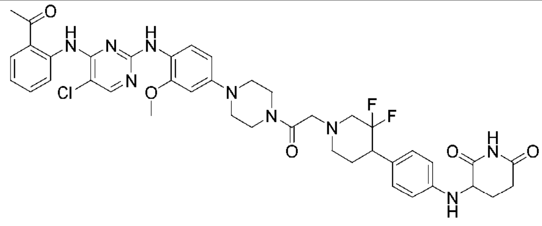<br>T-317 | 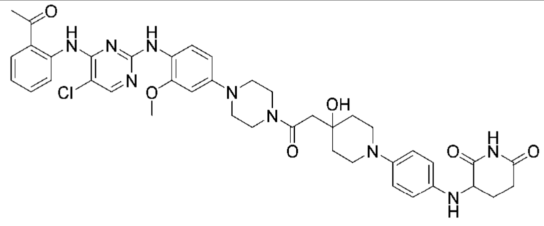<br>T-318 |
| 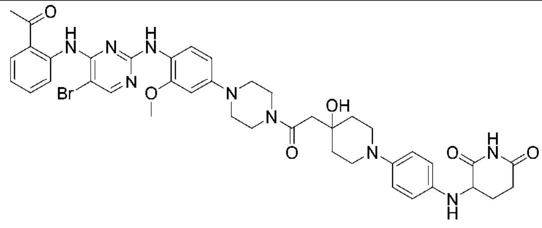<br>T-319 | 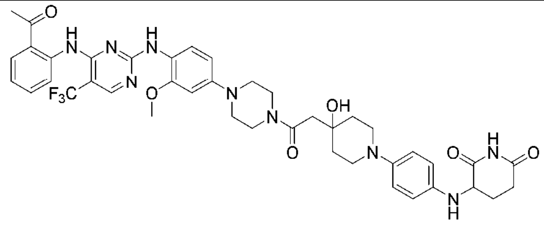<br>T-320 |
| 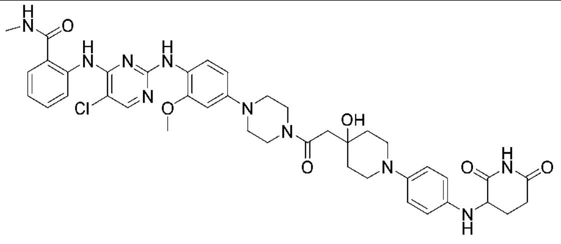<br>T-321 | 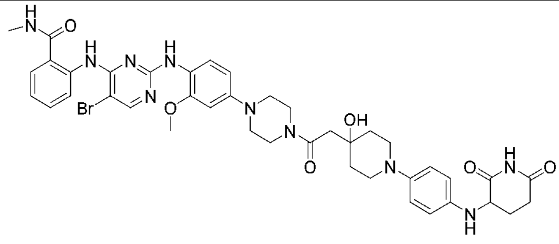<br>T-322 |
| 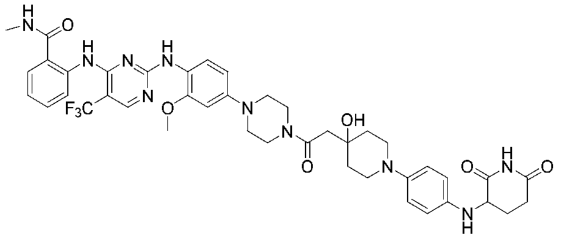<br>T-323 | 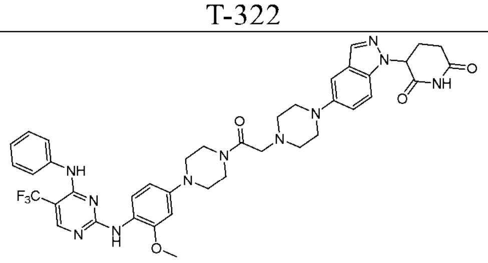<br>T-324 |
| 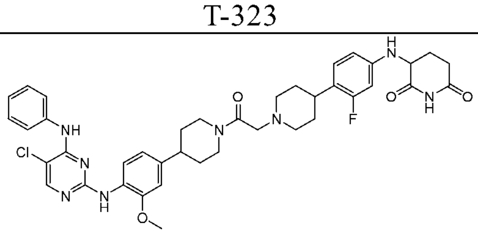<br>T-325 | 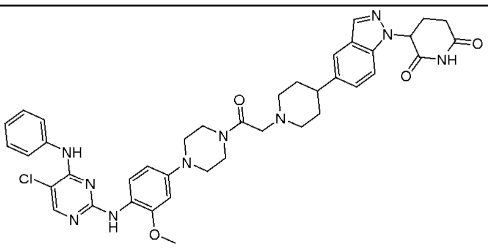<br>T-326 |

| | |
|---|---|
| 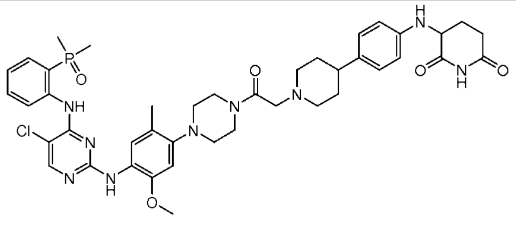 T-327 | 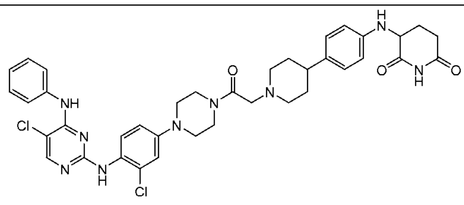 T-328 |
| 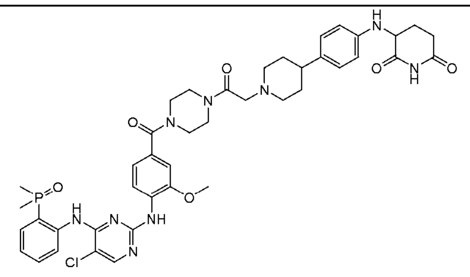 T-329 | 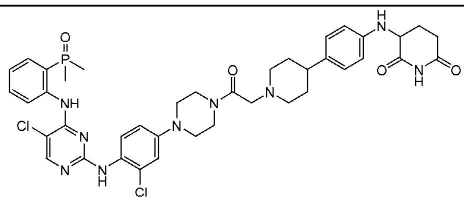 T-330 |
| 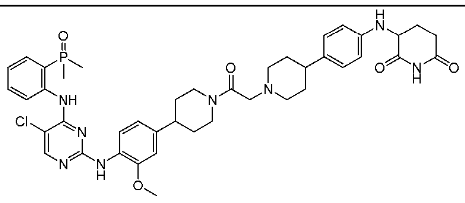 T-331 | 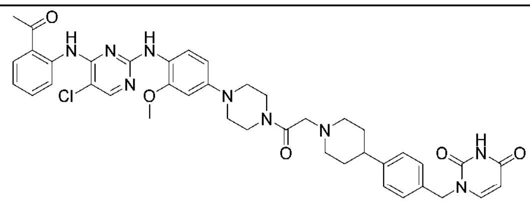 T-332 |
| 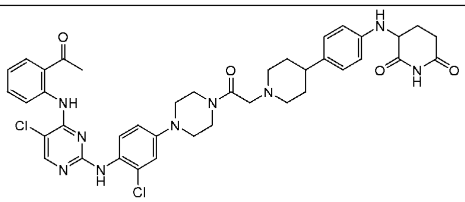 T-333 | 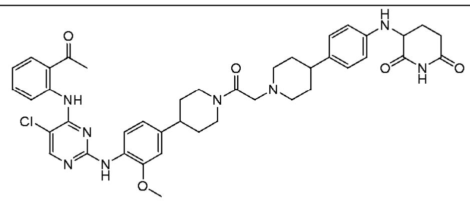 T-334 |
| 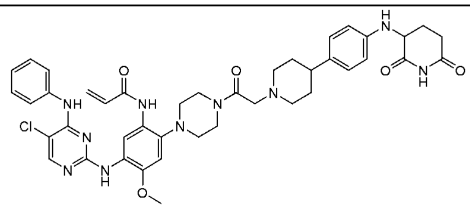 T-335 | 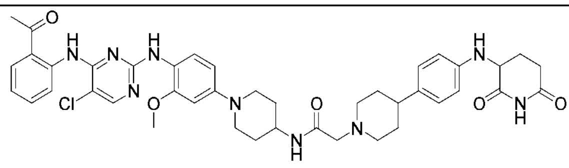 T-336 |
| 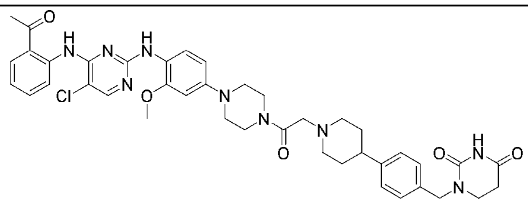 T-337 | 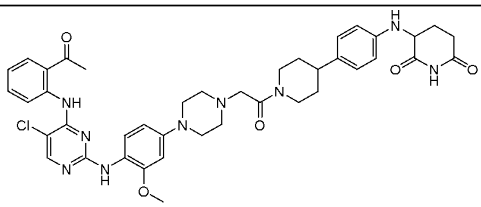 T-338 |
| 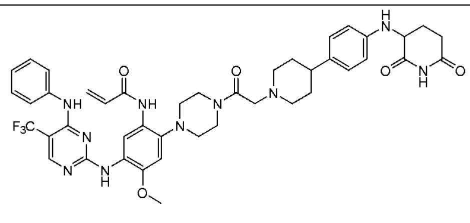 T-339 | 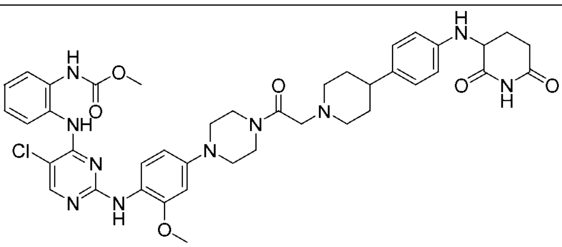 |

| | |
|---|---|
| | T-340 |
| 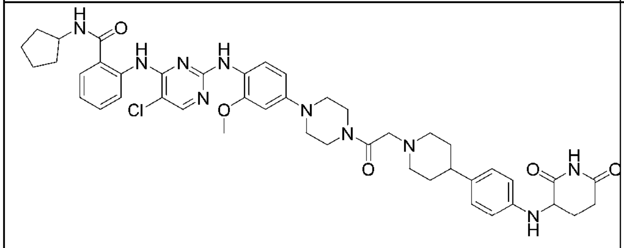<br>T-341 | 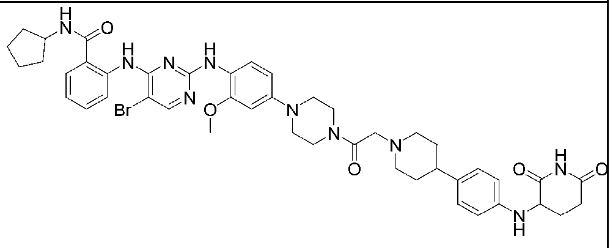<br>T-342 |
| 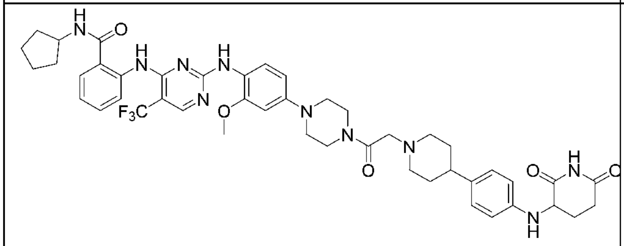<br>T-343 | 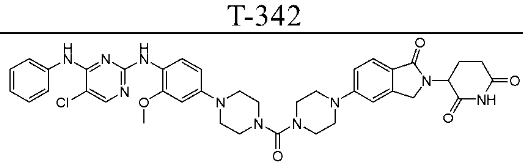<br>T-344 |
| 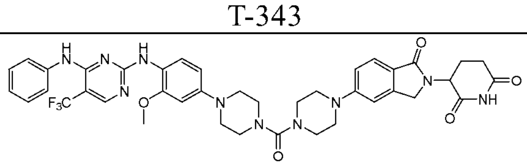<br>T-345 | 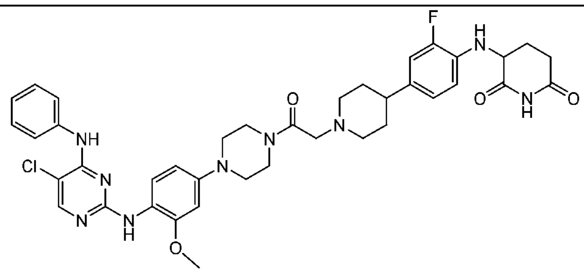<br>T-346 |
| 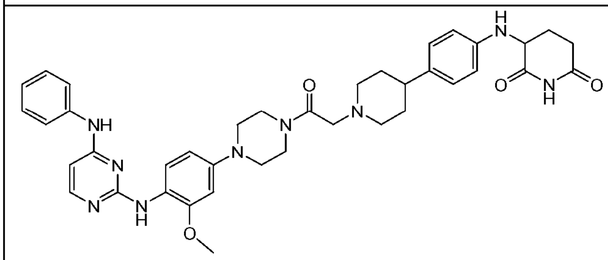<br>T-347 | 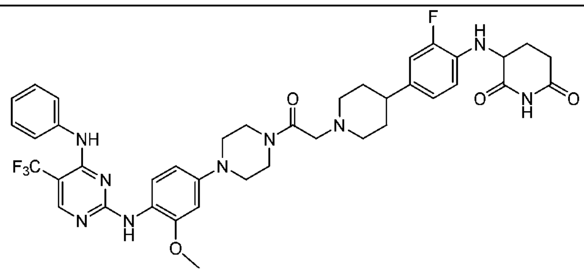<br>T-348 |
| 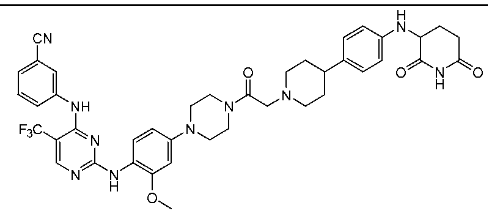<br>T-349 | 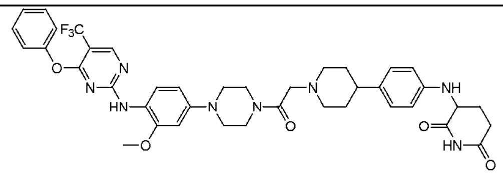<br>T-350 |

| | |
|---|---|
| 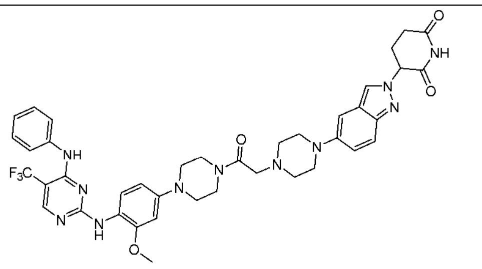<br>T-351 | 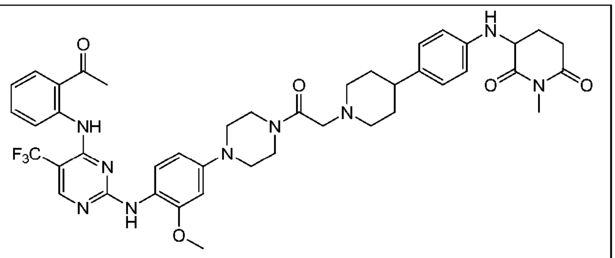<br>T-352 |
| 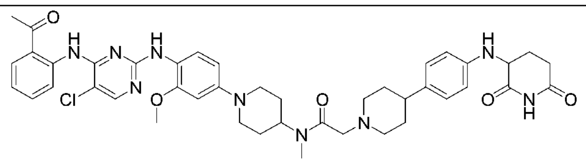<br>T-353 | 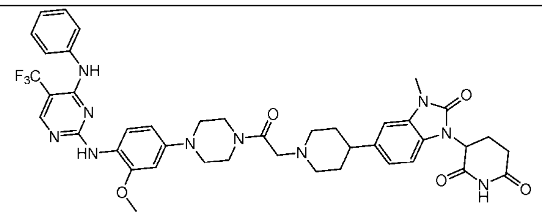<br>T-354 |
| 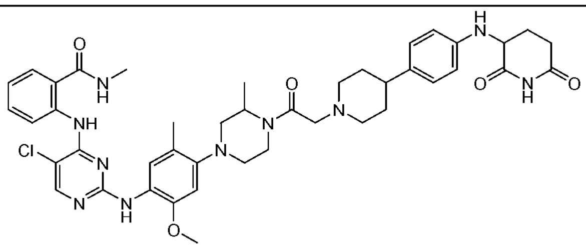<br>T-355 | 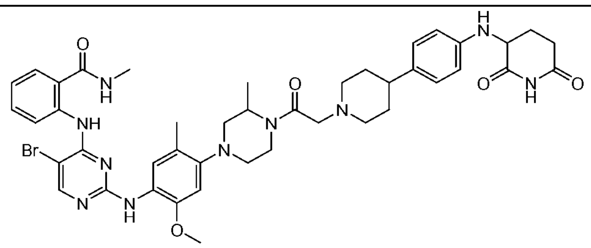<br>T-356 |
| 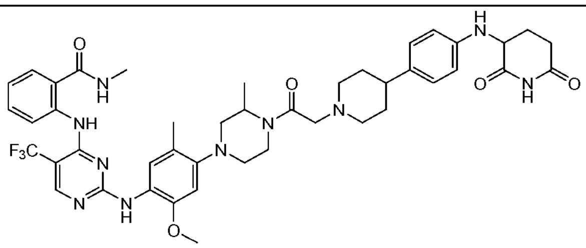<br>T-357 | 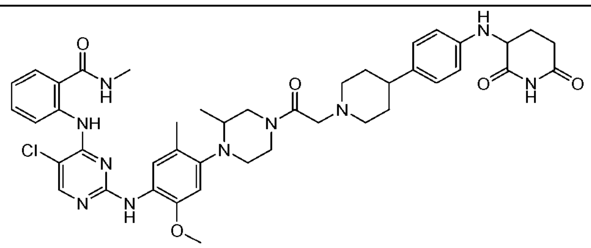<br>T-358 |
| 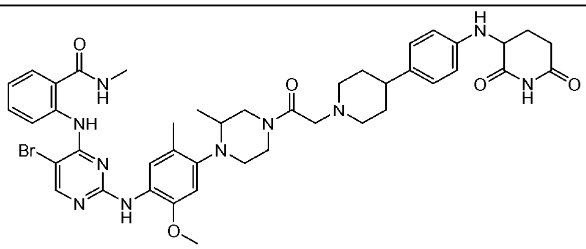<br>T-359 | 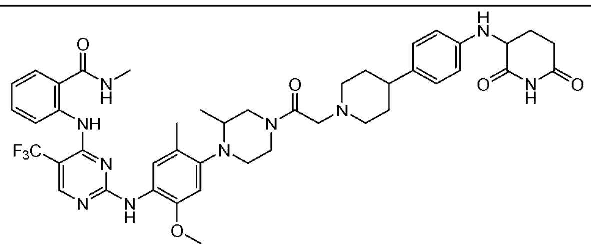<br>T-360 |
| 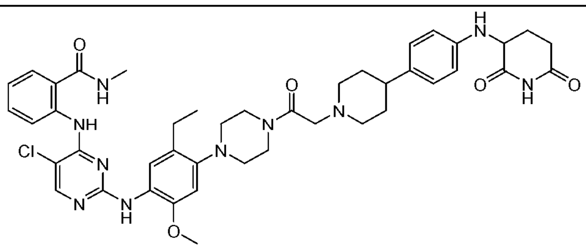<br>T-361 | 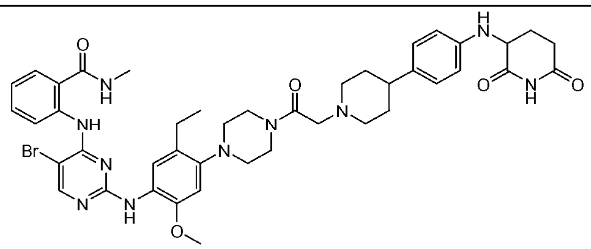<br>T-362 |

| | |
|---|---|
| 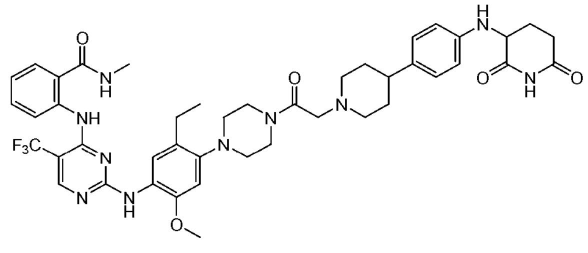 T-363 | 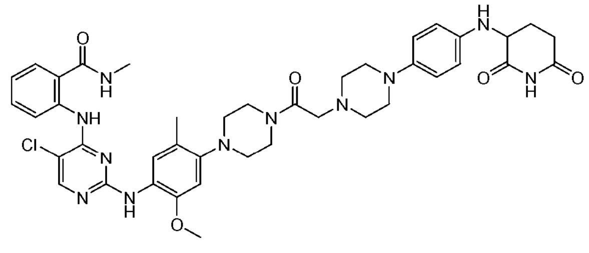 T-364 |
| 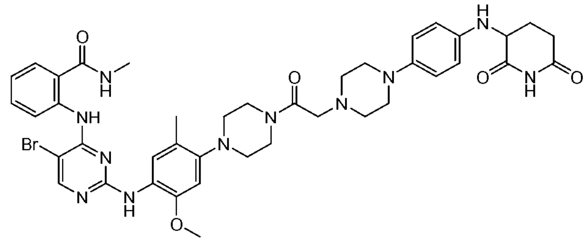 T-365 | 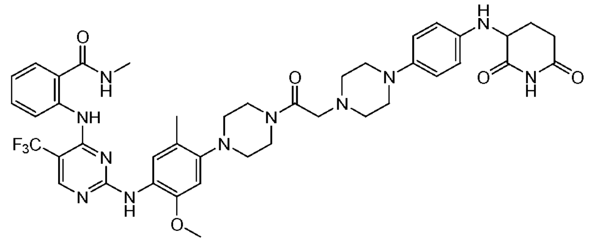 T-366 |
| 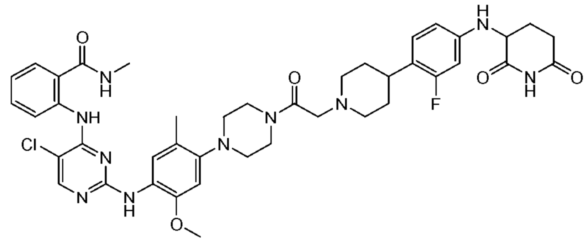 T-367 | 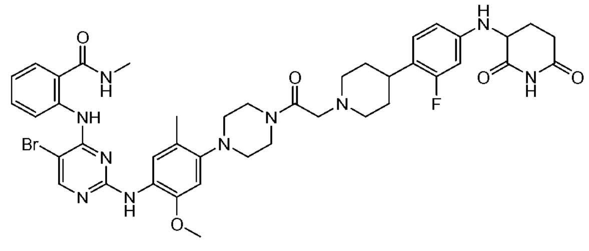 T-368 |
| 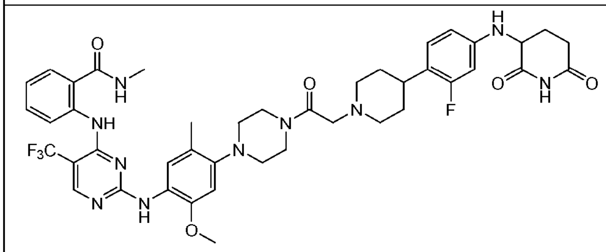 T-369 | 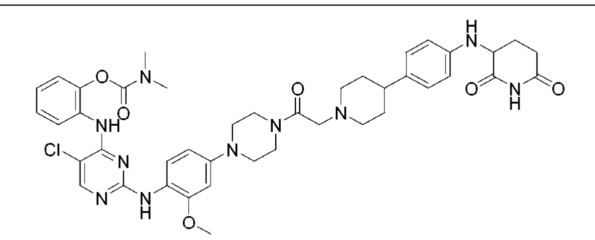 T-370 |
| 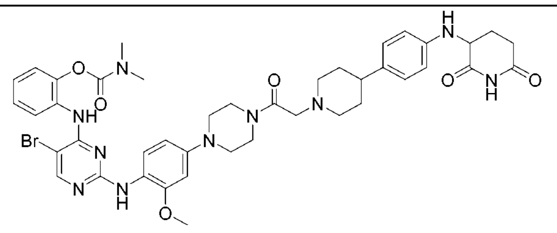 T-371 | 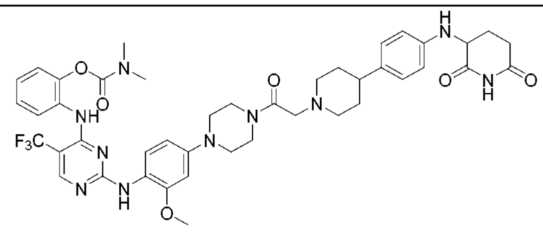 T-372 |
| 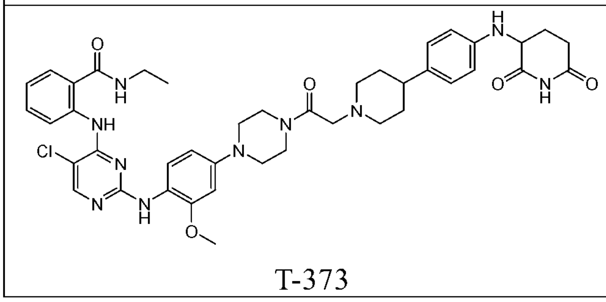 T-373 | 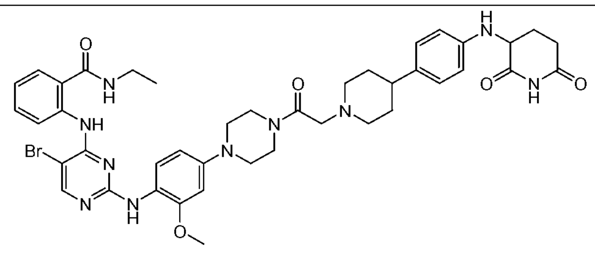 T-374 |

| | |
|---|---|
| 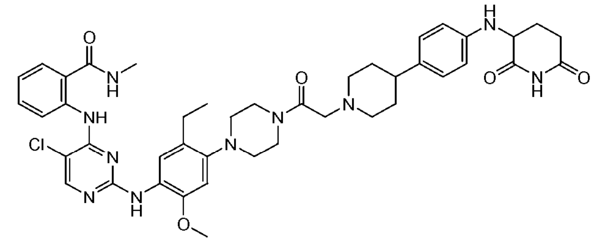 T-375 | 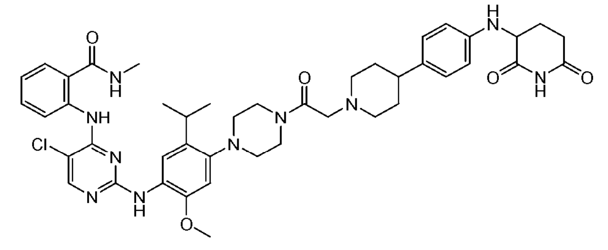 T-376 |
| 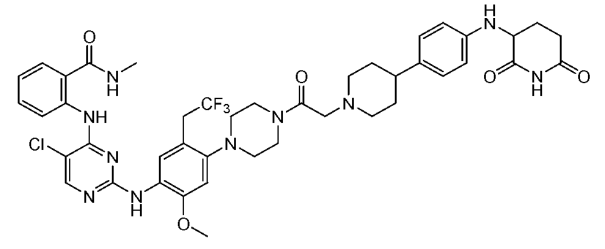 T-377 | 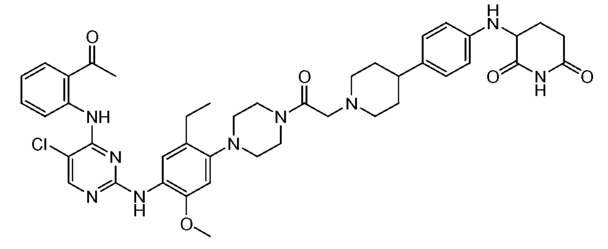 T-378 |
| 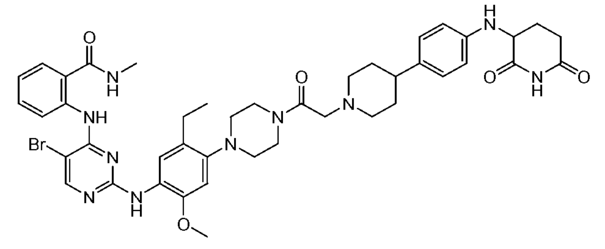 T-379 | 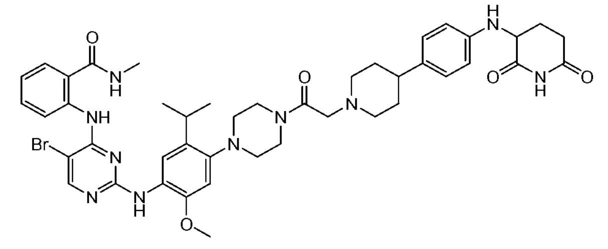 T-380 |
| 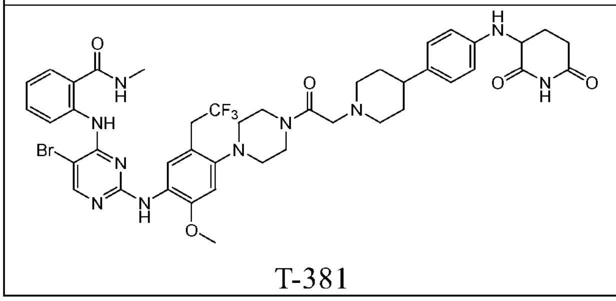 T-381 | 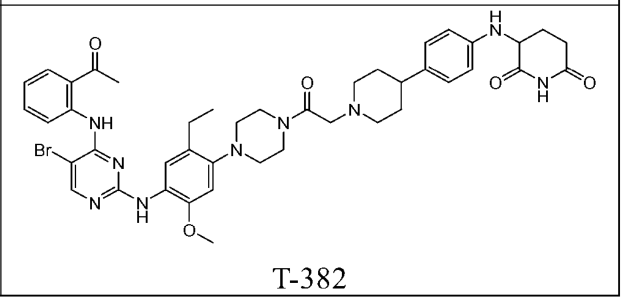 T-382 |

In another preferred embodiment, the compound is not selected from the group consisting of:

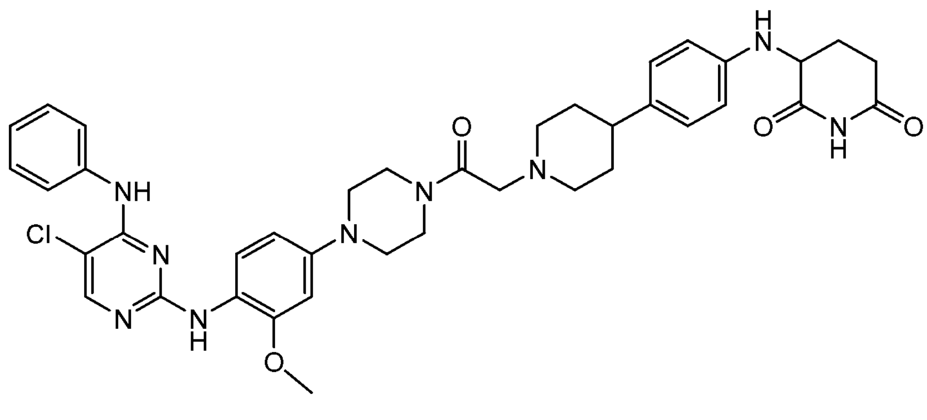

T-01

,

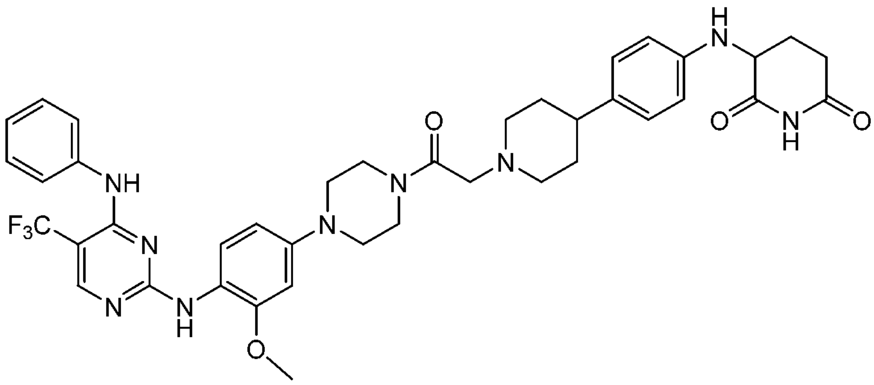

T-02

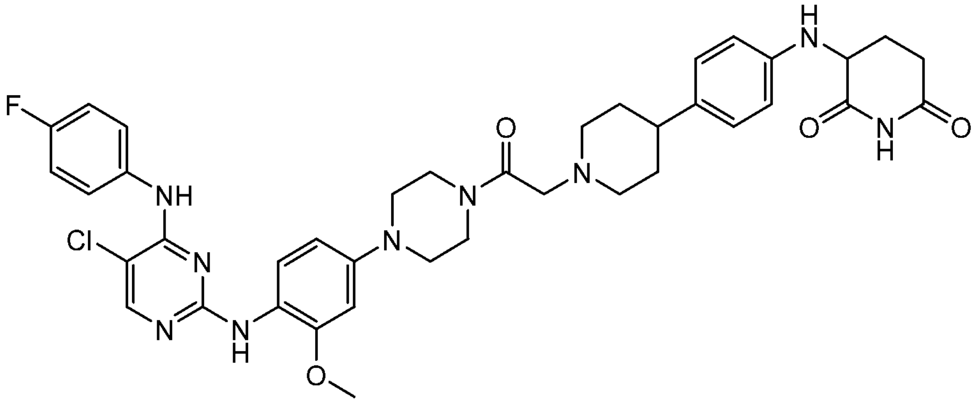

T-03

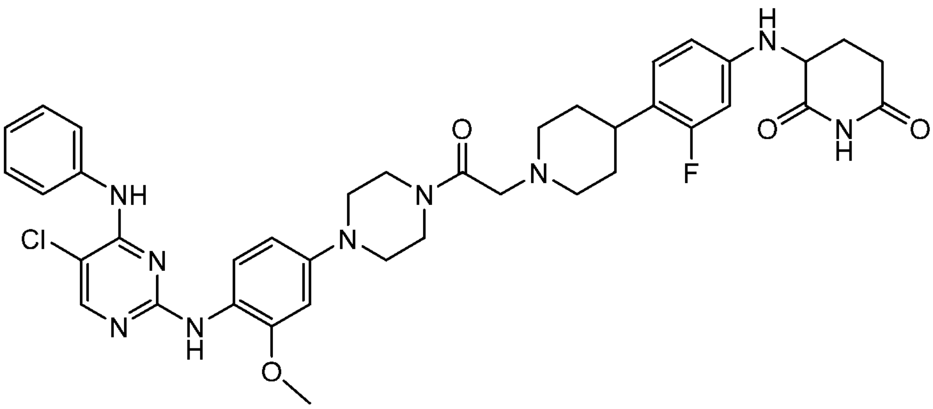

T-04

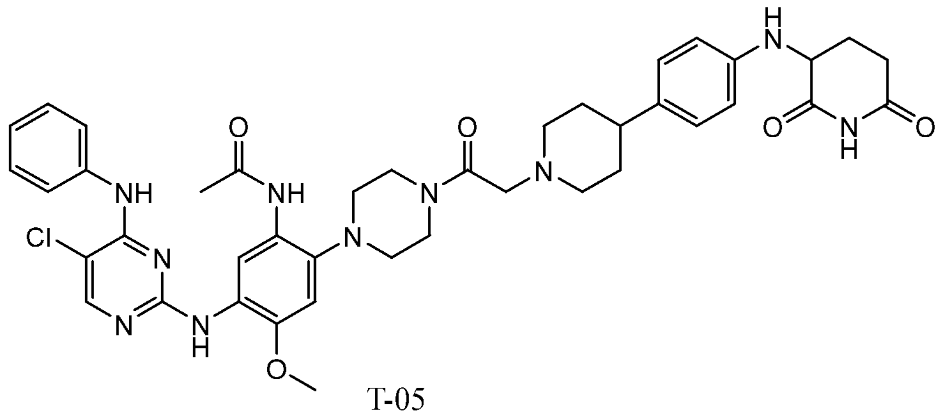

T-05

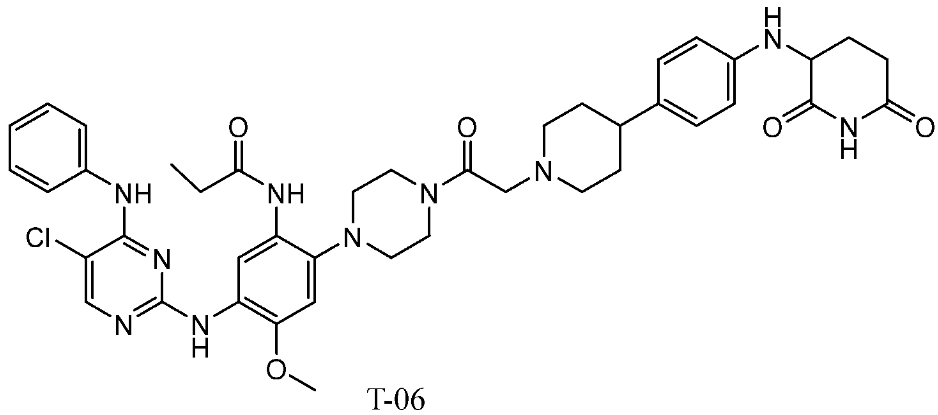

T-06

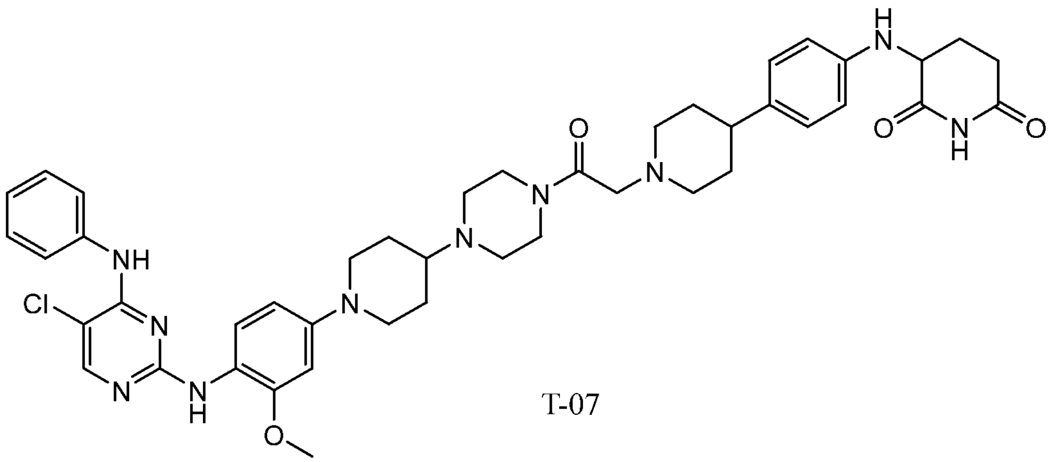

T-07

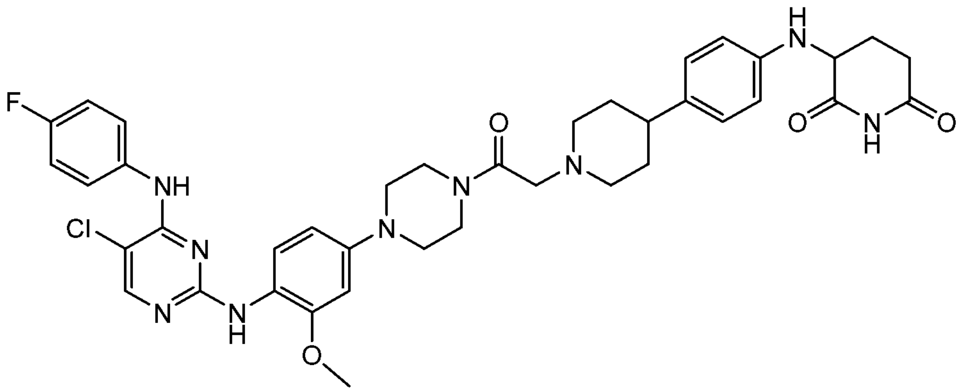

T-08

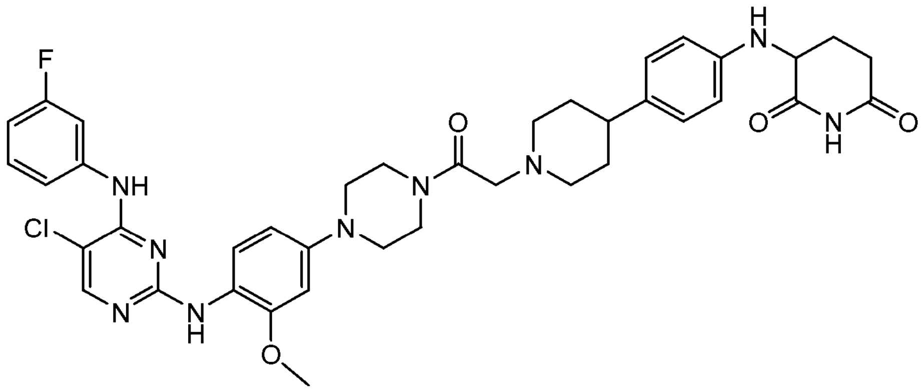

T-09

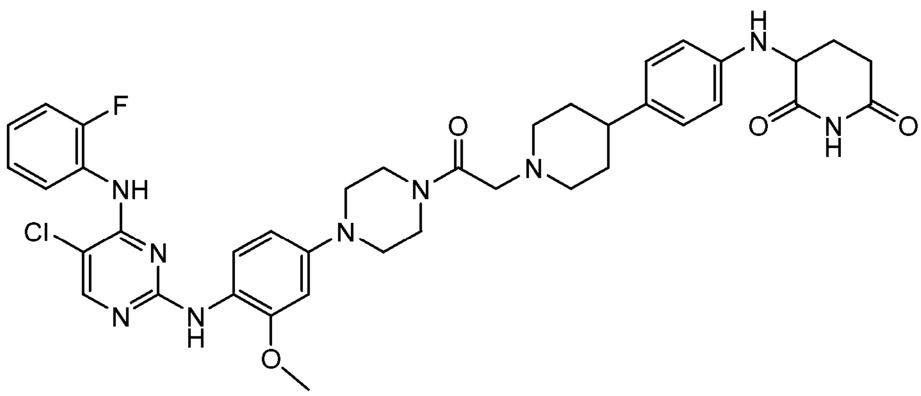

T-10

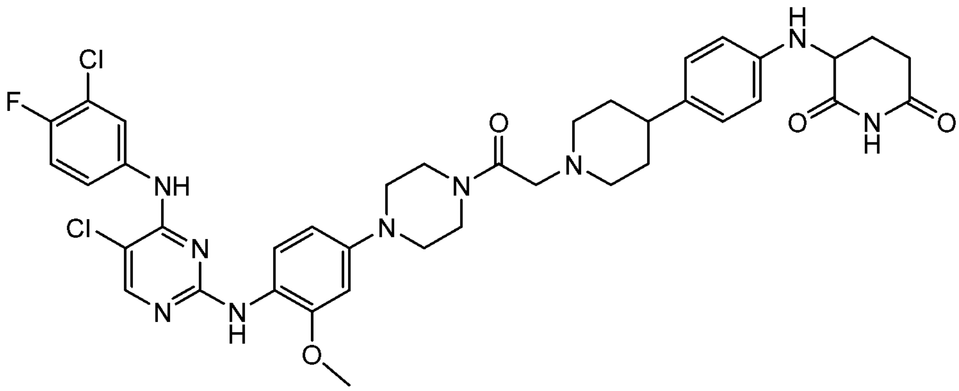

T-11

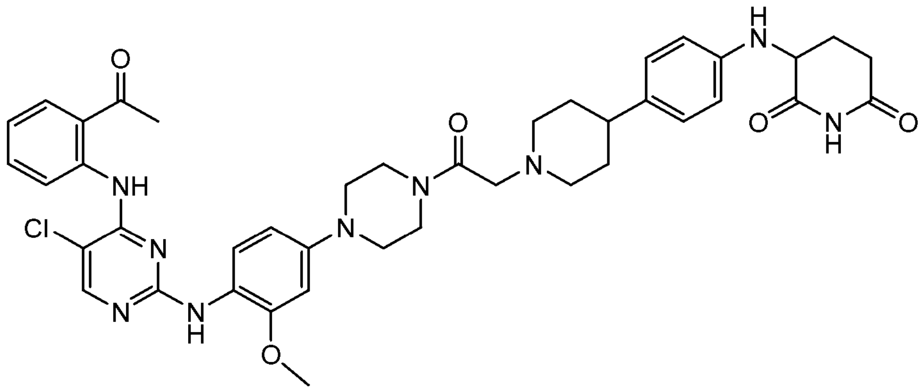

T-12

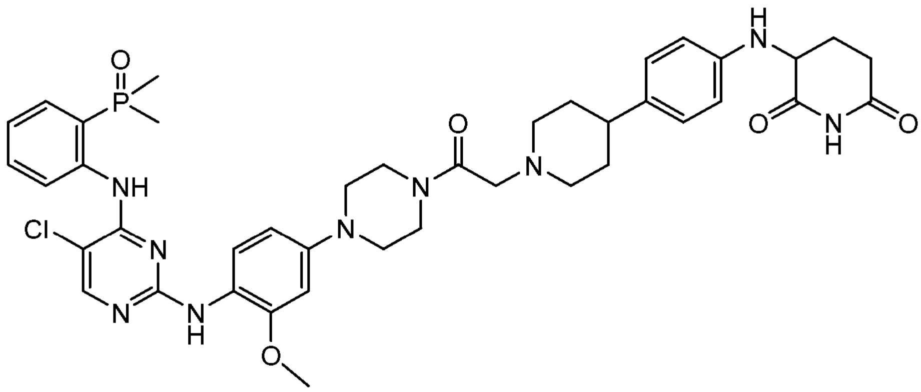

T-13

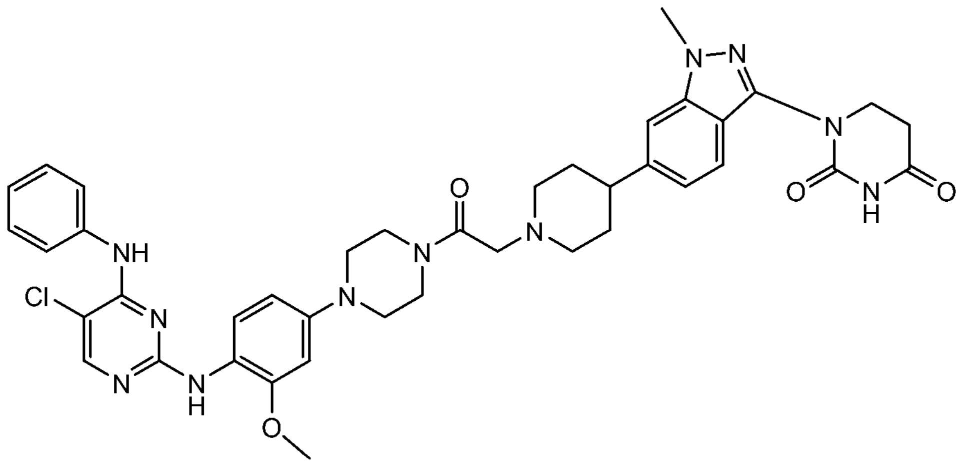

T-14

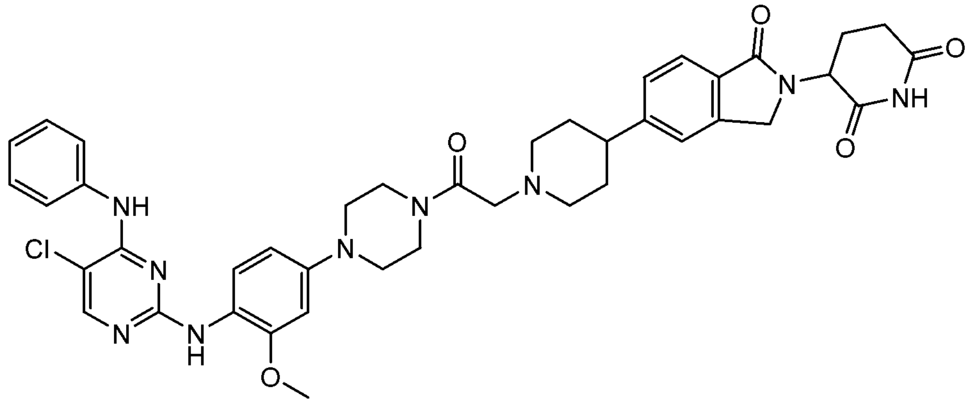

T-15

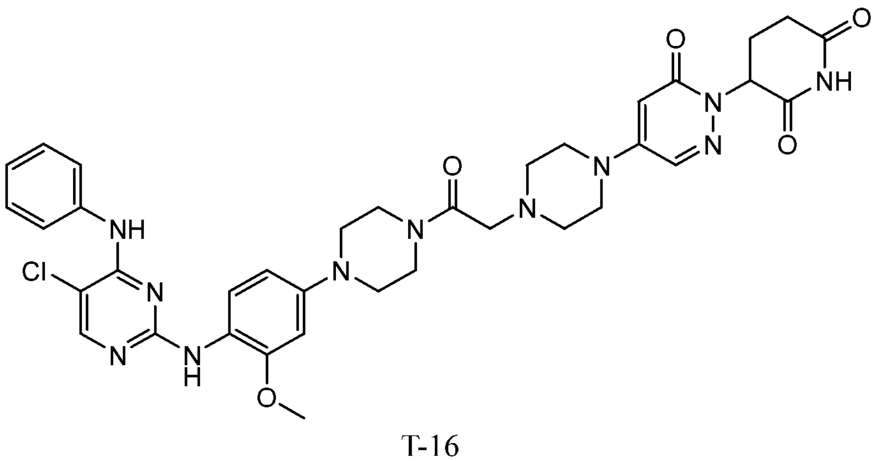

T-16

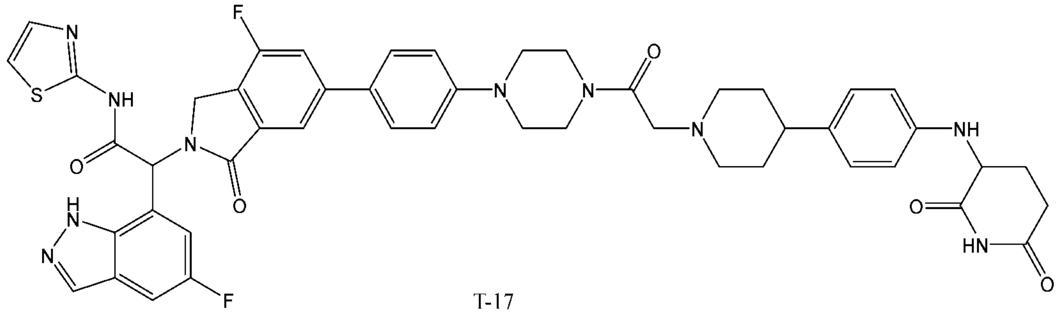

T-17

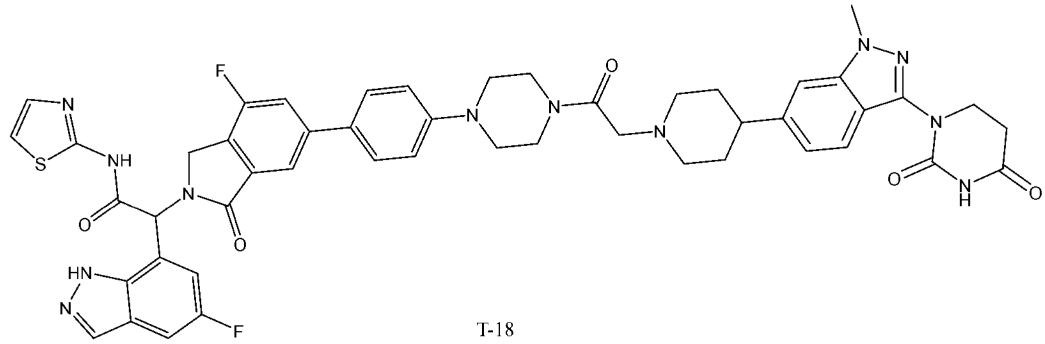

T-18

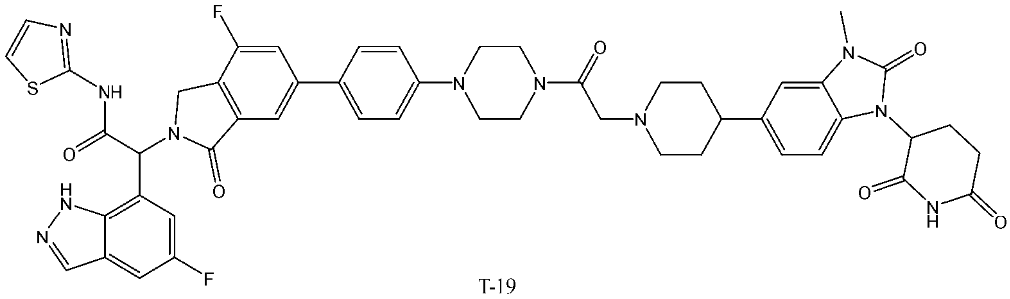

T-19

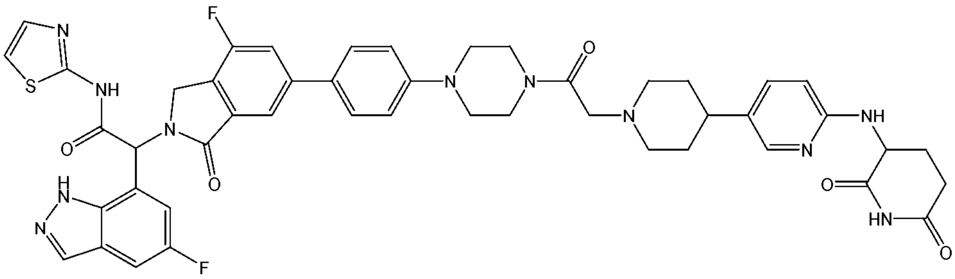

T-20

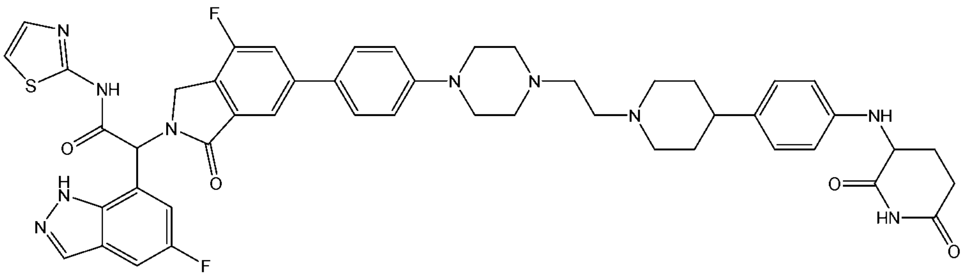

T-21

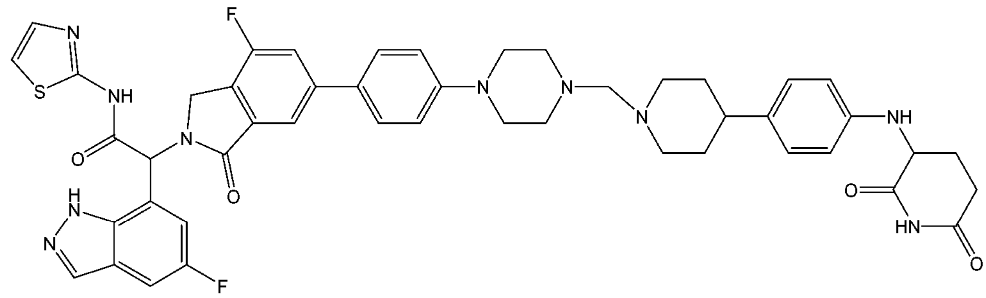

T-22

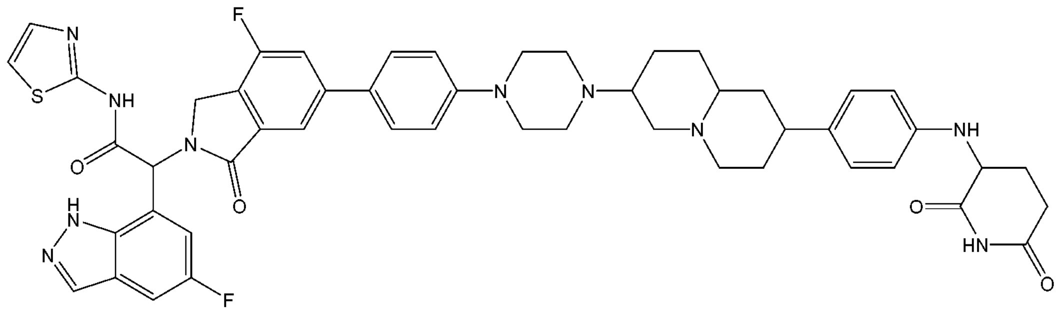

T-23

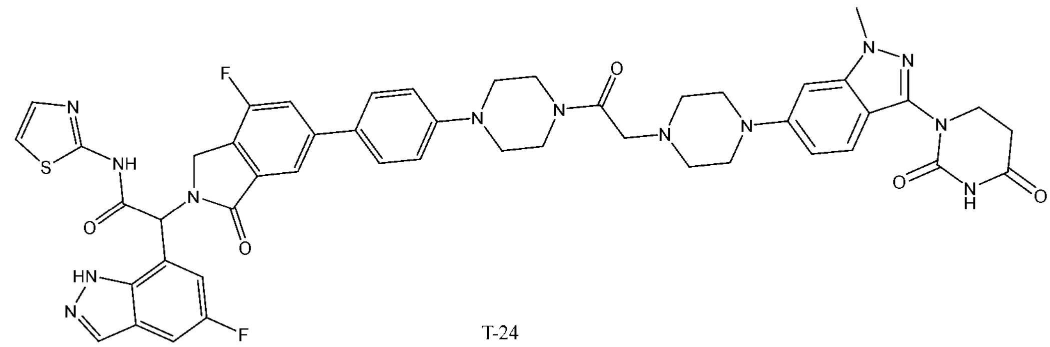

T-24

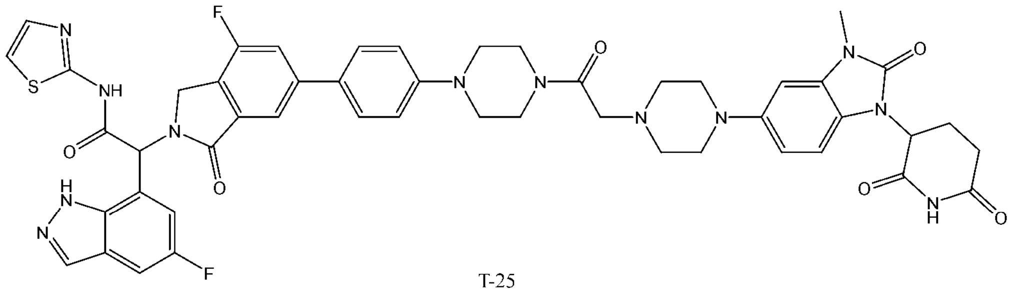

T-25

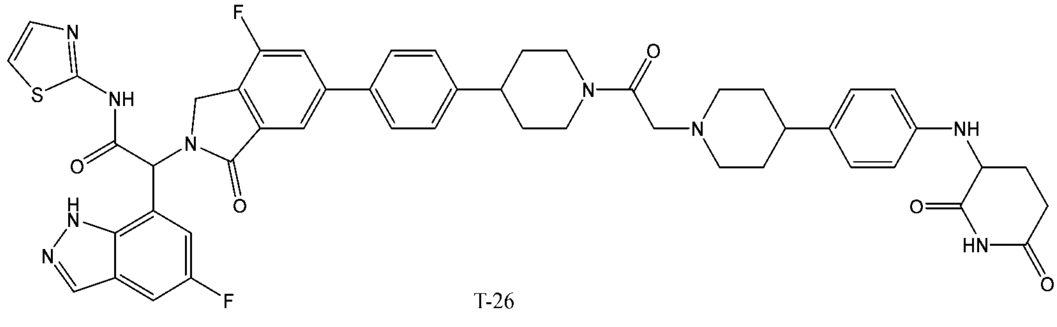

T-26

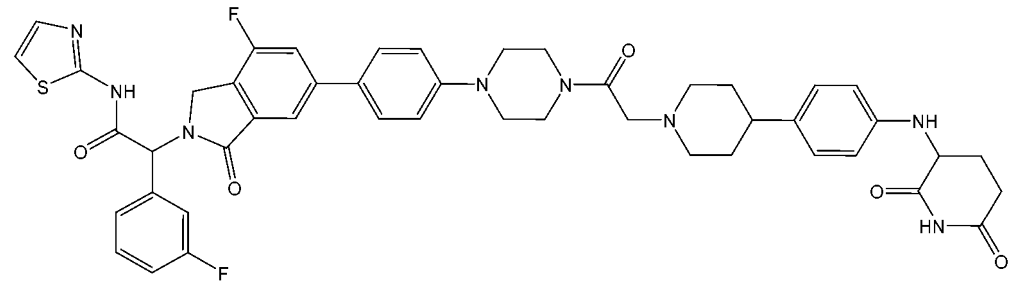

T-27

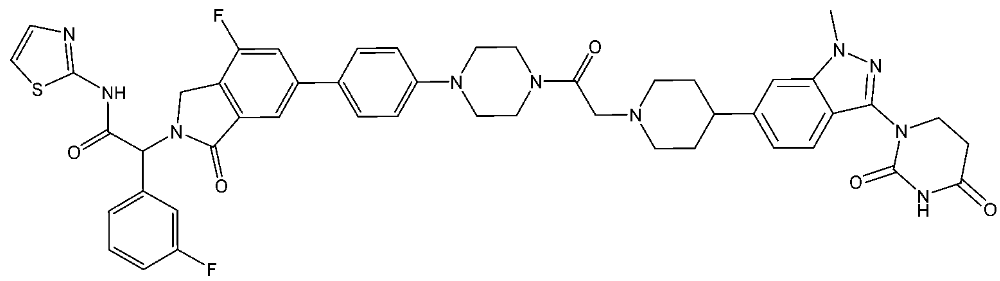

T-28

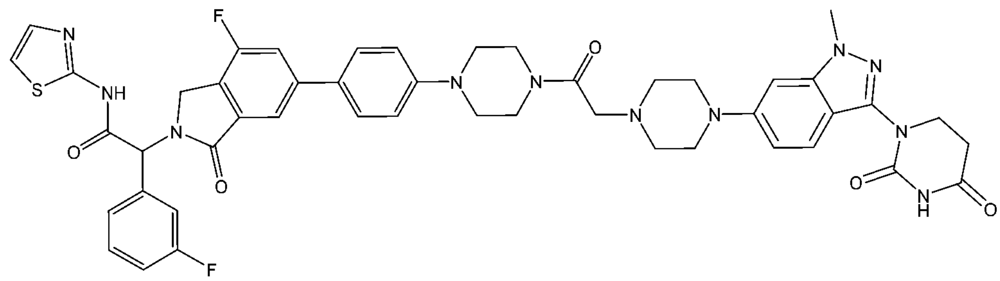

T-29

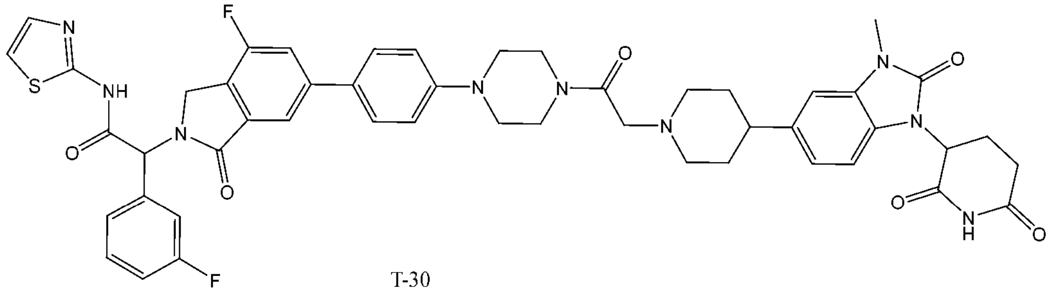

T-30

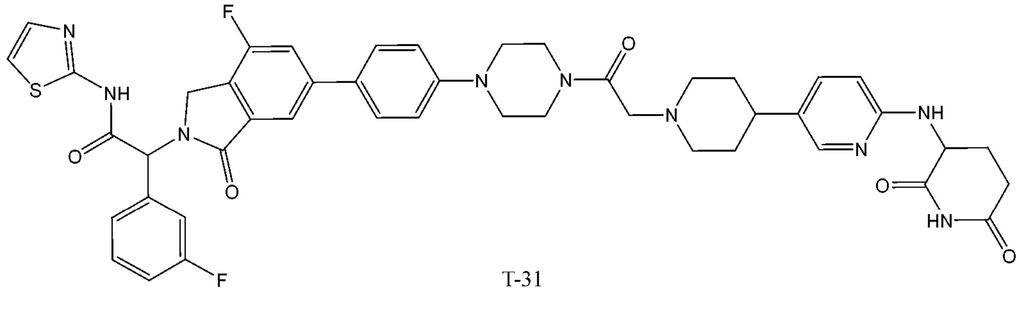

T-31

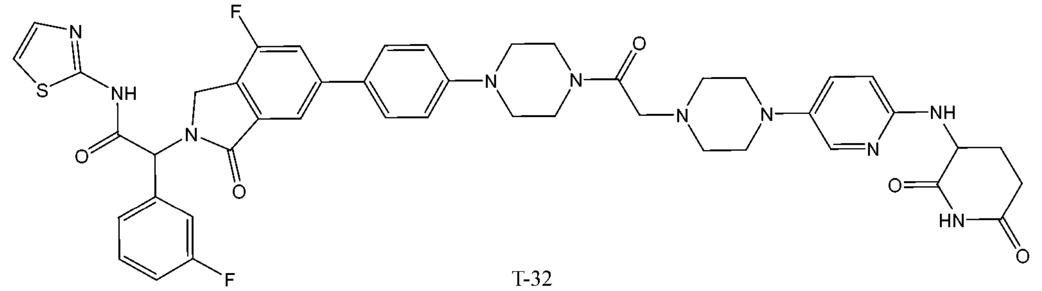

T-32

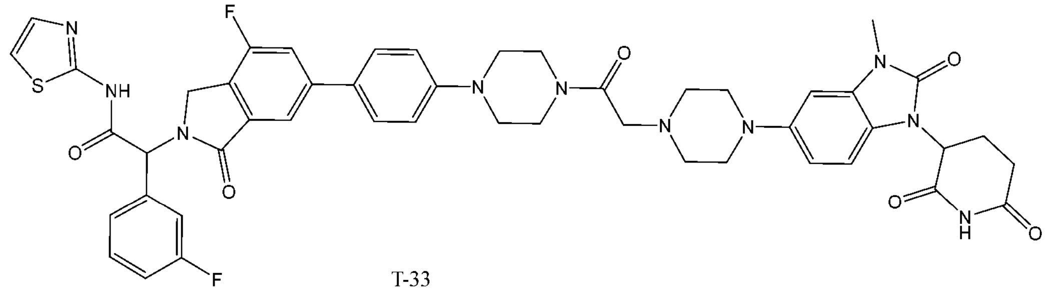

T-33

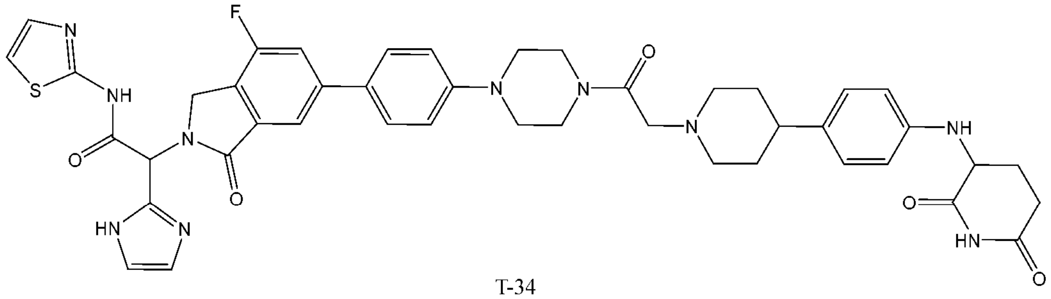

T-34

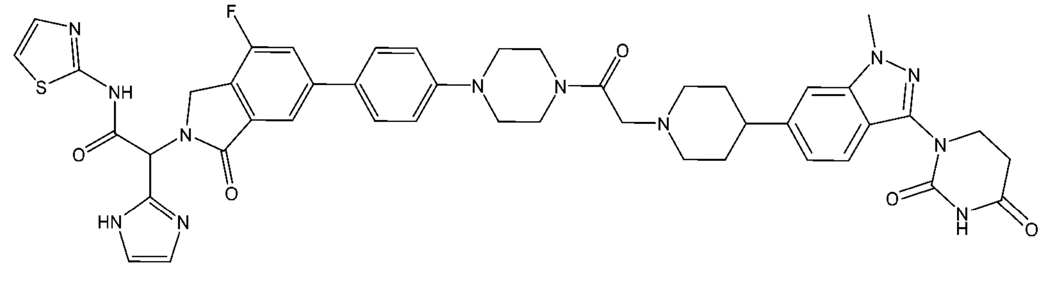

T-35

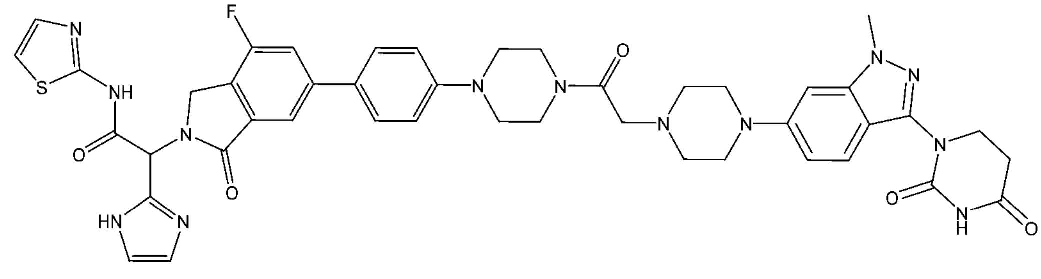

T-36

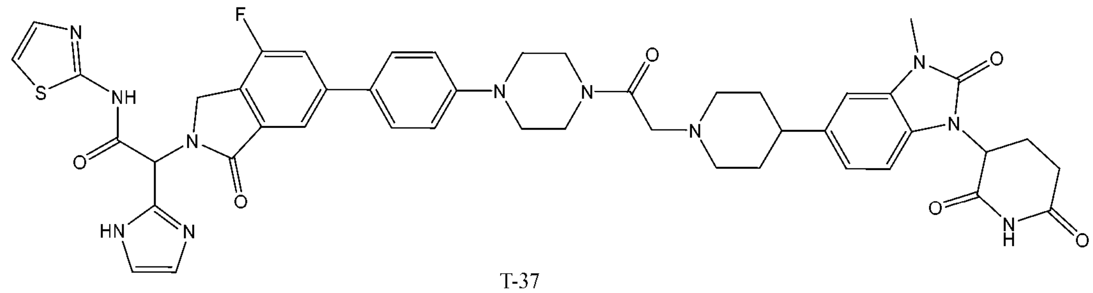

T-37

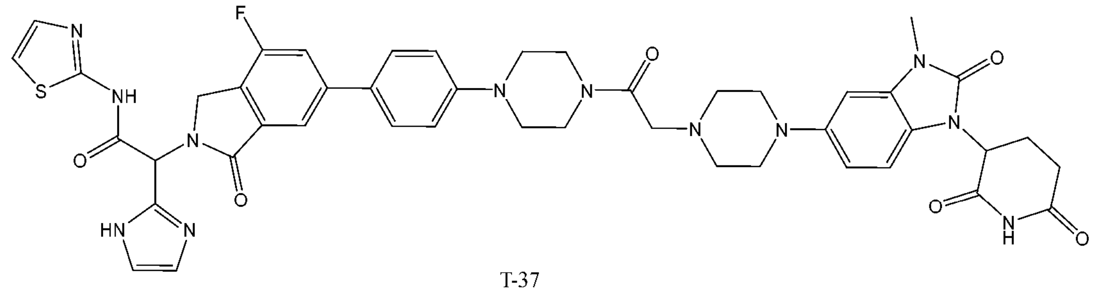

T-37

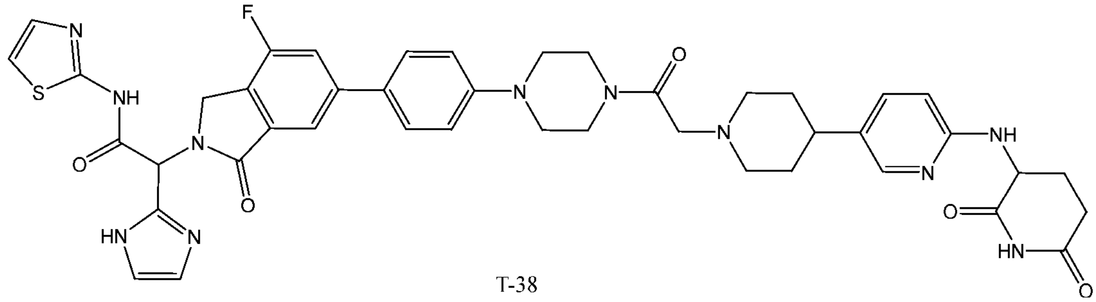

T-38

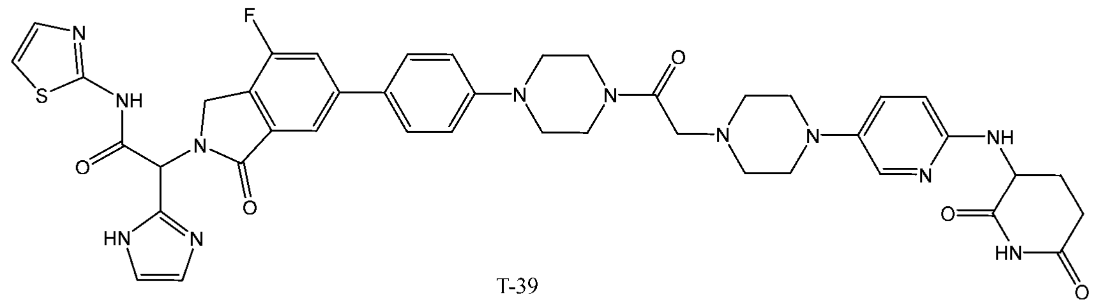

T-39

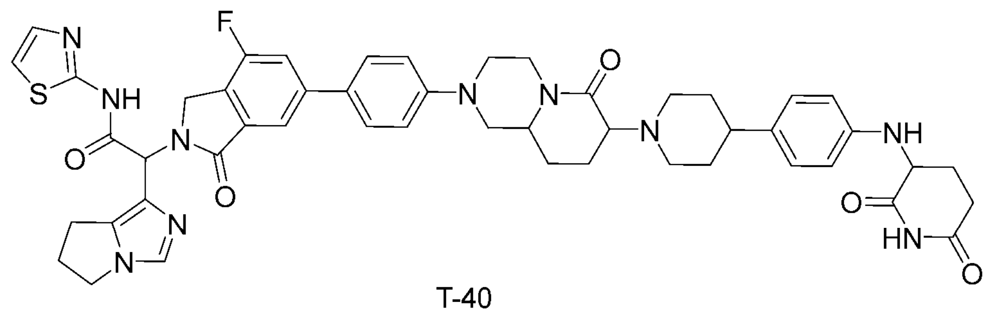

T-40

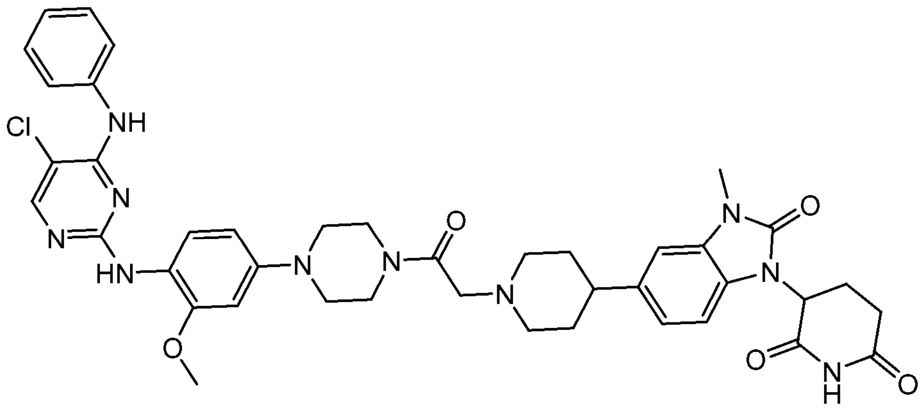

T-41

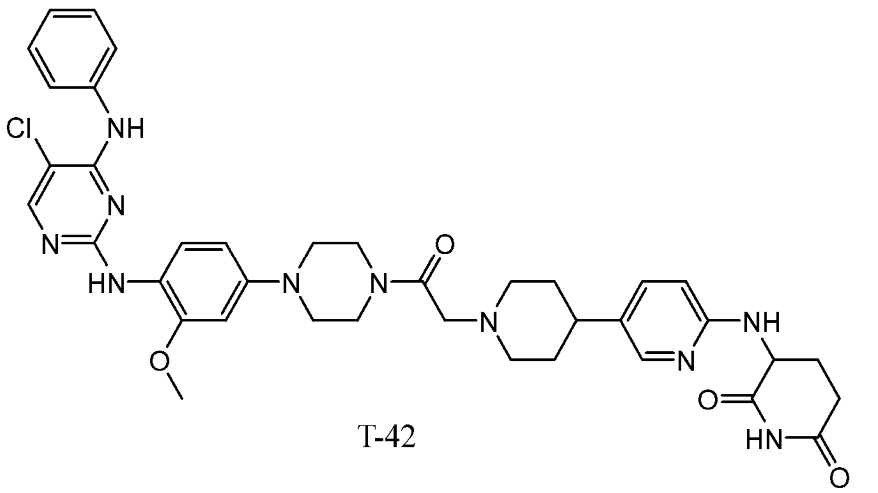

T-42

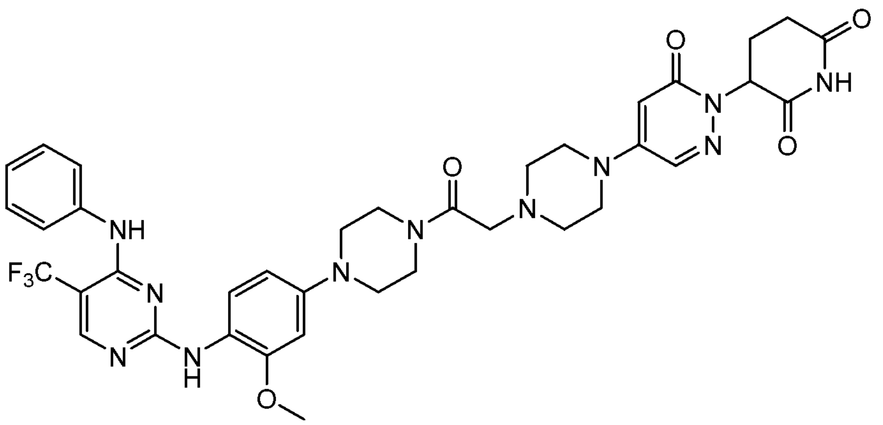

T-43

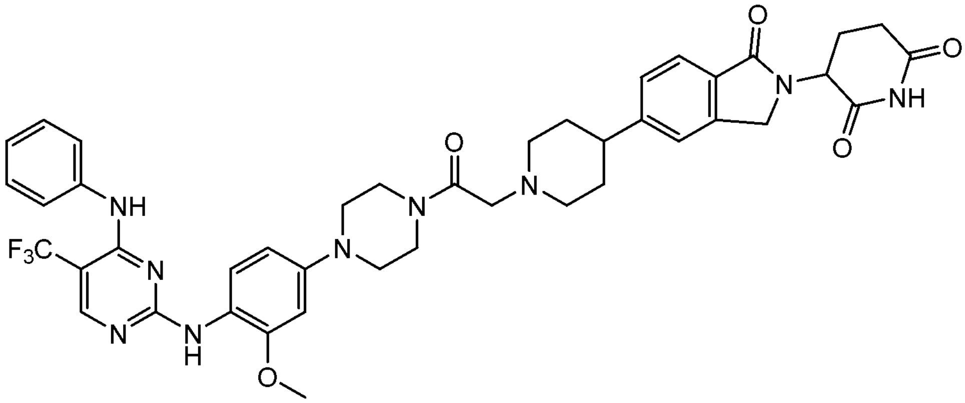

T-44

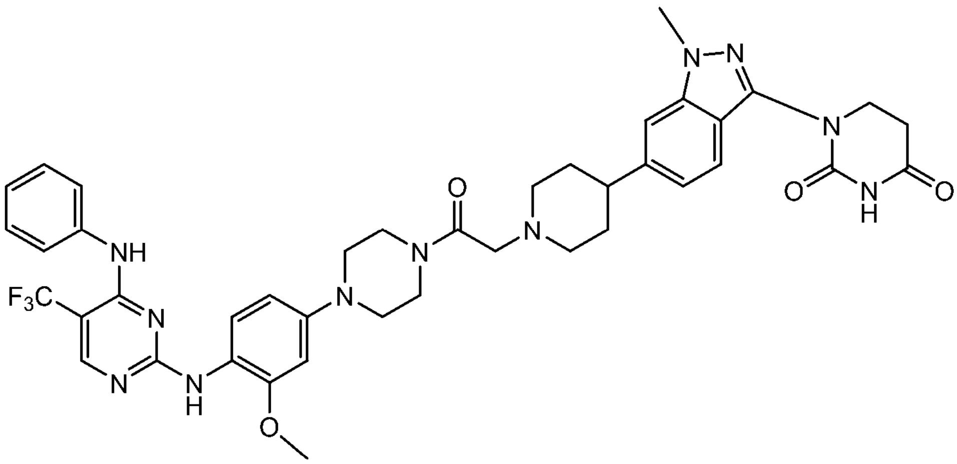

T-45

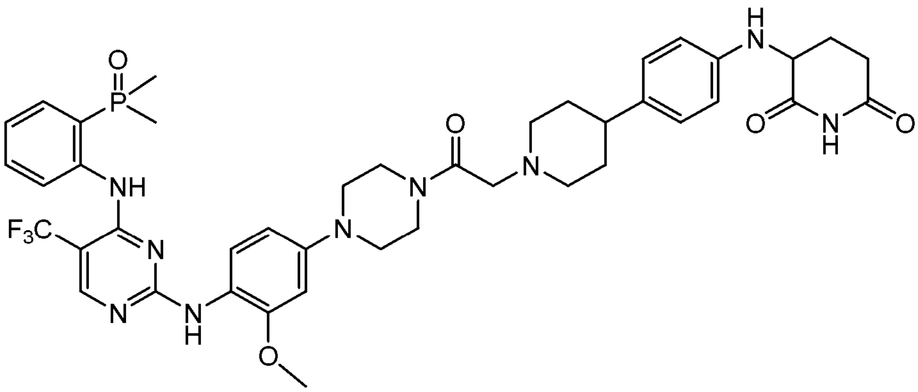

T-46

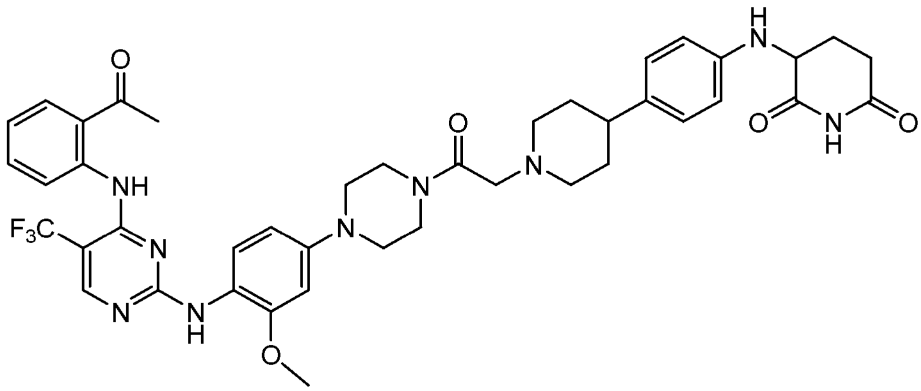

T-47

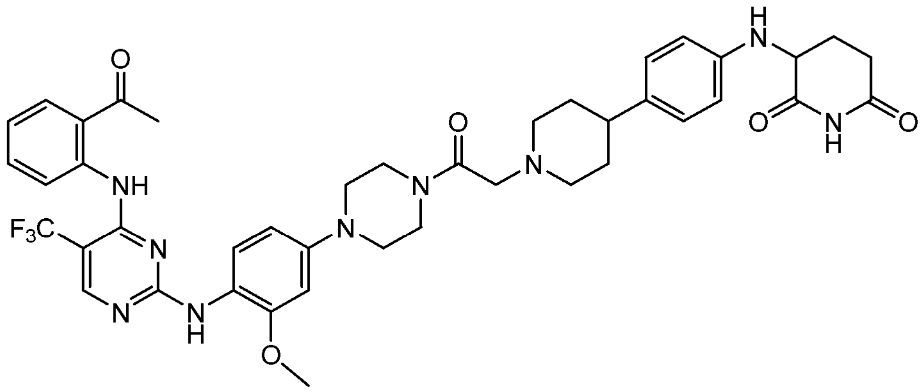

T-48

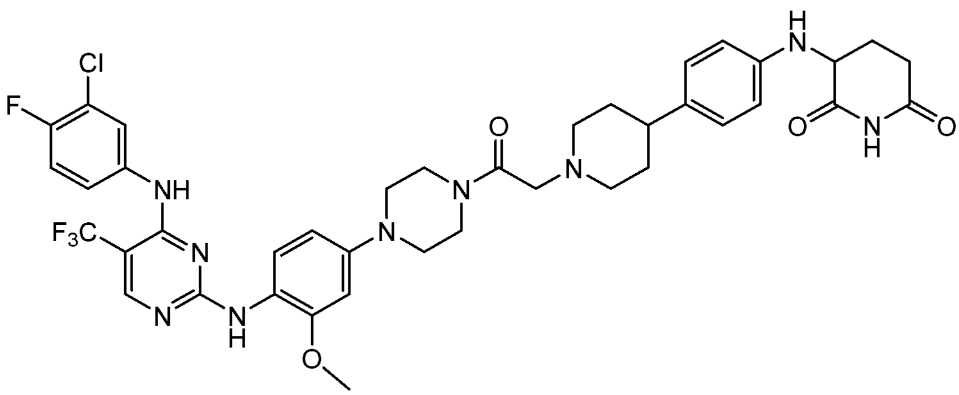

T-49

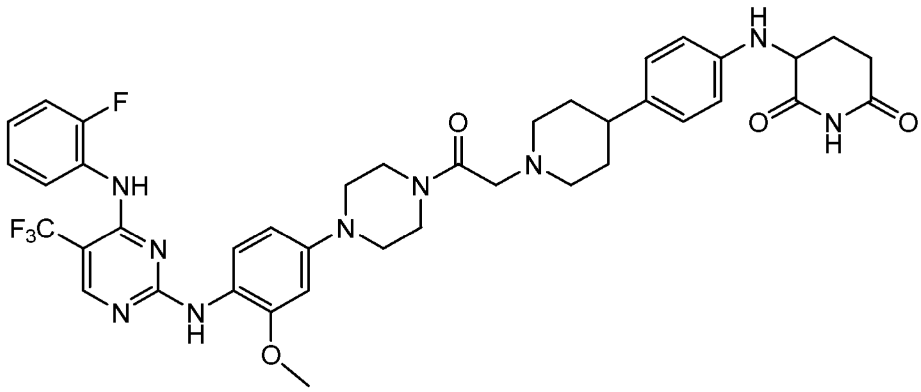

T-50

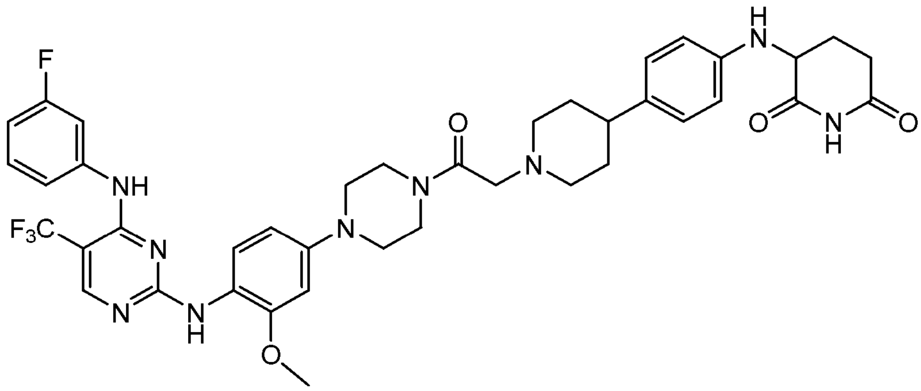

T-51

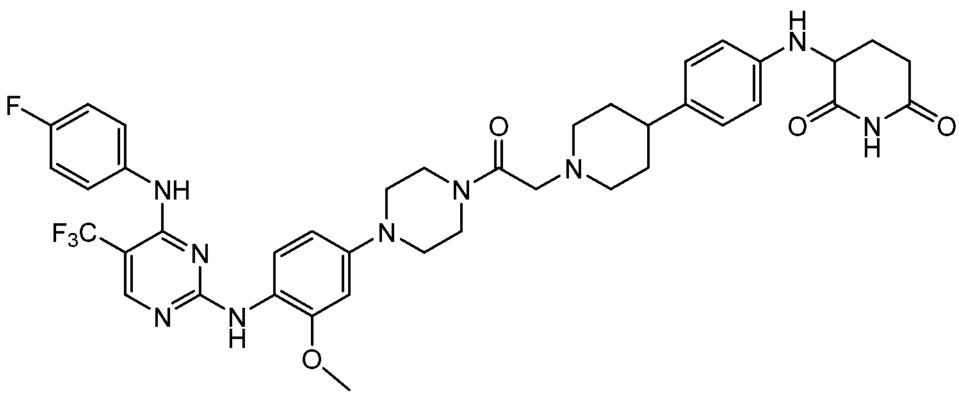

T-52

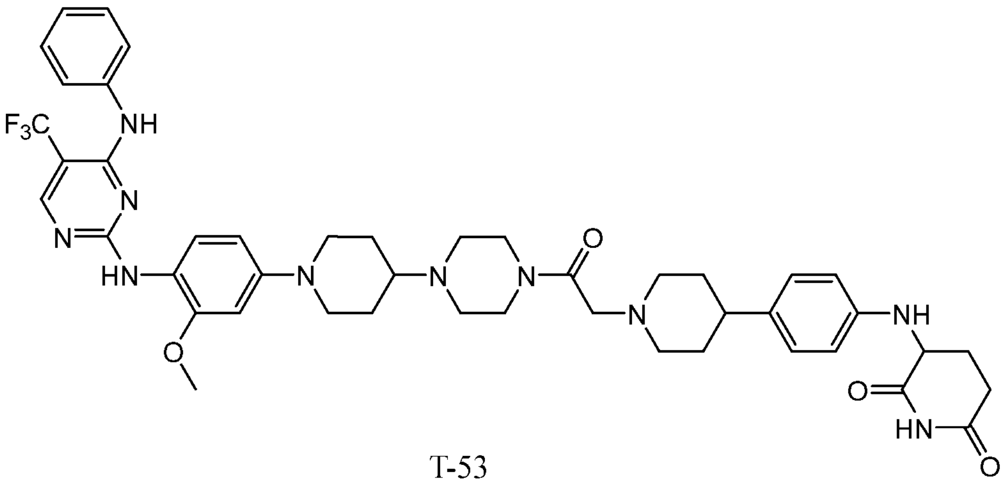

T-53

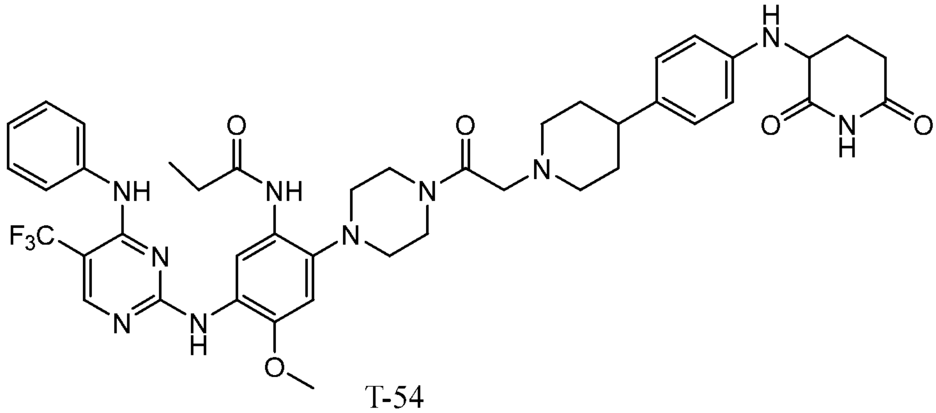

T-54

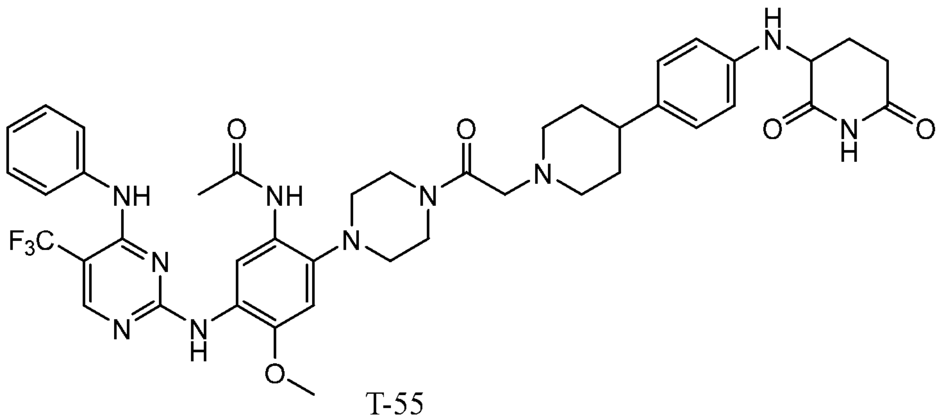

T-55

,

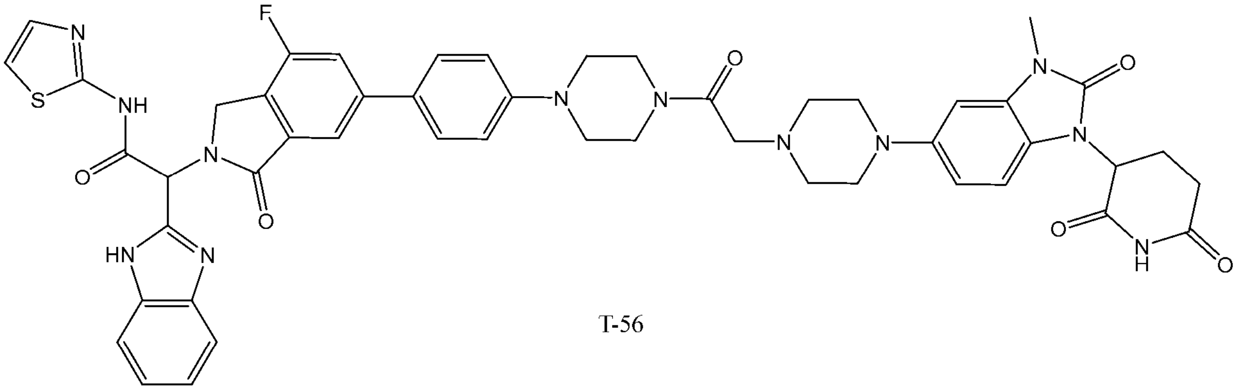

T-56

,

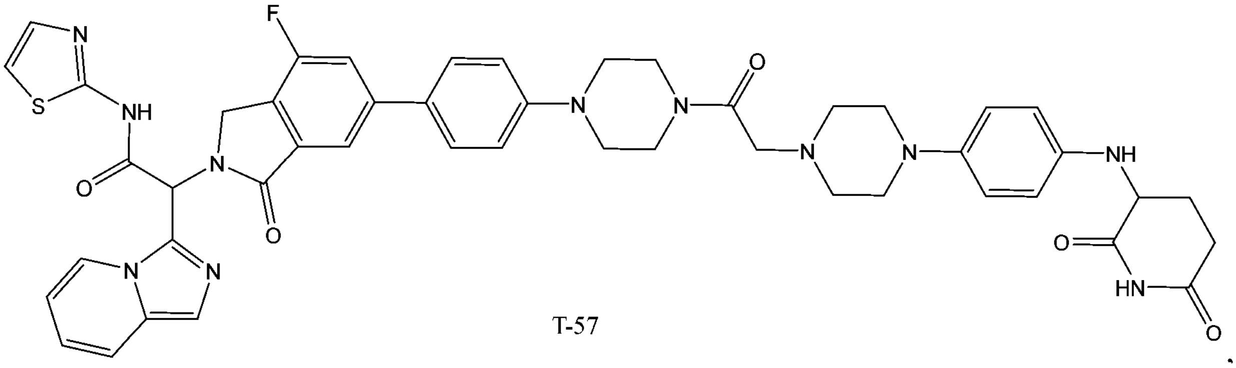

T-57

,

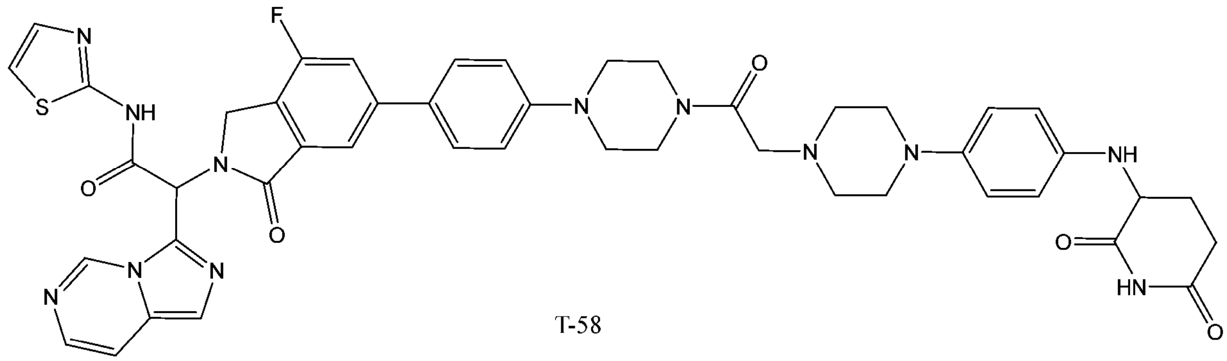

T-58

and

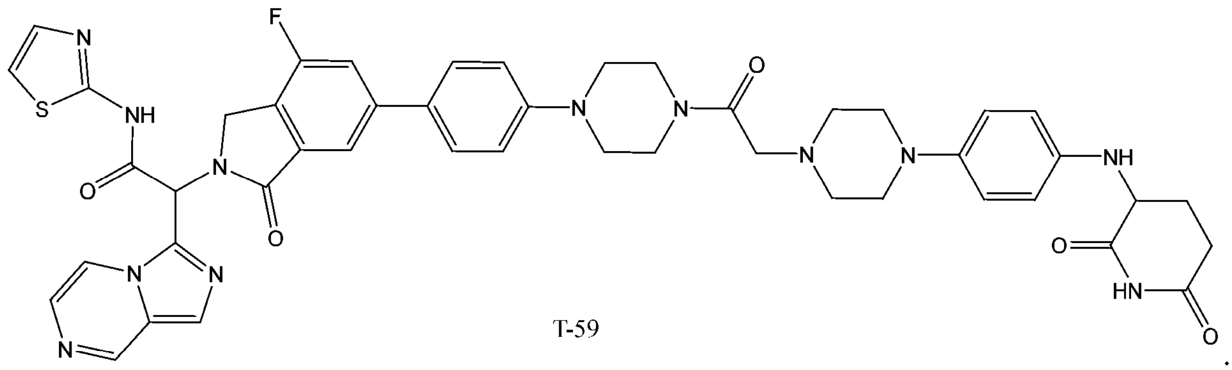

T-59

.

In the second aspect of the present invention, provided is a pharmaceutical composition comprising a safe and effective amount of the compound as described in the first aspect of the present invention, and a pharmaceutically acceptable carrier.

In another preferred embodiment, the pharmaceutical composition further comprises a second active ingredient selected from the group consisting of: gefitinib, erlotinib, ectinib, lapatinib, XL647, NVP-AEE-788, ARRY-334543, vandetanib, PF00299804, cetuximab, panitumumab, pertuzumab, zalumumab, nimotuzumab, MDX-214, CDX-110, IMC-11F8, CNF2024, tanespimycin, alvespimycin, IPI-504 and NVP-AUY922.

In the third aspect of the present invention, provided is a use of the compound according to the first aspect of the present invention, and the use is selected from the group consisting of:

1) preparation of a medicament for modulating EGFR kinase activity or treating EGFR-related diseases; and
2) preparation of a medicament for degrading EGFR proteins.

In another preferred embodiment, the EGFR-related disease is an EGFR resistance mutation-related disease.

In another preferred embodiment, the EGFR resistance mutation is selected from the group consisting of: EGFRT790M, EGFRC797S, T780M, C797S, DEL19, L858R, DEL19/T790M, DEL10/T790M/C797S, L858R/T790M/C797S, and combinations thereof.

In another preferred embodiment, the EGFR-related disease is selected from the group consisting of: inflammation, cancer, cardiovascular disease, infection, immune disease, and metabolic disease.

In another preferred embodiment, the cancer is selected from the group consisting of: lung cancer (including lung adenocarcinoma, non-small cell lung cancer), breast cancer, prostate cancer, colorectal cancer, liver cancer, pancreatic cancer, ovarian cancer, leukemia, neuroblastoma, gastric cancer, kidney cancer, esophageal cancer, uterine cancer, glioma, head and neck cancer.

In another preferred embodiment, the EGFR-related disease is non-small cell lung cancer with EGFR mutations selected from the group consisting of: T790M/L858R, T790M/L858R/C797S, L858R, L858R/C797S.

In the fourth aspect of the present invention, provided a method for preventing and/or treating EGFR resistance mutation-related diseases, comprising steps of:

1) Determining the EGFR activation mutation status of a patient;
2) If the patient's EGFR activation mutation status meets the treatment criteria, administering the compound according to the first aspect of the present invention to the patient.

In another preferred embodiment, the expression of "patient's EGFR activation mutation status meets the treatment criteria" means that the patient has EGFR mutations selected from the group consisting of: T790M/L858R, T790M/L858R/C797S, L858R and L858R/C797S.

In another preferred embodiment, the EGFR resistance mutation-related disease is selected from the group consisting of: lung cancer (including lung adenocarcinoma, non-small cell lung cancer), breast cancer, prostate cancer, colorectal cancer, liver cancer, pancreatic cancer, ovarian cancer, leukemia, neuroblastoma, stomach cancer, kidney cancer, esophageal cancer, uterine cancer, glioma, and head and neck cancer.

In another preferred embodiment, the EGFR resistance mutation-related disease is non-small cell lung cancer.

In another preferred embodiment, the step of "determining the EGFR activation mutation status of a patient" is determined through the cobas EGFR mutation test v2.

It should be understood that within the scope of the present invention, the above-mentioned technical features of the present invention and the technical features specifically described in the following (such as embodiments) can be combined with each other to form a new or preferred technical solution. Limited to space, it will not be repeated here.

## Description of the drawings

Figure 1 is a schematic diagram of the cell preparation steps in Test Example 2.
Figure 2 is a schematic diagram of the detection steps in Test Example 2.

## Detailed Description of the Invention

After long-term and in-depth research, the inventors of the present invention have unexpectedly prepared a compound with excellent EGFR degradation performance through structural optimization. The compound exhibits excellent inhibitory and/or therapeutic effects on EGFR-related diseases (especially diseases related to EGFR resistance mutations), and also has excellent pharmacokinetic properties. On this basis, the present invention is completed.

**Terms**

In the present invention, unless otherwise specified, the terms used herein have the ordinary meanings known to those skilled in the art.

When the substituent is described by the conventional chemical formula written from left to right, the substituent also includes the chemically equivalent substituent obtained when writing the structural formula from right to left. For example, $-CH_2O-$ is equivalent to $-OCH_2-$.

"Alkyl (alone or as a part of other groups)" means a monovalent straight or branched saturated hydrocarbon group comprising only carbon and hydrogen atoms and containing from 1 to 12 carbon atoms. Alkyl is preferably C1-C6 alkyl group (i.e., containing 1, 2, 3, 4, 5 or 6 carbon atoms). Examples of alkyl include, but are not limited to, methyl, ethyl, propyl, isopropyl, isobutyl, sec-butyl, tert-butyl, pentyl, n-hexyl, octyl, dodecyl, and the like. In the present invention, alkyl is also intended to encompass substituted alkyl, i.e. one or more positions in the alkyl are substituted, in particular by 1- 4 substituents, which may be substituted in any position. "Haloalkyl" refers to an alkyl as defined herein in which one or more of the hydrogens are replaced by the same or different halogens. Examples of haloalkyl include $-CH_2Cl$, $-CH_2CF_3$, $-CH_2CCl_3$, perfluoroalkyl (e.g., $-CF_3$), and the like.

The term "alkylene" refers to a divalent group of an alkyl, such as $-CH_2-$, $-CH_2CH_2-$, and $-CH_2CH_2CH_2-$.

"Alkoxy (alone or as a part of other groups)" means an alkyl to which an oxy group is attached, having an alkyl-O-structure, wherein the alkyl has the definition as described above, preferably, the alkoxy is C1-C6 alkoxy. Alkoxy includes, but is not limited to, methoxy, ethoxy, propoxy, tert-butoxy, and the like. "Haloalkoxy" refers to a group of formula - OR, wherein R is haloalkyl as defined herein. Examples of haloalkoxy include, but are not limited to, trifluoromethoxy, difluoromethoxy, 2,2,2-trifluoroethoxy, and the like.

"Alkenyl (alone or as a part of other groups)" means an aliphatic group containing at least one double bond, typically having from 2 to 20 carbon atoms. In the present invention, "C2-C6 alkenyl" means an alkenyl containing 2, 3, 4, 5 or 6 carbon atoms. Examples of alkenyl include, but are not limited to, vinyl, propenyl, butenyl, 1-methyl-2-buten-1-yl, and the like. In the present invention, alkenyl includes substituted alkenyl.

The term "alkenylene" refers to an alkenyl with two connection sites. For example, "vinylidene" means the group -CH=CH-. Alkenylene may also be in an unsubstituted form or in a substituted form having one or more substituents.

"Alkynyl (alone or as a part of other groups)" means a straight or branched hydrocarbon chain containing more than 2 carbon atoms and characterized by one or more triple bonds, typically having from 2 to 20 carbon atoms. In the present invention, "$C_{2-6}$ alkynyl" means an alkynyl having 2, 3, 4, 5, or 6 carbon atoms. Alkynyl includes, but is not limited to, ethynyl, propynyl, and 3-hexynyl. One of the triple bonded carbons may optionally be the connection site for the alkynyl substituent. In the present invention, the alkynyl also includes a substituted alkynyl.

The term "alkynylene" refers to an alkynyl with two attachment points. For example, "ethynyl" represents a group of -C≡C-. Alkynylene may also be in an unsubstituted form or in a substituted form having one or more substituents.

"Cycloalkyl" refers to a monovalent saturated carbocyclic group comprising a mono- or bicyclic ring having 3- 12, preferably 3- 10, more preferably 3- 8 ring atoms. The cycloalkyl group may optionally be substituted with one or more substituents, wherein each substituent is independently hydroxyl, alkyl, alkoxy, halogen, haloalkyl, amino, monoalkylamino or dialkylamino. Examples of cycloalkyl include, but are not limited to, cyclopropyl, cyclobutyl, cyclopentyl, cyclohexyl, cycloheptyl, and the like.

"Cycloalkoxy" refers to groups having formula -OR, wherein R is a cycloalkyl as defined herein. Exemplary cycloalkyloxy include cyclopropyloxy, cyclobutyloxy, cyclopentyloxy, cyclohexyloxy, and the like. "Cycloalkylalkyl" means (cycloalkyl)-alkyl-wherein cycloalkyl and alkyl are as disclosed herein. "Cycloalkylalkyl" is bonded to the parent molecule structure through the cycloalkyl.

"Heteroaryl" refers to a monocyclic (e.g., 5 or 6-membered), bicyclic (e.g., 8- to 10-membered) or tricyclic group having 5 to 12 ring atoms and containing at least one aromatic ring which comprises 1, 2, or 3 ring heteroatoms selected from N, O and S, with the remaining ring atoms being C. It should be clear that the connection site of the heteroaryl should be located on the aromatic ring. Examples of heteroaryl groups include, but are not limited to: imidazolyl, oxazolyl, isoxazolyl, thiazolyl, isothiazolyl, oxadiazolyl, thiadiazolyl, pyrazinyl, thiophenyl, furanyl, pyranyl, pyridyl, pyrrolyl, pyrazolyl, pyrimidinyl, quinolyl, isoquinolyl, benzo-furyl, benzofuryl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl and the like. Heteroarylene refers to a heteroaryl with two connection sites.

"Heterocyclic system" means monocyclic, bicyclic and polycyclic systems in which at least one ring is saturated or partially unsaturated (but not aromatic) and the ring contains at least one heteroatom. The heterocyclic system may be attached to the side group at any heteroatom or carbon atom, which produces a stable structure and any of the ring atoms may optionally be substituted.

"Heterocyclyl" means a monovalent group of a heterocyclic system, usually a stable monocyclic (e.g., 3- 8 membered, i.e., 3, 4, 5, 6, 7, or 8-membered) or bicyclic (e.g., 5- 12 membered, i.e., 5, 6, 7, 8, 9, 10, 11, or 12-membered)

ring, or a polycyclic ring (e.g., 7- 14 membered, i.e., 7, 8, 9, 10, 11, 12, 13, or 14-membered), including fused, spiro and/or bridged ring structures that are saturated, or partially unsaturated, and contain carbon atoms and 1, 2, 3, or 4 heteroatoms independently selected from N, O, and S. Representative heterocyclyl include the following ring systems, wherein (1) each ring is non-aromatic and at least one ring comprises a heteroatom, e.g., tetrahydrofuranyl, tetrahydropyranyl, tetrahydrothiophenyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl, and quinuclidinyl; (2) at least one ring is non-aromatic and contains heteroatoms, and at least one other ring is an aromatic carbocyclic ring, e.g., 1,2,3,4-tetrahydroquinolinyl, 1,2,3,4-tetrahydroisoquinolinyl; and (3) at least one ring is non-aromatic and contains heteroatoms, and at least one other ring is aromatic and contains heteroatoms, e.g., 3,4-dihydro-1H- pyrano[4, 3-c]pyridine and 1,2,3,4-tetrahydro-2,6-diazanaphthalene. Heterocyclylene are heterocyclyls having two attachment sites. Preferably in the present invention, the heterocyclylene is a bicyclic ring, wherein one ring is heteroaryl and is connected to the rest of the general formula through the heteroaryl. Preferably in the present invention, the heterocyclylene is a 5- 6-membered monocyclic heterocyclylene, or an 8- 10-membered bicyclic heterocyclylene.

"Heterocyclic alkyl" means an alkyl group substituted with a heterocyclyl, wherein heterocyclyl and alkyl are defined as above.

When the substituent is a non-terminal substituent, it is a -ylene form of the corresponding group, e.g., alkyl corresponds to alkylene, cycloalkyl corresponds to cycloalkylene, heterocyclyl corresponds to heterocyclylene, alkoxy corresponds to alkyleneoxy, and the like.

In the present invention, each of the aforementioned alkyl, alkoxy, cycloalkyl, heteroalkyl, aryl, heteroaryl, cycloheteroalkyl, alkenyl, alkynyl, heterocyclic, heterocyclyl, and the like may be substituted or unsubstituted.

For the purposes of the present invention, the term "substituted" refers to one or more hydrogen atoms on a specific group are substituted by a specific substituent. The specific substituent is a substituent described above, or a substituent appearing in the Examples. Unless specifically stated, a specific substituted group may have a substituent selected from a particular group at any substitutable site of the group, and the substituents may be the same or different at various positions. It should be understood by those skilled in the art that the combinations of substituents contemplated by the present invention are those that are stable or chemically realizable. Typical substitutions include but are not limited to one or more of the following groups: such as hydrogen, deuterium, halogen (e.g., monohalogenated or polyhalogenated substituent, the latter such as trifluoromethyl or an alkyl containing $Cl_3$), cyano, nitro, oxo (e.g.=O), trifluoromethyl, trifluoromethoxy, cycloalkyl, alkenyl, alkynyl, heterocyclyl, aryl, $OR_a$, $SR_a$, $S(=O)R_c$, $S(=O)_2R_c$, $P(=O)_2R_c$, $S(=O)_2OR_e$, $P(=O)_2OR_c$, $NR_bR_c$, $NR_bS(=O)_2R_e$, $NR_bP(=O)_2R_c$, $S(=O)_2NR_bR_c$, $P(=O)_2NR_bR_c$, $C(=O)OR_d$, $C(=O)R_a$, $C(=O)NR_bR_c$, $OC(=O)R_a$, $OC(=O)NR_bR_c$, $NR_bC(=O)OR_c$, $NR_dC(=O)NR_bR_c$, $NR_dS(=O)_2NR_bR_c$, $NR_dP(=O)_2NR_bR_c$, $NR_bC(=O)R_a$, or $NR_bP(=O)_2R_c$, wherein, $R_a$ can independently be hydrogen, deuterium, alkyl, cycloalkyl, alkenyl, alkynyl, heterocycle, or aromatic ring, $R_b$, $R_c$ and $R_a$ can independently be hydrogen, deuterium, alkyl, cycloalkyl, heterocycle, or aromatic ring, or Rb and Rc together with N atoms can form a heterocycle; $R_c$ can independently be hydrogen, alkyl, cycloalkyl, alkenyl, alkynyl, heterocycle, or aromatic ring. Exemplary substituents such as alkyl, cycloalkyl, alkenyl, cycloalkenyl, alkynyl, heterocycle or aromatic ring as described above can be optionally substituted. The substituents are, for example (but not limited to): halogen, hydroxyl, cyano, carboxy (-COOH), C1-C6 alkyl, C2-C6 alkenyl, C2-C6 alkynyl, C3-C8 cycloalkyl, 3- to 12-membered heterocyclyl, aryl, heteroaryl, C1-C8 aldehyde, C2-C10 acyl, C2-C10 ester group, amino, C1-C6 alkoxy, C1-C10 sulfonyl, C1-C6 ureido, etc.

"Cyano" means -CN.

"Nitro" means $-NO_2$.

"Hydroxyl" means -OH.

"Amino" means $-NH_2$ or RNH-, wherein R is a ketocarbonyl, sulfonyl, sulfonamido, $R_a$-C(=O)-, $R_aR_b$N-C(=O)-, etc., wherein $R_a$ and $R_b$ are alkyl, cycloalkyl, aryl, or heteroaryl, etc.

"Halogen (halogenated)" means any halogen group, e.g., -F, -Cl, -Br, or -I.

"Deuterated compound" means a compound in which one or more hydrogen atoms (H) are substituted by deuterium atoms (D).

For the purposes of the present invention, the term "more" refers independently to 2, 3, 4 or 5.

## Compound

The present invention provides a compound, the compound is a compound of Formula I, or a pharmaceutically acceptable salt thereof, a stereoisomer, a tautomer, a hydrate, a solvate, an isotopic compound, or a prodrug thereof,

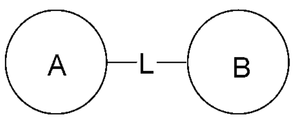

Formula I

wherein, each group is as defined above.

In another preferred embodiment, any one of the respective groups in the compound is independently the corresponding group in the specific compound, respectively.

As used herein, the term "pharmaceutically acceptable salt" refers to a salt formed by the compound of the invention with an acid or base that is suitable for use as a drug. Pharmaceutically acceptable salts include inorganic and organic salts. A preferred class of salts are those formed by the compounds of the present invention with acids. Acids suitable for forming salts include, but are not limited to: inorganic acids such as hydrochloric, hydrobromic, hydrofluoric, sulphuric, nitric, and phosphoric acids, etc.; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulphonic acid, ethanesulfonic acid, p-toluenesulphonic acid, benzenesulphonic acid, naphthalenesulphonic acid, etc.; and amino acids such as proline, phenylalanine, aspartic acid, glutamic acid, etc.

Another preferred class of salts are salts formed by the compounds of the present invention with a base, such as alkali metal salts (e.g., sodium or potassium salts), alkaline earth metal salts (e.g., magnesium or calcium salts), ammonium salts (e.g., lower alkanolammonium salts, and other pharmaceutically acceptable amine salts), such as methylamine salts, ethylamine salts, propylamine salts, dimethylamine salts, trimethylamine salts, diethylamine salts, triethylamine salts, tert-butylamine salts, ethylenediamine salts, hydroxyethylamine salts, dihydroxyethylamine salts, trihydroxyethylamine salts, and amine salts formed from morpholine, piperazine, and lysine, respectively.

The term "solvate" refers to a complex formed by the coordination of the compound of the present invention with solvent molecules in a specific ratio. "Hydrate" refers to a complex formed by the coordination of the compound of the present invention with water.

In addition, the compound of the present invention also includes prodrug of the compound of Formula I. The term "prodrug" includes a compound which itself may be biologically active or inactive, but which, when administered by suitable means, undergoes metabolism or chemical reactions *in vivo* to yield a compound of formula I, or a salt or solution consisting of a compound of formula I. The prodrug includes, but are not limited to, carboxylate ester, carbonate ester, phosphate ester, nitrate ester, sulfate ester, sulfone ester, sulfoxide ester, amine compound, carbamate, azo compound, phosphoramide, glucoside, ether, acetaldehyde acetal of the compound, and the like .

The compounds, salts, or solvates of the present invention, may exist in tautomeric forms (e.g., amides and imine ethers). All of these tautomers are part of the present invention.

All stereoisomers of the compounds (e.g., those asymmetric carbon atoms that may exist as a result of various substitutions), including both their enantiomeric and diastereomeric forms, are within the contemplated scope of the present invention. The individual stereoisomer of the compound of the present invention may be present free of other isomers (e.g., as a pure or substantially pure optical isomer with specific activity), or may also be present as mixtures, such as racemates, or as mixtures with all other stereoisomers, or portions thereof. The chiral centers of the present invention have the S or R configuration, as defined by the 1974 recommendations of the International Union of Pure and Applied Chemistry (IUPAC). The racemic forms may be resolved by physical methods, such as fractional crystallization, or by crystallization of their corresponding diastereomeric derivatives, or by separation via chiral column chromatography. Individual optical isomers may be obtained from the racemic form by suitable methods including, but not limited to, conventional methods, such as, salt formation with optically active acids followed by recrystallization.

The compounds of the present invention, as obtained sequentially by preparation, separation and purification of the compounds have a purity by weight equal to or greater than 90%, e.g., equal to or greater than 95%, equal to or greater than 99% ("very pure" compounds) as listed in the description. Such "very pure" compounds of the present invention are herein also included as part of the present invention.

All configurational isomers of the compounds of the present invention are encompassed within the scope of this invention, whether as mixture, or in pure or very pure forms. The definition of the compounds of the present invention encompasses both the cis (Z) and trans isomers of alkenes, as well as the cis and trans isomers of carbocycles and heterocycles..

Throughout the specification, groups and substituents may be selected to provide stable fragments and compounds.

Specific functional groups and definitions of chemical terms are detailed below. For the purposes of the present invention, the chemical elements correspond to those defined in Periodic Table of the Elements, CAS version, Handbook of Chemistry and Physics, 75th Ed. Definitions of specific functional groups are also described therein. In addition, the basic principles of organic chemistry, as well as specific functional groups and reactivity, are described in "Organic Chemistry," Thomas Sorrell, University Science Books, Sausalito: 1999, which is incorporated by reference in its entirety.

Certain compounds of the present invention may exist in specific geometric or stereoisomeric forms. The present invention covers all compounds, including their cis and trans isomers, R and S enantiomers, diastereomers, (D)-isomers, (L)-isomers, racemic mixtures and other mixtures. Additionally, the asymmetric carbon atom may represent a substituent, such as alkyl. All isomers, as well as mixtures thereof, are encompassed within the present invention.

Mixtures of isomers in accordance with the present invention may contain isomers in various ratios. For example,

A mixture of two isomers may be presnet in ratios such as: 50:50, 60:40, 70:30, 80:20, 90:10, 95:5, 96:4, 97:3, 98:2, 99:1, or 100:0. All such ratios of isomers are within the scope of the present invention. Similar ratios, which are readily understood by one of ordinary skill in the art, and ratios for mixtures of more complex isomers, are also intended to be within the scope of the present invention.

The present invention also includes isotopically labeled compounds, equivalent to the compounds as disclosed herein, but for the fact that one or more atoms are replaced by an atom having an atomic mass or mass number different from the atomic mass or mass number usually found in nature. Examples of isotopes that may be classified as isotopes of the compounds of the present invention include hydrogen, carbon, nitrogen, oxygen, phosphorus, sulphur, fluorine and chlorine isotopes, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$, and $^{36}Cl$, respectively. Compounds of the present invention, or enantiomers, diastereomers, isomers, or pharmaceutically acceptable salts, or solvates thereof, which contains isotopes or other isotopic atoms of the above-mentioned compounds are within the scope of the present invention. Certain isotopically labeled compounds of the present invention, e.g., the radioisotopes of $^{3}H$ and $^{14}C$ are also included and are useful in tissue distribution experiments of drugs and substrates. Tritium, i.e.,$^{3}H$ and carbon-14, i.e.,$^{14}C$, which are relatively easy to prepare and detect are preferred among the isotopes. In addition, substitutions with heavier isotopes, such as deuterium, i.e., $^{2}H$, can afford certain therapeutic advantages resulting from greater metabolic stability, for example, increased *in vivo* half-life, or reduced dosage requirements, and may therefore be preferred in some circumstances. Isotopically labeled compounds can be prepared in a general way by carrying out the procedures disclosed in the examples herein, by substituting a readily available isotopically labeled reagent for a non-isotopically labeled reagent.

If the synthesis of a specific enantiomer of the compound of the invention is to be devised, it can be prepared by asymmetric synthesis, or derivatized with a chiral auxiliary, the resulting diastereomeric mixture can be separated, and the chiral auxiliary can then be removed to obtain the pure enantiomer. Alternatively, if a molecule contains a basic functional group, such as an amino acid, or an acidic functional group, such as a carboxyl group, a suitable optically active acid or base can be used to form a diastereoisomeric salt with it, which can then be separated by conventional means, such as by separation and crystallization, or by chromatography, and the pure enantiomer is then obtained.

As described herein, the compounds of the present invention may be expanded by the addition of any number of substituents or functional groups. Generally, the term "substituted", whether preceded or followed by the term "optionally", (the formula of the present invention includes formula of substituents), refers to the substitution of a hydrogen atom in a given structure with a specified substituent. When a plurality of positions in a given structure are replaced by a plurality of specific substituents, the substituent at each position may be the same or different. The term "substituted" as used herein includes all permissible substitutions of organic compounds. Broadly speaking, permissible substituents include acyclic, cyclic, branched and unbranched, carbocyclic and heterocyclic, aromatic and non-aromatic organic compounds. For the purposes of the present invention, e.g., a heteroatom, such as nitrogen, may have hydrogen substituents or any of the permissible organic compounds described above to supplement its valence. Furthermore, the present invention is not intended to limit in any way the permissible substitution of organic compounds. The present invention contemplates that combinations of substituents and variable groups which results in stable compounds are desirable in the treatment of diseases. The term "stable" herein refers to a stable compound that is sufficiently robust to maintain its structural integrity for a period of time sufficient for detection, and preferably, for a period of time sufficient to be effective for the purposes described herein.

Metabolites of the compounds of the present invention and pharmaceutically acceptable salts thereof, as well as prodrugs that can be transformed in vivo to the structures of the compounds of the present invention and pharmaceutically acceptable salts thereof, are also included in the scope of the claims of the present invention.

It should be understood that each example of the present invention specifically describes the preparation method of the compound of formula I of the present invention, but these specific methods do not constitute any limitations on the present invention. The compounds of the present invention can also be conveniently made by optionally combining various synthetic methods described in this specification or known in the art, and such combinations can be readily carried out by those skilled in the field to which the present invention belongs.

Typically, unless otherwise specified, all raw materials and reagents used in the preparation process of the compounds of the present invention are commercially available.

Typically, in the preparative process, each reaction is usually carried out under inert gas, in a suitable solvent, at 0 to 150 °C, and the reaction time is usually 2- 24 hours.

**Pharmaceutical composition and mode of administration**

The present invention also provides a pharmaceutical composition, which comprises the compound and a pharmaceutically acceptable carrier.

Due to the excellent anti-tumor activity of the compounds of the present invention, the compounds of the present invention, and various crystal forms thereof, pharmaceutically acceptable inorganic or organic salts, hydrates or solvates

thereof, as well as pharmaceutical compositions containing the compounds of the present invention as the main active ingredient can be used for the treatment, prevention and alleviation of tumor-related diseases.

The pharmaceutical compositions of the present invention comprise the compound of the present invention or the pharmaceutically acceptable salt thereof within the safe and effective amount range, and a pharmaceutically acceptable excipient or carrier. In which, "safe and effective amount" means that the amount of the compound is sufficient to significantly improve the condition without causing serious side effects. Generally, the pharmaceutical composition contains 1-2000 mg of the compounds of the present invention/dose, more preferably, 10-1000 mg of the compounds of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc..

The pharmaceutical composition can be in the form of injections, capsules, tablets, pills, powders, or granules.

There is no special limitation on the administration mode for the compound or pharmaceutical compositions of the present invention, and the representative administration mode includes (but is not limited to): oral, intratumoral, rectal, parenteral (intravenous, intramuscular or subcutaneous), and topical administration.

The dosage forms of the pharmaceutical compositions of the present invention include (but are not limited to): injections, tablets, capsules, aerosols, suppositories, films, pills, liniments, controlled-release or sustained-release or nano-formulations.

Solid dosage forms for oral administration include capsules, tablets, pills, powders and granules. In these solid dosage forms, the compound is mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants, such as talc, calcium stearate, magnesium stearate, solid polyethylene glycol, sodium lauryl sulfate, or mixtures thereof. In capsules, tablets, and pills, the dosage form may also include a buffer.

Solid dosage forms such as tablets, sugar pills, capsules, pills, and granules can be prepared using coating and shell materials, such as casings and other well-known materials in the art. They can contain an opaque agent, and the active compound or compound in the composition can be released in a delayed mode in a given portion of the digestive tract. Examples of embedding components that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers, suspending agents, sweeteners, corrigents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders for re-dissolution into sterile injectable solutions or dispersions. Suitable aqueous and non-aqueous carriers, diluents, solvents or excipients include water, ethanol, polyols, and their suitable mixtures.

The dosage forms of the compound of the present invention for topical administration include ointments, powders, patches, sprays, and inhalants. The active ingredients are mixed under sterile conditions with physiologically acceptable carriers and any preservatives, buffers, or propellants that may be necessary.

The compound of the present invention can be administered alone, or in combination with other pharmaceutically acceptable other compounds (such as anti-tumor drugs).

The treatment method of the present invention can be administered alone, or in combination with other treatment methods or drugs.

The compound of formula I can be administered in combination with other drugs known to treat or ameliorate similar medical conditions. When administered in combination, the mode of administration and dosage of the known drug may be unchanged, while the compound of formula I is administered simultaneously or subsequently. When the compound of formula I of the present invention is administered in combination with one or more other drugs, a pharmaceutical composition containing both one or more known drugs and the compound of formula I may preferably be used. Pharmaceutical combination also includes the administration of a compound of Formula I with one or more other known drugs during overlapping time periods. When the compound of formula I is administered in combination with one or more other drugs, the dose of the compound of formula I or the known drug may be lower than their respective doses when administered alone.

The drugs or active ingredients that can be used in combination with the compound of formula I include, but are not limited to: gefitinib, erlotinib, icotinib, lapatinib, XL647, NVP-AEE-788, ARRY-334543, vandetanib, PF00299804, Cetuximab, Panitumumab, pertuzumab, zalutumumab, nimotuzumab, MDX-214, CDX-110, IMC-11F8, CNF2024, tanespimycin, alvespimycin, IPI-504, NVP-AUY922.

When the pharmaceutical composition is used, a safe and effective amount of the compound of the present invention is administered to a mammal (such as a human or a rat) in need of treatment, wherein the dosage at the time of administration is the pharmaceutically effective dosage, and for people having a body weight of 60kg, the daily dose is usually 1~2000 mg, preferably 50~1000 mg. Of course, for the specific dosage, factors, such as the route of administration and the patient's health status, which are within the skill range of a skilled physician, should also be considered.

Compared with the prior art, the main advantages of the present invention include:

(1) The compounds of the present invention exhibit excellent EGFR degradation performance;
(2) The compounds of the present invention have excellent inhibitory and/or therapeutic effects on EGFR-related diseases, especially diseases with EGFR resistance mutations, including but not limited to diseases selected from the group consisting of: H1975 (T790M/L858R), HCC827 (19DEL), and PC-9 (19DEL);
(3) The compounds of the present invention have excellent pharmacokinetic properties;
(4) The compounds of the present invention are suitable for preventing and/or treating diseases selected from the group consisting of: EGFR-sensitive mutant cancers, and diseases with secondary drug resistance arising from current EGFR treatments;
(5) The compounds of the present invention have excellent safety.

The present invention will be further described below in conjunction with specific embodiments. It is to be understood that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and parts are calculated by weight.

Unless otherwise defined, all professional and scientific terms used herein have the same meanings as commonly understood by those skilled in the art. In addition, any methods and materials similar or equivalent to those described can be applied to the method of the present invention. The preferred embodiments and materials described herein are for exemplary purposes only.

**Example 1**

The compound synthesized in the present invention:

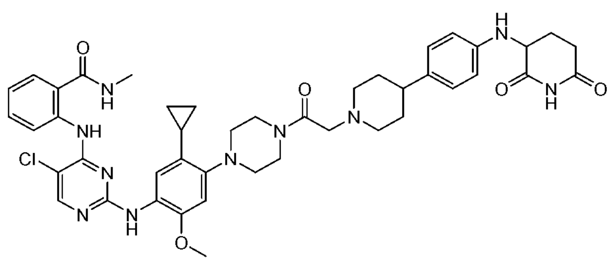

T-001

The experimental procedure is as follows:

I. Synthesis of Intermediate M1

The synthesis route is as follows:

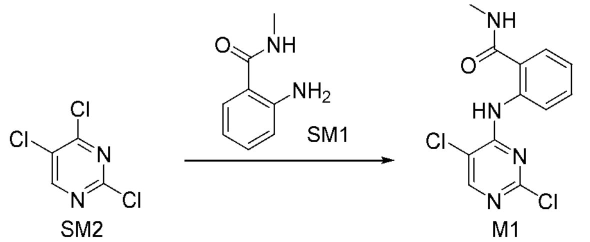


Synthesis of intermediate SM1

M1: To a 100 mL single-necked flask was added 10 g (1.0 eq) of Compound SM1, a mixture of 43g (5.0 eq) of N, N-diisopropylethylamine (DIPEA) and 100 mL of isopropanol was added, and the mixture was stirred uniformly at room temperature. Then 12.2 g (1.0 eq) of Compound SM2 was slowly added dropwise. After the addition was completed, the reaction was purged with nitrogen three times. Under nitrogen protection, the temperature was raised to reflux, and the reaction was stirred overnight. TLC analysis monitored the completion of the reaction. The solvent was evaporated to dryness under reduced pressure, and 50 mL of ethyl acetate (EA) was added to the residue for slurry at room temperature for 30 minutes. The mixture was suction-filtered, and the filter cake was rinsed twice with 15 mL of ethyl acetate. The collected filter cake was Intermediate M1 (17.4 g).

II. Synthesis of Intermediate M2

The synthesis route is as follows:

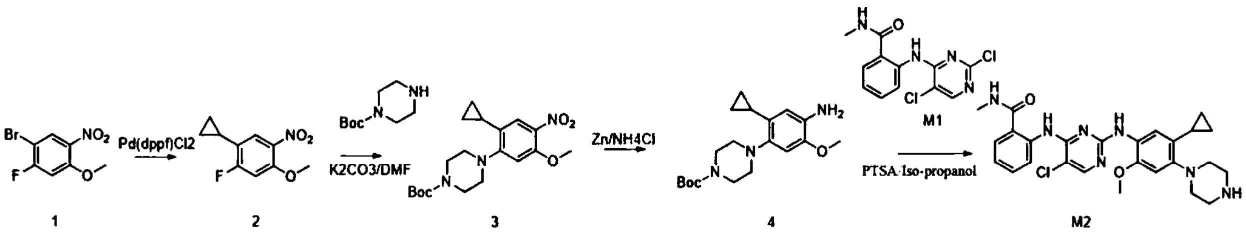


1. Synthesis of compound 2

In a 100 mL three-necked flask, 200 mg of Compound 1 (1.0 eq), 138 mg of cyclopropylboronic acid (2.0 eq), 167 mg of potassium carbonate (1.5 eq), 4 mL of 1,4-dioxane, and 0.8 mL of water were mixed uniformly, and the mixture was protected under nitrogen atmosphere. Then 70 mg of [1,1'-bis(diphenylphosphino)ferrocene]dichloropalladium (0.12 eq) was added. The temperature was raised to 100 °C, and the reaction was carried out overnight. The completion of the reaction was monitored by TLC, then the mixture was filtered through a diatomaceous earth pad, and the mother liquor was collected. The mother liquor was rotary-evaporated to remove the solvent. Subsequently, 30 mL of water was added, and the mixture was extracted three times with 15 mL of ethyl acetate each time. The combined organic phases were dry loaded for sample application, and then subjected to column chromatography to obtain 150 mg of Compound 2. LC-MS [M+1]: 212.

2. Synthesis of compound 3

To a 50 mL single-necked flask was added 120 mg of Compound 2 (1.0 eq), 160 mg of N-Boc piperazine (1.5 eq), 235 mg of potassium carbonate (3.0 eq), and 2.5 mL of N,N-dimethylformamide. Under nitrogen protection, the temperature was raised to 110 °C, and the reaction was carried out overnight. The completion of the reaction was monitored by TLC, then the reaction solution was extracted with ethyl acetate, and the organic phase was dried over anhydrous sodium sulfate, spun-dried, and subjected to column chromatography to obtain 100 mg of Compound 3. LC-MS[M+1]: 478.

3. Synthesis of compound 4

In a 50 mL round-bottom flask, Compound 3 (100 mg, 1.0 eq) was dissolved in 3 mL of ethanol. Then zinc powder (82 mg, 6.0 eq.), ammonium chloride (112 mg, 10.0 eq.), and water (0.6 mL) were added. The reaction mixture was refluxed at 90 °C overnight. The completion of the reaction was monitored by LCMS. The reaction mixture was directly mixed with 500 mg of silica gel (100-200 mesh) for sample application, and then purified by column chromatography (EA in PE from 0% to 75%) to obtain the target product 4 (60 mg, yellow oil). LC-MS[M+1]: 348.

4. Synthesis of intermediate M2

To a 50 mL single-necked flask was added Compound 4 (60 mg, 1.0 eq), intermediate M1 (51 mg, 1.0 eq), p-toluenesulfonic acid monohydrate (40 mg, 1.2 eq), and 4 mL of isopropanol. The mixture was uniformly mixed, heated to reflux, and reacted overnight. The completion of the reaction was monitored by LCMS. The reaction solution was adjusted to pH > 7 with a saturated aqueous sodium bicarbonate solution, and extracted with ethyl acetate. The organic phase was washed with saturated saline and dried over anhydrous sodium sulfate, then mixed with 500 mg of silica gel (100-200 mesh) for sample application, and purified by column chromatography (MeOH in DCM from 0% to 20%) to obtain the target product M2 (50 mg, brown solid). LC-MS[M+1]: 508.

III. Synthesis of Intermediate M3

The synthesis route is as follows:

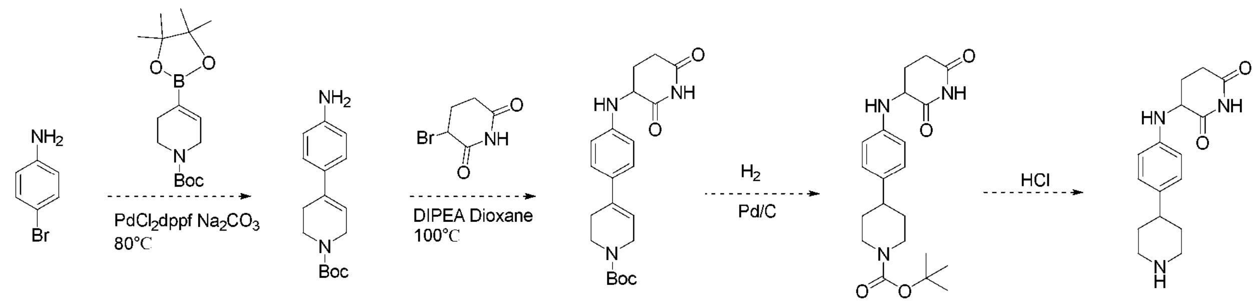


1 2 3 4 5

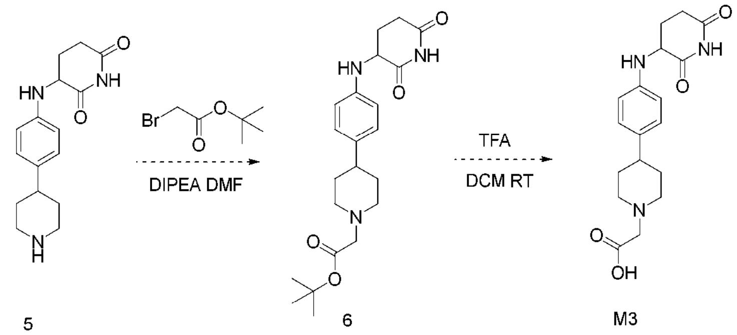


5 6 M3

1. Synthesis of compound 2

In a 250 mL three-necked flask, 5 g of Compound 1, 10.8 g of N-Boc-1,2,5,6-tetrahydropyridin-4-yl boronic acid pinacol ester, 60 mL of tetrahydrofuran, 24 mL of methanol, 12 mL of water, 6.8 g of sodium carbonate, and 0.43 g of $PdCl_2$ (dppf) were mixed uniformly. After purging with nitrogen, the temperature was raised to 80 °C, and the reaction was carried out overnight. The completion of the reaction was monitored by TLC, then the reaction was extracted with ethyl acetate, dried over anhydrous sodium sulfate, spun-dried, and separated by column chromatography to obtain 5.8 g of Compound 2. LC-MS[M+1]: 275.

2. Synthesis of compound 3

In a 100 ml three-necked flask, 5.1 g of compound 2, 7.1 g of 3-bromopiperidin-2,6-dione, 7.2 g of N, N-diisopropylethylamine were dissolved in 51 ml of 1,4-dioxane, then the reaction was warmed up to 100 °C and reacted overnight. The completion of the reaction was monitored by TLC, then the reaction solution was extracted with dichloromethane, dried with anhydrous sodium sulfate, spun-dried and separated by column chromatography to give 3.6 g of compound 3. LC-MS[M+1]: 386.

3. Synthesis of compound 4

To an autoclave reactor was added 3.6 g of compound 2, 0.72 g of palladium carbon, and 36 ml of anhydrous ethanol, and charged with hydrogen gas at 0.7 MPa. Then the mixture was reacted for two days, filtered, and rotary-evaporated to remove the solvent to obtain 1.2 g of compound 4. LC-MS[M+1]: 388.

4. Synthesis of compound 5

To a 100 ml single-necked flask was added 1.2 g of compound 4, and 30 ml of methanol was added to dissolve it. Then 4M solution of hydrochloric acid in dioxane was added dropwise under a cooling ice-water bath. After dropwise addition, the reaction was carried out at 40 °C for 2 h, and rotary evaporated to remove the solvent to give 1.4 g of compound 5. LC-MS[M+1]: 288.

5. Synthesis of compound 6

To a 100ml single-necked flask was added 0.8g of compound 5, 8ml of N,N-dimethylformamide, N,N-diisopropylethylamine, and tert butyl bromoacetate, and stirred overnight at room temperature. The completion of the reaction was monitored by TLC, and then the reaction solution was extracted with ethyl acetate and water. The organic phase was dried and filtered, and then subjected to column chromatography to obtain 300mg of compound 6, LC-MS[M+1]: 402.

6. Synthesis of compound M3

To a 100 ml single-necked vial was added 100 mg of compound 6, 1 ml of dichloromethane, and 1.6 ml of trifluoroacetic acid dropwise, and stirred at room temperature overnight. The completion of the reaction was monitored by TLC, and then the reaction solvent was rotary evaporated to remove the solven to obtain 150 mg of M3, LC-MS [M-1]: 344.

VI. Synthesis of compound T-001

The synthesis route is as follows:

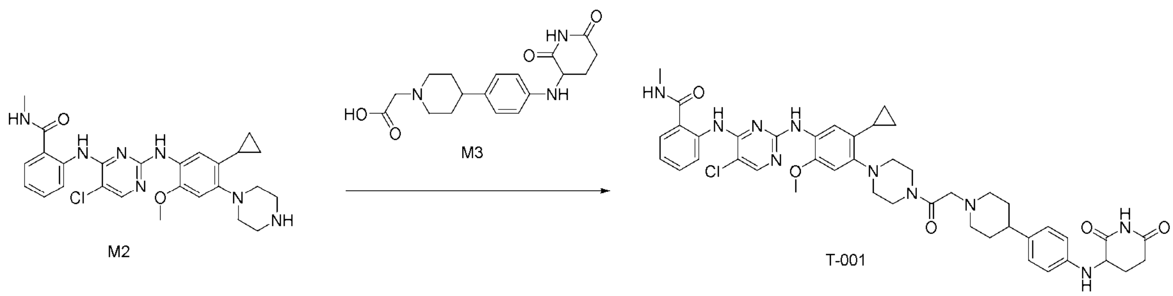


Compound M2 (50 mg, 1.0 eq) and M3 (70 mg, 1.0 eq) were dissolved in 2 mL of N,N-dimethylformamide in a 50 mL round-bottom flask. The mixture was cooled to 0 °C in an ice-water bath, and N,N-diisopropylethylamine (0.2 mL, 10.0 eq) and HATU (50 mg, 1.3 eq) were added under stirring. The reaction was carried out in the ice-water bath for 1 hour under nitrogen protection, then allowed to warm up to room temperature naturally. The completion of the reaction was monitored by TLC, then the reaction solution was extracted with ethyl acetate and water; the organic phase was dried and filtered, and subjected to column chromatography to obtain 15 mg of Compound T-001. LC-MS [M+1]: 833. HNMR: 1H NMR (400 MHz, DMSO-d6) δ 11.67 (s, 1H), 10.79 (s, 1H), 8.74 (q, J = 4.6 Hz, 1H), 8.65 - 8.45 (m, 1H), 8.25 (s, 1H), 8.12 (s, 1H), 7.71 (dd, J = 8.0, 1.6 Hz, 1H), 7.27 (d, J = 8.1 Hz, 1H), 7.04 (t, J = 7.6 Hz, 1H), 6.73 (s, 1H), 6.62 (d, J = 8.1 Hz, 2H), 5.71 (d, J = 7.7 Hz, 1H), 4.37 (t, J = 5.1 Hz, 0H), 4.32 - 4.22 (m, 1H), 3.75 (s, 3H), 3.71 (s, 5H), 3.50 - 3.39 (m, 5H), 3.17 (d, J = 5.1 Hz, 0H), 3.05 (t, J =

4.8 Hz, 2H), 2.98 (s, 2H), 2.83 - 2.67 (m, 4H), 2.60 (ddd, J = 17.6, 14.1, 4.4 Hz, 1H), 2.22 (td, J = 8.4, 4.4 Hz, 1H), 2.09 (dq, J = 13.4, 4.7 Hz, 1H), 1.85 (ddd, J = 25.7, 12.8, 8.0 Hz, 7H), 1.06 (t, J = 7.0 Hz, 1H), 0.88 (dd, J = 8.2, 5.5 Hz, 2H), 0.53 (q, J = 5.1, 4.6 Hz, 2H).

**Example 2**

The compound synthesized in the present invention:

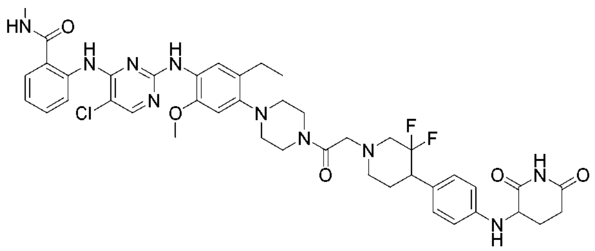

T-009

The experimental procedure is as follows:

I. Synthesis of Intermediate M1

The synthesis route is as follows:

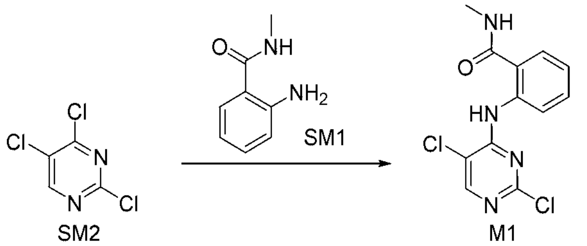
SM1
SM2
M1

Intermediate M1 was synthesized with reference to the method of Example 1

II. Synthesis of Intermediate M2

The synthesis route is as follows:

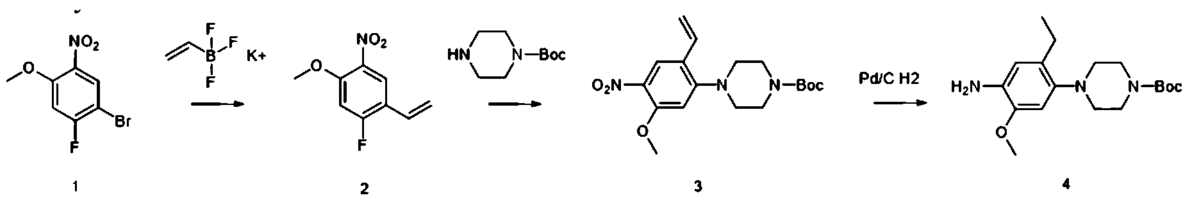

1
2
3
4

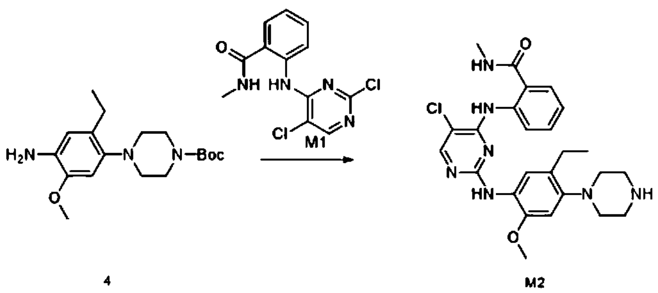
M1
4
M2

1. Synthesis of compound 2

In a 100 mL three-necked flask, 500 mg of Compound 1 (1.0 eq.), 536 mg of potassium vinyltrifluoroborate (2.0 eq.), 165 mg of potassium carbonate (1.5 eq.), 5 mL of 1,4-dioxane, and 1 mL of water were mixed uniformly, and the mixture was protected with nitrogen. 175mg of [1,1'-bis(diphenylphosphino)ferrocene]palladium(II) chloride (0.12 eq.) was added, and the mixture was heated to 100 °C to react overnight. The reaction was monitored by TLC until completion, then filtered through a diatomaceous earth pad, and the mother liquor was collected. The solvent was removed from the mother liquor by rotary evaporation, after which 30 mL of water, and 15 mL of ethyl acetate were added for three extractions. The organic phases were were combined and dry loaded for sample application, then subjected to column chromatography to obtain 320 mg of Compound 2.

2. Synthesis of compound 3

In a 100 mL single-necked flask, 320 mg of Compound 2, 600 mg of N-Boc piperazine, and 666 mg of potassium carbonate were dissolved in 30 mL of DMF. The mixture was heated to 110 °C and reacted for 18 hours. The solvent was removed by rotary evaporation; water was added to the residue, and the mixture was extracted with ethyl acetate. The organic phase was dried over anhydrous sodium sulfate, spun-dried and subjected to column chromatography to obtain 400 mg of Compound 3. LC-MS[M+1]: 366.

3. Synthesis of compound 4

In a 100 mL single-necked flask, 300 mg of Compound 3 and 15 mg of palladium on carbon were added to 5 mL of methanol. The mixture was reacted at room temperature for 18 hours, and then filtered through a diatomaceous earth pad. The mother liquor was collected, dried over anhydrous sodium sulfate, spun-dried and subjected to column chromatography to obtain 250 mg of Compound 4 as a brown solid. LC-MS[M+1]: 336.

4. Synthesis of intermediate M2

Compound 4 (223 mg, 1.0 eq), intermediate M1 (250 mg, 1.0 eq), p-toluenesulfonic acid monohydrate (156 mg, 1.2 eq), and 4 mL of isopropanol were added into a 50 mL single-necked flask. The mixture was uniformly mixed and heated to reflux overnight, and the completion of the reaction was monitored by LCMS. Saturated aqueous sodium bicarbonate solution was added to the reaction solution to adjust the pH to >7. The mixture was extracted with ethyl acetate; the organic phase was washed with saturated saline, dried over anhydrous sodium sulfate, then mixed with of silica gel (100-200 mesh) for sample application and purified by column chromatography (methanol in dichloromethane from 0% to 20%) to obtain the target product M2 (130 mg, brown solid). LC-MS[M+1]: 496.

III. Synthesis of Intermediate M3

The synthesis route is as follows:

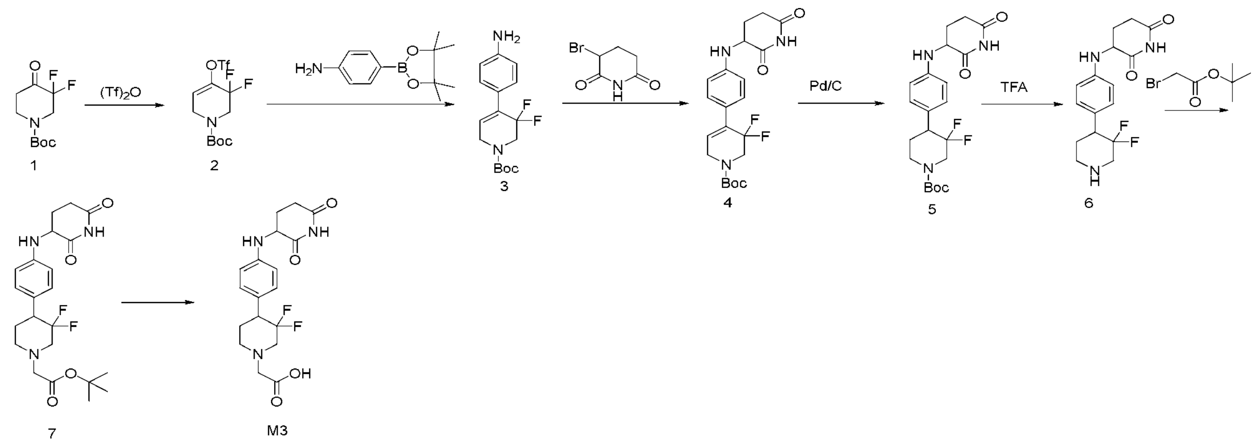


1. Synthesis of compound 2

In a 50 mL three-necked flask, Compound 1 (2.0 g, 8.5 mmol, 1.0 eq) and TEA (2.58 g, 25.5 mmol, 3.0 eq) were

dissolved in 20 mL of DMF, and the solution was cooled to 0 °C. Triflic anhydride ($(Tf)_2O$, 3.6 g, 12.76 mmol, 1.5 eq) was added to the mixture. The reaction solution was allowed to warm up to room temperature naturally and stirred overnight. The completion of the reaction was monitored by TLC (with potassium permanganate staining). The reaction solution was spun-dried, and the crude product was subjected to column chromatography (100% PE) to obtain crude Compound 2 as a yellow solid (841 mg, 45.6% yield). LC-MS[M+1]: 368. 1H NMR (400 MHz, DMSO-d6)$\delta$ 6.84 (s, 1H), 4.24 (q, J = 4.4Hz, 3H), 4.05 (t, J = 11.3 Hz,3H), 1.43 (s, 12H).

2. Synthesis of compound 3

To a 50 mL three-necked flask was added 4-aminophenylboronic acid pinacol ester (367 mg, 3.16 mmol, 1.2 eq), Compound 2 (700 mg, 1.9 mmol, 1.0 eq), and sodium carbonate (607 mg, 5.73 mmol, 3.0 eq). After purging with $N_2$ gas three times, 7 mL of dioxane and 1.75 mL of water were added. The mixture was purged with $N_2$ gas three more times, followed by the addition of $Pd(dppf)Cl_2$ (140 mg, 0.19 mmol, 0.1 eq). The reaction solution was stirred at 55 °C for 2 hours under $N_2$ gas protection. TLC analysis showed that the starting material had disappeared and a new spot had formed. The reaction solution was filtered, and the filtrate was spun-dried. The crude product was subjected to column chromatography (EA in PE from 0% to 40%) to obtain Compound 3 as a white solid (230 mg, 32.4% yield). LC-MS[M+1]: 311.

3. Synthesis of compound 4

In a 50 mL round-bottom flask, Compound 3 (230 mg, 0.74 mmol, 1.0 eq) and 3-bromopiperidine-2,6-dione (171 mg, 0.89 mmol, 1.2 eq) were dissolved in 2.3 mL of N,N-dimethylformamide, and sodium bicarbonate (125 mg, 0.65 mmol, 2.0 eq) was added to the mixture. The reaction solution was stirred at 70 °C for 16 hours under nitrogen protection. After cooling to room temperature, the completion of the reaction was monitored by TLC. The reaction solution was then diluted with EA (10 mL), and then 10 mL of water was added for partitioning. The aqueous phase was extracted with 10 mL of EA. The combined organic phases were dried over anhydrous sodium sulfate and filtered. The crude product was purified by column chromatography (EA in PE from 0% to 50%) to obtain Compound 4 (60 mg, 19% yield). LC-MS[M+1]: 422.

4. Synthesis of compound 5

In a 500 mL round-bottom flask, Compound 4 (70 mg, 0.16 mmol, 1.0 eq) was dissolved in 1 mL of methanol, and palladium on carbon (Pd/C, 7 mg) was added to the mixture. The reaction solution was stirred at room temperature for 16 hours under a hydrogen ($H_2$) balloon atmosphere. The completion of the reaction was monitored by LCMS, and then the reaction solution was filtered to obtain crude Compound 5 (70 mg, 100% crude yield). LC-MS[M+1]: 424.

5. Synthesis of compound 6

In a 50 mL round-bottom flask, Compound 5 (70 mg, 0.16 mmol, 1.0 eq) was dissolved in 2 mL of dichloromethane (DCM), and 0.5 mL of trifluoroacetic acid (TFA) was added to the mixture. The reaction solution was stirred at room temperature for 2 hours. The completion of the reaction was monitored by LCMS. The reaction solution was then evaporated to dryness to obtain Compound 6 (54 mg, 100% crude). LC-MS[M+1]: 324.

6. Synthesis of compound 7

In a 50 mL round-bottom flask, Compound 6 (54 mg, 0.17 mmol, 1.0 eq) and tert-butyl bromoacetate (40 mg, 0.2 mmol, 1.2 eq) were dissolved in 1 mL of N,N-dimethylformamide, and DIPEA(65 mg, 0.5 mmol, 3.0 eq) was added to the mixture. The reaction solution was stirred at room temperature for 12 hours. The completion of the reaction was monitored by TLC, the reaction solution was then diluted with EA (5 mL), and 5 mL of water was added for partitioning. The aqueous phase was extracted with 5 mL of EA. The combined organic phases were dried over anhydrous sodium sulfate and filtered. The crude product was purified by column chromatography (EA in PE from 0% to 60%) to obtain Compound 7 (45 mg, 61.6% yield). LC-MS[M+1]: 438.

7. Synthesis of compound M3

In a 50 mL round-bottom flask, Compound 7 (45 mg, 0.1 mmol, 1.0 eq) was dissolved in 2 mL of dichloromethane (DCM), and 0.5 mL of trifluoroacetic acid (TFA) was added to the mixture. The reaction solution was stirred at room temperature for 2 hours. The completion of the reaction was monitored by LCMS. The reaction solution was then evaporated to dryness to obtain Compound M3 (45 mg, 100% crude). LC-MS[M+1]: 382.

III. Synthesis of compound T-009

The synthesis route is as follows:

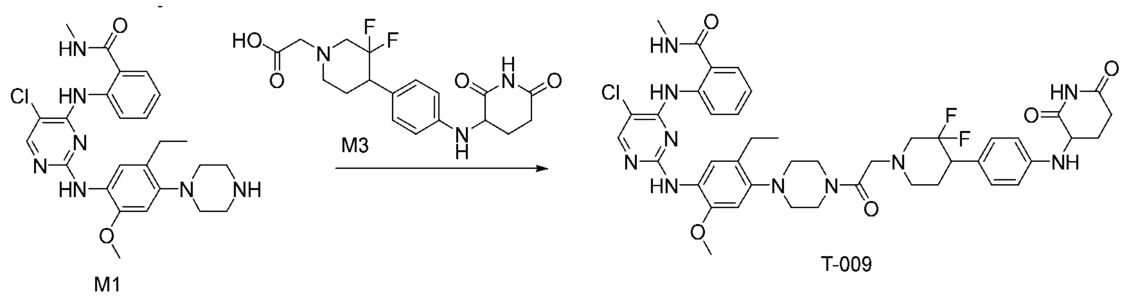


M1

T-009

In a 50 mL round-bottom flask, Compound M2 and Compound M3 were dissolved in 2 mL of N, N-dimethylformamide. The mixture was cooled to 0 °C in an ice-water bath, and N,N-diisopropylethylamine and HATU were added under stirring. The reaction was carried out in the ice-water bath for 1 hour under nitrogen protection, and then allowed to warm up to room temperature naturally. The completion of the reaction was monitored by TLC, then the reaction solution was extracted with ethyl acetate and water; the organic phase was dried and filtered, and subjected to column chromatography to obtain 100 mg of Compound T-009. LC-MS [M+1]: 860.

The following compounds were synthesized with reference to the method of Example 2:

| Compound No. | Structure | Characterization data: |
|---|---|---|
| T-078 | 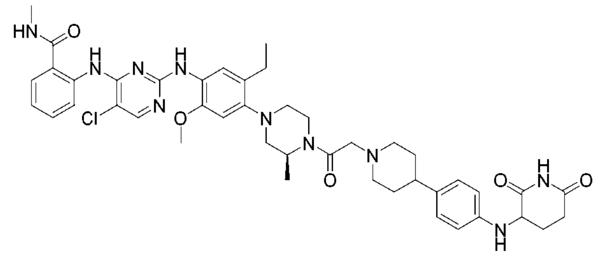 | LC-MS[M+1]: 837.8 |
| T-079 | 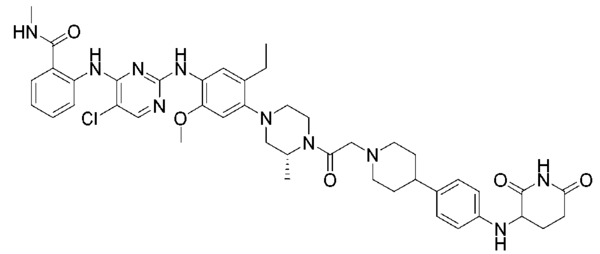 | LC-MS[M+1]:837.8 |
| T-080 | 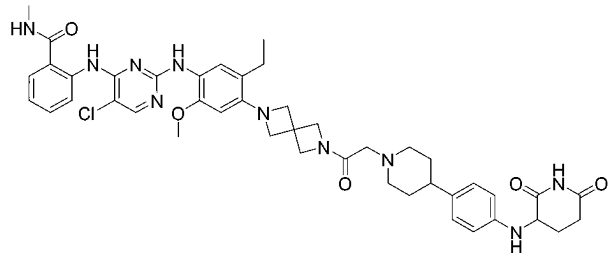 | LC-MS[M+1]: 835.6<br>1H NMR (400 MHz, DMSO-d6) δ 11.68 (s, 1H), 10.85 (s, 1H), 8.80 (d, J = 4.9 Hz, 1H), 8.65 (s, 1H), 8.20 (s, 1H), 8.15 (s, 1H), 7.77 (d, J = 8.1 Hz, 1H), 7.32 (s, 1H), 7.26 (s, 1H), 7.13 (t, J = 7.5 Hz, 1H), 7.03 (d, J = 8.2 Hz, 2H), 6.67 (d, J = 8.2 Hz, 2H), 6.23 (s, 1H), 5.73 (d, J = 7.5 Hz, 1H), 4.51 (s, 2H), 4.33 (dt, J = 12.0, 6.2 Hz, 1H), 4.15 (s, 2H), 4.09 (s, 4H), 3.80 (s, 3H), 3.07 (s, 2H), 2.97 (d, J = 10.5 Hz, 3H), 2.86 (d, J = 4.4 Hz, 3H), 2.82 - 2.73 (m, 2H), 2.66 (d, J = 4.4 Hz, 1H), 2.50 (d, J = 7.5 Hz, 2H), 2.37 (s, 1H), 2.17 (d, J = 12.9 Hz, 3H), 1.98 - 1.87 (m, 1H), 1.71 (q, J = 12.6, 11.8 Hz, 4H), 1.13 (t, J = 7.4 Hz, 3H). |
| T-109 | 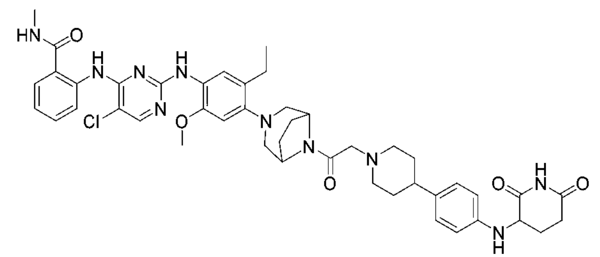 | LC-MS[M+1]: 849.6 |

**Example** 3

The compound synthesized in the present invention:

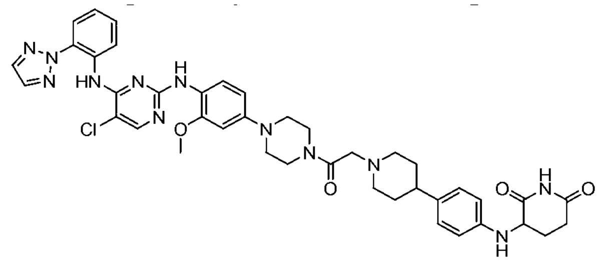

T-005

The experimental procedure is as follows:

I. Synthesis of Intermediate M1

The synthesis route is as follows:

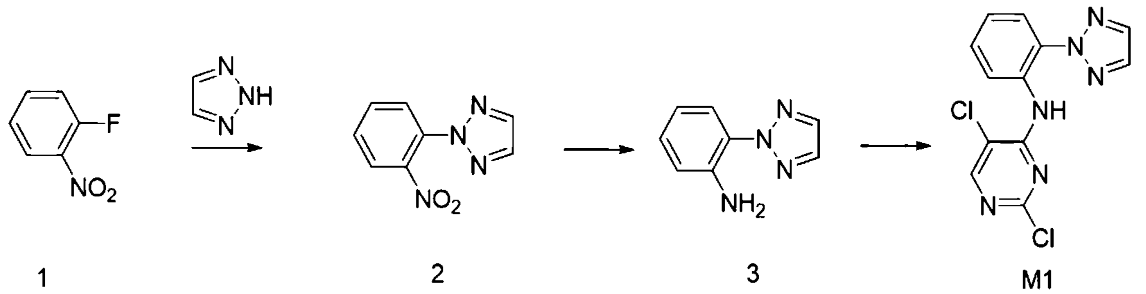

1. Synthesis of compound 2

To a 100 mL three-necked flask was added imidazole (587 mg, 1.2 eq) and 20 mL of tetrahydrofuran. The mixture was stirred to dissolve, and then cooled to 0 °C. Sodium hydride (400 mg, 1.4 eq) was added portion-wise to the reaction solution. After stirring for 30 minutes at this temperature, Compound 1 was added. The mixture was allowed to warm up to room temperature naturally, then heated to 60 °C and reacted overnight. The completion of the reaction was monitored by TLC, then excess sodium hydride was quenched with water. The reaction solution was extracted with ethyl acetate, and the organic phase was mixed with silica gel for sample application and subjected to column chromatography. The target fraction was collected, yielding 260 mg of the product.

2. Synthesis of compound 3

To a 50 mL single-necked flask was added compound 2 (260 mg, 1.0 eq), palladium on carbon catalyst (13 mg, 5 wt%), and 5 mL of methanol, and mixed uniformly. After purging with a hydrogen balloon three times, the reaction was carried out at room temperature under pressure maintenance for 20 hours. TLC analysis showed that the starting material had reacted completely, then the reaction solution was filtered through a diatomaceous earth pad, and the mother liquor was collected to obtain 160 mg of Compound 3.

3. Synthesis of intermediate M1

To a 100 mL single-necked flask was added 160 mg (1.0 eq) of Compound 3, a mixture of 0.8 mL (5.0 eq) of N,N-diisopropylethylamine (DIPEA) and 2 mL of isopropanol was added, and the mixture was stirred uniformly at room temperature. Then 183 mg (1.0 eq) of trichloropyrimidine was slowly added dropwise. After the addition was completed, the reaction was purged with nitrogen three times. Under nitrogen protection, the temperature was raised to reflux, and the reaction was stirred overnight. TLC analysis monitored the completion of the reaction. The solvent was evaporated to dryness under reduced pressure, and 50 mL of ethyl acetate (EA) was added to the residue for slurry at room temperature

for 30 minutes. The mixture was suction-filtered, and the filter cake was rinsed twice with 15 mL of ethyl acetate. The collected filter cake was Intermediate M1 (180 mg).

II. Synthesis of Intermediate M2

The synthesis route is as follows:

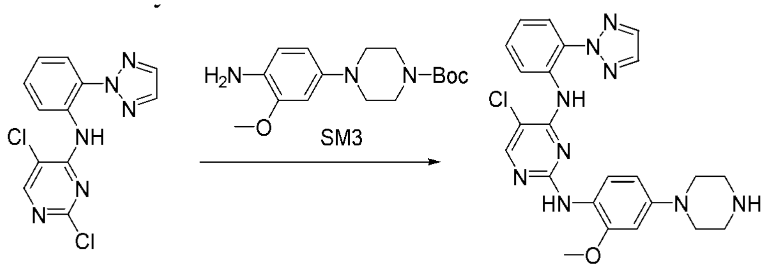


M1 M2

1. Synthesis of intermediate M2

To a 50 mL single-necked flask was added intermediate M1 (70 mg, 1.0 eq), SM3 (70 mg, 1.0 eq), p-toluenesulfonic acid monohydrate (52 mg, 1.2 eq), and 4 mL of isopropanol. The mixture was uniformly mixed and heated to reflux overnight, and the completion of the reaction was monitored by LCMS. Saturated aqueous sodium bicarbonate solution was added to the reaction solution to adjust the pH to >7. The mixture was extracted with ethyl acetate; the organic phase was washed with saturated saline and dried over anhydrous sodium sulfate, then mixed with silica gel (100-200 mesh) for sample application and purified by column chromatography (methanol in dichloromethane from 0% to 20%) to obtain the target product M2 (40 mg, brown solid). LC-MS[M+1]: 479.

III. Synthesis of compound T-005

The synthesis route is as follows:

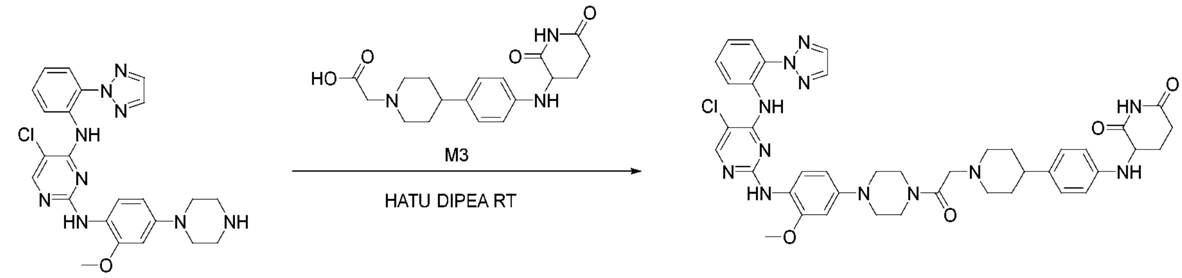


M2 T-005

In a 50 mL round-bottom flask, intermediate M2 and intermediate M3 were dissolved in 2 mL of N,N-dimethylformamide. The mixture was cooled to 0 °C in an ice-water bath, and N,N-diisopropylethylamine and HATU were added under stirring. The reaction was carried out in the ice-water bath for 1 hour under nitrogen protection, and then allowed to warm up to room temperature naturally. The completion of the reaction was monitored by TLC, and then the reaction solution was extracted with ethyl acetate and water; the organic phase was dried and filtered, and subjected to column chromatography to obtain 20 mg of Compound T-005. LC-MS [M+1]: 805; HNMR: 1H NMR (400 MHz, DMSO-d6) $\delta$ 10.84 (s,1H), 10.26 (s, 1H), 8.54 (d, J = 8.5 Hz, 1H),8.32 (s, 2H), 8.18 (s, 2H), 7.98 (dd, J = 8.2,1.6 Hz, 1H), 7.52 (d, J = 8.6 Hz, 1H), 7.41(t, J = 7.8 Hz, 1H), 7.31 (t, J = 7.8 Hz, 1H),7.03 (d, J = 8.0 Hz, 2H), 6.77 (d, J = 2.5 Hz,1H), 6.68 (d, J = 8.1 Hz, 2H), 6.56 (dd, J =8.7, 2.5 Hz, 1H), 5.76 (s, 1H), 4.33 (dd, J =9.7, 5.1 Hz, 1H), 3.85 (s, 3H), 3.73 (s, 5H),3.29 (s, 3H), 3.20 (s, 3H), 2.79 (td, J = 12.1,6.1 Hz, 2H), 2.65 (d, J = 4.4 Hz, 1H), 2.20 -2.10 (m, 2H), 1.99 - 1.76 (m, 5H), 1.33 (s,2H).

The following compounds were synthesized with reference to the method of Example 3:

| Compound No. | Structure | Characterization data: |
|---|---|---|
| T-007 | 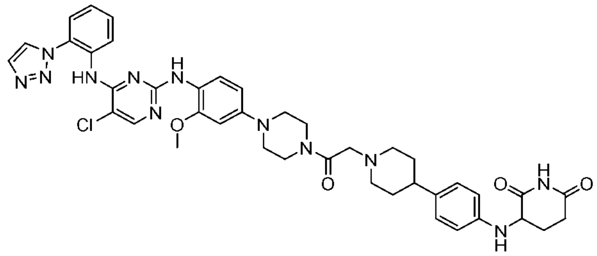 | LC-MS[M+1]: 805 |

**Example** 4

The compound synthesized in the present invention:

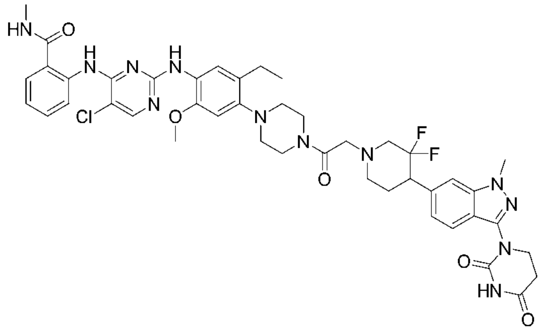

T-013

The experimental procedure is as follows:

I. Synthesis of Intermediate M1

The synthesis route is as follows:

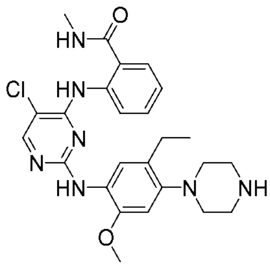

M1

Intermediate M1 was synthesized with reference to the method of Example 1

II. Synthesis of Intermediate M2

The synthesis route is as follows:

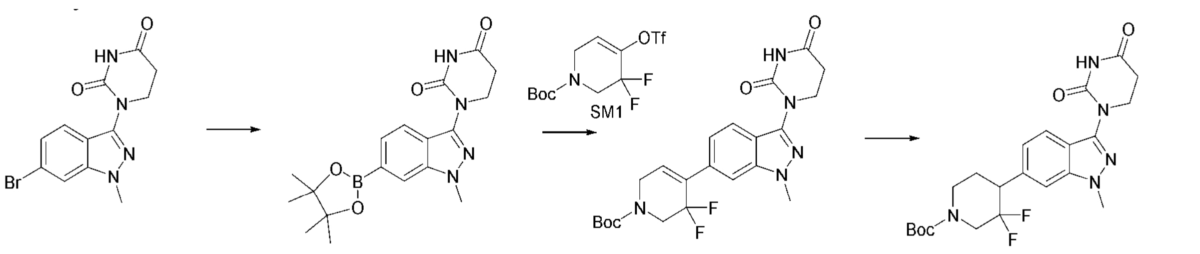


1 2 3 4

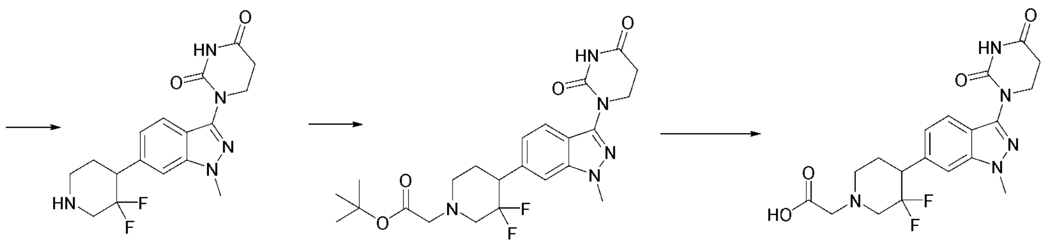

5 6 M2

1. Synthesis of compound 2

In a 100 mL single-necked flask, Compound 1 (1.0 g, 1.0 eq), bis(pinacolato)diboron (1.18 g, 1.2 eq), 15 mL of 1,4-dioxane, potassium acetate (911 mg, 3.0 eq), and $PdCl_2$(dppf) (113 mg, 0.12 eq) were mixed uniformly. After purging with nitrogen, the mixture was heated to 85 °C and reacted overnight. The completion of the reaction was monitored by TLC, and then the reaction solution was extracted with ethyl acetate, dried over anhydrous sodium sulfate, spun-dried and separated by column chromatography to obtain 1.2 g of Compound 2. LC-MS[M+1]: 372.

2. Synthesis of compound 3

In a 100 mL three-necked flask, 400 mg of Compound 2 (1.2 eq), 484 mg of SM1 (1.0 eq), 345 mg of potassium carbonate (3.0 eq), 10 mL of 1,4-dioxane, and 2.5 mL of water were mixed uniformly under nitrogen protection. Then, 80 mg of [1,1'-bis(diphenylphosphino)ferrocene]dichloropalladium (0.12 eq) was added, and the mixture was heated to 55 °C to react for 2 hour. The completion of the reaction was monitored by TLC, then the reaction solution was filtered through a diatomaceous earth pad, and the mother liquor was collected. The solvent was removed from the mother liquor by rotary evaporation, after which 30 mL of water, and 15 mL of ethyl acetate were added to extract for three times. The organic phases were were combined and dry loaded for sample application, and then subjected to column chromatography to obtain 500 mg of Compound 3. LC-MS[M+1]: 462.

3. Synthesis of compound 4

To a 50 mL single-necked flask was added compound 3 (500 mg, 1.0 eq), palladium on carbon catalyst (50 mg, 5 wt%), and 5 mL of methanol, and mixed uniformly. After purging with a hydrogen balloon three times, the reaction was carried out at room temperature under pressure maintenance for 20 hours. TLC analysis showed that the starting material had reacted completely, then the reaction solution was filtered through a diatomaceous earth pad, and the mother liquor was collected to obtain 400 mg of Compound 4.

4. Synthesis of compound 5

To a 100 ml single-necked flask was added 400 g of compound 4, and 10 ml of tetrahydrofuran was added to dissolve it. Then 4M solution of dioxane hydrochloride was added dropwise under a cooling ice-water bath. After the completion of dropwise addition, the reaction was carried out at 40 °C for 2 hours. The solvent was removed by rotary evaporation to obtain 330 g of compound 5. LC-MS[M+1]: 364.

5. Synthesis of compound 6

To a 100 ml single-necked flask was added 330 mg of compound 5, 4 ml of N,N-dimethylformamide, 1.2 mL of N,N-diisopropylethylamine(5.0 eq), and tert-butyl bromoacetate(195 mg, 1.1 eq), and stirred at room temperature overnight. The completion of the reaction was monitored by TLC. Then the reaction solution was extracted with ethyl acetate and water; the organic phase was dried and filtered, and subjected to column chromatography to obtain 370 mg of Compound 6. LC-MS [M+1]: 479.

6. Synthesis of intermediate M2

To a 100 ml single-necked flask was added 350 mg of compound 6 and 3 ml of dichloromethane. Then 2 mL of trifluoroacetic acid (TFA) was added dropwise to the mixture, and the resulting mixture was stirred at room temperature overnight. The completion of the reaction was monitored by TLC, and the solvent was then removed by rotary evaporation, to obtain 150 mg of M2. LC-MS [M-1]: 420.

III. Synthesis of compound T-013

The synthesis route is as follows:

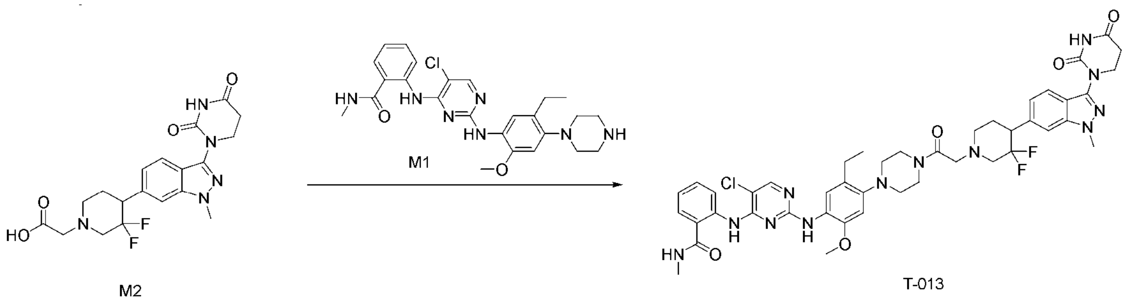
M2 M1 T-013

In a 50 mL round-bottom flask, Compound M2 (150 mg, 1.0 eq) and M1 (176 mg, 1.0 eq) were dissolved in 4 mL of N, N-dimethylformamide. The mixture was cooled to 0 °C in an ice-water bath; N, N-diisopropylethylamine and HATU (176 mg, 1.3 eq) were added under stirring, and the reaction was carried out in the ice-water bath for 1 hour under nitrogen protection. The mixture was then allowed to warm up to room temperature naturally. The completion of the reaction was monitored by TLC, and then the reaction solution was extracted with ethyl acetate and water; the organic phase was dried and filtered, and subjected to column chromatography to obtain 90 mg of Compound T-013. LC-MS [M+1]: 899.6.

1H NMR (400 MHz, DMSO-d6) δ11.69 (s, 1H), 10.64 (s, 1H), 8.80 (s,1H), 8.70 - 8.54 (m, 1H), 8.29 (s,1H), 8.21 (s, 1H), 7.76 (d, J = 7.8 Hz,1H), 7.71 - 7.52 (m, 3H), 7.38 (s,1H), 7.13 (q, J = 8.5, 7.6 Hz, 2H),6.88 (s, 1H), 4.12 - 3.93 (m, 6H),3.79 (d, J = 18.4 Hz, 5H), 3.68 (s,3H), 3.53 - 3.51 (m, 1H), 3.20 (d, J =7.6 Hz, 2H), 3.10 (s, 1H), 2.98 (s,2H), 2.93 - 2.78 (m, 8H), 2.69 (d, J =7.4 Hz, 1H), 2.33 (d, J = 12.9 Hz,1H), 2.05 (s, 1H), 1.94 (d, J = 13.0Hz, 1H), 1.16 (t, J = 7.6 Hz, 3H).

The following compounds were synthesized with reference to the method of Example 4:

| Compound No. | Structure | Characterization data: |
|---|---|---|
| T-014 | 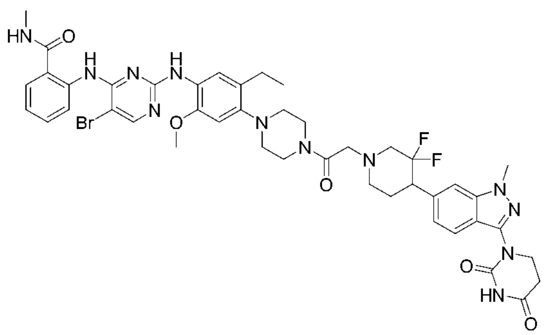 | LC-MS[M+1]: 945.8 |

(continued)

| Compound No. | Structure | Characterization data: |
|---|---|---|
| T-043 | 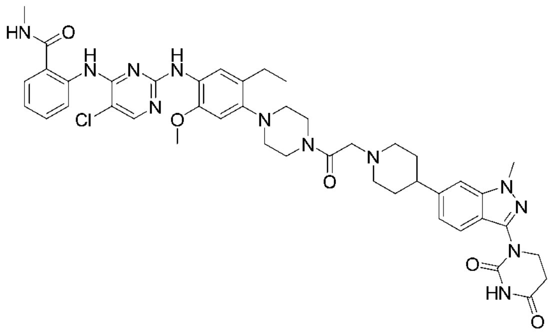 | LC-MS[M+1]: 863.7 |
| T-045 | 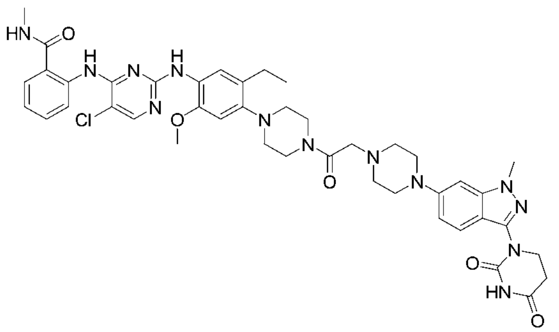 | LC-MS[M+1]: 864.6 |
| T-047 | 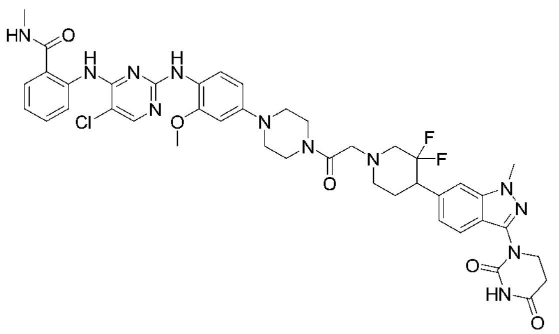 | LC-MS[M+1]: 871.7 |
| T-049 | 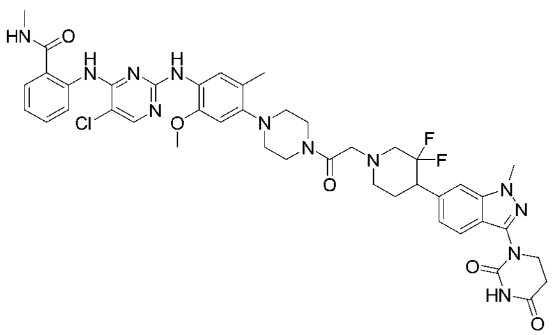 | LC-MS[M+1]: 885.6 |

(continued)

| Compound No. | Structure | Characterization data: |
|---|---|---|
| T-086 | 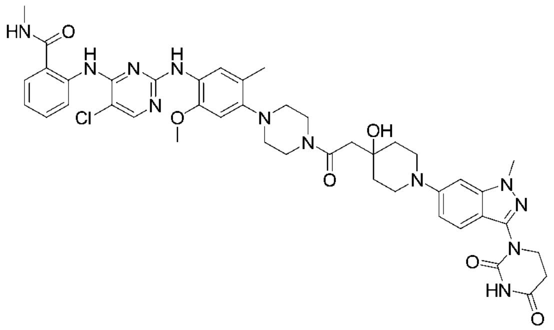 | LC-MS[M+1]: 865.4<br>1H NMR (400 MHz, DMSO-d6) δ 11.68 (s, 1H), 10.60 (s, 1H), 8.66 (d, J = 8.4 Hz, 1H), 8.22 (d, J = 4.5 Hz, 2H), 7.80 (dd, J = 8.0, 1.6 Hz, 1H), 7.59 (s, 1H), 7.51 (d, J = 9.0 Hz, 1H), 7.42 (t, J = 8.0 Hz, 1H), 7.17 (t, J = 7.6 Hz, 1H), 7.00 (dd, J = 9.1, 1.9 Hz, 1H), 6.91 (d, J = 2.0 Hz, 1H), 6.82 (s, 1H), 5.07 (s, 1H), 3.96 (d, J = 3.1 Hz, 5H), 3.85 (s, 3H), 3.78 (d, J = 7.9 Hz, 4H), 3.61 (d, J = 12.2 Hz, 2H), 3.26 (t, J = 11.2 Hz, 2H), 2.97 - 2.77 (m, 9H), 2.67 (s, 2H), 2.26 (s, 3H), 1.91 - 1.74 (m, 4H), 1.31 (d, J = 7.3 Hz, 1H). |

**Example** 5

The compound synthesized in the present invention:

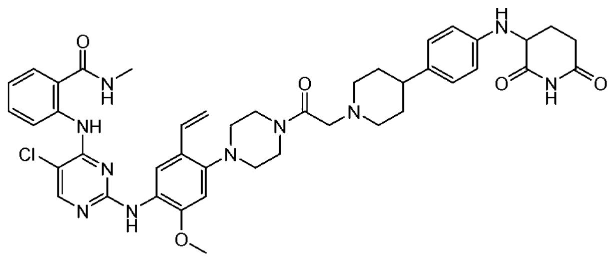

T-003

The experimental procedure is as follows:

I. Synthesis of Intermediate M1

The synthesis route is as follows:

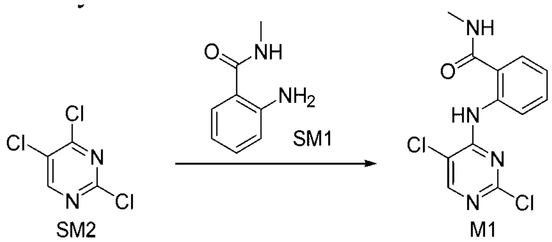

Intermediate M1 was synthesized with reference to the method of Example 1

II. Synthesis of Intermediate M2

The synthesis route is as follows:

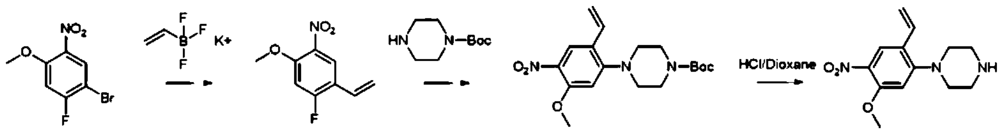


1 2 3 4

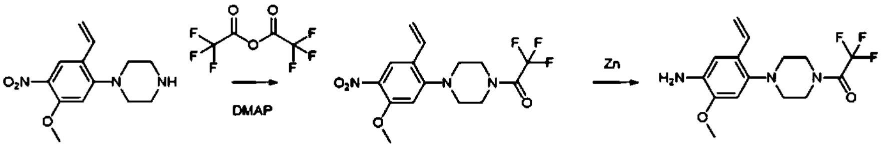


4 5 6

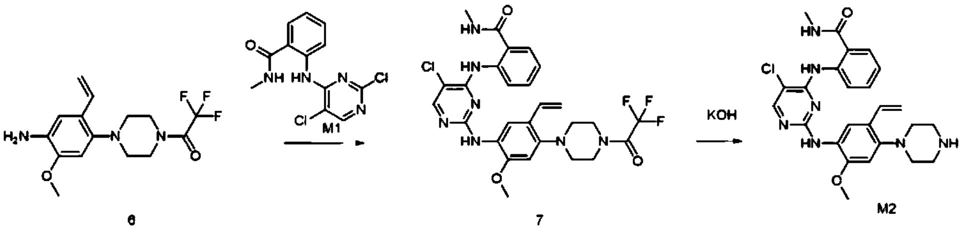


6 7 M2

1. Synthesis of compound 2

In a 100 mL three-necked flask, 500 mg of Compound 1 (1.0 eq.), 536 mg of potassium vinyltrifluoroborate (2.0 eq.), 165 mg of potassium carbonate (1.5 eq.), 5 mL of 1,4-dioxane, and 1 mL of water were mixed uniformly, and the mixture was protected with nitrogen. 175mg of [1,1'-bis(diphenylphosphino)ferrocene]palladium(II) chloride (0.12 eq.) was added, and the mixture was heated to 100 °C to react overnight. The completion of the reaction was monitored by TLC, then the reaction solution was filtered through a diatomaceous earth pad, and the mother liquor was collected. The solvent was removed from the mother liquor by rotary evaporation, after which 30 mL of water, and 15 mL of ethyl acetate were added to extract for three times. The organic phases were combined and dry loaded for sample application, and then subjected to column chromatography to obtain 320 mg of Compound 2.

2. Synthesis of compound 3

In a 100 mL single-necked flask, 320 mg of Compound 2, 600 mg of N-Boc piperazine, and 666 mg of potassium carbonate were dissolved in 30 mL of DMF. The mixture was heated to 110 °C and reacted for 18 hours. The solvent was removed by rotary evaporation; water was added to the residue, and the mixture was extracted with ethyl acetate. The organic phase was dried over anhydrous sodium sulfate, spun-dried and and subjected to column chromatography to obtain 400 mg of Compound 3. LC-MS[M+1]: 366.

3. Synthesis of compound 4

In a 100 mL single-necked flask, 300 mg of Compound 3, 3 mL of hydrogen chloride (HCl)/1,4-dioxane solution, and 5 mL of dichloromethane (DCM) were mixed. The mixture was reacted at room temperature for 6 hours, and then the reaction solution was spun-dried under reduced pressure to obtain 217 mg of Compound 4 as a yellow solid. LC-MS[M+1]: 264.

4. Synthesis of compound 5

In a 100 mL single-necked flask, 217 mg of Compound 4 was dissolved in 3 mL of dichloromethane (DCM). Then 202 mg of 4-dimethylaminopyridine (DMAP) was added, and the mixture was reacted at room temperature for 5 minutes. Subsequently, 346 mg of trifluoroacetic anhydride was added, and the reaction was carried out at room temperature for 18 hours. The reaction solution was spun-dried under reduced pressure to obtain 295 mg of Compound 5 as a yellow solid. LC-MS[M+1]: 360.

5. Synthesis of compound 6

In a 100 mL single-necked flask, 295 mg of Compound 5 was dissolved in 5 mL of ethanol and 1 mL of water. Then 320 mg of Zn powder and 435 mg of solid ammonium chloride were added. The mixture was refluxed at 100 °C for 4 hours. The Zn powder was filtered out through a diatomaceous earth pad. The mother liquor was rotary evaporated to remove the solvent under reduced pressure, and then subjected to column chromatography to obtain 100 mg of Compound 6 (100 mg). LC-MS[M+1]: 330.

6. Synthesis of compound 7

To a 50 mL single-necked flask was added 100 mg of Compound 6, 90 mg of M1, 347 mg of trifluoroacetic acid (TFA), and 3 mL of isopropanol. The mixture was refluxed at 100 °C for 18 hours. The reaction solution was quenched with saturated aqueous sodium bicarbonate solution, and then extracted with dichloromethane (DCM). The organic phase was dried over anhydrous sodium sulfate, spun-dried, and subjected to column chromatography to obtain 111 mg of Compound 7 as a yellow solid. LC-MS[M+1]: 590.

7. Synthesis of intermediate M2

To a 50 mL single-necked flask was added Compound 7 (111 mg, 1.0 eq), potassium hydroxide (105 mg, 10.0 eq), 5 mL of methanol, and 1 mL of water. The mixture was mixed uniformly, and then heated to 60 °C for 4 hours. The completion of the reaction was monitored by LCMS. The reaction solution was then quenched by adding water, and then extracted with dichloromethane (DCM). The organic phase was washed with saturated saline and dried over anhydrous sodium sulfate, then mixed with silica gel (100-200 mesh) for sample application, and purified by column chromatography (MeOH in DCM 0% to 10%) to obtain the target product M2 (60 mg, brown solid). LC-MS[M+1]: 493.

III. Synthesis of compound T-003

The synthesis route is as follows:

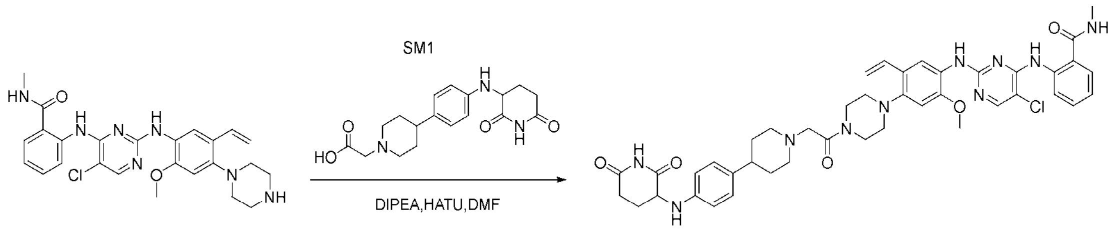


M2

T-003

In a 50 mL round-bottom flask, 60 mg of Compound M2 and 87 mg of SM1 were dissolved in 3 mL of N,N-dimethylformamide. The mixture was cooled to 0 °C in an ice-water bath, 1 mL of N,N-diisopropylethylamine and 48 mg of HATU were added under stirring, and the reaction was carried out in the ice-water bath for 1 hour under nitrogen protection. After that, the mixture was allowed to warm up to room temperature naturally, and reacted for another 2 hours. The completion of the reaction was monitored by TLC. The reaction solution was then extracted with ethyl acetate and water; the organic phase was dried and filtered, and subjected to column chromatography to obtain 100 mg of Compound T-003. LC-MS [M+1]: 821. 1H NMR (400 MHz, DMSO-d6) δ 11.75 (s, 1H), 10.86 (s, 1H), 8.86 (d, J = 4.8 Hz, 1H), 8.63 (s, 1H), 8.43 (s, 1H), 8.20 (s, 1H), 7.83 - 7.72 (m, 2H), 7.29 (s, 1H), 7.13 - 6.96 (m, 4H), 6.81 (s, 1H), 6.65 (d, J = 8.6 Hz, 2H), 5.74 (d, J = 7.5 Hz, 1H), 5.59 (d, J = 17.8 Hz, 1H), 5.18 (d, J = 11.3 Hz, 1H), 4.32 (ddd, J = 11.8, 7.4, 4.8 Hz, 1H), 3.84 (s, 5H), 3.72 (s, 2H), 3.28 (s, 2H), 2.97 (d, J = 32.9 Hz, 6H), 2.85 (d, J = 4.5 Hz, 3H), 2.65 (d, J = 4.4 Hz, 1H), 2.20 - 1.84 (m, 5H), 1.77 (d, J = 11.9 Hz, 2H), 1.66 (d, J = 12.6 Hz, 2H).

**Example 6**

The compound synthesized in the present invention:

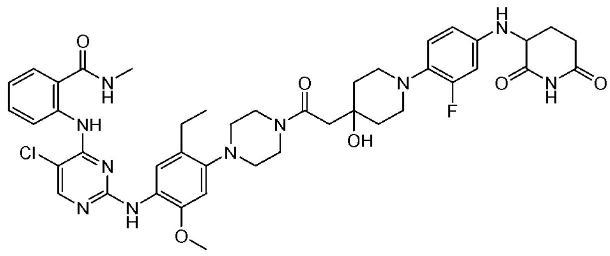

T-037

The experimental procedure is as follows:

I. Synthesis of Intermediate M1

The synthesis route is as follows:

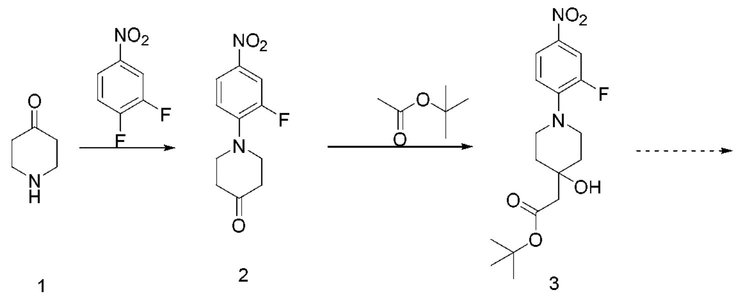

1 2 3

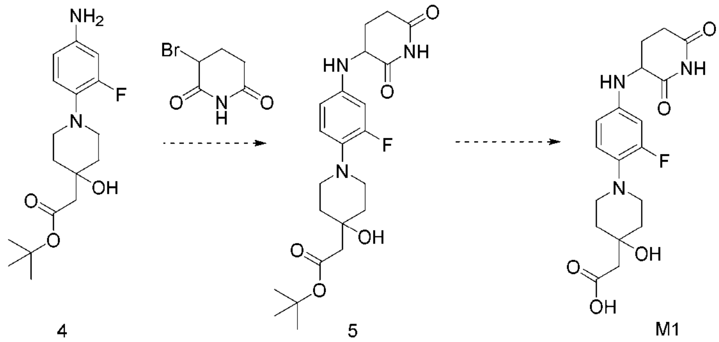

4 5 M1

1. Synthesis of compound 2

In a 50 mL three-necked flask, 4-piperidone (1.06 g, 6.9 mmol, 1.1 eq) and DIPEA (2.4 g, 18.6 mmol, 3.0 eq) were dissolved in 10 mL of DMF. Then 3,4-difluoronitrobenzene (1 g, 6.29 mmol, 1.0 eq) was added to the mixture. After purging with $N_2$ for 3 times, the mixture was stirred at 110 °C overnight. The completion of the reaction was monitored by TLC. The reaction solution was diluted with EA (10 mL) and stirred for 5 minutes, and then 10 mL of water was added. The mixture was allowed to stand for partitioning. The aqueous phase was extracted once with 10 mL of EA. The combined organic phases were washed with saturated NaCl solution (15 mL × 3), dried over anhydrous sodium sulfate, and filtered. The filtrate was spun-dried, and the crude product was subjected to column chromatography (EA in PE from 0% to 40%) to obtain crude Compound 2 as a yellow solid (629 mg, 42% yield). LC-MS[M+1]: 239.

2. Synthesis of compound 3

To a 100 mL three-necked flask was added tert-Butyl acetate (367 mg, 3.16 mmol, 1.2 eq). After purging with $N_2$ gas three times, anhydrous tetrahydrofuran (10 mL) was added. The mixture was cooled to -78 °C, and LDA (lithium diisopropylamide) was slowly added to the reaction system, followed by stirring at -78 °C for 45 minutes. Tert-butyl acetate (367 mg, 3.16 mmol, 1.2 eq) was dissolved in anhydrous tetrahydrofuran (10 mL) and slowly added dropwise to the reaction system. The reaction solution was stirred at -78 °C for 2 hours. TLC analysis showed that the starting material had

disappeared and a new spot had formed. Saturated aqueous $NH_4Cl$ solution (10 mL) was added to the reaction solution to quench the reaction. EA was added to the mixture, and then allowed to stand for partitioning. The organic phase was washed with saturated saline (10 mL × 3), dried over anhydrous sodium sulfate, and filtered. The filtrate was spun-dried, and the crude product was subjected to column chromatography (EA in PE from 0% to 40%) to obtain Compound 3 as a yellow solid (781 mg, 83.2% yield). LC-MS[M+1]: 355.

3. Synthesis of compound 4

In a 100 mL three-necked flask, Compound 3 (781 mg, 2.2 mmol, 1.0 eq), ammonium chloride (1.4 g, 21.87 mmol, 10.0 eq), and iron powder (741 mg, 13.2 mmol, 6.0 eq) were dispersed in 10 mL of a solvent mixture of ethanol and water at a ratio of 4:1. After purging with $N_2$ three times, the mixture was heated to reflux for 18 hours. TLC analysis showed that the starting material had disappeared. The solvent was removed by rotary evaporation. The crude product was subjected to column chromatography (EA in PE from 0% to 50%) to obtain Compound 4 as a yellow solid (669 mg, 93.6% yield). LC-MS [M+1]: 325.

4. Synthesis of compound 5

In a 100 mL round-bottom flask, Compound 4 (669 mg, 2.06 mmol, 1.0 eq) and 3-bromopiperidin-2,6-dione (595 mg, 3.09 mmol, 1.5 eq) were dissolved in 6.69 mL of N,N-dimethylformamide (DMF). Sodium bicarbonate (347 mg, 4.13 mmol, 2.0 eq) was added to the mixture. Then the reaction solution was stirred at 70 °C for 16 hours under nitrogen protection. After cooling to room temperature, the completion of the reaction was monitored by TLC. The reaction solution was then diluted with EA (10 mL), and 100 mL of water was added for partitioning. The aqueous phase was extracted with 10 mL of EA. The combined organic phases were dried over anhydrous sodium sulfate and filtered. The crude product was purified by column chromatography (EA in PE from 0% to 57%) to obtain Compound 5 (570 mg, 63.6% yield). LC-MS[M+1]: 436.

5. Synthesis of compound M1

In a 50 mL round-bottom flask, Compound 5 (290 mg, 0.667 mmol, 1.0 eq) was dissolved in 3 mL of dichloromethane (DCM), and 0.5 mL of trifluoroacetic acid (TFA) was added to the mixture. The reaction solution was stirred at room temperature for 2 hours. The completion of the reaction was monitored by LCMS. The reaction solution was evaporated to dryness to obtain Compound M1 (253 mg, 100% crude). LC-MS[M+1]: 381.

II. Synthesis of compound T-037

The synthesis route is as follows:

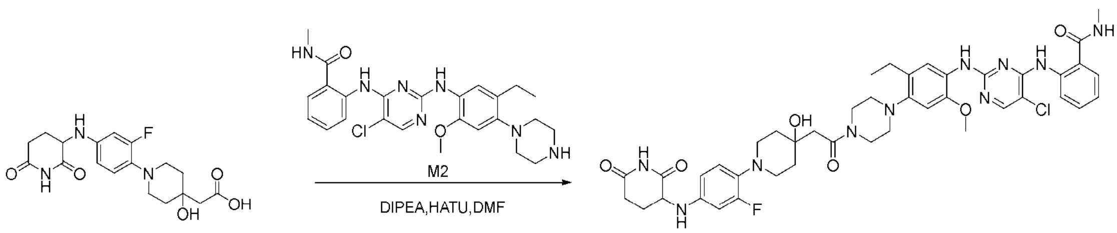


M1

T-037

1. Compound M2 was synthesized with reference to Example 2.
2. Synthesis of T-037

In a 50 mL round-bottom flask, Compound M1 (253 mg, 0.66 mmol, 1.0 eq) and Compound M2 (330 mg, 0.66 mmol, 1.0 eq) were dissolved in 3.3 mL of N, N-dimethylformamide (DMF), and N, N-diisopropylethylamine (258 mg, 1.98 mmol, 3.0 eq) was added to the mixture. The reaction solution was cooled to 0 °C in an ice-water bath under nitrogen protection. Then HATU (304 mg, 0.8 mmol, 1.2 eq) was added to the reaction solution under stirring, and the reaction was carried out in the ice-water bath for 2 hours under nitrogen protection. The mixture was allowed to warm up to room temperature naturally, and the completion of the reaction was monitored by TLC. The reaction solution was then diluted with EA (10 mL), and 10 mL of water was added. After stirring for 5 minutes, the mixture was allowed to stand for partitioning. The aqueous phase was extracted with 10 mL of EA. The combined organic phases were dried over

anhydrous sodium sulfate and filtered. The crude product was purified by column chromatography (MeOH in DCM from 0% to 3%) to obtain Compound T-037 (295 mg, 51.69% yield). LC-MS[M+1]: 857. 1H NMR (400 MHz, DMSO-d6) δ 11.63 (s,1H), 10.81 (s, 1H), 8.76 (q, J = 4.6 Hz, 1H),8.57 (d, J = 8.2 Hz, 1H), 8.19 (d, J = 31.9Hz, 2H), 7.84- 7.62 (m, 1H), 7.50 (s, 1H),7.32 (t, J = 7.8 Hz, 1H), 7.08 (t, J = 7.5 Hz,1H), 6.84 (d, J = 20.3 Hz, 2H), 6.51 (dd, J =15.0, 2.5 Hz, 1H), 6.42 (dd, J = 8.8, 2.5 Hz,1H), 5.81 (s, 1H), 4.91 (s, 1H), 4.26 (dt, J =12.6, 6.2 Hz, 1H), 3.74 (d, J = 24.6 Hz, 7H),2.99 - 2.82 (m, 7H), 2.80 (d, J = 4.4 Hz,3H), 2.74 (s, 1H), 2.66 - 2.53 (m, 5H), 2.09(dq, J = 13.4, 4.8 Hz, 1H), 1.92 - 1.73 (m,3H), 1.69 (d, J = 12.4 Hz, 2H), 1.24 (d, J =8.0 Hz, 1H), 1.09 (t, J = 7.4 Hz, 3H).

The following compounds were synthesized with reference to the method of Example 6:

| Compound No. | Structure | Characterization data: |
|---|---|---|
| T-015 | 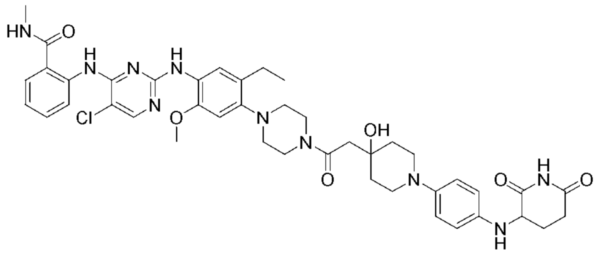 | LC-MS[M+1]: 811.8 |
| T-067 | 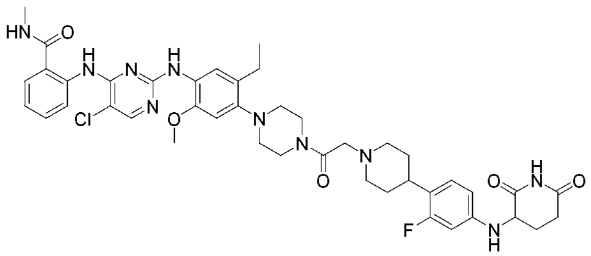 | LC-MS[M+1]: 841.4 |
| T-068 | 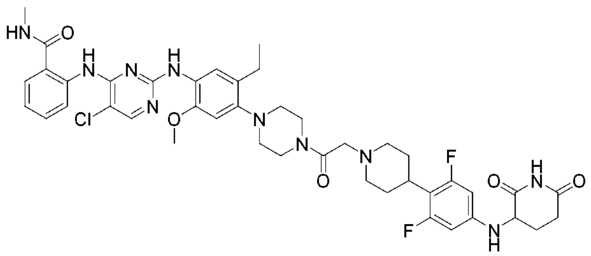 | LC-MS[M+1]: 859.6 |
| T-069 | 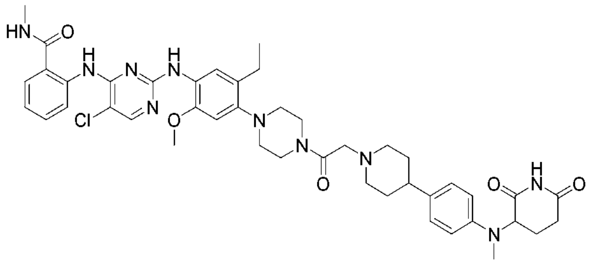 | LC-MS[M+1]: 837.9 |
| T-070 | 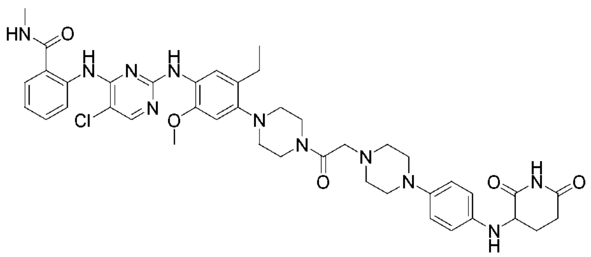 | LC-MS[M+1]: 826.5 |

(continued)

| Compound No. | Structure | Characterization data: |
|---|---|---|
| T-071 | 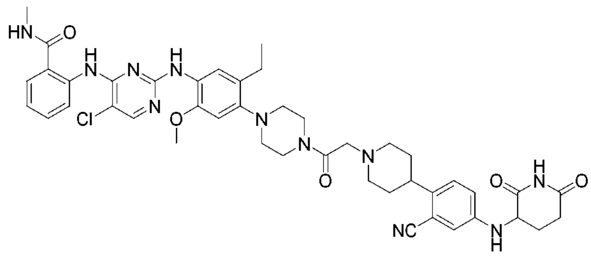 | LC-MS[M+1]: 848.8 |
| T-073 | 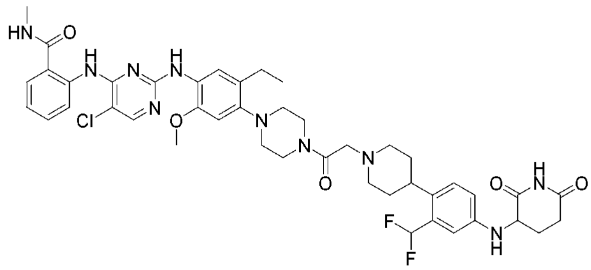 | LC-MS[M+1]: 873.6 |
| T-074 | 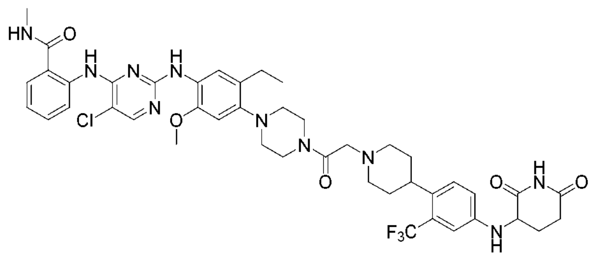 | LC-MS[M+1]: 891.7 |
| T-090 | 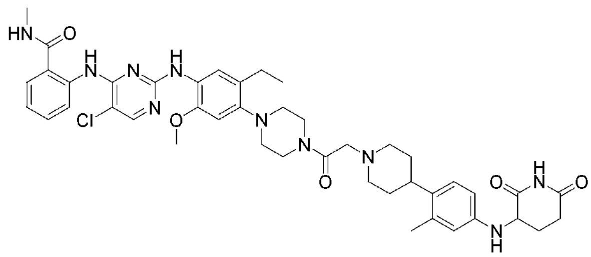 | LC-MS[M+1]: 837.6 |

**Example 7**

The compound synthesized in the present invention:

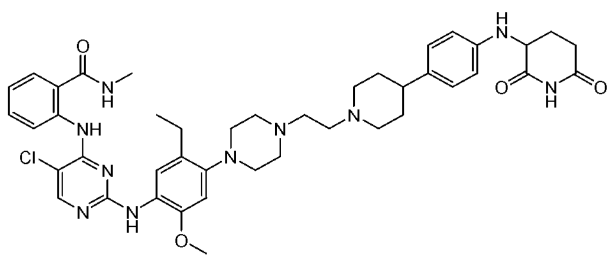

T-023

The experimental procedure is as follows:

I. Synthesis of Intermediate M3

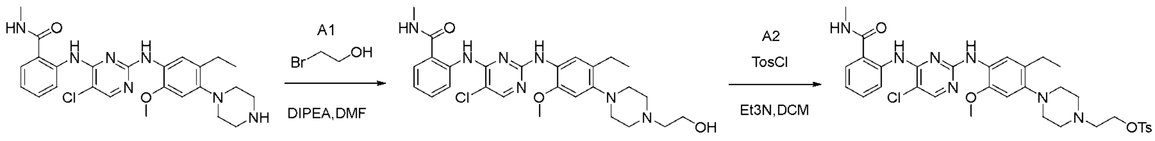


M2 2 M3

1. Intermediate M2 was synthesized with reference to Example 2.
2. Synthesis of compound 2

To a 50 mL three-necked flask was added 600 mg of pre-weighed M2 and dissolved in 6 mL of DMF. Then 188 mg of A1 and 469 mg of DIPEA were added. After purging with nitrogen ($N_2$) three times, the reaction was conducted at 90 °C overnight. TLC monitoring indicated the formation of a new spot in the reaction. Water was added to the reaction solution, and the mixture was extracted with ethyl acetate. The organic phase was dried over anhydrous sodium sulfate, and the crude product was subjected to column chromatography to obtain 448 mg of Compound 2. LC-MS[M+1]: 540.

3. Synthesis of intermediate M3

In a 50 mL single-necked flask, 448 mg of Compound 2 and 252 mg of DIPEA were dissolved in 5 mL of dichloromethane (DCM). Separately, 206 mg of A2 was dissolved in 2 mL of DCM, and this A2 solution was slowly added dropwise to the above reaction mixture. After purging with nitrogen ($N_2$) three times, the reaction was carried out at room temperature for 2 hours. TLC monitoring was performed until the amount of Compound 2 no longer decreased. The reaction solution was directly spun-dired under reduced pressure, and dry loaded for sample application, and subjected to column chromatography to obtain 286 mg of intermediate M3. LC-MS[M+1]: 694.

II. Synthesis of compound T-023

The experimental procedure is as follows:

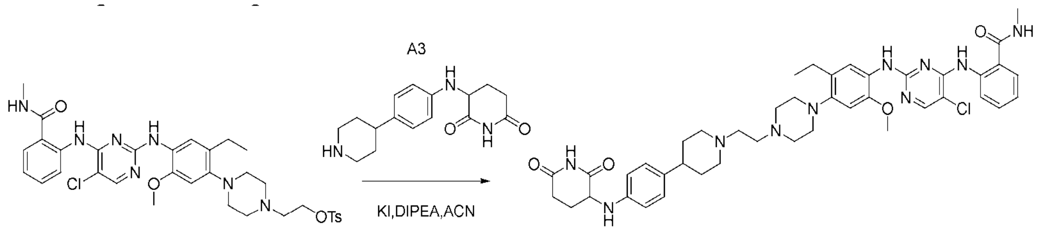


M3 T-023

In a 50 mL round-bottom flask, 186 mg of Compound M3 and 119 mg of A3 were dissolved in 3 mL of acetonitrile. Then 82 mg of KI and 159 mg of DIPEA were added. After purging with nitrogen ($N_2$) three times, the reaction was carried out at 75 °C for 3 hours. The completion of the reaction was monitored by TLC. The reaction solution was then spun-dried under reduced pressure, and dry loaded for sample application, and subjected to column chromatography to obtain 190 mg of Compound T-023. LC-MS [M+1]: 809. 1H NMR (400 MHz, DMSO-d6) δ 11.72 (s, 1H), 10.87 (s, 1H), 8.90 (q, J = 4.6 Hz, 1H), 8.63 (d, J = 8.5 Hz, 1H), 8.28 (s, 1H), 8.20 (s, 1H), 7.82 (d, J = 7.9 Hz, 1H), 7.54 (s, 1H), 7.37 (t, J = 8.0 Hz, 1H), 7.14 (t, J = 7.6 Hz, 1H), 7.04 (d, J = 8.2 Hz, 2H), 6.86 (s, 1H), 6.70 (d, J = 8.3 Hz, 2H), 5.82 (d, J = 8.9 Hz, 1H), 4.35 (ddd, J = 11.9, 7.5, 4.8 Hz, 1H), 3.83 (s, 3H), 3.23 (s, 1H), 2.96 (t, J = 4.7 Hz, 5H), 2.86 (d, J = 4.5 Hz, 4H), 2.84 - 2.69 (m, 6H), 2.64 (q, J = 7.5 Hz, 4H), 2.00 - 1.82 (m, 4H), 1.57 - 1.23 (m, 6H), 1.14 (t, J = 7.5 Hz, 3H).

The following compounds were synthesized with reference to the method in Example 7:

| Compound No. | Structure | Characterization data: |
|---|---|---|
| T-021 | 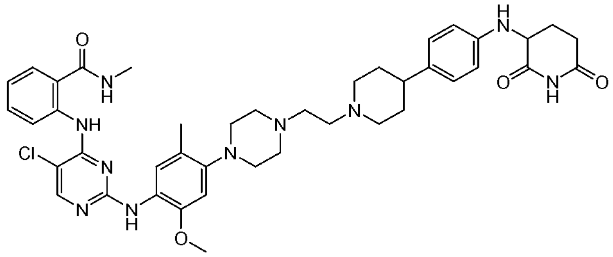 | LC-MS[M+1]: 795.6 HNMR: 1H NMR (400 MHz, DMSO-d6) δ 11.65 (s, 1H), 10.85 (s, 1H), 8.81 (d, J = 5.9 Hz, 1H), 8.63 (s, 1H), 8.20 (d, J = 6.5 Hz, 2H), 7.78 (d, J = 7.8 Hz, 1H), 7.52 (s, 1H), 7.39 (d, J = 8.5 Hz, 1H), 7.16 (t, J = 7.6 Hz, 1H), 7.02 (d, J = 8.0 Hz, 2H), 6.80 (s, 1H), 6.67 (d, J = 8.0 Hz, 2H), 5.73 (d, J = 7.4 Hz, 1H), 4.33 (s, 1H), 3.83 (s, 4H), 3.11 (s, 3H), 2.94 (s, 4H), 2.86 (d, J = 4.5 Hz, 4H), 2.80 (s, 2H), 2.67 (s, 3H), 2.42 (s, 1H), 2.18 (d, J = 25.8 Hz, 6H), 1.92 (d, J = 12.5 Hz, 2H), 1.72 (d, J = 28.7 Hz, 5H) |
| T-024 | 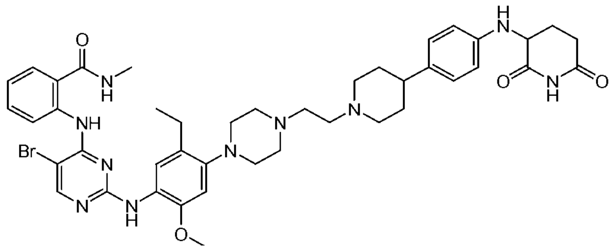 | LC-MS[M+1]: 853.5 |
| T-92 | 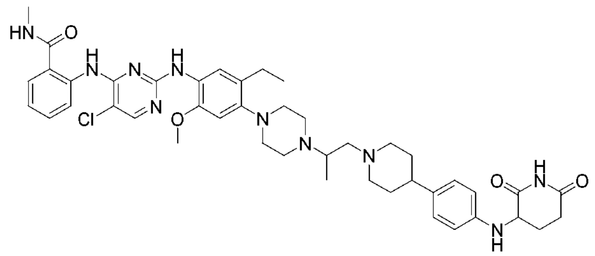 | LC-MS[M+1]: 823.5 |
| T-94 | 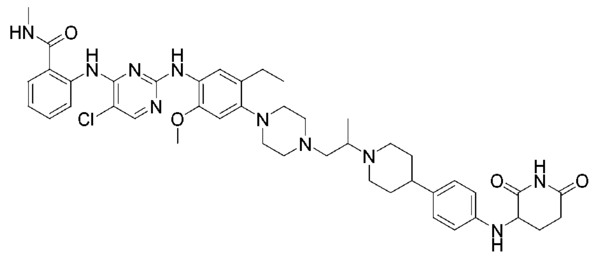 | LC-MS[M+1]: 823.5 |

**Example 8**

The compound synthesized in the present invention:

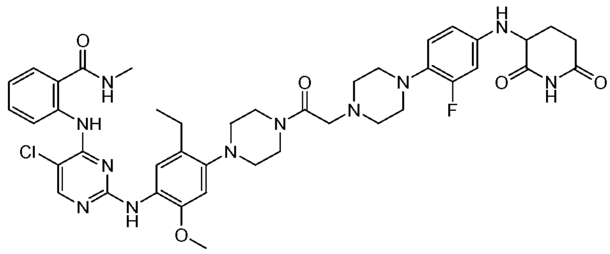

T-029

The experimental procedure is as follows:

I. Synthesis of Intermediate M1

The synthesis route is as follows:

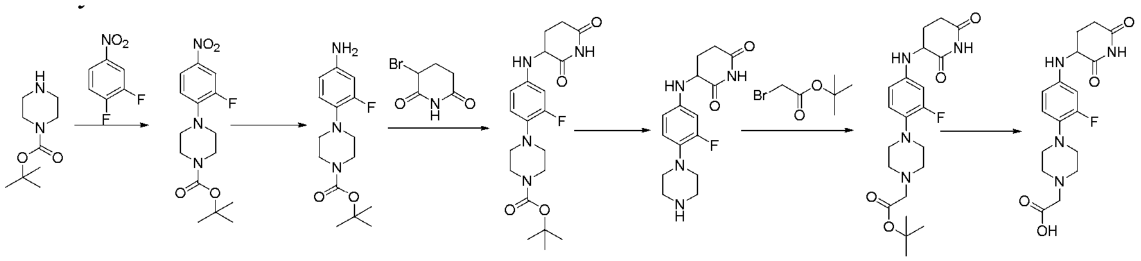

1 2 3 4 5 6 7

1. Synthesis of compound 2

In a 50 mL three-necked flask, 1-tert-butoxycarbonylpiperazine (0.5 g, 3.14 mmol, 1.0 eq) and potassium carbonate (1300 mg, 9.42 mmol, 3.0 eq) were added to 5 mL of DMF. Then 3,4-difluoronitrobenzene (877 mg, 4.71 mmol, 1.5 eq) was added to the mixture. After purging with $N_2$ three times, the mixture was stirred at 90 °C overnight. The completion of the reaction was monitored by TLC. The reaction solution was then diluted with EA (10 mL) and stirred for 5 minutes, followed by the addition of water (10 mL). The mixture was allowed to stand for partitioning. The aqueous phase was extracted once with EA (10 mL). The combined organic phases were washed with saturated NaCl solution (15 mL × 3), dried over anhydrous sodium sulfate, and filtered. The filtrate was spun-dried, and the crude product was subjected to column chromatography (EA in PE from 0% to 40%) to obtain crude Compound 2 as a yellow solid (1.13 g, 110% yield). LC-MS[M+1]: 326.

2. Synthesis of compound 3

In a 50 mL three-necked flask, Compound 2 (1.13 g, 3.47 mmol, 1.0 eq), ammonium chloride (445 mg, 6.95 mmol, 2.0 eq), and iron powder (584 mg, 10.4 mmol, 3.0 eq) were dispersed in 12.5 mL of a solvent mixture of ethanol and water at a ratio of 5:1. The mixture was heated to reflux and reacted for 4 hours. TLC analysis showed that the starting material had disappeared. The solvent was removed by rotary evaporation, and the crude product was subjected to column chromatography (EA in PE from 0% to 40%) to obtain Compound 3 as a colorless oil (946 mg, 92.3% yield). LC-MS [M+1]: 151.

3. Synthesis of compound 4

In a 50 mL round-bottom flask, Compound 3 (946 mg, 3.2 mmol, 1.0 eq) and 3-bromopiperidin-2,6-dione (923 mg, 4.8 mmol, 1.5 eq) were dissolved in 10 mL of N,N-dimethylformamide (DMF), and sodium bicarbonate (538 mg, 6.4 mmol, 2.0 eq) was added to the mixture. The reaction solution was stirred at 70 °C for 16 hours under nitrogen protection. After cooling to room temperature, the completion of the reaction was monitored by TLC. The reaction solution was then diluted with EA (10 mL), and then 10 mL of water was added for partitioning. The aqueous phase was extracted with 10 mL of EA. The combined organic phases were dried over anhydrous sodium sulfate and filtered. The crude product was purified by column chromatography (EA in PE from 0% to 55%) to obtain Compound 4 (798 mg, 61.4% yield). LC-MS[M+1]: 407.

4. Synthesis of compound 5

In a 50 mL round-bottom flask, Compound 4 (798 mg, 1.96 mmol, 1.0 eq) was dissolved in 10 mL of dichloromethane (DCM), and trifluoroacetic acid (2 mL) was added to the mixture. The reaction solution was stirred at room temperature for 2 hours. The completion of the reaction was monitored by LCMS. The reaction solution was then evaporated to dryness to obtain Compound 5 (600 mg, 100% crude). LC-MS[M+1]: 307.

5. Synthesis of compound 6

In a 50 mL round-bottom flask, Compound 5 (300 mg, 1.96 mmol, 1.0 eq) and tert-butyl bromoacetate (460 mg, 2.36 mmol, 1.2 eq) were dissolved in 6 mL of N, N-dimethylformamide, and DIPEA (761 mg, 5.9 mmol, 3.0 eq) was added to the mixture. The reaction solution was stirred at room temperature for 12 hours. The completion of the reaction was monitored by TLC. The reaction solution was diluted with EA (10 mL), and water (10 mL) was added for partitioning. The aqueous phase was extracted with EA(10 mL). The combined organic phases were dried over anhydrous sodium sulfate and filtered. The crude product was purified by column chromatography (EA in PE from 0% to 70%) to obtain Compound 6 (707 mg, 85.6% yield). LC-MS[M+1]: 421.

6. Synthesis of Compound 7 (i.e. M1)

In a 50 mL round-bottom flask, Compound 6 (300 mg, 0.71 mmol, 1.0 eq) was dissolved in 3 mL of dichloromethane, and trifluoroacetic acid (1 mL) was added to the mixture. The reaction solution was stirred at room temperature for 2 hours. The completion of the reaction was monitored by LCMS. The reaction solution was then evaporated to dryness to obtain Compound M1 (260 mg, 100% crude). LC-MS[M+1]: 365.

II. Synthesis of T-029

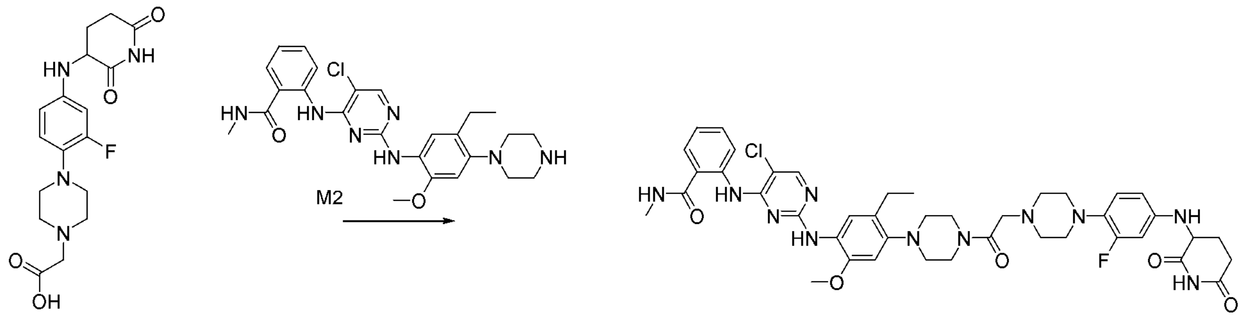
M2

M1

T-029

1. Compound M2 was synthesized with reference to Example 2.
2. Synthesis of T-029

In a 50 mL round-bottom flask, Compound 7 (260 mg, 0.71 mmol, 1.0 eq) and Compound M2 (352.6 mg, 0.71mmol, 1.0 eq) were dissolved in 3 mL of N, N-dimethylformamide, and N, N-diisopropylethylamine (276 mg, 2.14 mmol, 3.0 eq) was added to the mixture. The reaction solution was cooled to 0 °C in an ice-water bath under nitrogen protection. Then HATU (325 mg, 0.85 mmol, 1.2 eq) was added to the reaction solution under stirring, and the reaction was carried out in the ice-water bath for 2 hours under nitrogen protection. The mixture was allowed to warm up to room temperature naturally, and the completion of the reaction was monitored by TLC. The reaction solution was then diluted with EA (10 mL), and 10 mL of water was added. After stirring for 5 minutes, the mixture was allowed to stand for partitioning. The aqueous phase was extracted with 10 mL of EA. The combined organic phases were dried over anhydrous sodium sulfate and filtered. The crude product was purified by column chromatography (MeOH in DCM from 0% to 3%) to obtain Compound T-029 (244 mg, 40.73% yield). LC-MS[M+1]: 842.36. 1H NMR (400 MHz, DMSO-d6) δ 11.63(s, 1H), 10.81 (s, 1H), 8.75 (q, J = 4.6 Hz,1H), 8.57 (d, J = 8.4 Hz, 1H), 8.22 (s,1H), 8.14 (s, 1H), 7.71 (d, J = 7.9 Hz,1H), 7.51 (s, 1H),7.31 (t, J = 7.9 Hz, 1H),7.06 (t, J = 7.6 Hz, 1H), 6.84 (d, J = 9.4Hz, 1H), 6.80 (s, 1H), 6.52 (dd, J = 15.0,2.5 Hz, 1H), 6.41 (dd, J = 8.8, 2.5 Hz,1H), 5.84 (d, J = 7.7 Hz, 1H), 3.74 (d, J =14.5 Hz, 5H), 3.64 (s, 1H), 3.29 (d, J = 9.3Hz, 1H), 2.89 (s, 5H), 2.85 - 2.78 (m, 6H),2.73 (td, J = 12.3, 6.1 Hz, 2H), 2.62 (q, J= 7.1 Hz, 6H), 2.08 (dq,J = 13.3, 4.7 Hz,1H), 1.85 (qd, J =

12.3, 4.7 Hz, 1H), 1.23(d, J = 10.7 Hz, 1H), 1.09 (t, J = 7.5 Hz,3H).

**Example 9**

The compound synthesized in the present invention:

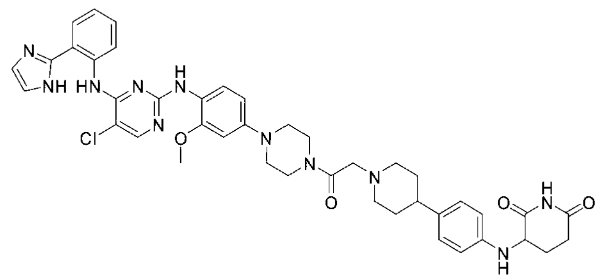

T-011

The synthesis route is as follows:

I. Synthesis of Intermediate M1

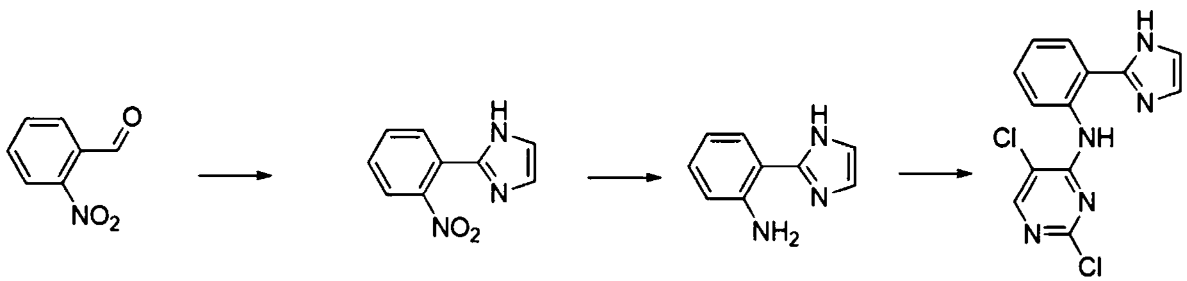

1 2 3 M1

1. Synthesis of compound 2

To a 100 mL three-necked flask was added Compound 1 (500 mg, 1.0 eq) and 2 mL of methanol, and the mixture was stirred to dissolve. Then glyoxal (960 mg, 2.0 eq) was added to the reaction solution. After mixing evenly, the mixture was cooled to 0 °C, and 4 mL of ammonia water was added. The mixture was allowed to warm up to room temperature naturally and then reacted overnight. After the completion of the reaction was monitored by TLC, the reaction solution was then extracted with ethyl acetate. The organic phase was mixed with silica gel for sample application and subjected to column chromatography. The target fraction was collected to obtain 330 mg of the product. LC-MS [M+1]: 190.

2. Synthesis of compound 3

To a 50 mL three-necked flask was added compound 2 (330 mg, 1.0 eq), palladium on carbon catalyst (16 mg, 5 wt%), and 5 mL of methanol and mixed uniformly. After purging with a hydrogen balloon three times, the reaction was carried out at room temperature under pressure maintenance for 20 hours. TLC analysis showed that the starting material had reacted completely. Then the reaction solution was filtered through a diatomaceous earth pad, and the mother liquor was collected and subjected to column chromatography to obtain 230 mg of Compound 3, LC-MS[M+1]:160.

3. Synthesis of intermediate M1

To a 100 mL single-necked flask was added 230 mg (1.0 eq) of Compound 3, and a mixture of 1.3 mL (5.0 eq) of N, N-diisopropylethylamine and 2 mL of isopropanol was added. The mixture was stirred uniformly at room temperature. Then 530 mg (2.0 eq) of trichloropyrimidine was slowly added dropwise. After the addition was completed, the reaction was purged with nitrogen three times. Under nitrogen protection, the temperature was raised to reflux, and the reaction was

stirred overnight. TLC analysis monitored the completion of the reaction. The reaction solution was evaporated to dryness under reduced pressure to remove the solvent, and 50 mL of ethyl acetate (EA) was added to the residue for slurry at room temperature for 30 minutes. The mixture was suction-filtered, and the filter cake was rinsed twice with 15 mL of ethyl acetate. The collected filter cake was Intermediate M1 (300 mg), LC-MS[M+1]: 305.

II. Synthesis of Intermediate M2

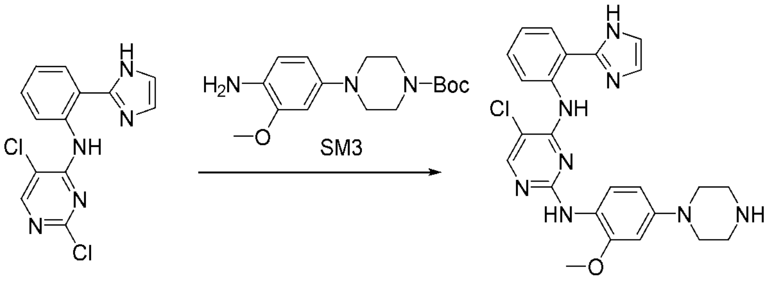


M1 M2

To a 50 mL single-necked flask was added intermediate M1 (150 mg, 1.0 eq), SM3 (150 mg, 1.0 eq), p-toluenesulfonic acid monohydrate (112 mg, 1.2 eq), and 4 mL of isopropanol. The mixture was uniformly mixed and heated to reflux overnight, and the completion of the reaction was monitored by LCMS. Saturated aqueous sodium bicarbonate solution was added to the reaction solution to adjust the pH to >7. The mixture was extracted with ethyl acetate; the organic phase was washed with saturated saline and dried over anhydrous sodium sulfate, then mixed with silica gel (100-200 mesh) for sample application, and purified by column chromatography (MeOH in DCM 0% to 20%) to obtain the target product M2 (100 mg, brown solid). LC-MS[M+1]: 476.

III. Synthesis of compound T-011

The synthesis route is as follows:

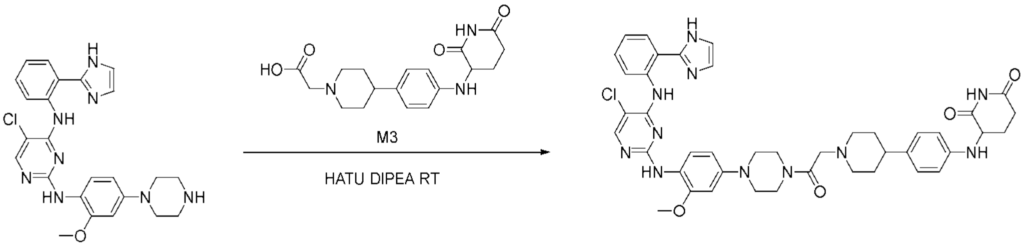


M2 T-011

In a 50 mL round-bottom flask, intermediate M2 and intermediate M3 were dissolved in 2 mL of N, N-dimethylformamide. The mixture was cooled to 0 °C in an ice-water bath, and N, N-diisopropylethylamine and HATU were added under stirring. The reaction was carried out in the ice-water bath for 1 hour under nitrogen protection, and then allowed to warm up to room temperature naturally. The completion of the reaction was monitored by TLC. Then the reaction solution was extracted with ethyl acetate and water; the organic phase was dried and filtered, and subjected to column chromatography to obtain 33 mg of Compound T-011. LC-MS [M+1]: 804.

The following compounds were synthesized with reference to the method of Example 9:

| Compound No. | Structure | Characterization data: |
| --- | --- | --- |
| T-017 | | LC-MS[M+1]: 818.90 |

**Example 10**

The compound synthesized in the present invention:

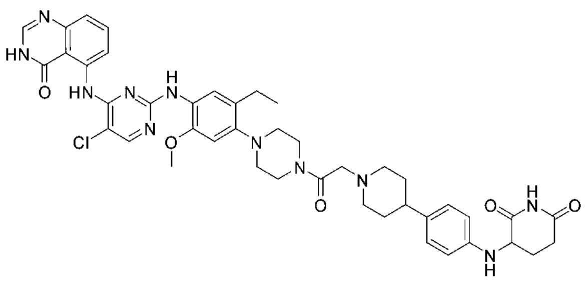

T-076

I. Synthesis of Intermediate M1

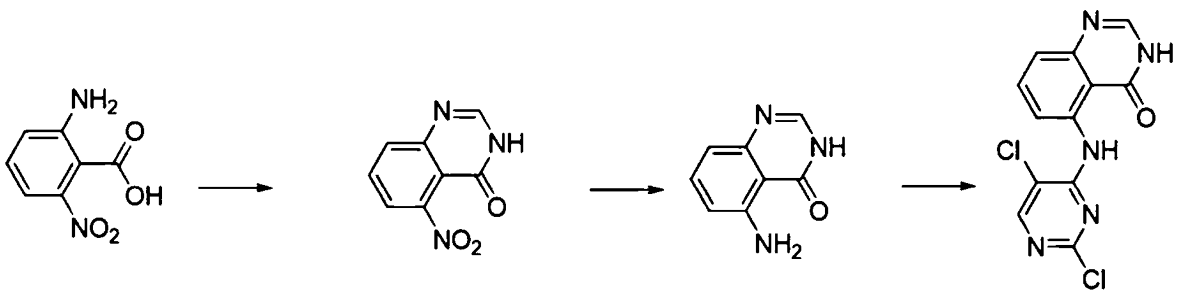

1 2 3 M1

1. Synthesis of compound 2

To a 100 mL three-necked flask was added Compound 1 (1 g, 1.0 eq) and formamidine acetate (1.15 g, 2.0 eq), followed by the addition of 15 mL of ethylene glycol monomethyl ether. The mixture was heated to 100 °C and reacted overnight. After the completion of the reaction was monitored by TLC, the pH of the reaction solution was adjusted to > 7 using saturated aqueous sodium bicarbonate solution. The reaction solution was extracted with ethyl acetate, and the organic phase was mixed with silica gel for sample application and then subjected to column chromatography. The target fraction was collected to obtain 720 mg of the product.

2. Synthesis of compound 3

To a 50 mL single-necked flask was added compound 2 (720 mg, 1.0 eq), palladium on carbon catalyst (36 mg, 5 wt%), and 10 mL of methanol, and mixed uniformly. After purging with a hydrogen balloon three times, the reaction was carried out at room temperature under pressure maintenance for 20 hours. TLC analysis showed that the starting material had reacted completely. Then the reaction solution was filtered through a diatomaceous earth pad, and the mother liquor was collected and subjected to column chromatography to obtain 450 mg of Compound 3. LC-MS[M+1]: 162.

3. Synthesis of intermediate M1

To a 100 mL single-necked flask was added 300 mg (1.0 eq) of Compound 3, a mixture of 1.6 mL (5.0 eq) of N, N-diisopropylethylamine and 5 mL of isopropanol was added, and the mixture was stirred uniformly at room temperature. Then 682 mg (2.0 eq) of trichloropyrimidine was slowly added dropwise. After the addition was completed, the reaction solution was purged with nitrogen three times. Under nitrogen protection, the temperature was raised to reflux, and the reaction was stirred overnight. TLC analysis monitored the completion of the reaction. The reaction solution was evaporated to dryness under reduced pressure to remove the solvent, and 50 mL of ethyl acetate (EA) was added to the residue for slurry at room temperature for 30 minutes. The mixture was suction-filtered, and the filter cake was rinsed twice with 15 mL of ethyl acetate. The collected filter cake was Intermediate M1 (250 mg), LC-MS[M+1]: 307.

II. Synthesis of Intermediate M2

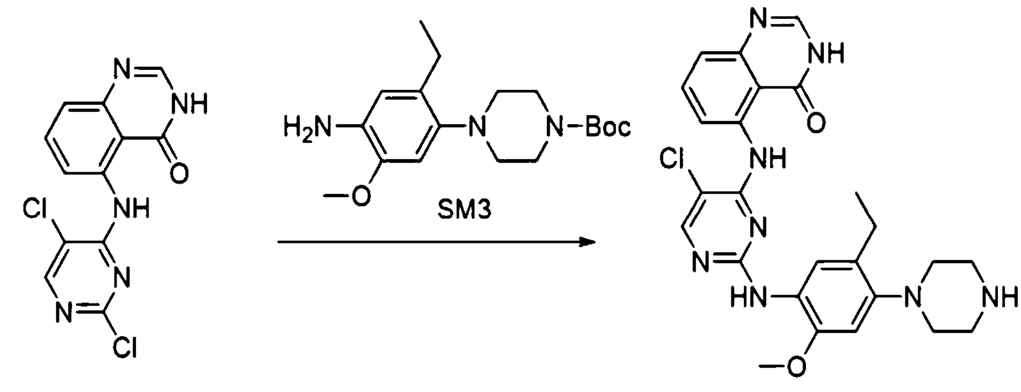


M1 M2

To a 50 mL single-necked flask was added intermediate M1 (250 mg, 1.0 eq), SM3 (250 mg, 1.0 eq), p-toluenesulfonic acid monohydrate (184 mg, 1.2 eq), and 4 mL of isopropanol. The mixture was uniformly mixed and heated to reflux overnight, and the completion of the reaction was monitored by LCMS. Saturated aqueous sodium bicarbonate solution was added to the reaction solution to adjust the pH to >7. The mixture was extracted with ethyl acetate; the organic phase was washed with saturated saline and dried over anhydrous sodium sulfate, then mixed with silica gel (100-200 mesh) for sample application and purified by column chromatography (MeOH in DCM from 0% to 20%) to obtain the target product M2 (60 mg, brown solid). LC-MS[M+1]: 507.

III. Synthesis of compound T-076

The synthesis route is as follows:

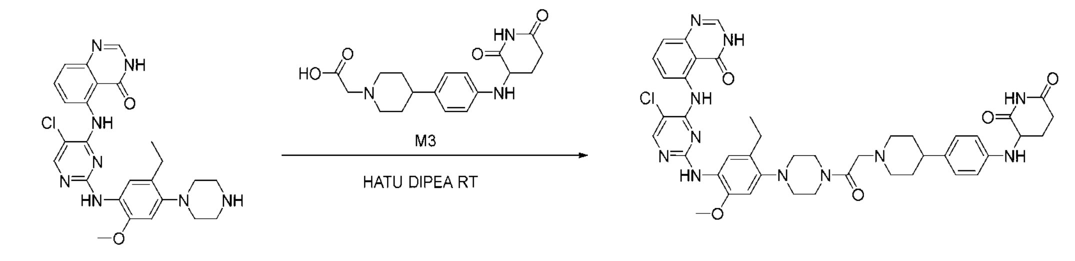


M2 T-076

In a 50 mL round-bottom flask, intermediate M2 (60mg, 1.0eq) and intermediate M3 were dissolved in 2 mL of N, N-dimethylformamide. The mixture was cooled to 0 °C in an ice-water bath, and N, N-diisopropylethylamine and HATU were added under stirring. The reaction was carried out in the ice-water bath for 1 hour under nitrogen protection, and then allowed to warm up to room temperature naturally. The completion of the reaction was monitored by TLC. Then the reaction solution was extracted with ethyl acetate and water; the organic phase was dried and filtered, and subjected to column chromatography to obtain 11 mg of Compound T-076. LC-MS [M+1]: 834.6. 1H NMR (400 MHz, DMSO-d6) δ 12.57 (s, 1H), 12.52 (s, 1H), 10.86 (s, 1H), 8.80 (s, 1H), 8.55 (s, 1H), 8.27 (d, J = 1.7 Hz, 1H), 8.18 (s, 1H), 7.58 (s, 1H), 7.51 (d, J = 8.1 Hz, 1H), 7.32 (d, J = 8.1 Hz, 1H), 7.02 (d, J = 8.1 Hz, 2H), 6.89 (s, 1H), 6.66 (d, J = 8.1 Hz, 2H), 5.74 (d, J = 7.5 Hz, 1H), 4.37 - 4.28 (m, 1H), 3.89 - 3.76 (m, 5H), 3.73 (s, 2H), 3.57 (s, 1H), 3.09 (s, 2H), 3.00 (s, 2H), 2.92 (s, 2H), 2.73 (q, J = 7.5 Hz, 3H), 2.45 (s, 2H), 2.27 (s, 2H), 2.19 - 2.10 (m, 2H), 2.06 (d, J = 1.9 Hz, 1H), 1.93 (dd, J = 12.4, 8.1 Hz, 2H), 1.80 (s, 2H), 1.72 (s, 2H), 1.21 (t, J = 7.5 Hz, 3H).

**Comparative Example 1 Control Compound 1**

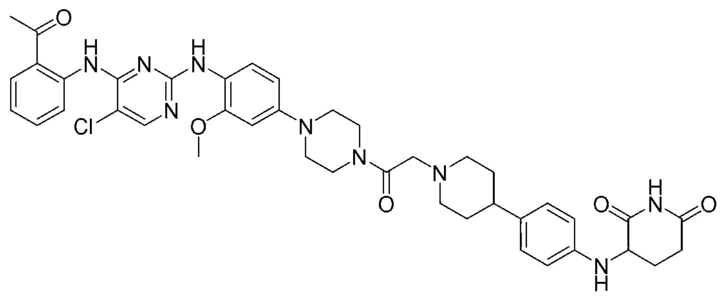

The experimental procedure is as follows:

I. Synthesis of Intermediate M1

The synthesis route is as follows:

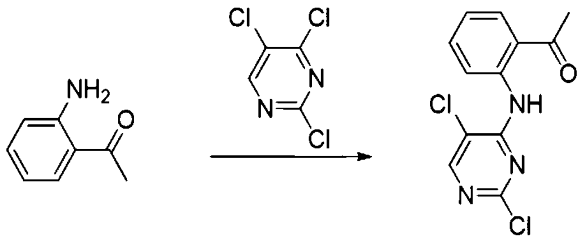

M1

Intermediate M1 was synthesized with reference to the method of Example 1

II. Synthesis of Intermediate M2

The synthesis route is as follows:

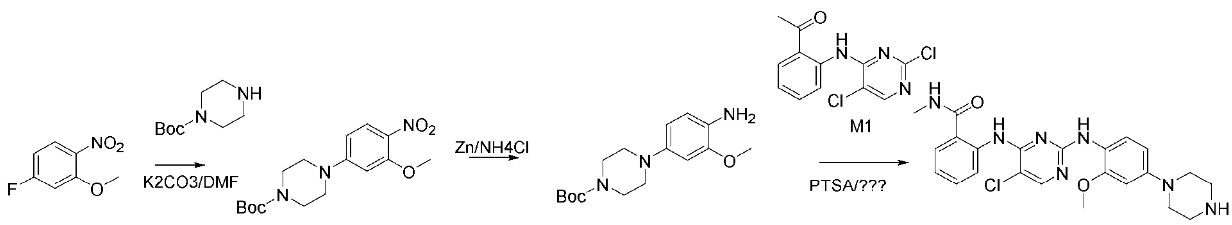


M1

2 3 4 M2

Intermediate M2 was synthesized with reference to the method of Example 1

III. Synthesis of reference Control Compound 1

The synthesis route is as follows:

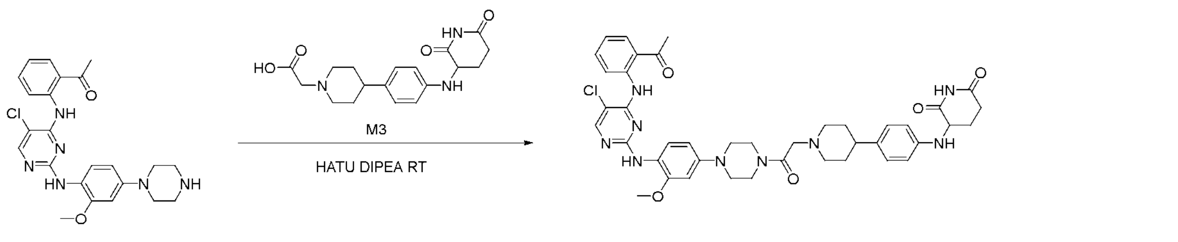


M3

M2

In a 50 mL round-bottom flask, Compounds M2 and M3 were dissolved in 2 mL of N, N-dimethylformamide. The

mixture was cooled to 0 °C in an ice-water bath, and N, N-diisopropylethylamine and HATU were added under stirring. The reaction was carried out in the ice-water bath for 1 hour under nitrogen protection, and then allowed to warm up to room temperature naturally. The completion of the reaction was monitored by TLC. Then the reaction solution was extracted with ethyl acetate and water. The organic phase was dried and filtered, and subjected to column chromatography to obtain 50 mg of Control Compound 1. LC-MS [M+1]: 783; $^1$H NMR: 1H NMR (400 MHz, DMSO-d6) δ 11.83 (s, 1H), 10.79 (s, 1H), 8.77 (s, 1H), 8.36 (s, 1H), 8.16 (s, 1H), 8.04 (d, J = 8.0 Hz, 1H), 7.37 (d, J = 10.4 Hz, 2H), 7.06 (t, J = 7.6 Hz, 1H), 6.95 (d, J = 8.1 Hz, 2H), 6.76 - 6.68 (m, 2H), 6.57 (dd, J = 16.5, 7.3 Hz, 3H), 5.67 (d, J = 7.5 Hz, 1H), 4.25 (s, 0H), 3.77 (s, 5H), 3.64 (s, 2H), 3.23 (s, 4H), 3.14 (s, 2H), 2.94 (s, 2H), 2.78 - 2.70 (m, 0H), 2.67 (s, 3H), 2.58 (d, J = 4.3 Hz, 0H), 2.34 (s, 1H), 2.15 - 2.03 (m, 3H), 1.85 (dt, J = 12.2, 6.1 Hz, 1H), 1.72 (d, J = 11.7 Hz, 2H), 1.58 (d, J = 12.3 Hz, 2H).

**Tests of the inhibitory activity of the compounds on cell growth.**

Test example 1 Cell antiproliferative assay

I. Experimental materials and equipment:

H1975 has EGFR T790M/L858R dual mutation, PC-9 has EGFR exon 19 deletion mutation, and HCC827 has exon 19 deletion mutation. CellCounting Lite 2.0, Trypsin EDTA, 37 °C CO2 incubator, cell counter, EnVision Serial No.1050454.

II. Experimental preparation:

1. 96-well plate seeding

(A) Trypsin EDTA was used to digest log-phase cells, medium was added to terminate the digestion reaction, and a cell suspension was prepared by blowing and mixing with a pipette.
(B) Cell concentration was determined using Vi-cell, and a suspension of 15,000-25,000 cells per milliliter was made depending on the purpose of the experiment and the characteristics of the cells.
(C) After the cell suspension was ready, it was gently mixed and each well was added with 100μL so that the cell density to be tested was 1,500- 2,500 per well.

2. Compound treatment

Compound dilution

A) About 2 mg of compound was weighed, and the volume of DMSO required was calculated according to the formula: compound mass (mg)*compound purity (%)/compound molecular weight*1000.
(B) The inoculated cell culture plates were incubated in an incubator. About 24 hours later, compounds were added at gradient concentrations.
(C) 10mM of compound reservoir solution was diluted to 50mM with medium, and 50mM of the compound solution was added sequentially to the second column of the deep-well plate, and 375ml of medium containing 0.5% DMSO was added to the third to eleventh columns.
D) Gradient dilution: 125 μl of solution was pipetted from the second column and added to the third column, after mixing; another 125 μl of solution was pipetted from the third column and added to the fourth column, and the operation was repeated until the tenth column.
(E) 25 μl of compound was pipetted from the deep-well plate into a 96-well culture plate using a multichannel pipette, and each compound was run in triplicate on the 96-well plate. A 1:4 concentration gradient was established on the 96-well plate, with a final highest concentration of 10,000 nM.

3. Addition of CTG and readings

(A) 96-well plates were incubated in an incubator for 72 h. The effect of the compounds was observed under an

inverted microscope.

(B) 25 μl of CTG solution was added to each well. The plate was incubated on a shaker for 10 minutes and the OD value of each well was read.

4. Data analysis

The survival rate ( % Cell Viability) was calculated using the following formula:

$$\%Cell\ Viability=100\%\times(Lum_Sample - Lum_LC)/(Lum_HC - Lum_LC)$$

Lum_HC: Cell reading of 0.1% DMSO control group
Lum_Sample: Cell readings of compound addition group
Lum_LC: Reading of blank medium
IC50 values were obtained by curve fitting using GraphPad Prism 8 software.

As shown in Table 1, AA<10nM; 10nM<A≤100nM; 100nM<B<1000nM; C≥1000nM.

Table 1

| Compound | H1975 | PC-9 | HCC827 |
|---|---|---|---|
| T-001 | A | B | B |
| T-003 | A | B | A |
| T-007 | A | B | B |
| T-011 | A | B | B |
| T-017 | A | B | B |
| T-021 | AA | B | A |
| T-023 | AA | B | A |
| T-029 | A | B | B |
| T-037 | A | B | B |
| T-043 | AA | B | A |
| T-045 | B | B | B |
| T-047 | A | B | B |
| T-049 | AA | B | A |
| T-067 | AA | B | B |
| T-079 | A | B | B |
| T-080 | AA | A | A |
| T-086 | B | B | B |
| T-109 | A | A | A |

As can be seen from Table 1, the compounds of the present invention showed excellent inhibitory effects on H1975 (human lung adenocarcinoma cells), PC-9 (human lung cancer cells) and HCC827 (human non-small cell lung cancer cells).

Test Example 2 EGFR PROTAC HTRF Experiment

1. Instruments and reagents

| Reagent | Supplier | Model |
|---|---|---|
| HTRF TOTAL-EGFR DETECTION kit | Cisbio | 64NG1PEG |
| Consumables | Supplier | Model |
| HTRF 96 well low volume plate | Cisbio | 66PL96025 |
| 96 well cell culture plate | Coring | 3599 |
| Instruments | Supplier | Model |
| Vortex Mixer | / | QL-861 |
| Microplate Centrifuge | / | BE-6100 |
| Microplate constant temperature oscillator | Hangzhou Allsheng Instrument Co., Ltd | MB100-4A |
| EnVision | PerkinElmer | 1050454 |
| Cell lines | Supplier | |
| Baf3-19del-T780M-C797S | KYINNO Biotechnology Co., Ltd | |
| Baf3- L858R-T780M-C797S | KYINNO Biotechnology Co., Ltd | |

Among them, Baf3-19del-T780M-C797S is Stable Ba/F3 clone expressing exogenous EGFR gene bearing T790M, C797S and L858R triple amino acid mutations, purchased from KYINNO Biotechnology; Baf3-L858R-T780M-C797S is Stable Ba/F3 clone expressing exogenous EGFR gene bearing exon19 E746_A750 deletion, T790M, C797S triple mutations, purchased from KYINNO Biotechnology.

2 Experimental steps:

1) Cell preparation:

| Adherent cells | Suspended cells |
|---|---|
| 50 μl of cells were seeded in a 96-well cell culture plate and incubated overnight in a 37 °C incubator. | 25 μl of cells were seeded in a 96-well cell culture plate and incubated overnight in a 37 °C incubator. |
| Usually, 50K cells/well were seeded, and the seeding density can be adjusted appropriately according to cells type. | |
| 50 μl of compound (2 x) was added to the cells | 50 μl of compound (6 x) was added to the cells |
| Incubated in a 37 °C incubator for 16 hours | |
| The supernatant was aspirated | The supernatant was not aspirated |

2) Reagent preparation:

| Adherent cells | | Suspended cells |
|---|---|---|
| 1 volume lysis buffer 4X +3 volumes distilled water | 1 volume Blocking reagent+99 volumes lysis buffer 1X | 1 volume Blocking reagent+24 volumes lysis buffer 4X |

According to a volume of 2 μl of antibody per well, Eu and d2 antibodies were separately diluted 20-fold with detection buffer. The two antibody solutions were mixed evenly.

3) Testing:

| Adherent cells | Suspended cells |
|---|---|
| 50 µl of lysis buffer (1 ×) was added and incubated at room temperature for at least 30 minutes | 10 µl of lysis buffer (4 ×) was added and incubated at room temperature for at least 30 minutes |
| Appropriate supplemented lysis buffer was added and shaken at room temperature for cultivation. The lysis culture time can be optimized, and the lysis volume can be reduced to 25 µl. | |
| The lysed cells were mixed evenly with a pipette and 16 µL of it was then transfered from the 96-well cell culture plate to a 96 well low volume plate. (16 µL of lysis buffer was added to the control well) | |
| 4 µL of the mixed antibody solution was added to the detection buffer and the plate was sealed with a sealing membrane. The plate was then centrifuged briefly using a microplate centrifuge. The plate was incubated for 4 hours at room temperature, protected from light. The maximum signal was reached after the incubation period and remained stable for 24 hours. Therefore, readings can be taken anytime between the incubation period and 24 hours. | |

3. Results and Calculations

Open the EnVision software, select the HTRF protocol, set up the plate, and then read the plate.

The fitting curve, DC50, and Dmax of the compound can be generated using GraphPad Prism.

As shown in Table 2, for DC50, AA ≤ 10nM; 10nM<A≤100nM; 100nM<B<1000nM; C≥1000nM; For Dmax, 70% ≤ A ≤ 100%; 50%≤B<70%; C < 50%; NA is untested.

Table 2

| Compound | BAF3-LTC | | BAF3-DTC | |
|---|---|---|---|---|
| | DC50(nM) | Dmax(%) | DC50(nM) | Dmax(%) |
| T-001 | B | A | | |
| T-003 | A | A | A | A |
| T-007 | B | A | | |
| T-011 | B | B | | |
| T-013 | AA | A | A | A |
| T-014 | AA | A | AA | A |
| T-015 | B | A | B | B |
| T-021 | B | A | B | A |
| T-023 | A | A | A | A |
| T-029 | B | A | A | B |
| T-037 | B | B | B | B |
| T-067 | A | A | A | A |
| T-068 | A | A | A | A |
| T-080 | A | A | A | A |
| T-090 | A | A | B | A |
| T-109 | A | B | A | B |

Among them, BAF3-DTC: Baf3-19del-T780M-C797S is Stable Ba/F3 clone expressing exogenous EGFR gene bearing T790M, C797S and L858R triple amino acid mutations, purchased from KYINNO Biotechnology; BAF3-LTC:

Baf3-L858R-T780M-C797S is Stable Ba/F3 clone expressing exogenous EGFR gene bearing exon19 E746_A750 deletion, T790M, C797S triple mutations, purchased from KYINNO Biotechnology.

According to Table 2, the compounds of the present invention exhibit excellent degradation performance towards EGFR cells containing L858R-T780M-C797S triple mutations and 19del-T780M-C797S triple mutations.

**Pharmacokinetic study of the compound**

Test Example 3 Pharmacokinetic Test on Male SD Rats

1.1 Experimental animals

Healthy adult SD rats, male, a total of 3, 6-8 weeks old; Weighing 200-300 grams.

1.2. Instruments and reagents

1.2. 1 Instruments

Analytical balance, animal weight scale, magnetic stirrer, gavage needle, freeze centrifuge, single channel manual pipette, liquid chromatography-mass spectrometry, etc.

1.2.2 Reagents

EDTA-$Na_2$ anticoagulant: 11.2 g of EDTA-$Na_2$ was weighted and placed into a reagent bottle. 100 mL of physiological saline was added and the mixture was shaken until completely dissolved. After preparation, the solution was aliquoted into 1.5 mL centrifuge tubes, each containing approximately 20 uL, for use in whole blood sample collection.

2 Experimental procedures

2.1. Drug preparation

Approximately 10 mg of the test sample was accurately weighed. DMSO accounting for 10% of the total volume was added to dissolve the sample. Subsequently, 0.5% MC solvent accounting for 90% of the total volume was slowly added while stirring. The mixture was sonicated and vortexed evenly, and a visually homogeneous preparation solution was obtained, with a concentration of 1 mg/mL. This solution should be freshly prepared immediately before use.

0.2 mL of the sample was pipetted into a 1.5 mL centrifuge tube and stored at -80°C for concentration analysis of the dosing solution.

2.2 Animal Preparation

Animals were kept in rat cages and fasted for no less than 10 hours on the day before the experiment, but were allowed to drink water. The animals were individually weighed and marked on their tails. Prior to dosing, blank blood samples were collected from each animal via the tail vein.

2.3. Administration

Administration route: gavage (p.o.)
Dosage concentration: 1mg/ml
Dosage: 10 mg/kg
Dosage volume: 10 mL/kg

Operation process: The rat was grasped with a left hand protected by a bite-resistant glove and held in an upright position. The gavage needle was inserted through the mouth and advanced towards the pharynx. The needle was advanced gently, ensuring no significant resistance was felt. The drug was then injected into the stomach.

2.4 Sample Collection

At 0.5 h, 1 h, 2 h, 4 h, 6 h, 8 h, 12 h, and 24 h post-dosing, 0.1-0.2 mL of whole blood was collected from each test animal into EDTA-$Na_2$ anticoagulant tubes. The tubes were mixed by inverting 3-4 times. The samples were centrifuged at 2000 × g and 4 °C for 5 minutes. The upper plasma layer was to separated, promptly transferred to -80 °C for storage until analysis. The blood was collected via tail vein.

3 Sample analysis and data processing

3.1 Sample Analysis

A quantitative detection method for the test compound was established using a Shimadzu liquid chromatography system and a Triple Quad™ 6500+ AB mass spectrometry (MS) system. This method was used to analyze the concentration of the parent drug in plasma. The variation of the analysis results was controlled using quality control (QC) samples, and the accuracy of the QC samples was required to be within the range of 80%-120%.

3.2 Data Processing

The main pharmacokinetic parameters were calculated using the non-compartmental model (NCA) with the WinNonlin Phoenix software. These parameters include: area under the concentration-time curve (AUC(0-t) and AUC(0-∞)), elimination half-life ($T_1/_2$), maximum plasma concentration (Cmax), and time to reach maximum plasma concentration (Tmax), etc.

The results are shown in Table 3.

Table 3

| Compound | Tmax (h) | Cmax (ng/ml) | AUClast (h*ng/ml) | AUCinf (h*ng/ml) | HL_ (h) | MRTlast(h) |
|---|---|---|---|---|---|---|
| Control Compound 1 | 5.33 | 227 | 2055 | 2090 | 3.47 | 7.32 |
| T-013 | 4.00 | 899 | 6689 | 6734 | 3.09 | 6.27 |
| T-014 | 3.33 | 721 | 4510 | 4557 | 3.37 | 5.88 |
| T-015 | 3.33 | 2443 | 25814 | 26077 | 3.18 | 6.83 |
| T-023 | 6.00 | 4063 | 61581 | 76320 | 9.28 | 10.0 |
| T-029 | 4.67 | 1010 | 7903 | 8213 | 2.51 | 5.65 |
| T-037 | 2.67 | 2287 | 24042 | 24369 | 2.61 | 7.49 |
| T-067 | 4.00 | 1023 | 10960 | 11299 | 4.39 | 7.78 |
| T-074 | 5.33 | 1116 | 16947 | 20225 | 7.97 | 9.66 |
| T-078 | 6.00 | 714 | 8603 | 10150 | 5.45 | 9.06 |
| T-079 | 4.67 | 673 | 7716 | 7994 | 4.02 | 4.67 |

From Table 3, it can be seen that the compounds of the present invention have better pharmacokinetic effects than the control compound 1.

All documents referred to in the present inention are incorporated by reference herein as if each document is individually incorporated by reference. Further, it should be understood that upon reading the above teachings of the present invention, various modifications or changes may be made to the present invention by those skilled in the art, and those equivalents also fall within the scope defined by the appended claims of the present invention.

**Claims**

1. A compound, wherein the compound is a compound of Formula I, or a pharmaceutically acceptable salt thereof, a stereoisomer, a tautomer, a hydrate, a solvate, an isotopic compound, or a prodrug thereof,

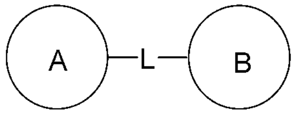
Formula I

wherein,

L is selected from the group consisting of:

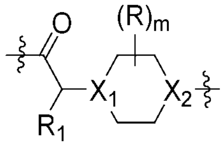,
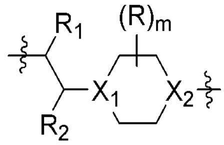,

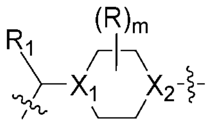,
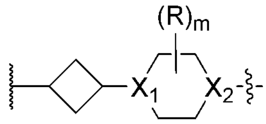
and
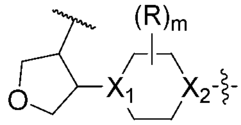;

ring A is selected from the group consisting of:

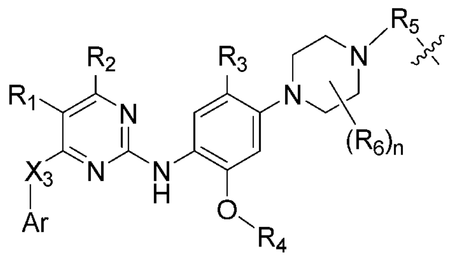

and

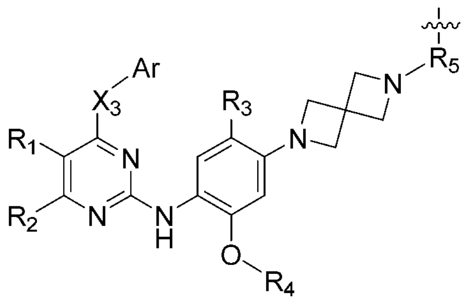;

ring B is selected from the group consisting of:

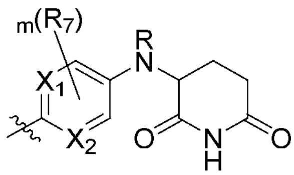,

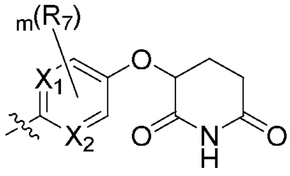, 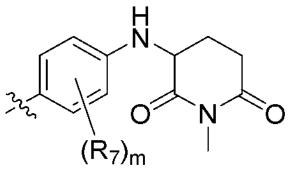, and 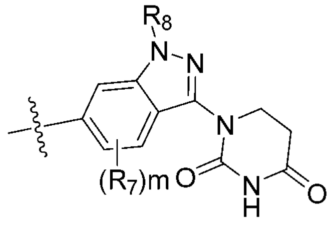;

in each formula:

each of $X_1$ and $X_2$ is independently selected from the group consisting of: CR and N;
each $X_3$ is independently selected from the group consisting of: NH, O and none;
each Ar is independently selected from the group consisting of: substituted or unsubstituted phenyl, substituted or unsubstituted 5-10 membered heteroaryl containing 1, 2, or 3 heteroatoms selected from N, O and S,

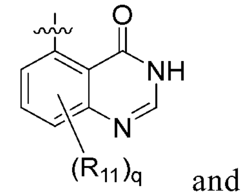 and 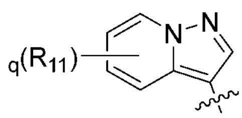;

the "substituted" refers to being substituted by one or more (such as 2, 3, or 4) substituents selected from the group consisting of: halogen, $C_{1-6}$ alkyl, $C_{1-6}$ haloalkyl, hydroxyl substituted $C_{1-6}$ alkyl, $C_{3-6}$ cycloalkyl, $C_{1-6}$ alkyl-NH -, $C_{1-6}$ alkoxy, $C_{1-6}$ haloalkoxy, $C_{3-6}$ halocycloalkyl, cyano, oxo, - $C(O)NR_9R_{10}$, -CO-$C_{1-6}$alkyl, - CO-$C_{3-6}$ cycloalkyl, -CO-$C_{1-6}$ haloalkyl, -CO-$C_{3-6}$ halocycloalkyl, and 5-6 membered heteroaryl containing 1, 2, or 3 heteroatoms selected from N, O and S;
each $R_1$ is independently selected from the group consisting of: hydrogen, deuterium, halogen, $C_{1-6}$ alkyl, $C_{1-6}$ haloalkyl, $C_{3-6}$ cycloalkyl, $C_{3-6}$ halocycloalkyl and cyano;
each of $R_2$ and $R_3$ is independently selected from the group consisting of: hydrogen, deuterium, halogen, $C_{1-6}$ alkyl, $C_{2-6}$ alkenyl, $C_{2-6}$ alkynyl, $C_{3-6}$ cycloalkyl, $C_{1-6}$ alkyl-NH-, $C_{1-6}$ alkoxy, $C_{1-6}$ haloalkoxy, $C_{1-6}$ haloalkyl, cyano, $C_{3-6}$ halocycloalkyl, - $NR_9C(O)R_{10}$, - $C(O)NR_9R_{10}$, methylsulfonyl,

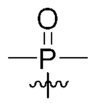,

unsubstituted or $C_{1-6}$ alkyl substituted 5-10 membered heteroaryl containing 1, 2, or 3 heteroatoms selected from N, O and S;
each $R_4$ is independently selected from the group consisting of: $C_{1-6}$ alkyl, $C_{1-6}$ haloalkyl, $C_{3-6}$ cycloalkyl and $C_{3-6}$ halocycloalkyl;
each $R_5$ is independently selected from the group consisting of: absent, $C_{1-6}$ alkyl, NR,

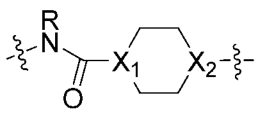, 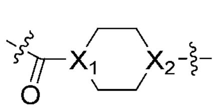 and 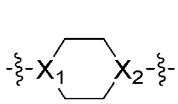;

each of $R_6$, $R_7$, and $R_8$ is independently selected from the group consisting of: H, halogen, $C_{1-6}$ alkyl, $C_{3-6}$ cycloalkyl, $C_{1-6}$ alkyl-NH-, $C_{1-6}$ alkoxy, $C_{1-6}$ haloalkoxy, $C_{1-6}$ haloalkyl, cyano, $C_{3-6}$ halocycloalkyl-$NR_9C(O)R_{10}$, -$C(O)NR_9R_{10}$, methylsulfonyl,

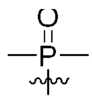,

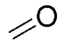

and hydroxyl, or $R_6$ together with the attached ring forms a 3-6 membered ring;
each $R_9$ is independently selected from the group consisting of: H, $C_{1-6}$ alkyl, $C_{1-6}$ haloalkyl, $C_{3-6}$ cycloalkyl and $C_{3-6}$ halocycloalkyl;
each $R_{10}$ is independently selected from the group consisting of: H, $C_{1-6}$ alkyl, $C_{2-6}$ alkenyl, $C_{2-6}$ alkynyl, $C_{3-6}$ cycloalkyl, $C_{1-6}$ alkoxy, $C_{1-6}$ haloalkoxy, $C_{1-6}$ haloalkyl, $C_{3-6}$ halocycloalkyl, methylsulfonyl and

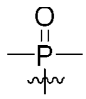 ;

each of m, n, and q is independently selected from the group consisting of: 0, 1, 2, 3, 4 and 5;
each $R_{11}$ is independently selected from the group consisting of: H, halogen, $C_{1-6}$ alkyl, $C_{1-6}$ haloalkyl, $C_{3-6}$ cycloalkyl and $C_{3-6}$ halocycloalkyl; and
each R is independently selected from the group consisting of: H, $C_{1-6}$ alkyl, hydroxyl, halogen and $C_{1-6}$ haloalkyl.

**2.** The compound according to claim 1, wherein

each Ar is independently selected from the group consisting of: substituted phenyl,

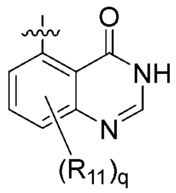 and 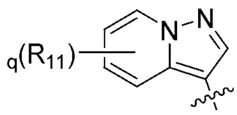 ;

the "substituted" refers to being substituted by one substituent selected from the group consisting of: -C(O) $NR_9R_{10}$ and 5-6 membered heteroaryl containing 1, 2, or 3 N atoms;
$R_{11}$, and q are as defined in claim 1;
$R_9$ is selected from the group consisting of: H and $C_{1-6}$ alkyl;
$R_{10}$ is selected from the group consisting of: H and $C_{1-6}$ alkyl;
and $R_9$ and $R_{10}$ are not simultaneously H.

**3.** The compound according to claim 1, wherein

each Ar is independently selected from the group consisting of: substituted phenyl,

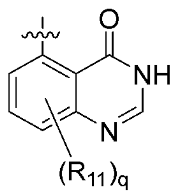 and 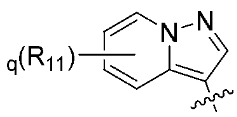 ;

the "substituted" refers to being substituted by one substituent selected from the group consisting of: -C(O) $NR_9R_{10}$ and 5-6 membered heteroaryl containing 1, 2, or 3 N atoms;
$R_{11}$, and q are as defined in claim 1;
$R_9$ is H; and
$R_{10}$ is $C_{1-6}$ alkyl.

**4.** The compound according to claim 1, wherein $R_3$ is selected from the group consisting of: H, $C_{1-6}$ alkyl, $C_{2-6}$ alkenyl, $C_{2-6}$ alkynyl, and $C_{3-6}$ cycloalkyl.

5. The compound according to claim 1, wherein the compound is represented by Formula II, Formula III, Formula IV, Formula V, Formula VI, or Formula VII,

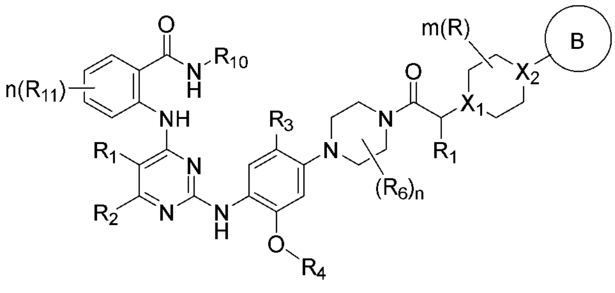

Formula II ,

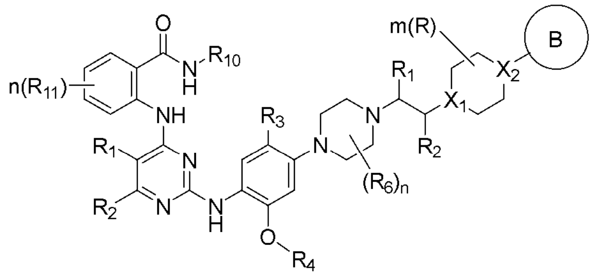

Formula III

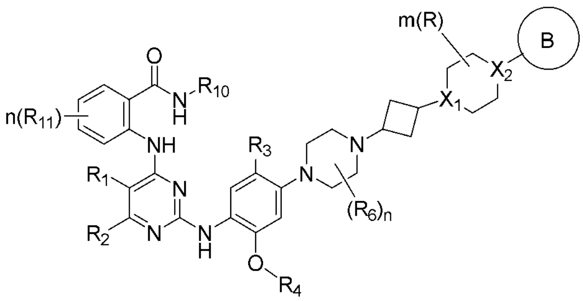

Formula IV ,

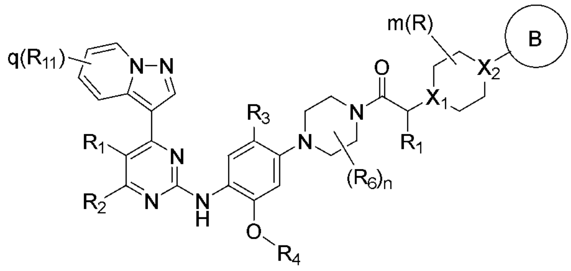

Formula V ,

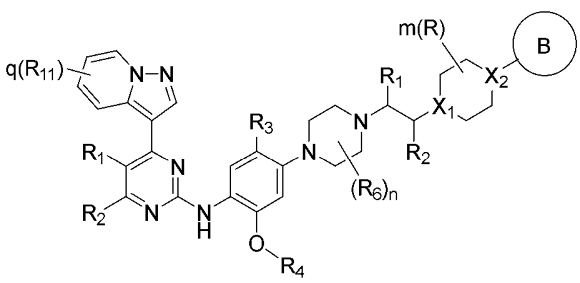

Formula VI ,

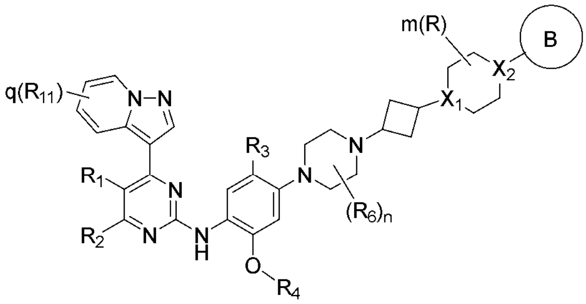

Formula VII ,

wherein, $R_4$ is a $C_{1-6}$ alkyl selected from the group consisting of: methyl, ethyl, propyl, isopropyl, butyl, isobutyl, tert butyl, neopentyl, tert-pentyl, and hexyl.

6. The compound according to claim 1, wherein the compound is selected from the group consisting of:

| | |
|---|---|
| 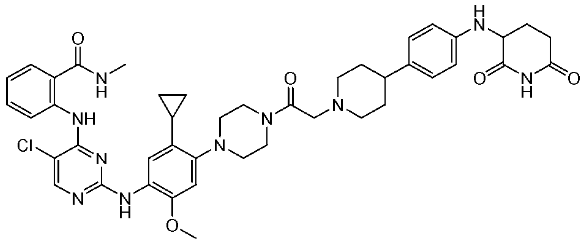 T-001 | 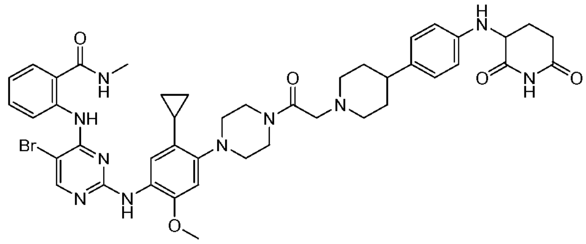 T-002 |
| 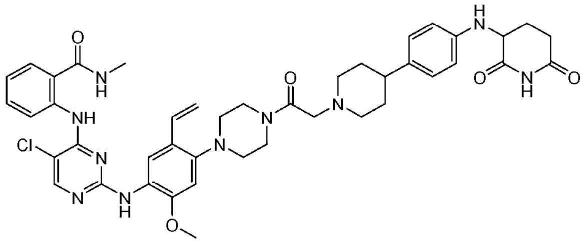 T-003 | 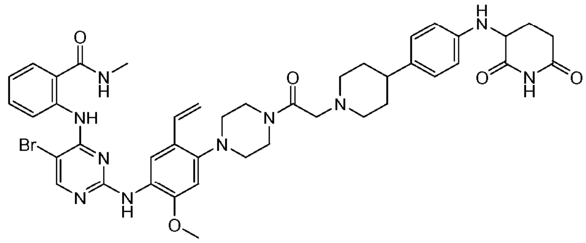 T-004 |

| | |
|---|---|
| 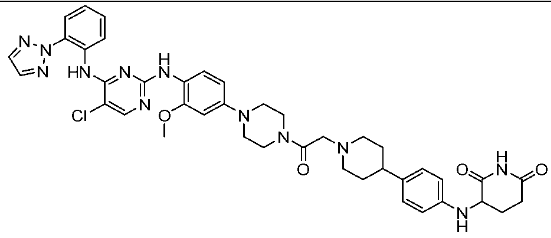<br>T-005 | 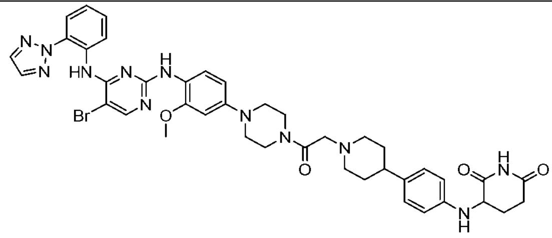<br>T-006 |
| 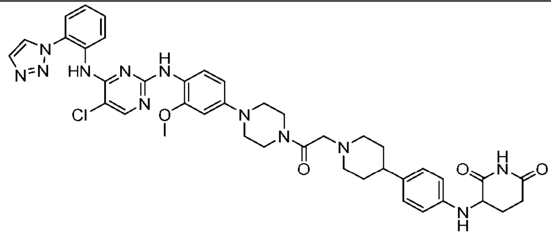<br>T-007 | 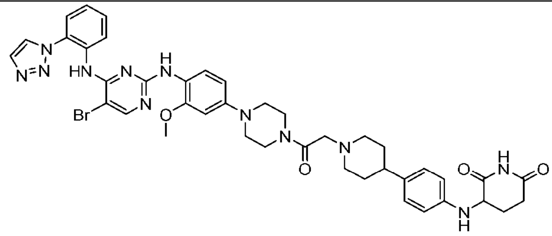<br>T-008 |
| 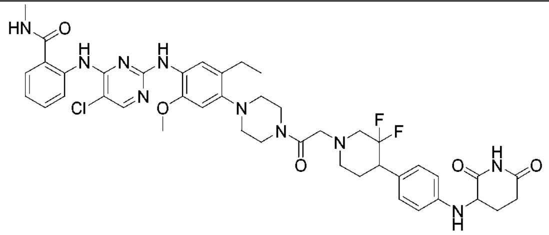<br>T-009 | 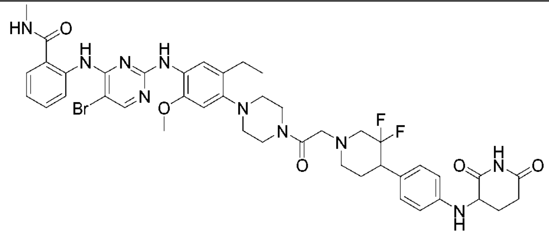<br>T-010 |
| 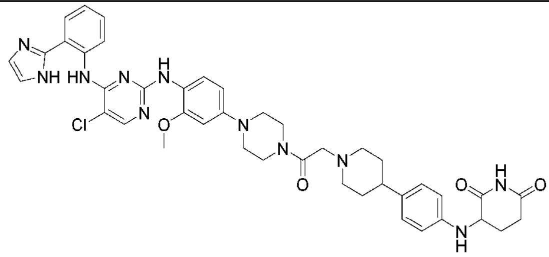<br>T-011 | 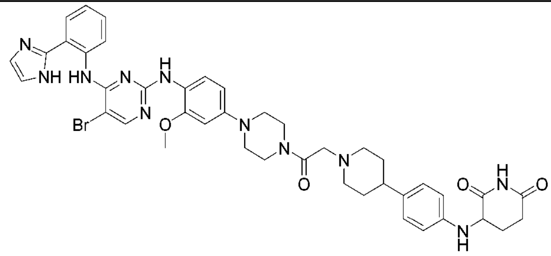<br>T-012 |
| 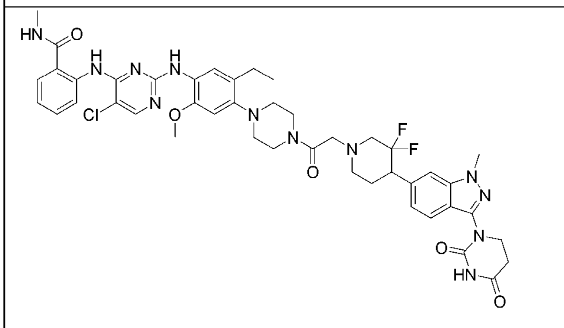<br>T-013 | 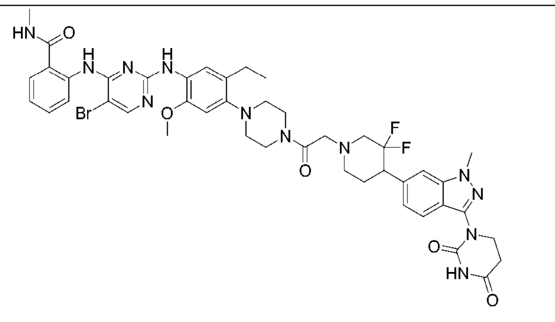<br>T-014 |
| 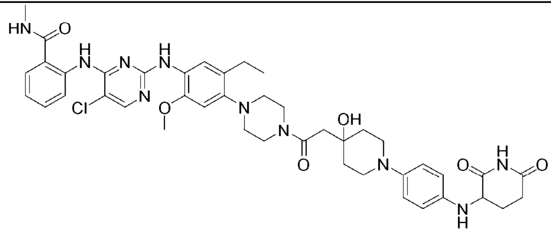<br>T-015 | 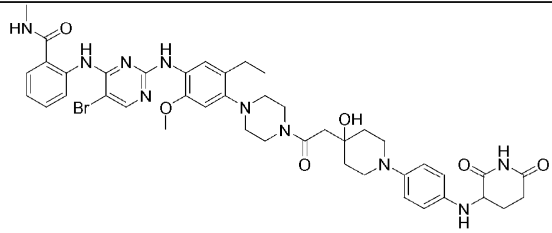<br>T-016 |

| | |
|---|---|
| 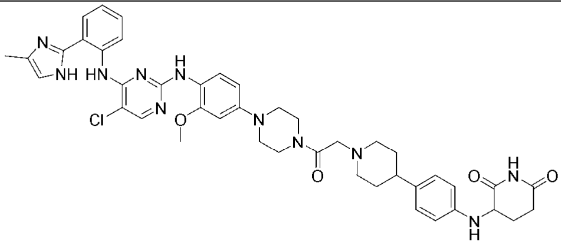 T-017 | 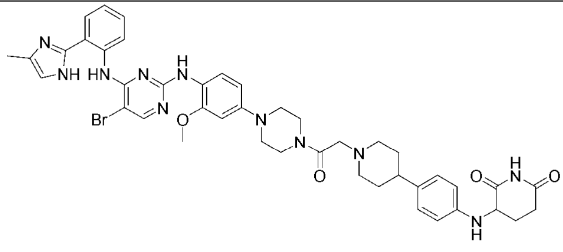 T-018 |
| 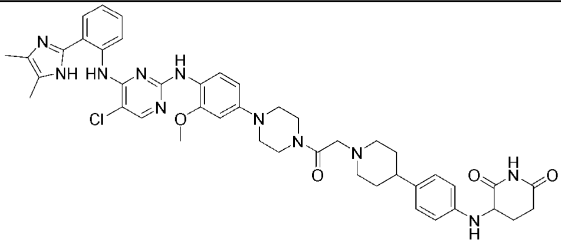 T-019 | 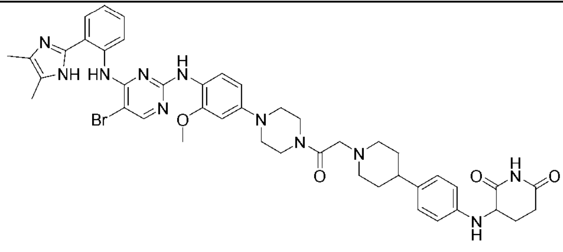 T-020 |
| 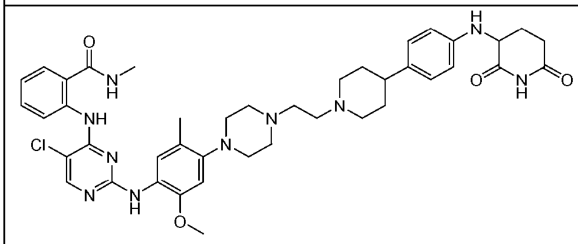 T-021 | 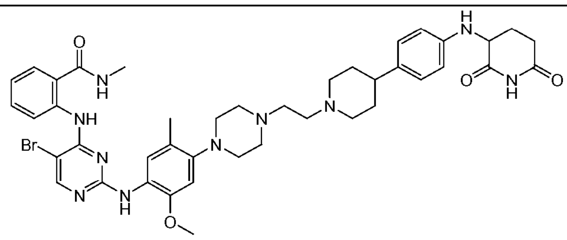 T-022 |
| 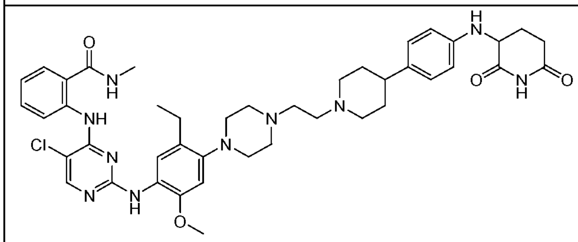 T-023 | 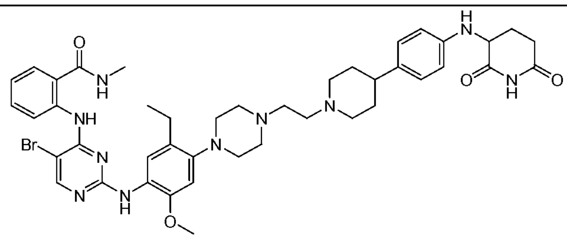 T-024 |
| 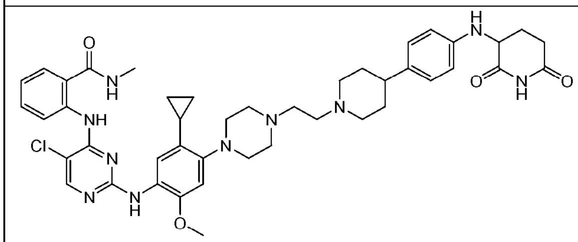 T-025 | 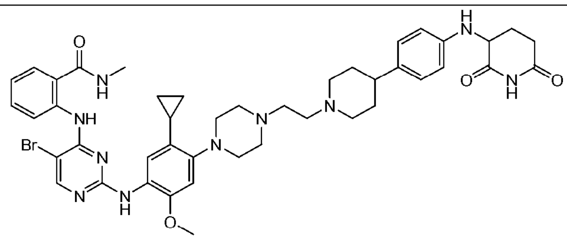 T-026 |
| 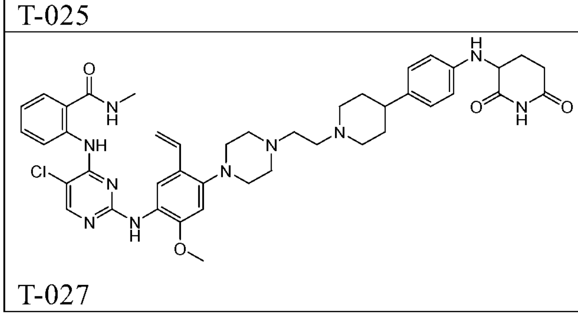 T-027 | 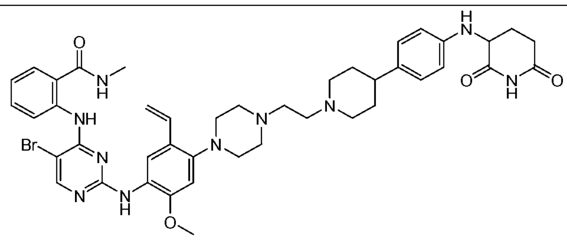 T-028 |

| | |
|---|---|
| 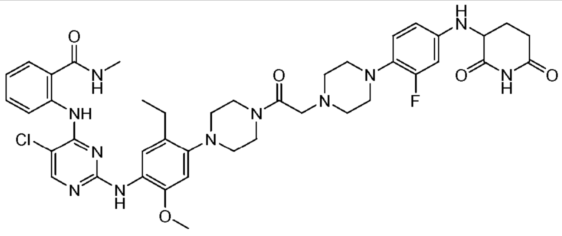<br>T-029 | 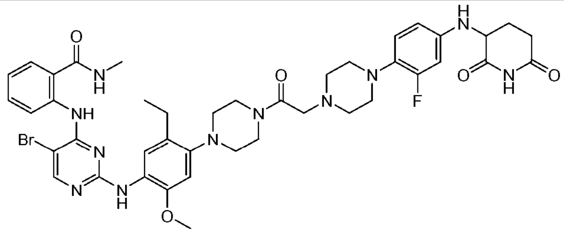<br>T-030 |
| 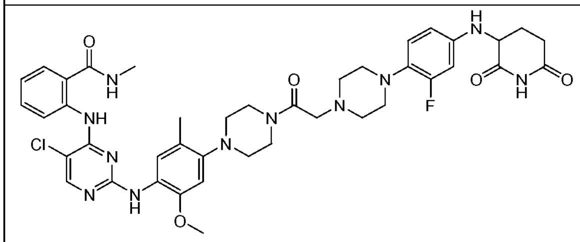<br>T-031 | 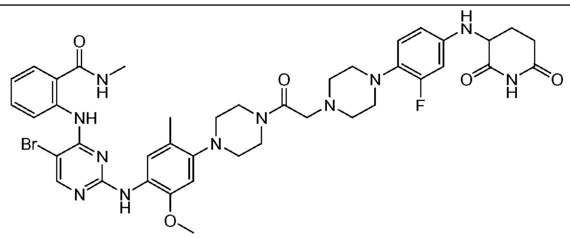<br>T-032 |
| 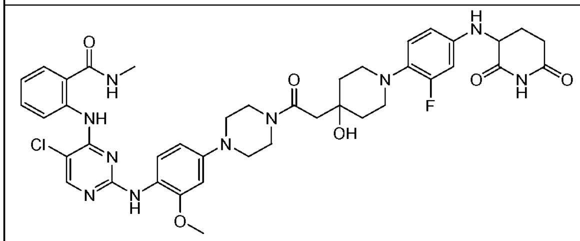<br>T-033 | 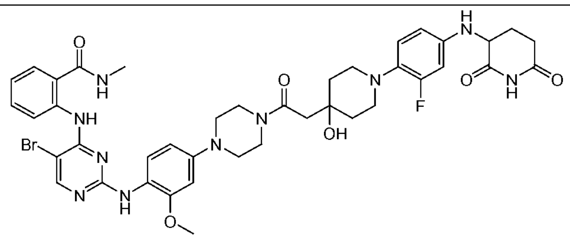<br>T-034 |
| 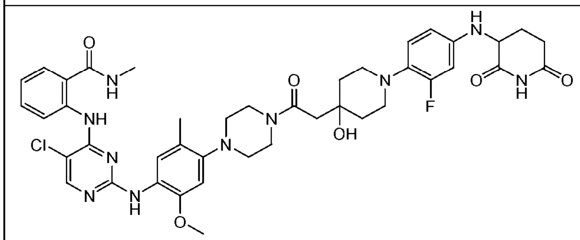<br>T-035 | 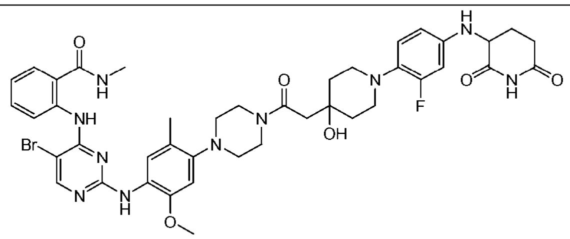<br>T-036 |
| 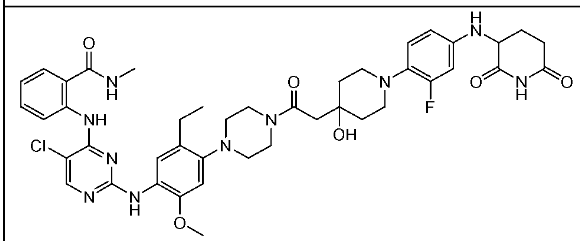<br>T-037 | 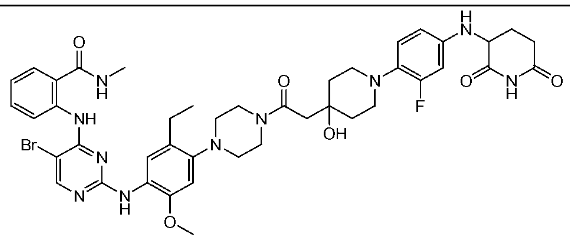<br>T-038 |
| 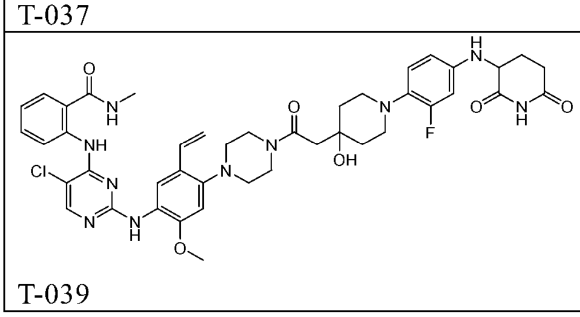<br>T-039 | 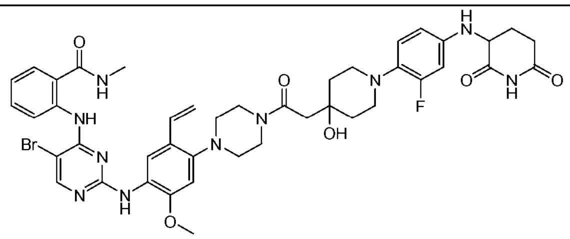<br>T-040 |

| | |
|---|---|
| 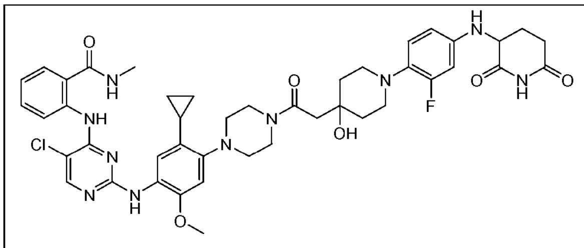 T-041 | 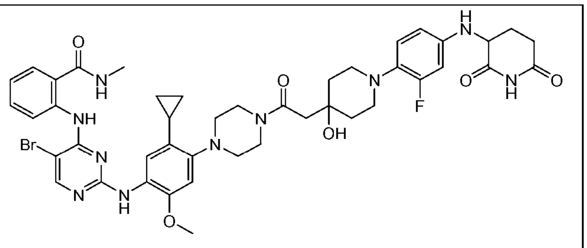 T-042 |
| 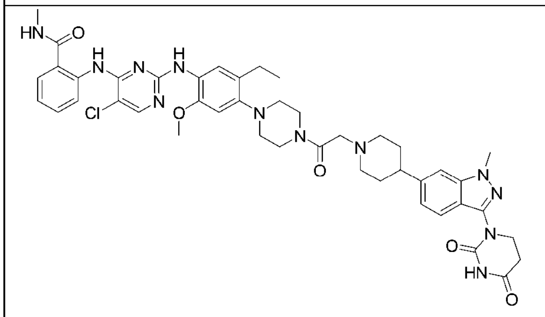 T-043 | 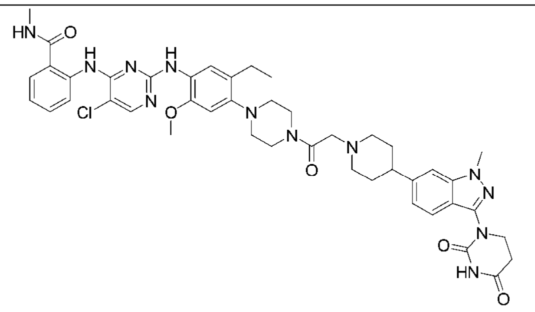 T-044 |
| 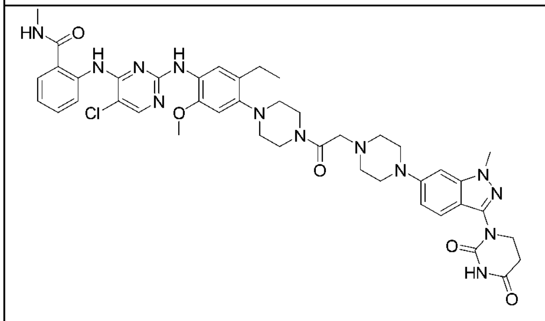 T-045 | 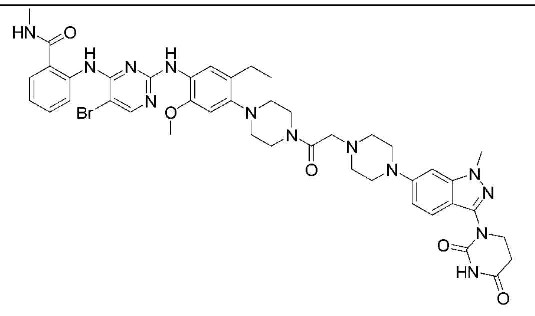 T-046 |
| 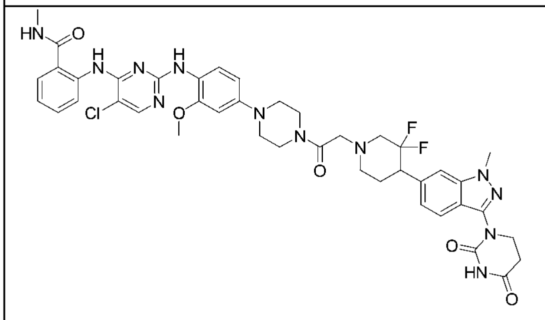 T-047 | 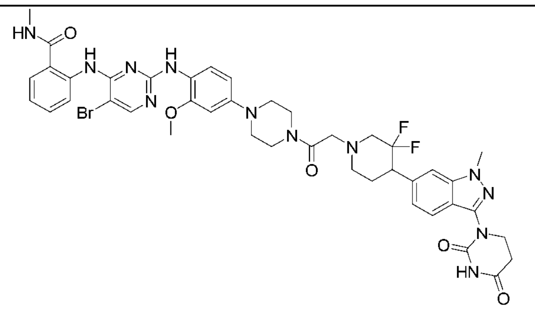 T-048 |
| 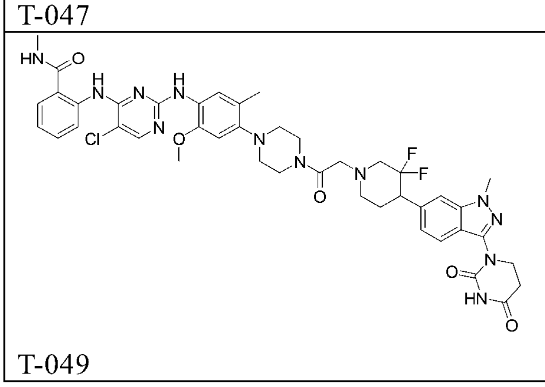 T-049 | 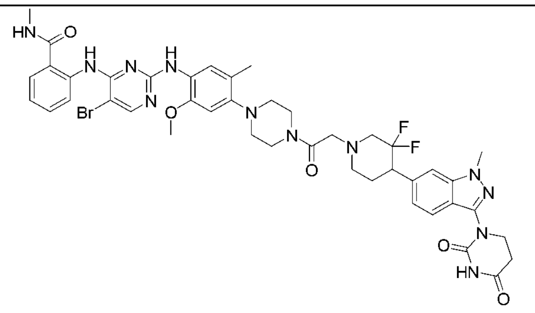 T-050 |

| | |
|---|---|
| 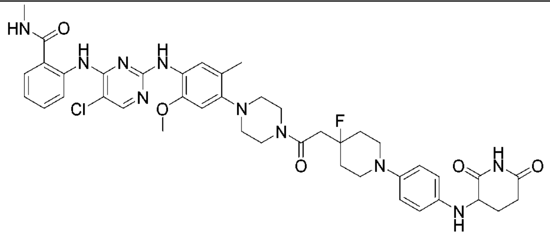 T-051 | 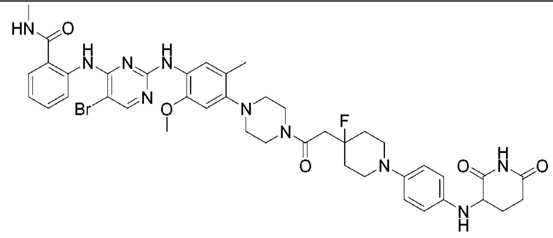 T-052 |
| 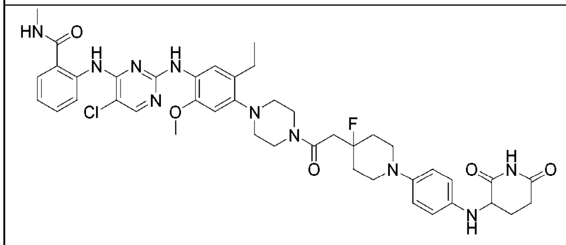 T-053 | 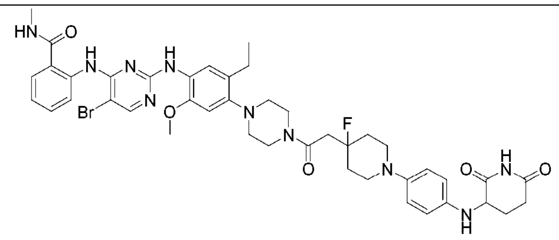 T-054 |
| 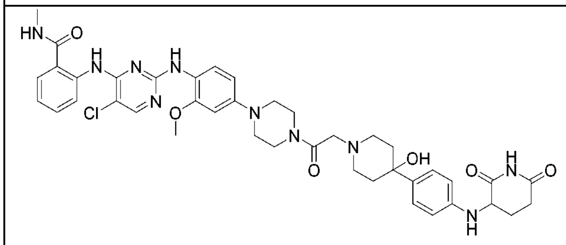 T-055 | 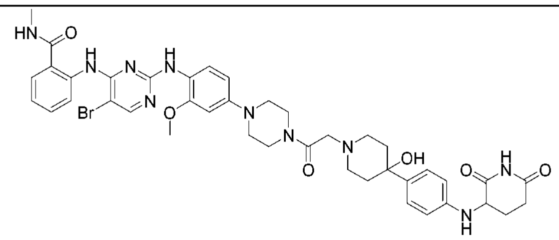 T-056 |
| 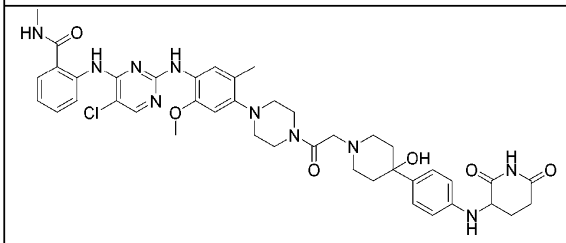 T-057 | 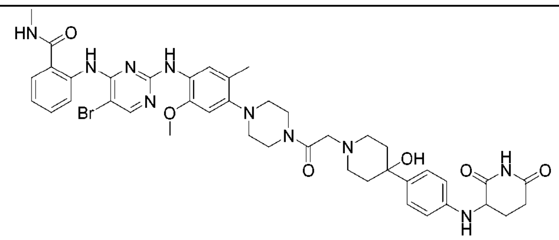 T-058 |
| 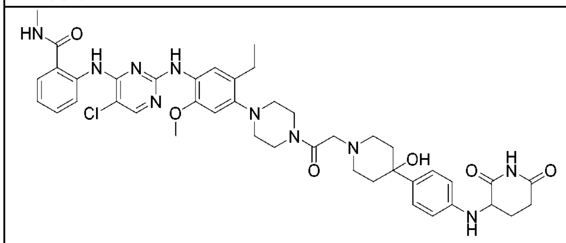 T-059 | 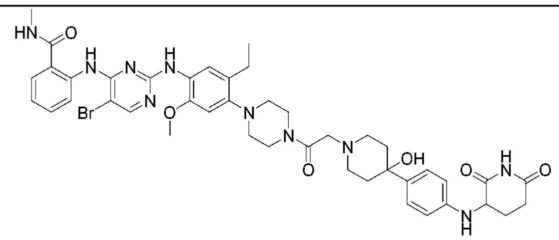 T-060 |
| 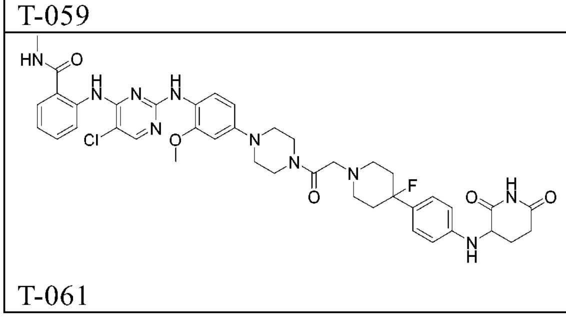 T-061 | 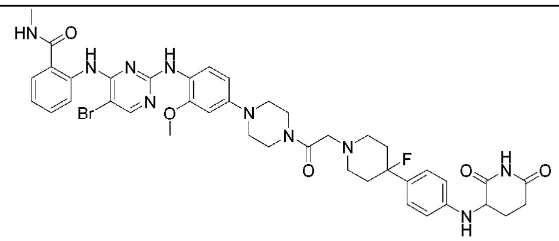 T-062 |

| | |
|---|---|
| 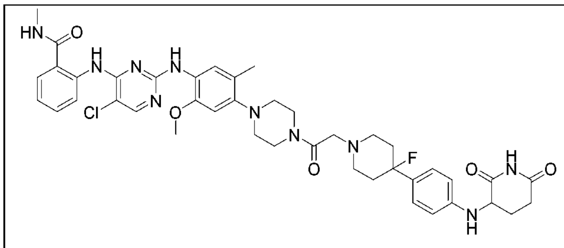 T-063 | 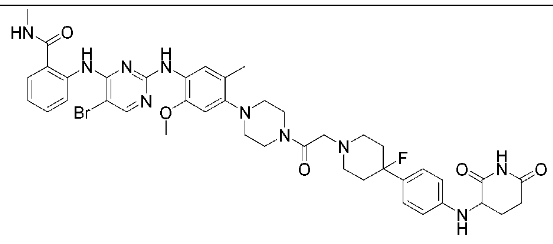 T-064 |
| 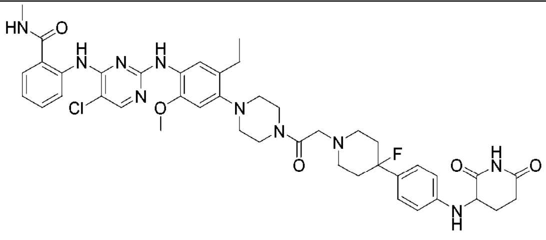 T-065 | 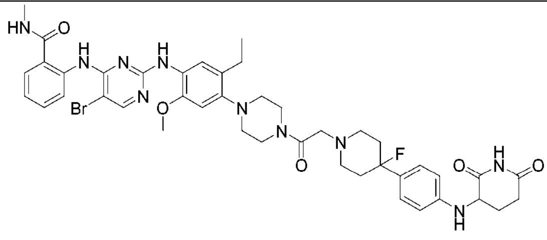 T-066 |
| 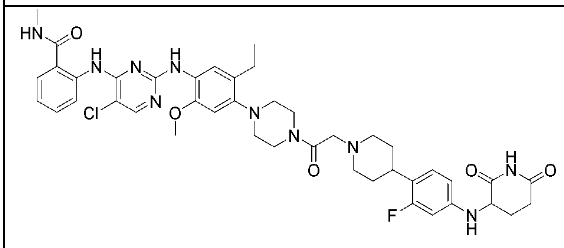 T-067 | 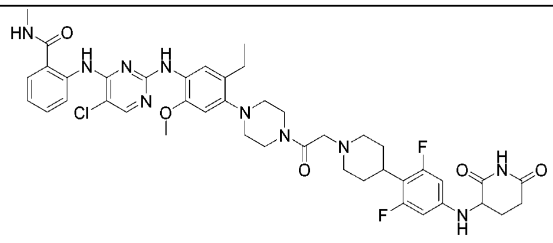 T-068 |
| 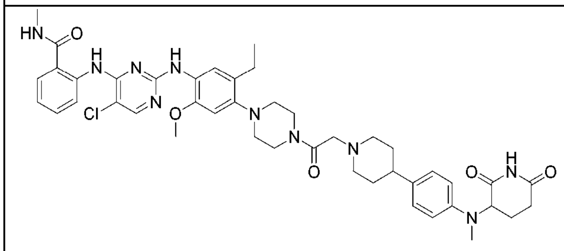 T-069 | 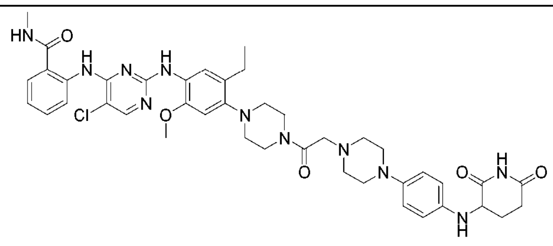 T-070 |
| 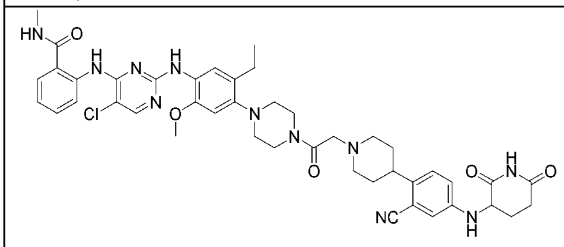 T-071 | 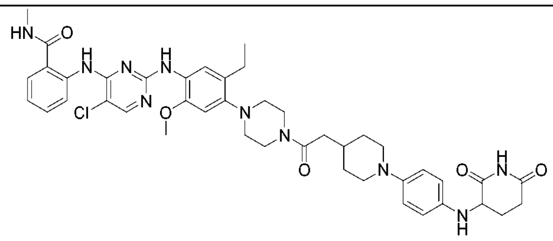 T072 |
| 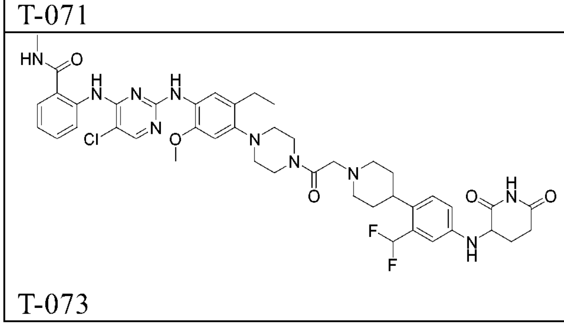 T-073 | 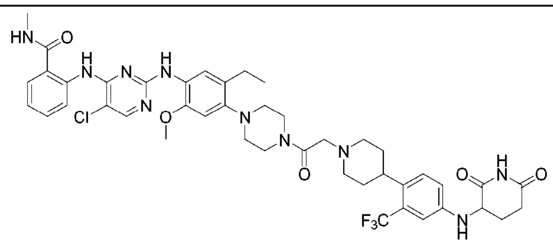 T-074 |

| | |
|---|---|
| 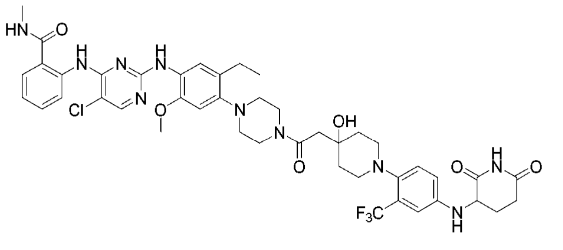<br>T-075 | 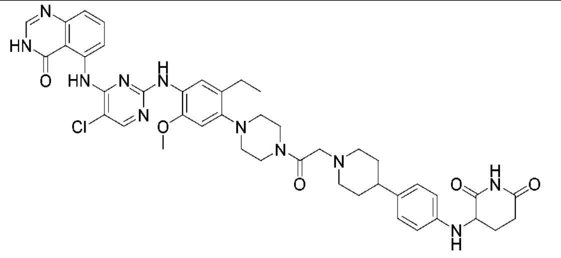<br>T-076 |
| 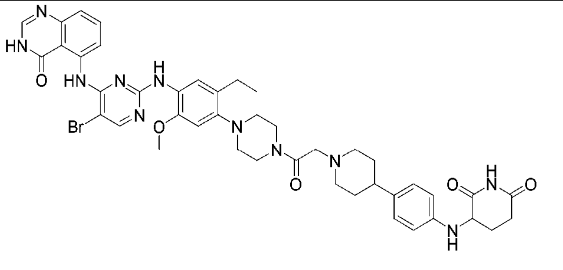<br>T-077 | 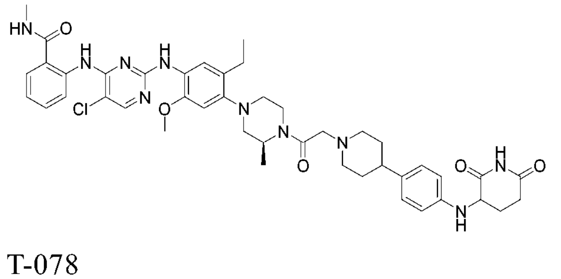<br>T-078 |
| 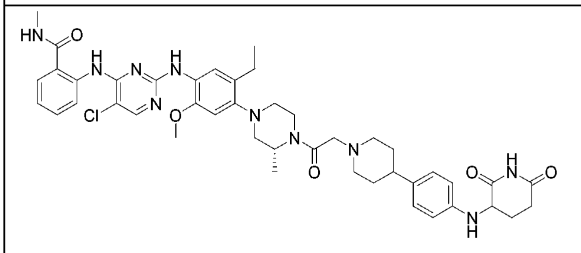<br>T-079 | 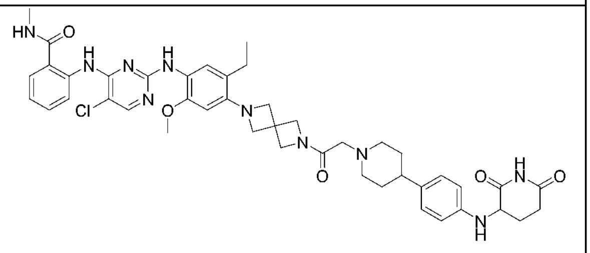<br>T-080 |
| 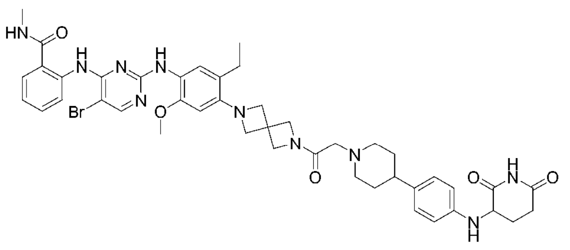<br>T-081 | 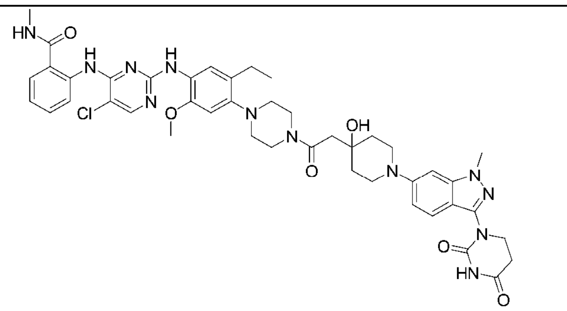<br>T-082 |
| 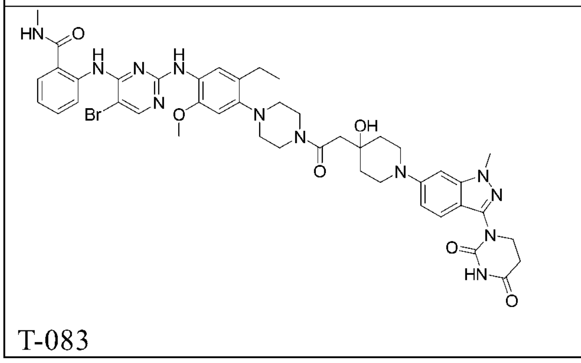<br>T-083 | 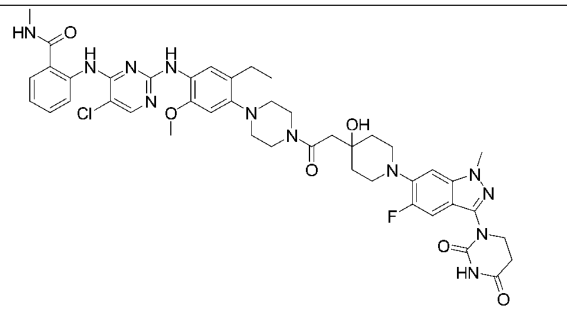<br>T-084 |

| | |
|---|---|
| 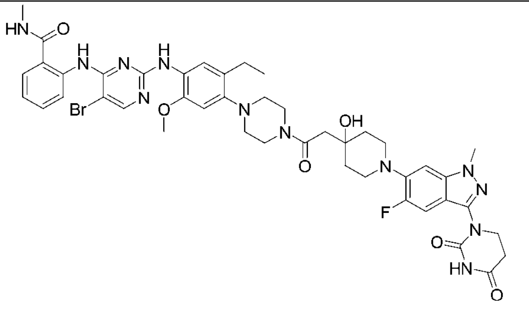 T-085 | 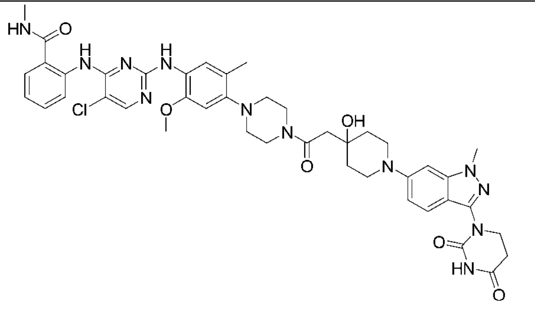 T-086 |
| 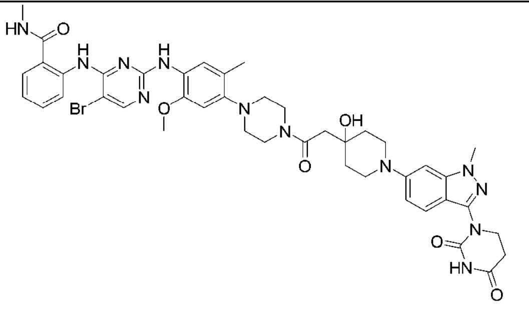 T-087 | 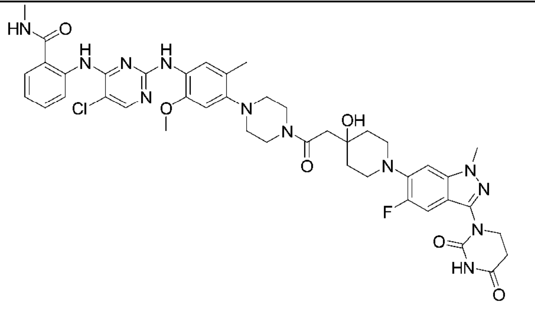 T-088 |
| 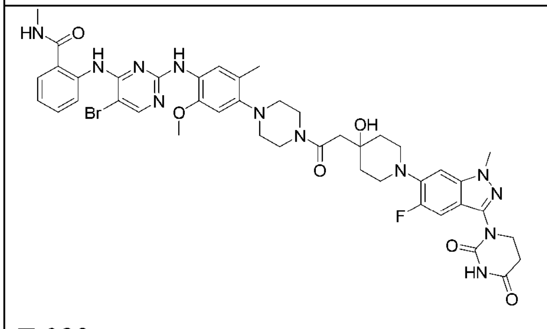 T-089 | 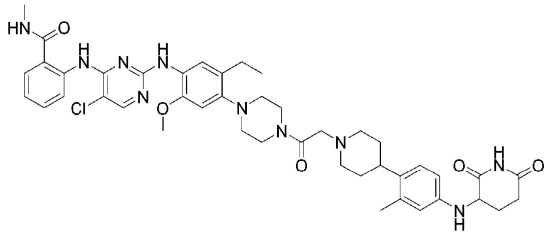 T-090 |
| 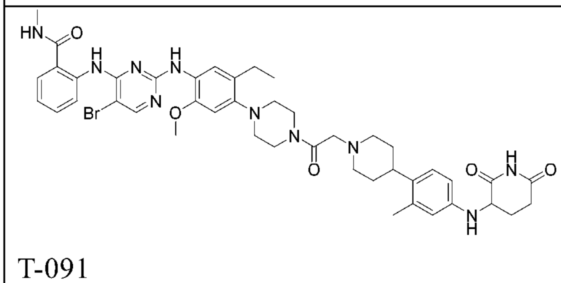 T-091 | 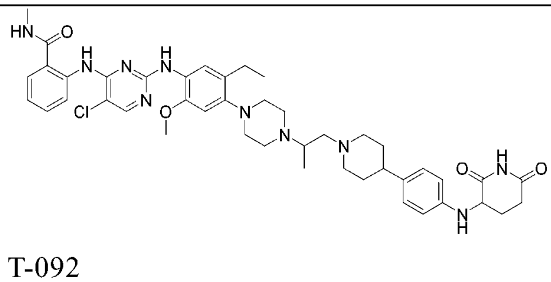 T-092 |
| 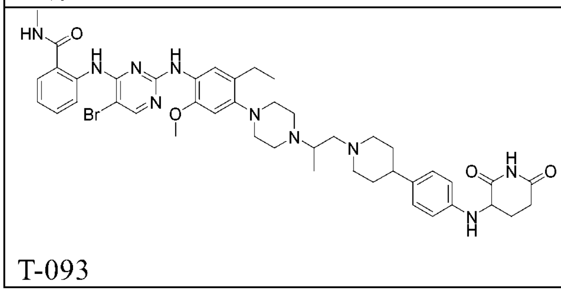 T-093 | 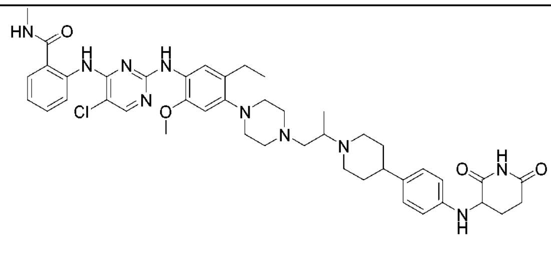 T-094 |

| | |
|---|---|
| 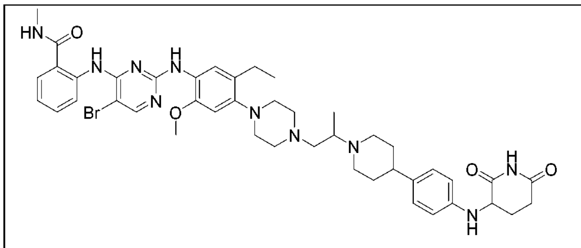<br>T-095 | 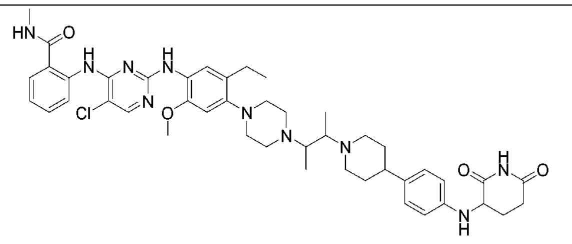<br>T-096 |
| 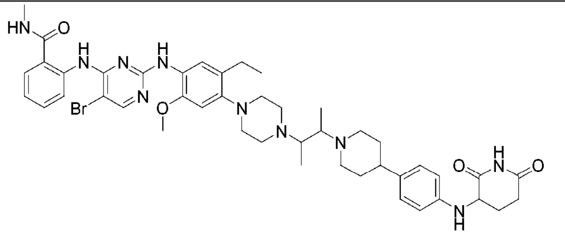<br>T-097 | 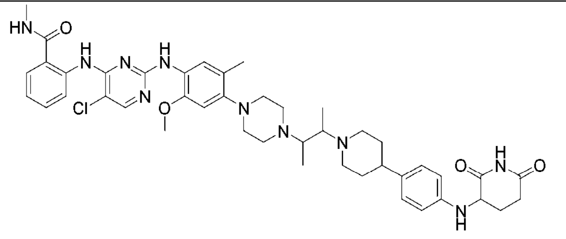<br>T-098 |
| 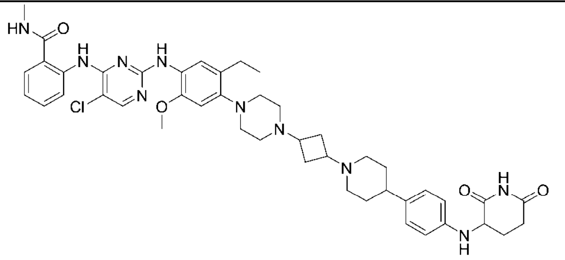<br>T-099 | 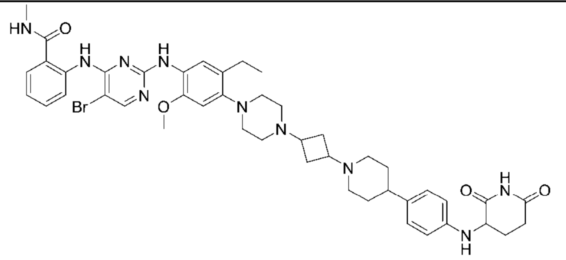<br>T-100 |
| 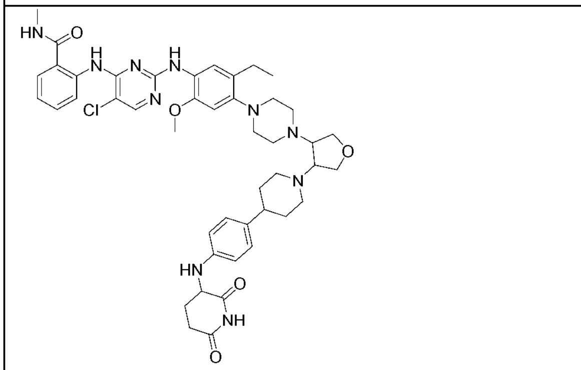<br>T-101 | 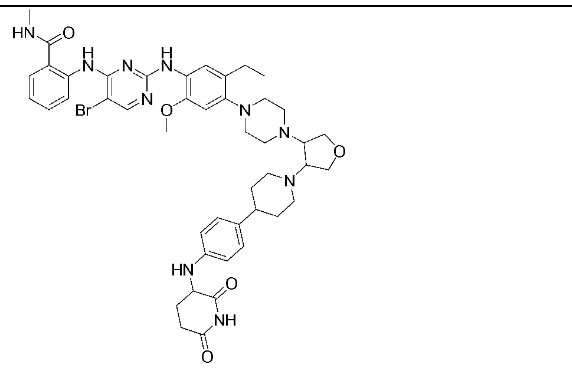<br>T-102 |
| 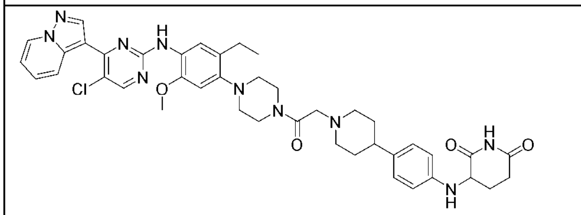<br>T-103 | 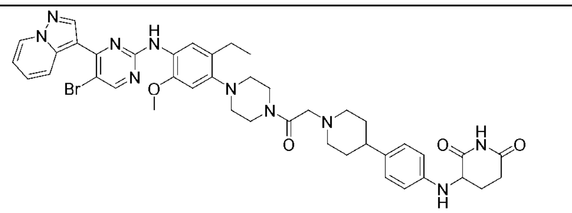<br>T-104 |
| 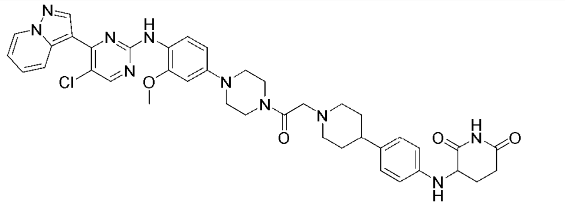<br>T-105 | 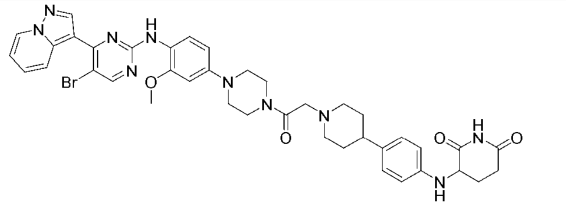<br>T-106 |

| | |
|---|---|
| 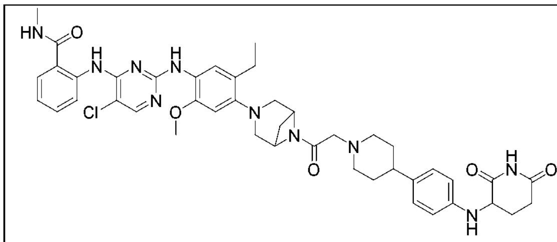 T-107 | 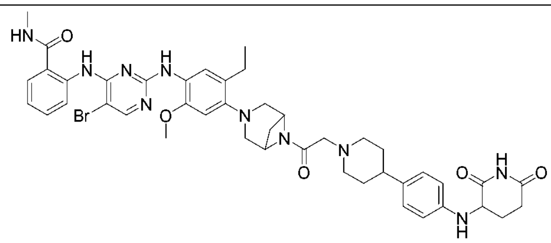 T-108 |
| 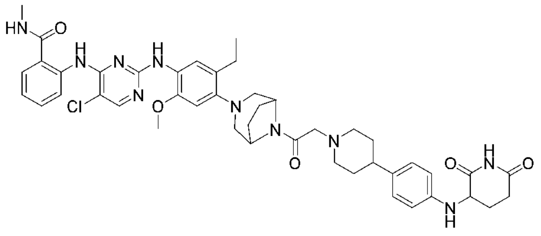 T-109 | 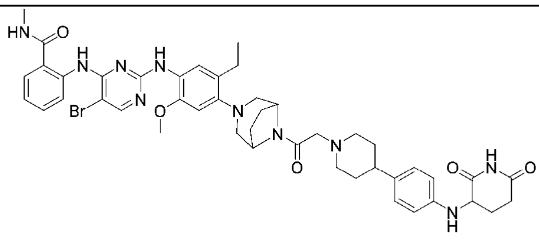 T-110 |
| 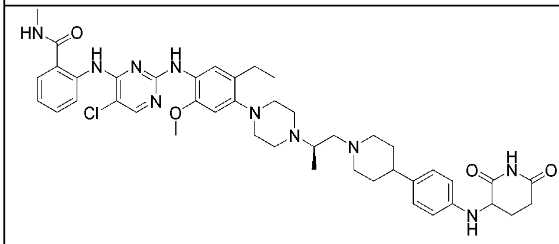 T-111 | 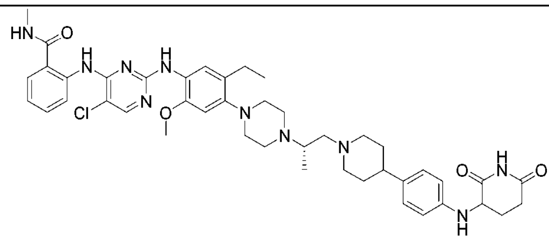 T-112 |
| 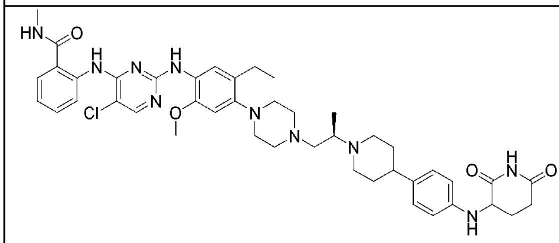 T-113 | 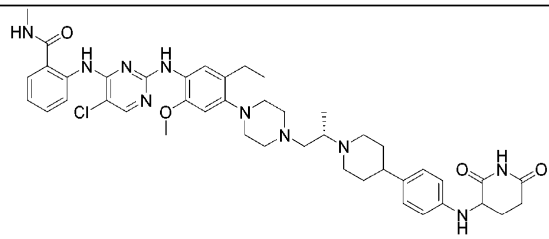 T-114 |
| 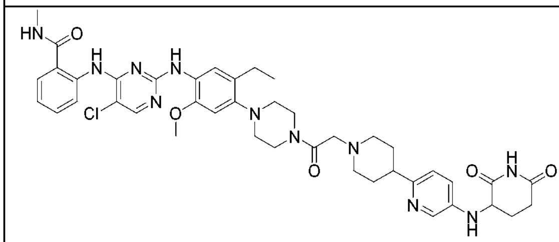 T-115 | 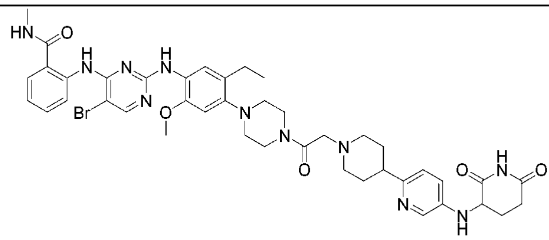 T-116 |
| 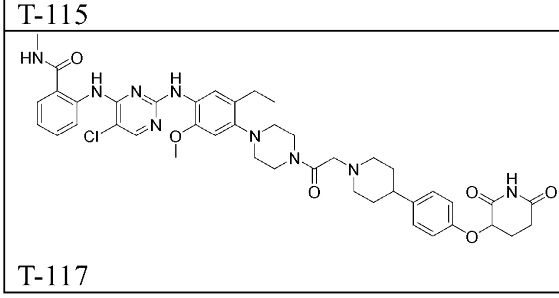 T-117 | 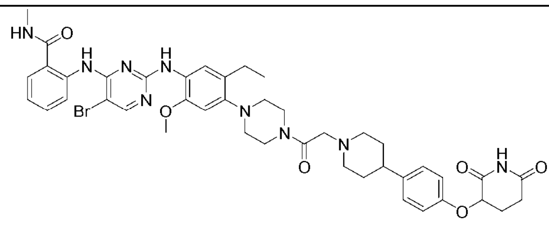 T-118 |

| | |
|---|---|
| 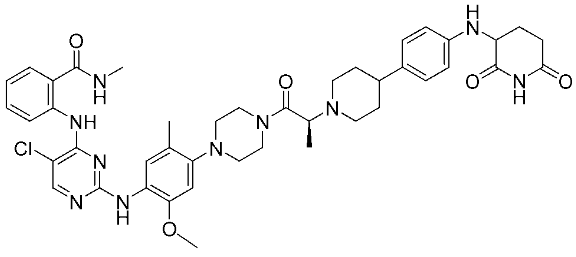<br>T-119 | 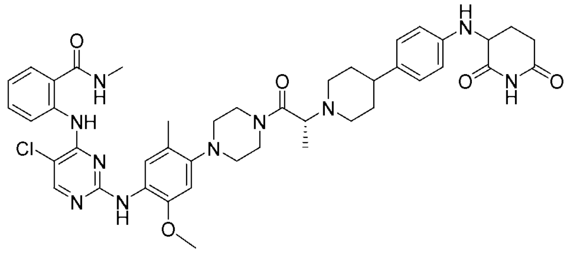<br>T-120 |
| 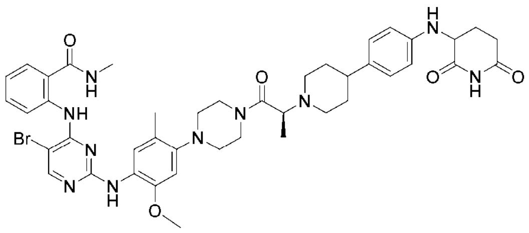<br>T-121 | 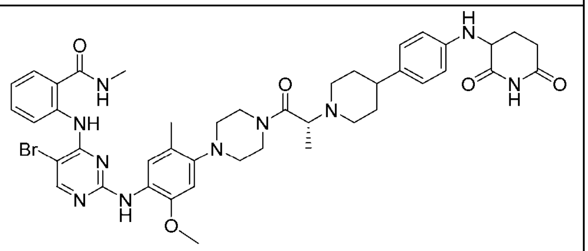<br>T-122 |
| 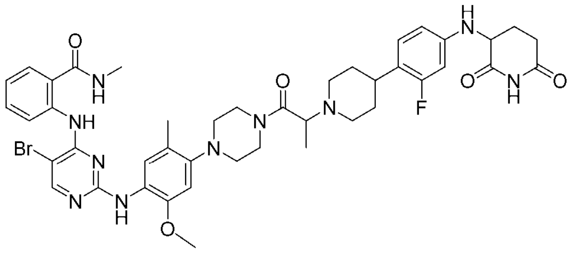<br>T-123 | 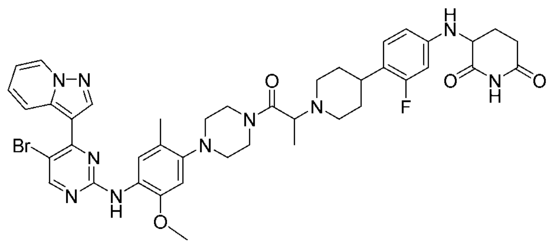<br>T-124 |
| 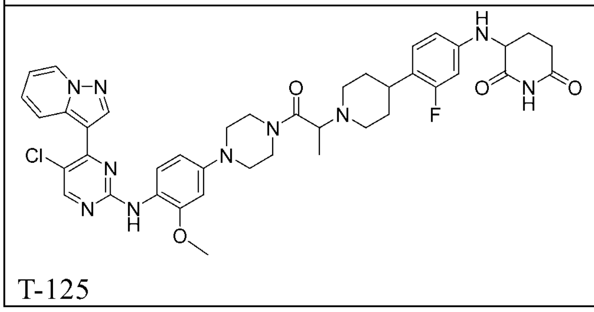<br>T-125 | 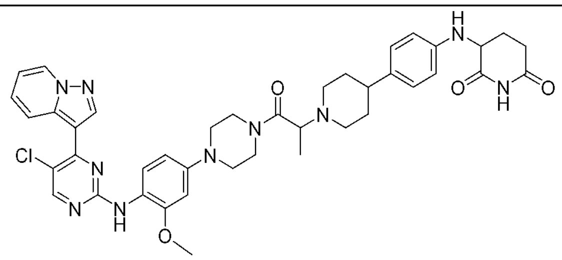<br>T-126 |

7. A pharmaceutical composition, comprising a safe and effective amount of the compound according to claim 1, and a pharmaceutically acceptable carrier.

8. A use of the compound according to claim 1, wherein the use is selected from the group consisting of:

   1) preparation of a medicament for modulating EGFR kinase activity or treating EGFR-related diseases; and
   2) preparation of a medicament for degrading EGFR proteins.

9. The use according to claim 8, wherein the EGFR-related disease is an EGFR resistance mutation-related disease.

10. The use according to claim 8, wherein the EGFR-related disease is selected from the group consisting of: inflammation, cancer, cardiovascular disease, infection, immune disease, and metabolic disease.

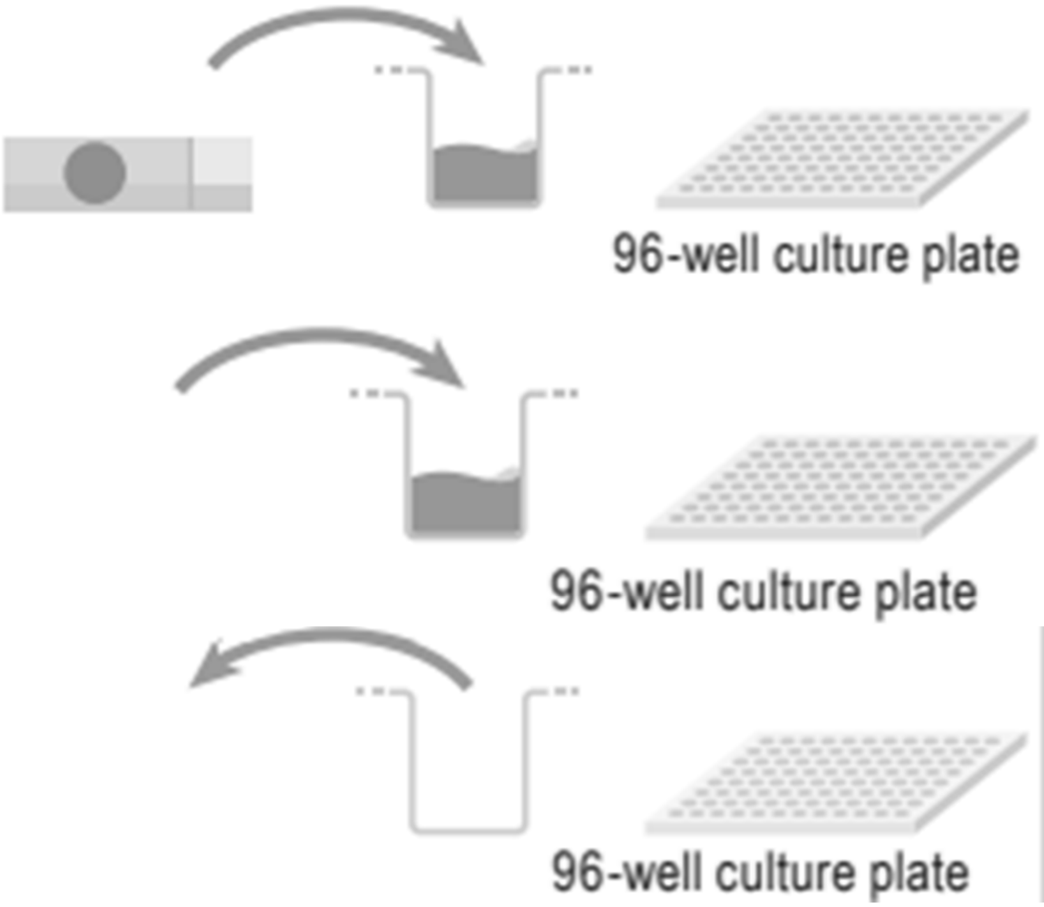
96-well culture plate
96-well culture plate
96-well culture plate

Figure 1

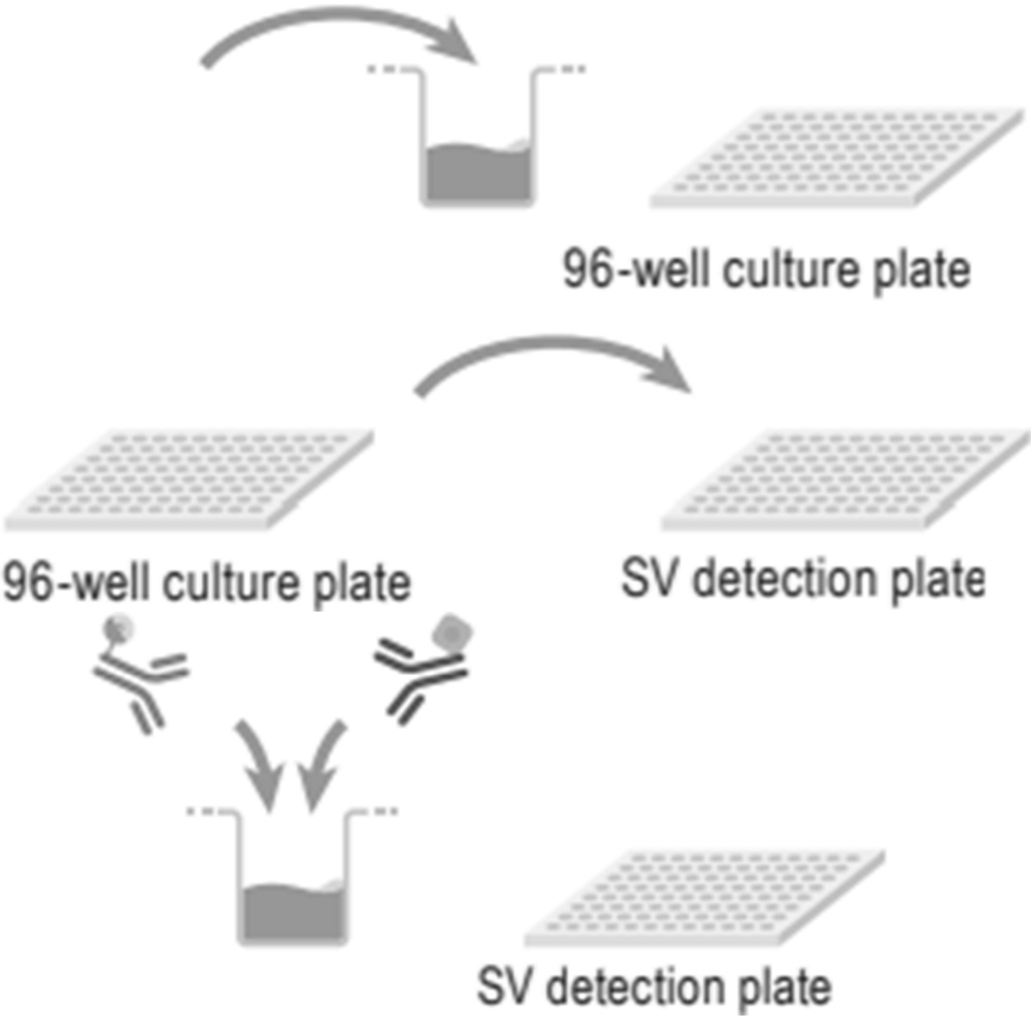
96-well culture plate
96-well culture plate
SV detection plate
SV detection plate

Figure 2

## INTERNATIONAL SEARCH REPORT

International application No.
**PCT/CN2024/093652**

**A. CLASSIFICATION OF SUBJECT MATTER**

C07D401/14(2006.01)i; C07D487/10(2006.01)i; C07D405/14(2006.01)i; C07D403/14(2006.01)i; A61K31/506(2006.01)i; A61K31/496(2006.01)i; A61P35/00(2006.01)i; A61P29/00(2006.01)i; A61P9/00(2006.01)i; A61P37/06(2006.01)i; A61P3/00(2006.01)i; A61P35/02(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of Knowledge, CAPLUS (STN), REGISTRY (STN): 嘧啶, 哌嗪, 酪氨酸激酶, pyrimid+, piperaz+, EGFR, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 116135852 A (TYK MEDICINES, INC.) 19 May 2023 (2023-05-19) claims 1-10 | 1-10 |
| PX | CN 116891502 A (SHANGHAI QILU PHARMACEUTICAL RESEARCH AND DEVELOPMENT CENTRE LTD.) 17 October 2023 (2023-10-17) claims 1-24 | 1-10 |
| X | WO 2022194269 A1 (SHANGHAI QILU PHARMACEUTICAL RESEARCH AND DEVELOPMENT CENTRE LTD.) 22 September 2022 (2022-09-22) claims 1-53, and description, page 43, paragraphs 4-5 | 1-10 |
| X | WO 2022068849 A1 (BEIGENE, LTD. et al.) 07 April 2022 (2022-04-07) claims 1-39, and description, page 3, lines 23-26 | 1-10 |
| A | WO 2022171123 A1 (BEIGENE, LTD. et al.) 18 August 2022 (2022-08-18) claims 1-46 | 1-10 |
| A | WO 2022012622 A1 (BEIGENE, LTD. et al.) 20 January 2022 (2022-01-20) claims 1-54 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 July 2024** | **25 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/093652

C. DOCUMENTS CONSIDERED TO BE RELEVANT

| Category* | Citation of document, with indication, where appropriate, of the relevant passages | Relevant to claim No. |
|---|---|---|
| A | WO 2022228547 A1 (SICHUAN HAISCO PHARMACEUTICAL CO., LTD.) 03 November 2022 (2022-11-03) claims 1-15 | 1-10 |
| A | WO 2023080732 A1 (UBIX THERAPEUTICS, INC.) 11 May 2023 (2023-05-11) claims 1-13 | 1-10 |

Form PCT/ISA/210 (second sheet) (July 2022)

**INTERNATIONAL SEARCH REPORT**
**Information on patent family members**

International application No.
**PCT/CN2024/093652**

| Patent document cited in search report | Publication date (day/month/year) | Patent family member(s) | Publication date (day/month/year) |
|---|---|---|---|
| CN 116135852 A | 19 May 2023 | WO 2023088385 A1 | 25 May 2023 |
| | | TW 202329964 A | 01 August 2023 |
| CN 116891502 A | 17 October 2023 | None | |
| WO 2022194269 A1 | 22 September 2022 | TW 202237597 A | 01 October 2022 |
| WO 2022068849 A1 | 07 April 2022 | US 2024025902 A1 | 25 January 2024 |
| | | TW 202221000 A | 01 June 2022 |
| WO 2022171123 A1 | 18 August 2022 | EP 4291193 A1 | 20 December 2023 |
| | | US 2024165243 A1 | 23 May 2024 |
| WO 2022012622 A1 | 20 January 2022 | US 2023265116 A1 | 24 August 2023 |
| WO 2022228547 A1 | 03 November 2022 | TW 202308655 A | 01 March 2023 |
| | | CA 3217325 A1 | 03 November 2022 |
| | | EP 4332100 A1 | 06 March 2024 |
| | | CL 2023003223 A1 | 10 May 2024 |
| | | IL 308103 A | 01 December 2023 |
| | | BR 112023022735 A2 | 02 January 2024 |
| | | AU 2022263747 A1 | 14 December 2023 |
| | | MX 2023012743 A | 09 November 2023 |
| | | JP 2024518363 A | 01 May 2024 |
| | | KR 20240005838 A | 12 January 2024 |
| WO 2023080732 A1 | 11 May 2023 | AU 2022380336 A1 | 30 May 2024 |
| | | CA 3237119 A1 | 11 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019149922 A
- WO 2021127561 A


### Non-patent literature cited in the description


- Handbook of Chemistry and Physics
- **THOMAS SORRELL**. Organic Chemistry. University Science Books, 1999
- **SAMBROOK et al.** Molecular Cloning: A Laboratory Manual. Cold Spring Harbor Laboratory Press, 1989